# EUROPEAN PATENT APPLICATION

(11) **EP 1 754 705 A2**
(43) Date of publication of application: **21.02.2007**
(21) Application number: 06015229.5
(22) Date of filing: 24.03.1999
(51) Int. Cl.: C07D 409/12, C07D 307/68, C07D 209/20, C07C 237/22, C07C 271/22, C07C 235/42, A61K 31/38, A61K 31/165, A61K 31/325, A61P 37/06, A61P 37/08, A61P 29/00

(54) **Antagonists for treatment of CD11/CD18 adhesion receptor mediated disorders**

(30) Priority: 27.03.1998 US 79732 P
(62) Divisional of application: 99912869.7
(71) Applicant: Genentech, Inc., South San Francisco CA 94080-4990 (US)
(72) Inventor: Burdick, Daniel, J., Burlingame, CA 94010 (US); McDowell, Robert, S., San Francisco, CA 94114 (US); Oare, David, Belmont, CA 94002 (US); Stanley, Mark, S., Pacifica, CA 94041 (US); Gadek, Thomas, R., Oakland, CA 94611 (US); Marsters, James, C., Oakland, CA 94611 (US); Reynolds, Mark, Millbrae, CA 94030 (US); Weese, Kenneth, J., South San Francisco, CA 94080 (US)
(74) Representative: Barz, Peter

(57) **Abstract**

The invention provides novel compounds that are useful for treating Mac-1 or LFA-1-mediated disorders such as inflammatory disorders, allergies, and autoimmune diseases.

## Description

### Field of the Invention

This invention relates to methods and therapeutic compositions for treating mammals, preferably humans, who suffer from or are susceptible to (CD11/CD18) adhesion receptor mediated disorders, especially leukocyte LFA-1 mediated disorders. In particular, it relates to methods for ameliorating or modulating immune responses such as those caused by inflammation, autoimmune responses and host-graft rejection, as exemplified by psoriasis, rheumatoid arthritis, asthma, multiple sclerosis, rejection following transplanted grafts and the like.

### Background of the Invention

### Inflammation

Human peripheral blood is composed principally of red blood cells, platelets and white blood cells or leukocytes. The family of leukocytes are further classified as neutrophils, lymphocytes (mostly B- and T-cell subtypes), monocytes, eosinophils and basophils. Neutrophils, eosinophils and basophils are sometimes referred to as "granulocytes" or "polymorphonuclear (PMN) granulocytes" because of the appearance of granules in their cytoplasm and their multiple nuclei. Granulocytes and monocytes are often classified as "phagocytes" because of their ability to phagocytose or ingest micro-organisms and foreign mater referred to generally as "antigens". Monocytes are so called because of their large single nucleus and these cells may in turn become macrophages. Phagocytes are important in defending the host against a variety of infections and together with lymphocytes are also involved in inflammatory disorders. The neutrophil is the most common leukocyte found in human peripheral blood followed closely by the lymphocyte. In a microliter of normal human peripheral blood, there are about 6,000 leukocytes, of which about 4,000 are neutrophils, 1500 are lymphocytes, 250 are monocytes, 150 are eosinophils and 25 are basophils.

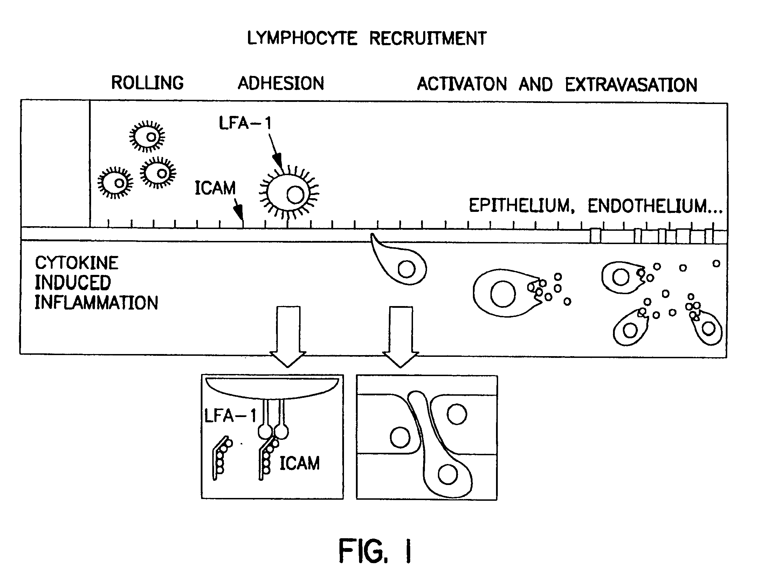

During an inflammatory response peripheral blood leukocytes are recruited to the site of inflammation or injury by a series of specific cellular interactions (see Fig. 1). The initiation and maintenance of immune functions are regulated by intercellular adhesive interactions as well as signal transduction resulting from interactions between leukocytes and other cells. Leukocyte adhesion to vascular endothelium and migration from the circulation to sites of inflammation is a critical step in the inflammatory response (Fig. 1). T-cell lymphocyte immune recognition requires the interaction of the T-cell receptor with antigen (in combination with the major histocompatibility complex) as well as adhesion receptors, which promote attachment of T-cells to antigen-presenting cells and transduce signals for T-cell activation. The lymphocyte function associated antigen-1 (LFA-1) has been identified as the major integrin that mediates lymphocyte adhesion and activation leading to a normal immune response, as well as several pathological states (Springer, T.A., Nature 346:425-434 (1990)). Intercellular adhesion molecules (ICAM) -1, -2, and -3, members of the immunoglobulin superfamily, are ligands for LFA-1 found on endothelium, leukocytes and other cell types. The binding of LFA-1 to ICAMs mediate a range of lymphocyte functions including lymphokine production of helper T-cells in response to antigen presenting cells, T-lymphocyte mediated target cells lysis, natural killing of tumor cells, and immunoglobulin production through T-cell-B-cell interactions. Thus, many facets of lymphocyte function involve the interaction of the LFA-1 integrin and its ICAM ligands. These LFA-1:ICAM mediated interactions have been directly implicated in numerous inflammatory disease states including; graft rejection, dermatitis, psoriasis, asthma and rheumatoid arthritis.

While LFA-1 (CD11a/CD18) on lymphocytes plays a key role in chronic inflammation and immune responses, other members of the leukocyte integrin family (CD11b/CD18, CD11c/CD18 and CD11d/CD18) also play important roles on other leukocytes, such as granulocytes and monocytes, particularly in early response to infective agents and in acute inflammatory response.

The primary function of polymorphonuclear leukocytes, derived from the neutrophil, eosinophil and basophil lineage, is to sense inflammatory stimuli and to emigrate across the endothelial barrier and carry out scavenger function as a first line of host defense. The integrin Mac-1(CD11b/CD18) is rapidly upregulated on these cells upon activation and binding to its multiple ligands which results in the release of oxygen derived free radicals, protease's and phospholipases. In certain chronic inflammatory states this recruitment is improperly regulated resulting in significant cellular and tissue injury. (Harlan, J. M., Acta Med Scand Suppl., 715:123 (1987); Weiss, S., New England J. of Med., 320:365 (1989)).

### LFA-1 (CD11a/CD18) and Mac-1 (CD11b/CD18)

The (CD11/CD18) family of adhesion receptor molecules comprises four highly related cell surface glycoproteins; LFA-1 (CD11a/CD18), Mac-1 (CD11b/CD18), p150.95 (CD11c/CD18) and (CD11d/CD18). LFA-1 is present on the surface of all mature leukocytes except a subset of macrophages and is considered the major lymphoid integrin. The expression of Mac-1, p150.95 and CD11d/CD18 is predominantly confined to cells of the myeloid lineage (which include neutrophils, monocytes, macrophage and mast cells). Functional studies have suggested that LFA-1 interacts with several ligands, including ICAM-1 (Rothleinet al., J. Immunol. 137:1270-1274 (1986), ICAM-2, (Staunton et al., Nature 339:361-364 (1989)), ICAM-3 (Fawcett et al., Nature 360:481-484 (1992); Vezeux et al., Nature 360:485-488, (1992); de Fougerolles and Springer, J. Exp. Med. 175:185-190 (1990)) and Telencephalin (Tian et at., J. Immunol. 158:928-936 (1997)).

The CD11/CD18 family is related structurally and genetically to the larger integrin family of receptors that modulate cell adhesive interactions, which include; embryogenesis, adhesion to extracellular substrates, and cell differentiation (Hynes, R. O., Cell 48:549-554 (1987); Kishimoto et al., Adv. Immunol. 46:149-182 (1989); Kishimotoet al., Cell 48:681-690 (1987); Ruoslahtiet al., Science 238:491-497 (1987).

Integrins are a class of membrane-spanning heterodimers comprising an α subunit in noncovalent association with a β subunit. The β subunits are generally capable of association with more than one α subunit and the heterodimers sharing a common β subunit have been classified as subfamilies within the integrin population (Larson and Springer, "Structure and function of leukocyte integrins," Immunol. Rev. 114:181-217 (1990)).

The integrin molecules of the CD11/CD18 family, and their cellular ligands, have been found to mediate a variety of cell-cell interactions, especially in inflammation. These proteins have been demonstrated to be critical for adhesive functions in the immune system (Kishimoto et al., Adv. Immunol. 46:149-182 (1989)). Monoclonal antibodies to LFA-1 have been shown to block leukocyte adhesion to endothelial cells (Dustin et al., J. Cell. Biol. 107:321-331 (1988); Smith et al., J. Clin. Invest. 83:2008-2017 (1989)) and to inhibit T-cell activation (Kuypers et al., Res. Immunol., 140:461 (1989)), conjugate formation required for antigen-specific CTL killing (Kishimotoet al., Adv. Immunol. 46:149-182 (1989)), T. cell proliferation (Davignon et al., J. Immunol. 127:590-595 (1981)) and NK cell killing (Krenskyet al., J. Immunol. 131:611-616 (1983)).

### ICAMs

ICAM-1 (CD54) is a cell surface adhesion receptor that is a member of the immunoglobulin protein super-family (Rothleinet al., J. Immunol. 137:1270-1274 (1986); Stauntonet al., Cell 52:925-933 (1988). Members of this superfamily are characterized by the presence of one or more Ig homology regions, each consisting of a disulfide-bridged loop that has a number of anti-parallel β-pleated strands arranged in two sheets. Three types of homology regions have been identified, each with a typical length and having a consensus sequence of amino acid residues located between the cysteines of the disulfide bond (Williams, A. F. et al. Ann Rev. Immunol. 6:381-405 (1988); Hunkapillar, T. et al. Adv. Immunol. 44:1-63 (1989). ICAM-1 is expressed on a variety of hematopoietic and non-hematopoietic cells and is upregulated at sites of inflammation by a variety of inflammatory mediators *(*Dustin et al., J. Immunol., 137:256-254 (1986)). ICAM-1 is a 90,000-110,000 Mᵣ glycoprotein with a low messenger RNA levels and moderate surface expression on unstimulated endothelial cells. LPS, IL-1 and TNF strongly upregulate ICAM-1 mRNA and surface expression with peak expression at approximately 18-24 hours (Dustinet al., J. Cell. Biol. 107:321-331 (1988); Stauntonet al., Cell 52:925-933 (1988)). ICAM-1 has five extracellular Ig like domains (designated Domains 1, 2, 3, 4 and 5 or D1, D2, D3, D4 and D5) and an intracellular or cytoplasmic domain. The structures and sequence of the domains is described by Staunton et al. (Cell 52:925-933 (1988)).

ICAM-1 was defined originally as a counter-receptor for LFA-1 (Springer et al., Ann. Rev. Immunol, 5:223-252 (1987); Marlin Cell 51:813-819 (1987); Simmons et al., Nature 331:624-627 (1988); Staunton Nature 339:61-64 (1989); Stauntonet al., Cell 52:925-933 (1988)). The LFA-1/ICAM-1 interaction is known to be at least partially responsible for lymphocyte adhesion (Dustinet al., J. Cell. Biol. 107:321-331 (1988); Mentzeret al., J. Cell. Physiol. 126:285-290 (1986)), monocyte adhesion (Amaout et al., J. Cell Physiol. 137:305 (1988); Mentzeret al., J. Cell. Physiol. 130:410-415 (1987); te Veldeet al., Immunology 61:261-267 (1987)), and neutrophil adhesion (Loet al., J. Immunol. 143(10):3325-3329 (1989); Smithet al., J. Clin. Invest. 83:2008-2017 (1989)) to endothelial cells. Through the development of function blocking monoclonal antibodies to ICAM-1 additional ligands for LFA-1 were identified, ICAM-2 and ICAM-3 (Simmons, Cancer Surveys 24, Cell Adhesion and Cancer, 1995) that mediate the adhesion of lymphocytes to other leukocytes as well as non-hematopoietic cells. Interactions of LFA-1 with ICAM-2 are thought to mediate natural killer cell activity (Helander et al., Nature 382:265-267 (1996)) and ICAM-3 binding is thought to play a role in lymphocyte activation and the initiation of the immune response (Simmons, ibid). The precise role of these ligands in normal and aberrant immune responses remains to be defined.

### Disorders Mediated by T Lymphocytes

Function blocking monoclonal antibodies have shown that LFA-1 is important in T-lymphocyte-mediated killing, T-helper lymphocyte responses, natural killing, and antibody-dependent killing (Springer et al., Ann. Rev. Immunol 5:223-252 (1987)). Adhesion to the target cell as well as activation and signaling are steps that are blocked by antibodies against LFA-1.

Many disorders and diseases are mediated through T lymphocytes and treatment of these diseases have been addressed through many routes. Rheumatoid arthritis (RA) is one such disorder. Current therapy for RA includes bed rest, application of heat, and drugs. Salicylate is the currently preferred treatment drug, particularly as other alternatives such as immunosuppressive agents and adrenocorticosteroids can cause greater morbidity than the underlying disease itself. Nonsteroidal anti-inflammatory drugs are available, and many of them have effective analgesic, anti-pyretic and anti-inflammatory activity in RA patients. These include cyclosporin, indomethacin, phenylbutazone, phenylacetic acid derivatives such as ibuprofen and fenoprofen, naphthalene acetic acids (naproxen), pyrrolealkanoic acid (tometin), indoleacetic acids (sulindac), halogenated anthranilic acid (meclofenamate sodium), piroxicam, and diflunisal. Other drugs for use in RA include anti-malarials such as chloroquine, gold salts and penicillamine. These alternatives frequently produce severe side effects, including retinal lesions and kidney and bone marrow toxicity. Immunosuppressive agents such as methotrexate have been used only in the treatment of severe and unremitting RA because of their toxicity. Corticosteroids also are responsible for undesirable side effects (e.g., cataracts, osteoporosis, and Cushing's disease syndrome) and are not well tolerated in many RA patients.

Another disorder mediated by T lymphocytes is host rejection of grafts after transplantation. Attempts to prolong the survival of transplanted allografts and xenografts, or to prevent host versus graft rejection, both in experimental models and in medical practice, have centered mainly on the suppression of the immune apparatus of the host/recipient. This treatment has as its aim preventive immunosuppression and/or treatment of graft rejection. Examples of agents used for preventive immunosuppression include cytotoxic drugs, anti-metabolites, corticosteroids, and anti-lymphocytic serum. Nonspecific immunosuppressive agents found particularly effective in preventive immunosuppression (azathioprine, bromocryptine, methylprednisolone, prednisone, and most recently, cyclosporin A) have significantly improved the clinical success of transplantation. The nephrotoxicity of cyclosporin A after renal transplantation has been reduced by co-administration of steroids such as prednisolone, or prednisolone in conjunction with azathioprine. In addition, kidneys have been grafted successfully using anti-lymphocyte globulin followed by cyclosporin A. Another protoeol being evaluated is total lymphoid irradiation of the recipient prior to transplantation followed by minimal immunosuppression after transplantation.

Treatment of rejection has involved use of steroids, 2-amino-6-aryl-5-substituted pyrimidines, heterologous anti-lymphocyte globulin, and monoclonal antibodies to various leukocyte populations, including OKT-3. See generally J. Pediatrics, 111: 1004-1007 (1987), and specifically U.S. Pat. No. 4,665,077.

The principal complication of immunosuppressive drugs is infections. Additionally, systemic immunosuppression is accompanied by undesirable toxic effects (e.g., nephrotoxicity when cyclosporin A is used after renal transplantation) and reduction in the level of the hemopoietic stem cells. Immunosuppressive drugs may also lead to obesity, poor wound healing, steroid hyperglycemia, steroid psychosis, leukopenia, gastrointestinal bleeding, lymphoma, and hypertension.

In view of these complications, transplantation immunologists have sought methods for suppressing immune responsiveness in an antigen-specific manner (so that only the response to the donor alloantigen would be lost). In addition, physicians specializing in autoimmune disease strive for methods to suppress autoimmune responsiveness so that only the response to the self-antigen is lost. Such specific immunosuppression generally has been achieved by modifying either the antigenicity of the tissue to be grafted or the specific cells capable of mediating rejection. In certain instances, whether immunity or tolerance will be induced depends on the manner in which the antigen is presented to the immune system. Pretreating the allograft tissues by growth in tissue culture before transplantation has been found in two murine model systems to lead to permanent acceptance across MHC barriers. Lafferty et al., Transplantation, 22:138-149 (1976); Bowen et al., Lancet, 2:585-586 (1979). It has been hypothesized that such treatment results in the depletion of passenger lymphoid cells and thus the absence of a stimulator cell population necessary for tissue immunogenicity. Lafferty et al., Annu. Rev. Immunol., 1:143 (1983). See also Lafferty et al., Science, 188:259-261 (1975) (thyroid held in organ culture), and Gores et al., J. Immunol., 137:1482-1485 (1986) and Faustman et al., Proc. Natl. Acad. Sci. U.S.A., 78: 5156-5159 (1981) (islet cells treated with murine anti-Ia antisera and complement before transplantation). Also, thyroids taken from donor animals pretreated with lymphocytotoxic drugs and gamma radiation and cultured for ten days *in vitro* were not rejected by any normal allogeneic recipient (Gose and Bach, J.Exp.Med., 149:1254-1259 (1979)). All of these techniques involve depletion or removal of donor lymphocyte cells.

In some models such as vascular and kidney grafts, there exists a correlation between Class II matching and prolonged allograft survival, a correlation not present in skin grafts (Pescovitz et al., J.Exp.Med., 160:1495-1508 (1984); Conti et al., Transplant. Proc., 19: 652-654 (1987)). Therefore, donor-recipient HLA matching has been utilized. Additionally, blood transfusions prior to transplantation have been found to be effective (Opelz et al., Transplant. Proc., 4: 253 (1973); Persijn et al., Transplant. Proc., 23:396 (1979)). The combination of blood transfusion before transplantation, donor-recipient HLA matching, and immunosuppression therapy (cyclosporin A) after transplantation was found to improve significantly the rate of graft survival, and the effects were found to be additive (Opelz et al., Transplant. Proc., 17:2179 (1985)).

The transplantation response may also be modified by antibodies directed at immune receptors for MHC antigens (Bluestone et al., Immunol. Rev. 90:5-27 (1986)). Further, graft survival can be prolonged in the presence of antigraft antibodies, which lead to a host reaction that in turn produces specific immunosuppression (Lancaster et al., Nature, 315: 336-337 (1985)). The immune response of the host to MHC antigens may be modified specifically by using bone marrow transplantation as a preparative procedure for organ grafting. Thus, anti-T-cell monoclonal antibodies are used to deplete mature T-cells from the donor marrow inoculum to allow bone marrow transplantation without incurring graft-versus-host disease (Mueller-Ruchholtz et al., Transplant Proc., 8:537-541 (1976)). In addition, elements of the host's lymphoid cells that remain for bone marrow transplantation solve the problem of immunoincompetence occurring when fully allogeneic transplants are used.

As shown in Fig. 1, lymphocyte adherence to endothelium is a key event in the process of inflammation. There are at least three known pathways of lymphocyte adherence to endothelium, depending on the activation state of the T-cell and the endothelial cell. T-cell immune recognition requires the contribution of the T-cell receptor as well as adhesion receptors, which promote attachment of - cells to antigen-presenting cells and transduce regulatory signals for T-cell activation. The lymphocyte function associated (LFA) antigen-1 (LFA-1, CD11a/CD18, α_{L}β₂: where α_{L} is CD11a and β₂ is CD18) has been identified as the major integrin receptor on lymphocytes involved in these cell adherence interactions leading to several pathological states. ICAM-1, the endothelial cell immunoglobulin-like adhesion molecule, is a known ligand for LFA-1 and is implicated directly in graft rejection, psoriasis, and arthritis.

LFA-1 is required for a range of leukocyte functions, including lymphokine production of helper T-cells in response to antigen-presenting cells, killer T-cell-mediated target cell lysis, and immunoglobulin production through T-cell/B-cell interactions. Activation of antigen receptors on T-cells and B-cells allows LFA-1 to bind its ligand with higher affinity.

Monoclonal antibodies (MAbs) directed against LFA-1 led to the initial identification and investigation of the function of LFA-1 (Davignon et al., J. Immunol., 127:590 (1981)). LFA-1 is present only on leukocytes (Krenskey et al., J. Immunol., 131:611 (1983)), and ICAM-1 is distributed on activated leukocytes, dermal fibroblasts, and endothelium (Dustin et al., J. Immunol. 137:245 (1986)).

Previous studies have investigated the effects of anti-CD11a MAbs on many T-cell-dependent immune functions *in vitro* and a limited number of immune responses *in vivo. In vitro,* anti-CD11a MAbs inhibit T-cell activation (Kuypers et al., Res. Immunol., 140:461 (1989)), T-cell-dependent B-cell proliferation and differentiation (Davignon *et al., supra*; Fischer et al., J. Immunol., 136:3198 (1986)), target cell lysis by cytotoxic T-lymphocytes (Krensky *et al., supra*), formation of immune conjugates (Sanders et al., J. Immunol., 137:2395 (1986); Mentzer et al., J. Immunol., 135:9 (1985)), and the adhesion of T-cells to vascular endothelium (Lo et al., J. Immunol., 143:3325 (1989)). Also, the antibody 5C6 directed against CD11b/CD18 was found to prevent intra-islet infiltration by both macrophages and T cells and to inhibit development of insulin-dependent diabetes mellitis in mice (Hutchings et al., Nature, 348: 639 (1990)).

The observation that LFA-1:ICAM-1 interaction is necessary to optimize T-cell function *in vitro,* and that anti-CD11a MAbs induce tolerance to protein antigens (Benjamin et al., Eur. J. Immunol., 18:1079 (1988)) and prolongs tumor graft survival in mice (Heagy et al., Transplantation, 37: 520-523 (1984)) was the basis for testing the MAbs to these molecules for prevention of graft rejection in humans.

Experiments have also been carried out in primates. For example, based on experiments in monkeys it has been suggested that a MAb directed against ICAM-1 can prevent or even reverse kidney graft rejection (Cosimi et al., "Immunosuppression of Cynomolgus Recipients of Renal Allografts by R6.5, a Monoclonal Antibody to Intercellular Adhesion Molecule-1," in Springer et al. (eds.), Leukocyte Adhesion Molecules New York: Springer, (1988), p. 274; Cosimi et al., J. Immunology, 144:4604-4612 (1990)). Furthermore, the *in vivo.*administration of anti-CD11a MAb to cynomolgus monkeys prolonged skin allograft survival (Berlin et al., Transplantation, 53: 840-849 (1992)).

The first successful use of a rat anti-murine CD11a antibody (25-3; IgG1) in children with inherited disease to prevent the rejection of bone-marrow-mismatched haploidentical grafts was reported by Fischer et al., Lancet, 2: 1058 (1986). Minimal side effects were observed. See also Fischer et al., Blood, 77: 249 (1991); van Dijken et al., Transplantation, 49:882 (1990); and Perez et al., Bone Marrow Transplantation, 4:379 (1989). Furthermore, the antibody 25-3 was effective in controlling steroid-resistant acute graft-versus-host disease in humans (Stoppa et al., Transplant. Int., 4:3-7 (1991)).

However, these results were not reproducible in leukemic adult grafting with this MAb (Maraninchi et al., Bone Marrow Transplant, 4:147-150 (1989)), or with an anti-CD18 MAb, directed against the invariant chain of LFA-1, in another pilot study (Baume et al., Transplantation, 47: 472 (1989)). Furthermore, a rat anti-murine CD11a MAb, 25-3, was unable to control the course of acute rejection in human kidney transplantation (LeMauff et al., Transplantation, 52: 291 (1991)).

A review of the use of monoclonal antibodies in human transplantation is provided by Dantal and Soulillou, Current Opinion in Immunology, 3:740-747 (1991).

An earlier report showed that brief treatment with either anti-LFA-1 or anti-ICAM-1 MAbs minimally prolonged the survival of primarily vascularized heterotopic heart allografts in mice (Isobe et al., Science, 255:1125 (1992)). However, combined treatment with both MAbs was required to achieve long-term graft survival in this model.

Independently, it was shown that treatment with anti-LFA-1 MAb alone potently and effectively prolongs the survival of heterotopic (ear-pinnae) nonprimarily vascularized mouse heart grafts using a maximum dose of 4 mg/kg/day and treatment once a week after a daily dose (Nakakura et al., J. Heart Lung Transplant., 11:223 (1992)). Nonprimarily vascularized heart allografts are more immunogenic and more resistant to prolongation of survival by MAbs than primarily vascularized heart allografts (Warren et al., Transplant. Proc., 5:717 (1973); Trager et al., Transplantation, 47:587 (1989)). The latter reference discusses treatment with L3T4 antibodies using a high initial dose and a lower subsequent dose.

Another study on treating a sclerosis-type disease in rodents using similar antibodies to those used by Nakakura *et al., supra,* is reported by Yednock et al., Nature, 356:63-66 (1992).

Additional disclosures on the use of anti-LFA-1 antibodies and ICAM-1, ICAM-2, and ICAM-3 and their antibodies to treat LFA-1-mediated disorders include WO 91/18011 published 11/28/91, WO 91/16928 published 11/14/91, WO 91/16927 published 11/14/91, Can. Pat. Appln. 2,008,368 published 6/13/91, WO 90/03400, WO 90/15076 published 12/13/90, WO 90/10652 published 9/20/90, EP 387,668 published 9/19/90, WO 90/08187 published 7/26/90, WO 90/13281, WO 90/13316, WO 90/13281, WO 93/06864, WO 93/21953, WO 93/13210, WO 94/11400, EP 379,904 published 8/1/90, EP 346,078 published 12/13/89, U.S. Pat. No. 5,002,869, U.S. Pat. No. 5,071,964, U.S. Pat. No. 5,209,928, U.S. Pat. No. 5,223,396, U.S. Fat. No. 5,235,049, U.S. Pat. No. 5,284,931, U.S. Pat. No. 5,288,854, U.S. Pat. No. 5,354,659, Australian Pat. Appln. 15518/88 published 11/10/88, EP 289,949 published 11/9/88, and EP 303,692 published 2/22/89, EP 365,837, EP 314,863, EP 319,815, EP 468, 257, EP 362,526, EP 362, 531, EP 438,310.

Other disclosures on the use of LFA-1 and ICAM peptide fragments and antagonists include; U.S. Pat. No. 5,149,780, U.S. Pat. No. 5,288,854, U.S. Pat. No. 5,340,800, U.S. Pat. No. 5,424,399, U.S. Pat. No. 5,470,953, WO 90/03400, WO 90/13316, WO 90/10652, WO 91/19511, WO 92/03473, WO 94/11400, WO 95/28170, JP 4193895, EP 314,863, EP 362,526 and EP 362,531.

The above methods successfully utilizing anti-LFA-1 or anti-ICAM-1 antibodies, LFA-1 or ICAM-1 peptides, fragments or peptide antagonists represent an improvement over traditional immunosuppressive drug therapy. These studies demonstrate that LFA-1 and ICAM-1 are appropriate targets for antagonism. There is a need in the art to better treat disorders that are mediated by LFA-1 including autoimmune diseases, graft vs. host or host vs. graft rejection, and T-cell inflammatory responses, so as to minimize side effects and sustain specific tolerance to self- or xenoantigens. There is also a need in the art to provide a non-peptide or peptidomimetic antagonist to the LFA-1: ICAM-1 interaction.

At least one peptidomimetic antagonist of the LFA-1:ICAM-1 interaction has shown promise in various *in vitro* assays. 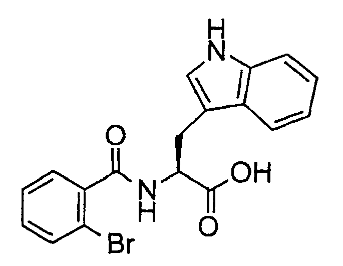 2-Bromobenzoyltryptophan exhibits IC₅₀'s of about 2µM and 10µM respectively in human LFA-1:ICAM-1 receptor binding and human T-cell adhesion assays described herein.

Recently, aminobenzoic acid derivatives of fluorene have been described in U.S. Patent 5,472,973 is useful anti-inflammatory agents. A representative compound is:

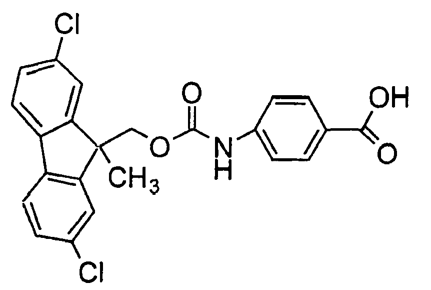

Objects of the Invention

Accordingly, it is an object of this invention to provide compositions and therapeutic methods for modulating adhesion between intracellular adhesion molecules (e.g. ICAM-1, -2 and - 3) and the leukocyte integrin family of receptors.

It is an object to antagonize CD11/CD18 receptors associated with leukocytes, especially Mac-1 and LFA-1-mediated disorders with minimal side effects.

It is an object to control inappropriate inflammatory responses and prevent damage to healthy tissue.

More specifically, it is an object to treat LFA-1-mediated disorders including: psoriasis; responses associated with inflammatory bowel disease (such as Crohn's disease and ulcerative colitis), dermatitis, meningitis, encephalitis, uveitis, allergic conditions such as eczema and asthma, conditions involving infiltration of T-cells and chronic inflammatory responses, skin hypersensitivity reactions (including poison ivy and poison oak); atherosclerosis, autoimmune diseases such as rheumatoid arthritis, systemic lupus erythematosus (SLE), diabetes mellitus, multiple sclerosis, Reynaud's syndrome, autoimmune thyroiditis, experimental autoimmune encephalomyelitis, Sjorgen's syndrome, juvenile onset diabetes, and immune responses associated with delayed hypersensitivity mediated by cytokines and T-lymphocytes typically found in tuberculosis, sarcoidosis, polymyositis, granulomatosis and vasculitis; pernicious anemia; diseases involving leukocyte diapedesis; CNS inflammatory disorder, multiple organ injury syndrome secondary to septicaemia or trauma; autoimmune hemolytic anemia; myasthemia gravis; antigen-antibody complex mediated diseases; all types of transplantations, including graft vs. host or host vs. graft disease, HIV and rhinovirus infection, pulmonary fibrosis, and the like. These and other objects will become apparent to one of ordinary skill in the art.

### Summary of the Invention

These objects are accomplished by providing a method and antagonist compositions for modulating adhesion between intracellular adhesion molecules (e.g. ICAM-1, -2 and -3) and the leukocyte integrin family of receptors. The method and antagonists are especially useful for treating CD11/CD18, especially Mac-1 and LFA-1-mediated disorders in a mammal, especially a human, comprising administering to the mammal a therapeutically effective amount of the antagonist. Suitable leukocyte integrin antagonists, especially Mac-1 and LFA-1 antagonists of this invention are represented by Structural Formula I below. Preferably, the LFA-1 antagonist is a specific antagonist of the leukocyte integrin CD11a(α_{L})/CD18(β₂). Such antagonists are especially useful to treat chronic LFA-1 mediated disorders. Preferably, these LFA-1 antagonists are used to treat: psoriasis, alopecia, organ transplant, inflammatory bowel disease (IBD), rheumatoid arthritis (RA), systemic lupus erythematosis (SLE), type-1 diabetes, multiple sclerosis (MS), asthma, graft verses host (GVH) disease, scleredoma, endometriosus and vitiligo. Optionally, certain compounds embraced by Formula I are also capable of antagonizing Mac-1 CD11b(αM)/CD18(β2) binding to ICAM-1 and additional ligands including iC3b, fibrinogen and Factor X. These compounds are therefore useful for inhibiting adhesion of neutrophils and leukocytes expressing both or either LFA-1 and Mac-1 in both chronic and acute leukocyte/neutrophil mediated disorders. More specifically these disorders include; ischemic reperfusion injury mediated by neutrophils such as acute myocardial infarction, restenosis following PTCA, invasive procedures such as cardiopulmanary bypass surgery, cerebral edema, stroke, traumatic brain injury, multiple sclerosis, systemic lupus erythematosis, hemorragic shock, burns, ischemic kidney disease, multiorgan failure, wound healing and scar formation, atherosclerosis as well as organ failure post-transplant.

The antagonist is represented by formula I

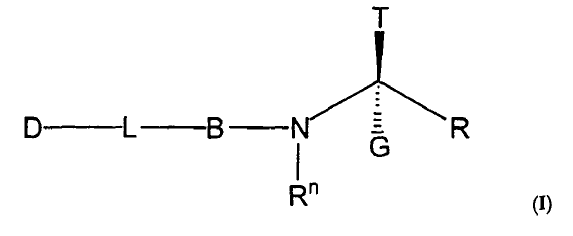

Where D is a mono-, bi-, or tricyclic saturated, unsaturated, or aromatic ring, each ring having 5-, 6- or 7 atoms in the ring where the atoms in the ring are carbon or from1-4 heteroatoms selected from; nitrogen, oxygen, and sulfur, where any sulfur ring atom may optionally be oxidized and any carbon ring atom may form a double bond with O, NRⁿ and CR¹R^{1'}, each ring nitrogen substituted with Rⁿ and any ring carbon substituted with R^{d}.

Optionally, D is an aromatic homocycle or aromatic heterocycle containing 1-3 heteroatoms selected from the group N, S and O, the homo- or hetero-cycles selected from: 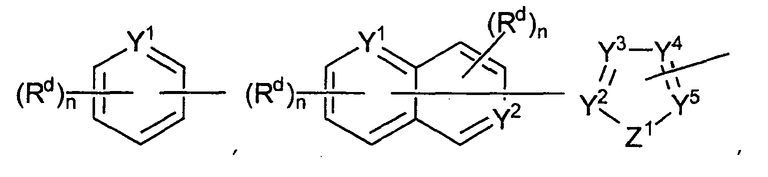 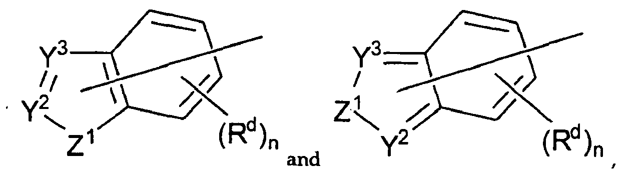 where Y¹, Y², Y³, Y⁴ and Y⁵ are CH, CR^{d} or N, Z¹ is O, S, NH or NRⁿ and n is 0-3.

More specifically, D may be:
1) a 5-member aromatic heterocycle or het selected from; 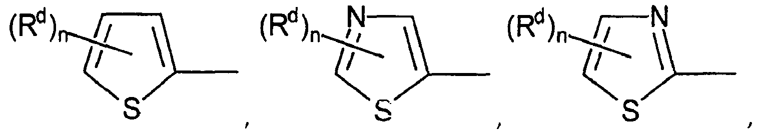 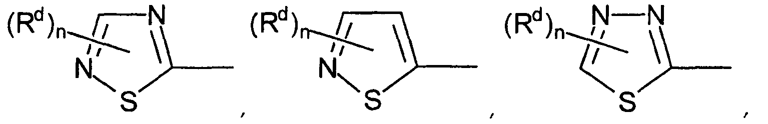 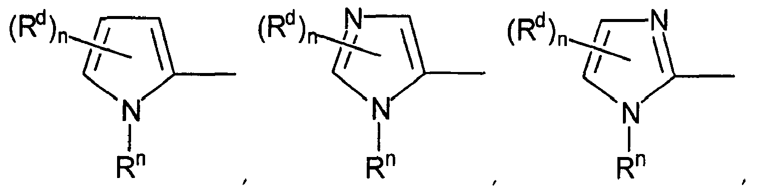 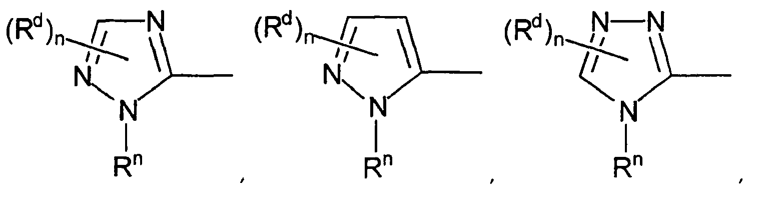 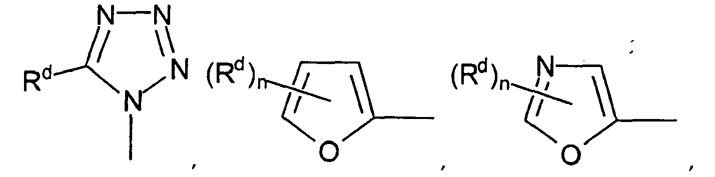 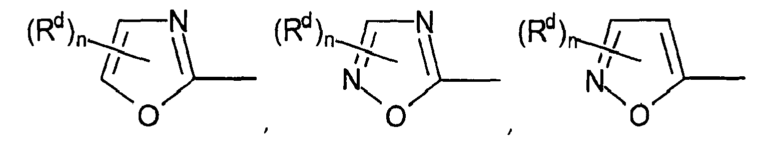 and 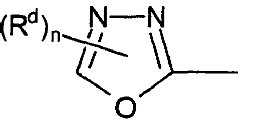
2) a 9-member aromatic heterobicycle selected from; 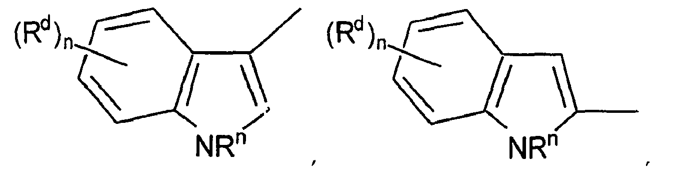 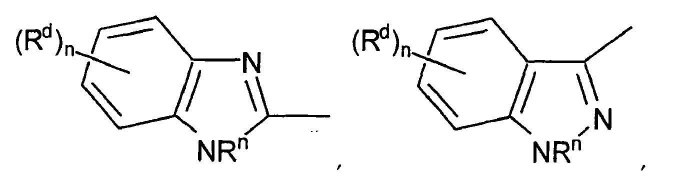 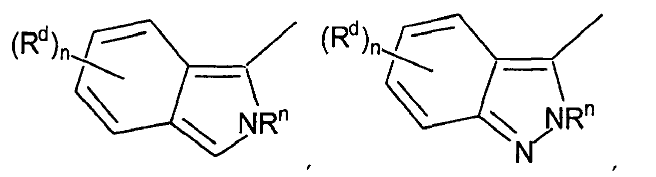 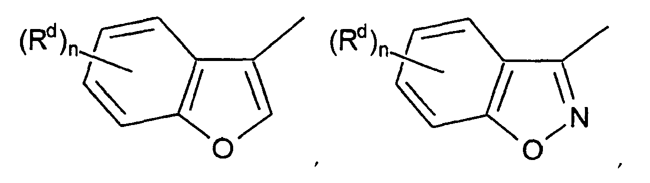 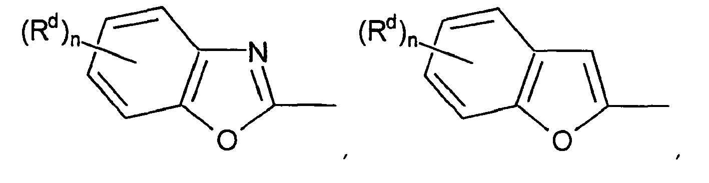 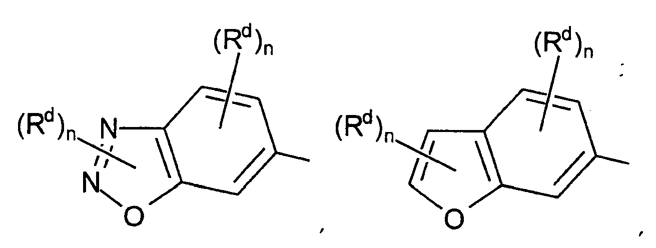 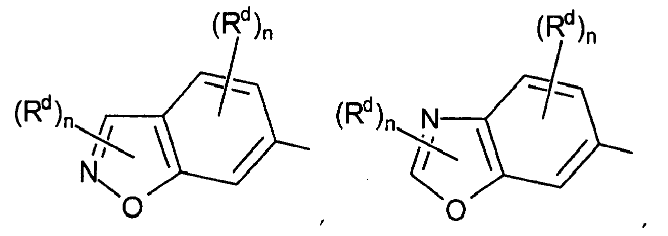 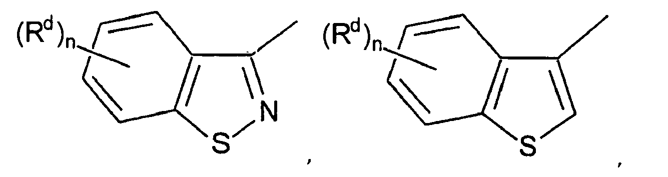 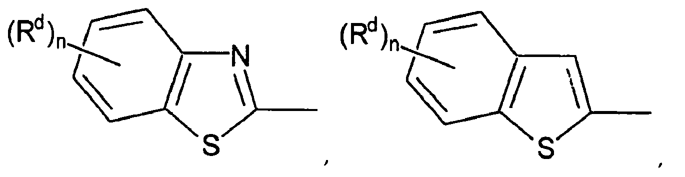 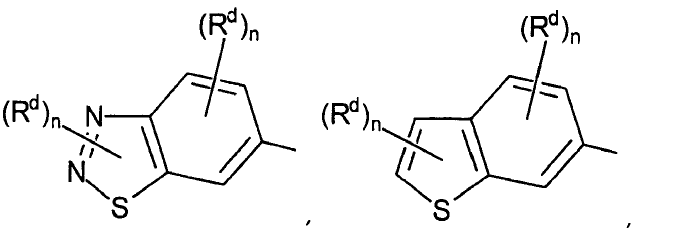 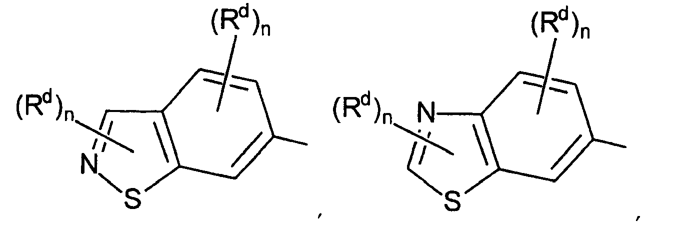 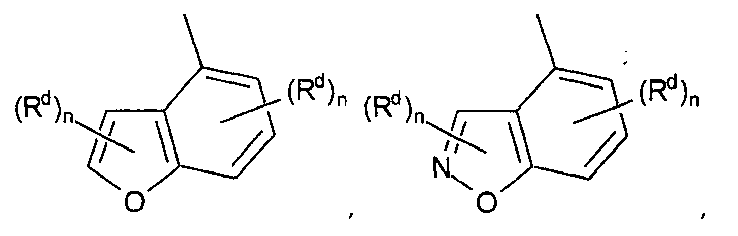 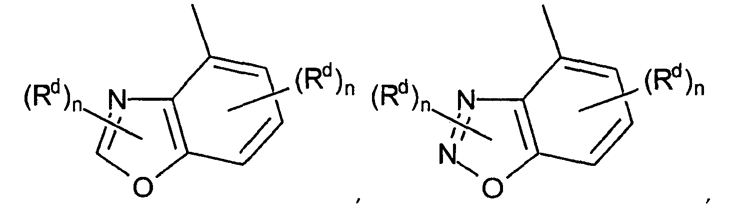 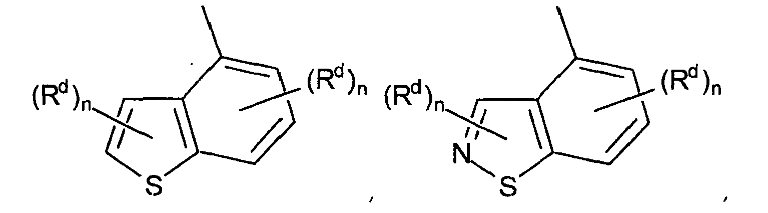 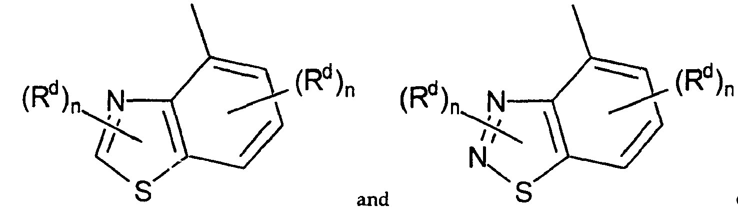 or
3) a 6-member aromatic hetero- or homocycle selected from; 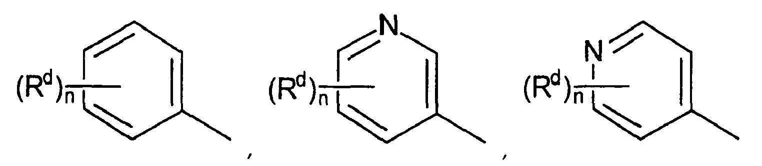 and 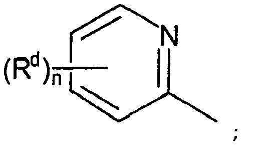
L is a bivalent linking group selected from

―L³―L²―L¹―,

―L⁴―L³―L²―L¹― and

―L⁵―L⁴―L³―L²―L¹―,

where:
L¹ may be oxo (O), S(O)ₛ, C(=O), C(=N-Rⁿ), C(=CR¹R^{1'}), C(R¹R^{1'}), C(R¹), C, het, N(Rⁿ) or N;
L² may be oxo (O), S(O)ₛ, C(=O), C(=N-O-R^{o}), C(=CR²R^{2'}), C(R²R^{2'}), C(R²), C, het, N(Rⁿ) or N;
L³ may be oxo (O), S(O)ₛ, C(=O), C(=N-O-R^{o}), C(=CR³R^{3'}), C(R³R^{3'}) C(R³), C, het, N(Rⁿ) or N;
L⁴ is absent or may be oxo (O), S(O)ₛ, C(=O), C(=N-O-R^{o}), C(=CR⁴R^{4'}), C(R⁴R^{4'}), C(R⁴), C, NRⁿ or N; and
L⁵ is absent or may be oxo (O), S(O)ₛ, C(=O), C(=N-Rⁿ), C(R⁵R^{5'}), C(=CR⁵R^{5'}), C(R⁵), C, NRⁿ or N;
provided that only one of L¹-L³ may be het and that when one of L¹-L³
is het the other L¹-L⁵ may be absent.

R¹, R^{1'},R², R^{2'} R³, R^{3'}, R⁴,R^{4'}, R⁵ and R^{5'} each are independently selected from R^{a}, R^{c} and U-Q-V-W. Optionally, R² and R^{2'} separately or together may form a saturated, unsaturated or aromatic fused ring with B through a substituent R^{P} on B, the fused ring containing 5, 6 or 7 atoms in the ring and optionally containing 1-3 heteroatoms selected from the group O, S and N, where any S or N may optionally be oxidized. Optionally, R³ and R^{3'} separately or together and R⁴ and R^{4'} separately or together may form a saturated, unsaturated or aromatic fused ring with D through a substituent R^{d} on D, the fused ring containing 5, 6 or 7 atoms in the ring and optionally containing 1-3 heteroatoms selected from the group O, S and N, where any S or N may optionally be oxidized. Also optionally, each R¹-R⁵, or NRⁿ together with any other R¹- R⁵ or NRⁿ may form a 5, 6 or 7 member homo- or heterocycle either saturated, unsaturated or aromatic optionally containing 1-3 additional heteroatoms selected from N, O and S, each cycle substituted with 0-3 R^{d}, where s is 0-2, and where any carbon or sulfur ring atom may optionally be oxidized.

More specifically, the bivalent linker L may be:

-(CR⁶R^{6'})ₒ-Ai-(CR⁸R^{8'})ₚ-,

-(CR⁶R^{6'})ₒ-het-(CR⁸R^{8'})ₚ-,

-(CR⁶=CR⁷)_{q}-Ai-(CR⁸R^{8'})ₚ-

and

-(CR⁶R^{6'})ₒ-Ai-(CR⁸=CR⁹)ᵣ-,

where Ai is selected from 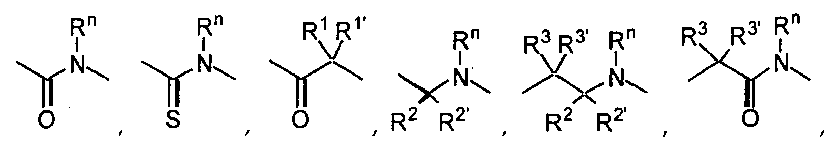 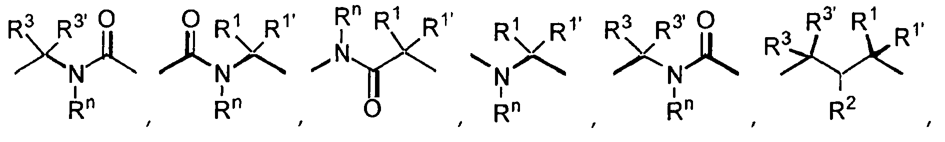 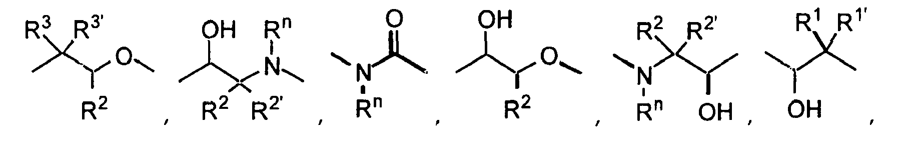 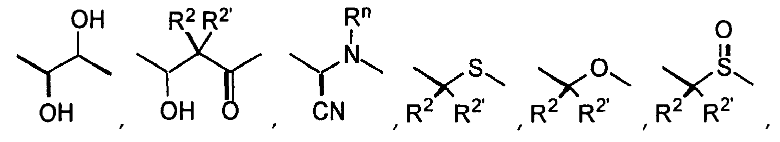 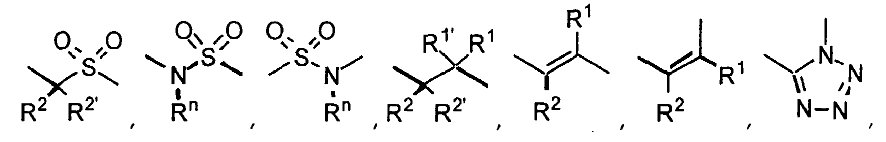 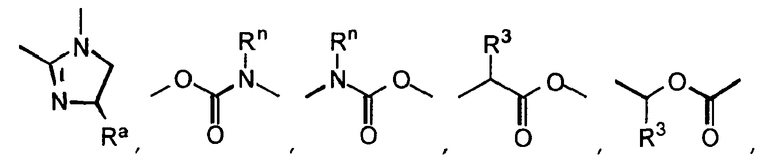 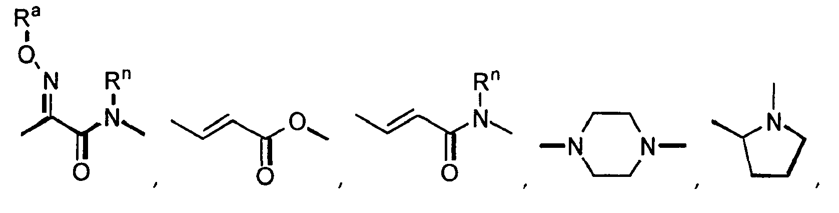 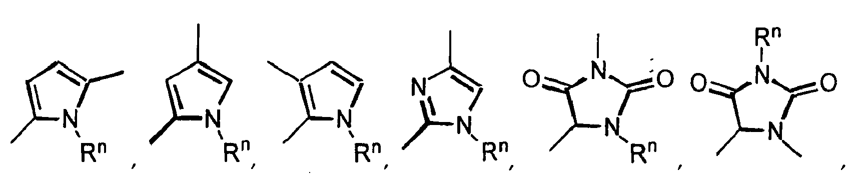 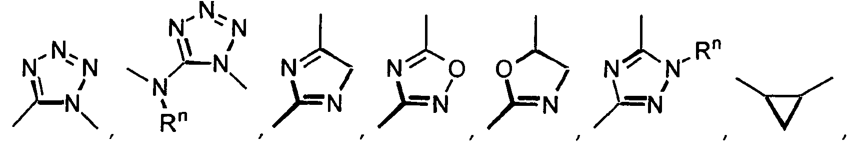 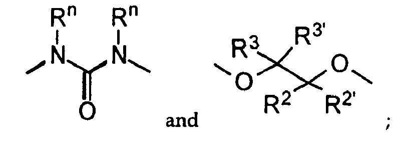 where o is 0-1, p is 0-1, q is 0-1 and r is 0-1.

het is any mono-, bi-, or tricyclic saturated, unsaturated, or aromatic ring where at least one ring is a 5-, 6- or 7-membered ring containing from one to four heteroatoms selected from the group nitrogen, oxygen, and sulfur, the 5-membered ring having from 0 to 2 double bonds and the 6- or 7-membered ring having from 0 to 3 double bonds and where any carbon or sulfur atoms in the ring may optionally be oxidized, and where any nitrogen heteroatom may optionally be quaternized and where any ring may contain from 0-3 R^{d}.

Optionally L is a bivalent linking group selected from the group:
-C₃-C₅-alkyl-, -C₃-C₅-alkenyl-, -CH₂C(=O)NH-, -CH₂NH-C(=O)-, -O-CH₂-C(=O)-, -CH₂-CH₂-C(=O)-, -CH=CH-C(=O)NH-CH₂-, -CH=CH-C(=O)NH-CH-(CH₃)-, -CH(OH)-CH₂-O-, -CH(OH)-CH₂-N(CH₃)-, -CH(OH)-CH₂CH₂-, -CH₂-CH₂-CH(OH)-, -O-CH₂-CH(OH)-, -O-CH₂-CH(OH)-CH₂-, -O-CH₂-CH₂-CH(OH)-, -O-CH₂-CH₂-O-, -CH₂-CH₂-CH₂-O-, -CH₂-CH(OH)-CH₂-O-, -CH₂-CH₂-O-, -CH-(CH₃)-NH-C(=O)-, -CH₂-NH-SO₂-, -NH-SO₂-CH₂-, -CH₂-SO₂NH-, -SO₂NH-CH₂-, - C(=O)-NH-C(=O)-, -NH-C(=O)-NH-, -NH-C(=O)-NH-CH₂-, -CH₂-NH-C(=O)-NH-, -C(=O)-NH-CH₂-C(=O)-NH -, -NH-C(=O)-O- and -O-C(=O)-NH-.

Optionally, specific D-L combinations are selected from: 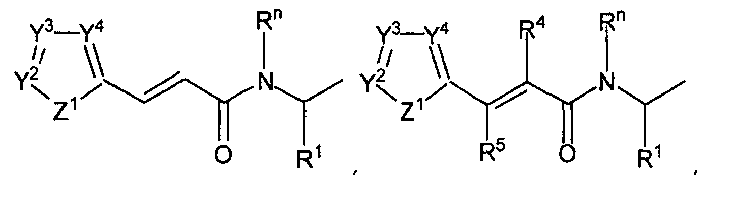 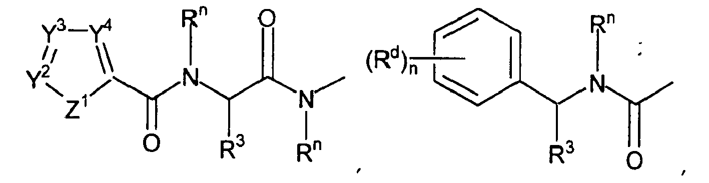 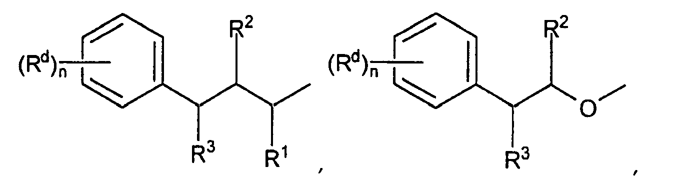 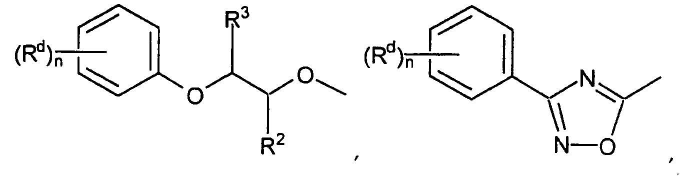 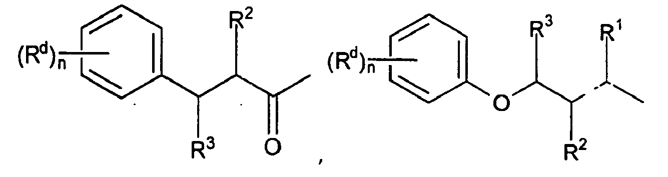 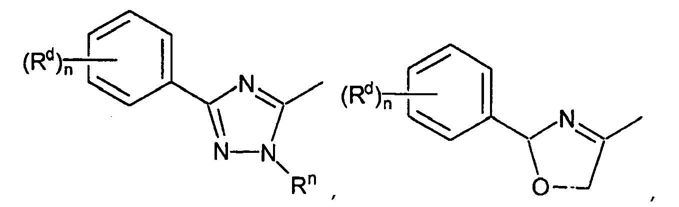 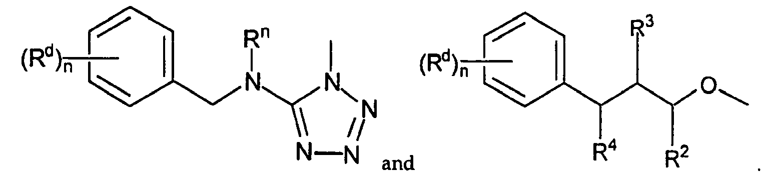 B is selected from the group 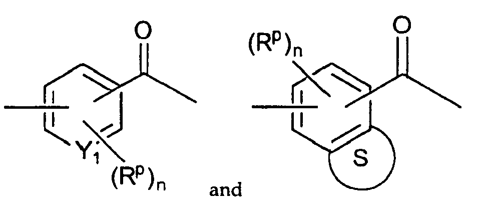 where 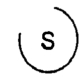 is a fused hetero- or homocyclic ring containing 5, 6 or 7 atoms, the ring being unsaturated, partially saturated or aromatic, the heteroatoms selected from 1-3 O, S and N.

Y₁ is selected from CH and N and n is 0-3.

G is selected from hydrogen and C₁-C₆alkyl, optionally G taken together with T may form a C₃-C₆cycloalkyl optionally substituted with -V-W.

T is selected from the group 1) a naturally occurring α-amino-acid side chain or derivatives thereto and U-Q-V-W.

U is an optionally substituted bivalent radical selected from the group; C₁-C₆alkyl, C₀-C₆alkyl-Q, C₂-C₆alkenyl-Q, and C₂-C₆alkynyl-Q, where the substituents on any alkyl, alkenyl or alkynyl are 1-3 R^{a}.

Q is absent or is selected from the group; -O-, -S(O)ₛ-, -SO₂-N(Rⁿ)-, -N(Rⁿ)-, -N(Rⁿ)-C(=O)-, -N(Rⁿ)-C(=O)-O-, -N(Rⁿ)-SO₂-, -C(=O)-, -C(=O)-O-, -het-, -C(=O)-N(Rⁿ)-, -PO(OR^{c})O- and -P(O)O-, where s is 0-2 and het is a mono- or bicyclic 5, 6, 7, 9 or 10 member heterocyclic ring, each ring containing 1-4 heteroatoms selected from N, O and S, where the heterocyclic ring may be saturated, partially saturated, or aromatic and any N or S being optionally oxidized, the heterocyclic ring being substituted with 0-3 R^{h}.

V is absent or is an optionally substituted bivalent group selected from C₁-C₆alkyl, C₃-C₈cycloalkyl, C₀-C₆alkyl-C₆-C₁₀aryl, and C₀-C₆alky-het, where the substituents on any alkyl are 1-3 R^{a} and the substituents on any aryl or het are 1-3 R^{d}.

W is selected from the group; hydrogen, -OR°, -SR^{m}, -NRⁿR^{n'}, -NH-C(=O)-O-R^{c}, -NH-C(=O)-NRⁿR^{n'}, -NH-C(=O)-R^{c}, -NH-SO₂-R^{s}, -NH-SO₂-NRⁿR^{n'}, -NH-SO₂-NH-C(=O)-R^{c}, -NH-C(=O)-NH-SO₂-R^{s}, -C(=O)-NH―C(=O)-O-R^{c}, -C(=O)-NH-C(=O)-R^{c}, -C(=O)-NH-C(=O)-NRⁿR^{n'},-C(=O)-NH-SO₂-R^{s}, -C(=O)-NH-SO₂-NRⁿR^{n'}, -C(=S)-NRⁿR^{n'}, -SO₂-R^{s},-SO₂-O-R^{s},-SO₂-NRⁿR^{n'},-SO₂-NH-C(=O)-O-R^{c}, -SO₂-NH-C(=O)-NRⁿR^{n'}, -SO₂-NH-C(=O)-R^{c}, -O-C(=O)-NRⁿR^{n'}, -O-C(=O)-R^{c}, -O-C(=O)-NH-C(=O)-R^{c}, -O-C(=O)-NH-SO₂-R^{s} and -O-SO₂-R^{s}.

R is selected from -C(=O)-R^{z}, -C(=O)-H, -CH₂(OH) and -CH₂O-C(=O)-C₁-C₆alkyl.

R^{a} is R^{a'} or R^{a''} substituted with 1-3 R^{a'}.

R^{a'} is selected from the group; hydrogen, halo(F. Cl, Br, I), cyano, isocyanate, carboxy, carboxy-C₁-C₁₁alkyl, amino, amino-C₁-C₈alkyl, aminocarbonyl, carboxamido, carbamoyl, carbamoyloxy, formyl, formyloxy, azido, nitro, imidazoyl, ureido, thioureido, thiocyanato, hydroxy, C₁-C₆alkoxy, mercapto, sulfonamido, het, phenoxy, phenyl, benzamido, tosyl, morpholino, morpholinyl, piperazinyl, piperidinyl, pyrrolinyl imidazolyl and indolyl.

R^{a"} is selected from the group C₀-C₁₀alkyl-Q-C₀-C₆alkyl, C₀-C₁₀alkenyl-Q-C₀-C₆alkyl, C₀-C₁₀alkynyl-Q-C₀-C₆alkyl, C₃-C₁₁cycloalkyl-Q-C₀-C₆alkyl, C₃-C₁₀cycloalkenyl-Q-C₀-C₆alkyl, C₁-C₆alkyl-C₆-C₁₂aryl-Q-C₀-C₆alkyl C₆-C₁₀aryl-C₁-C₆alkyl-Q-C₀-C₆alkyl, C₀-C₆alkyl-het-Q-C₀-C₆alkyl, C₀-C₆alkyl-Q-het-C₀-C₆alkyl, het-C₀-C₆alkyl-Q-C₀-C₆alkyl, C₀-C₆alkyl-Q-C₆-C₁₂aryl and Q-C₁-C₆alkyl.

R^{c} is selected from hydrogen and substituted or unsubstituted; C₁-C₁₀alkyl, C₂-C₁₀alkenyl, C₂-C₁₀alkynyl, C₃-C₁₁cycloalkyl, C₃-C₁₀cycloalkenyl, C₁-C₆alkyl-C₆-C₁₂aryl, C₆-C₁₀aryl-C₁-C₆alkyl, C₁-C₆alkyl-het, het-C₁-C₆alkyl, C₆-C₁₂aryl and het, where the substituents on any alkyl, alkenyl or alkynyl are 1-3 R^{a} and the substituents on any aryl or het are 1-3 R^{d}.

R^{d} is selected from R^{p} and R^{h}.

R^{h} is selected from the group OH, OCF₃, OR^{c}, SR^{m}, halo(F, Cl. Br, I), CN, isocyanate, NO₂, CF₃, C₀-C₆alkyl-NRⁿR^{n'}, C₀-C₆alkyl-C(=O)-NRⁿR^{n'}, C₀-C₆alkyl-C(=O)-R^{a}, C₁-C₈alkyl, C₁-C₈alkoxy, C₂-C₈alkenyl, C₂-C₈alkynyl, C₃-C₆cycloalkyl, C₃-C₆cycloalkenyl, C₁-C₆alkyl-phenyl, phenyl-C₁-C₆alkyl, C₁-C₆alkyloxycarbonyl, phenyl-C₀-C₆alkyloxy, C₁-C₆alkyl-het, het-C₁-C₆alkyl, SO₂-het, -O-C₆-C₁₂aryl, -SO₂-C₆-C₁₂aryl, -SO₂-C₁-C₆alkyl and het, where any alkyl, alkenyl or alkynyl may optionally be substituted with 1-3 groups selected from OH, halo(F, Cl, Br, I), nitro, amino and aminocarbonyl and the substituents on any aryl or het are 1-2 hydroxy, halo(F, Cl, Br, I), CF₃, C₁-C₆alkyl, C₁-C₆alkoxy, nitro and amino.

R^{m} is selected from S-C₁-C₆alkyl, C(=O)-C₁-C₆alkyl, C(=O)-NRⁿR^{n'}, C₁-C₆alkyl, halo(F, Cl, Br, I)-C₁-C₆alkyl, benzyl and phenyl.

Rⁿ is selected from the group R^{c}, NH-C(=O)-O-R^{c}, NH-C(=O)-R^{c}, NH-C(=O)-NHR^{c}, NH-SO₂-R^{s}, NH-SO₂-NH-C(=O)-R^{c}, NH-C(=O)-NH-SO₂-R^{s}, C(=O)-O-R^{c}, C(=O)-R^{c}, C(=O)-NHR^{c}, C(=O)-NH-C(=O)-O-R^{c}, C(=O)-NH-C(=O)-R^{c}, C(=O)-NH-SO₂-R^{s}, C(=O)-NH-SO₂-NHR^{s}, SO₂-R^{s}, SO₂-O-R^{s}, SO₂-N(R^{c})₂, SO₂-NH-C(=O)-O-R^{c}, SO₂-NH-C(=O)-O-R^{c} and SO₂-NH-C(=O)-R^{c}.

R^{n'} is selected from hydrogen, hydroxy and substituted or unsubstituted C₁-C₁₁alkyl, C₁-C₁₁alkoxy, C₂-C₁₀alkenyl, C₂-C₁₀alkynyl, C₃-C₁₁cycloalkyl, C₃-C₁₀cycloalkenyl, C₁-C₆alkyl-C₆-C₁₂aryl, C₆-C₁₀aryl-C₁-C₆alkyl, C₆-C₁₀aryl-C₀-C₆alkyloxy, C₁-C₆alkyl-het, het-C₁-C₆alkyl, C₆-C₁₂aryl, het, C₁-C₆alkylcarbonyl, C₁-C₈alkoxycarbonyl, C₃-C₈cycloalkylcarbonyl, C₃-C₈ cycloalkoxycarbonyl, C₆-C₁₁aryloxycarbonyl, C₇-C₁₁ arylalkoxycarbonyl, heteroarylalkoxycarbonyl, heteroarylalkylcarbonyl, heteroarylcarbonyl, heteroarylalkylsulfonyl, heteroarylsulfonyl, C₁-C₆alkylsulfonyl and C₆-C₁₀arylsulfonyl, where the substituents on any alkyl, alkenyl or alkynyl are 1-3 R^{a} and the substituents on any aryl, het or heteroaryl are 1-3 R^{d}.

Optionally, Rⁿ and R^{n'} taken together with the common nitrogen to which they are attached may from an optionally substituted heterocycle selected from morpholinyl, piperazinyl, thiamorpholinyl, pyrrolidinyl, imidazolidinyl, indolinyl, isoindolinyl, 1,2,3,4-tetrahydro-quinolinyl, 1,2,3,4-tetrahydro-isoquinolinyl, thiazolidinyl and azabicyclononyl, where the substituents are 1-3 R^{a}.

R^{o} is selected from hydrogen and substituted or unsubstituted C₁-C₆alkyl, C₁-C₆alkylcarbonyl, C₂-C₆alkenyl, C₂-C₆alkynyl, C₃-C₈cycloalkyl and benzoyl, where the substituents on any alkyl are 1-3 R^{a} and the substituents on any aryl are 1-3 R^{p}.

R^{p} is selected from the group; OH, halo(F, Cl. Br, I), CN, isocyanate, OR^{c}, SR^{m}, SOR^{c}, NO₂, CF₃, R^{c}, NRⁿR^{n'}, N(Rⁿ)-C(=O)-O-R^{c}, N(Rⁿ)-C(=O)-R^{c}, C₀-C₆alkyl-SO₂-R^{c}, C₀-C₆alkyl-SO₂-NRⁿR^{n'}, C(=O)-R^{c}, O-C(=O)-R^{c}, C(=O)-O-R^{c} and C(=O)-NRⁿR^{n'}, where the substituents on any alkyl, alkenyl or alkynyl are 1-3 R^{a} and the substituents on any aryl or het are 1-3 R^{d}.

R^{s} is a substituted or unsubstituted group selected from; C₁-C₈alkyl, C₂-C₈alkenyl, C₂-C₈alkynyl, C₃-C₈cycloalkyl, C₃-C₆cycloalkenyl, C₀-C₆alkyl-phenyl, phenyl-C₀-C₆alkyl, C₀-C₆alkyl-het and het-C₀-C₆alkyl, where the substituents on any alkyl, alkenyl or alkynyl are 1-3 R^{a} and the substituents on any aryl or het are 1-3 R^{d}.

R^{z} is a substituted or unsubstituted group selected from; hydroxy, C₁-C₁₁alkoxy, C₃-C₁₂cycloalkoxy, C₈-C₁₂aralkoxy, C₈-C₁₂arcycloalkoxy, C₆-C₁₀aryloxy, C₃-C₁₀alkylcarbonyloxyalkyloxy, C₃-C₁₀alkoxycarbonyloxyalkyloxy, C₃-C₁₀alkoxycarbonylalkyloxy, C₅-C₁₀cycloalkylcarbonyloxyalkyloxy, C₅-C₁₀cycloalkoxycarbonyloxyalkyloxy, C₅-C₁₀cycloalkoxycarbonylalkyloxy, C₈-C₁₂aryloxycarbonylalkyloxy, C₈-C₁₂aryloxycarbonyloxyalkyloxy, C₈-C₁₂arylcarbonyloxyalkyloxy, C₅-C₁₀alkoxyalkylcarbonyloxyalkyloxy, (Rⁿ)(R^{n'})N(C₁-C₁₀alkoxy) 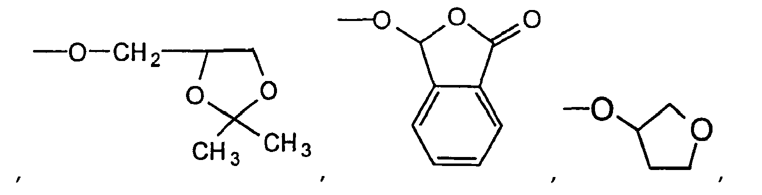 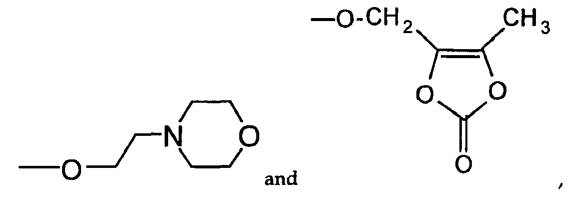 where the substituents on any alkyl, alkenyl or alkynyl are 1-3 R^{a} and the substituentson any aryl or het are 1-3 R^{d} and pharmaceutically acceptable salts thereof.

### Brief Description of the Drawings

**FIGURE 1** A cartoon illustrating lymphocyte recruitment to a site of infection is provided. Lymphocyte rolling and adhesion to ICAM expressing cells (leukocytes, endothelium, epithelium) is shown.
**FIGURE 2** A cartoon illustrating the human ICAM-1:LFA-1 receptor binding assay (protein/protein assay) is provided. Inhibition of the CD11a/CD18-ICAM-1 interaction is quantitated by adding known amounts of inhibitors to the protein/protein assay system described in Example 3.
**FIGURE 3** A cartoon illustrating the human T Cell Adhesion Assay described in Example 4 is provided.
**FIGURE 4** A cartoon illustrating the human T cell proliferation assay is provided. Cell proliferation is measured by tritiated thymidine uptake.
**FIGURE 5** A cartoon illustrating the human one way mixed lymphocyte response is provided. Cell proliferation is measured by tritiated thymidine uptake.

### Description of the Preferred Embodiments

### A. Definitions

The term "LFA-1-mediated disorders" refers to pathological states caused by cell adherence interactions involving the LFA-1 receptor on lymphocytes. Examples of such disorders include T-cell inflammatory responses such as inflammatory skin diseases including psoriasis; responses associated with inflammatory bowel disease (such as Crohn's disease and ulcerative colitis); adult respiratory distress syndrome; dermatitis; meningitis; encephalitis; uveitic; allergic conditions such as eczema and asthma and other conditions involving infiltration of T-cells and chronic inflammatory responses; skin hypersensitivity reactions (including poison ivy and poison oak); atherosclerosis; leukocyte adhesion deficiency; autoimmune diseases such as rheumatoid arthritis, systemic lupus erythematosus (SLE), diabetes mellitus, multiple sclerosis, Reynaud's syndrome, autoimmune thyroiditis, experimental autoimmune encephalomyelitis, Sjorgen's syndrome, type 1 diabetes, juvenile onset diabetes, and immune responses associated with delayed hypersensitivity mediated by cytokines and T-lymphocytes typically found in tuberculosis, sarcoidosis, polymyositis, granulomatosis and vasculitis; pernicious anemia; diseases involving leukocyte diapedesis; CNS inflammatory disorder, multiple organ injury syndrome secondary to septicaemia or trauma; autoimmune haemolytic anemia; myethemia gravis; antigen-antibody complex mediated diseases; all types of transplantations, including graft vs. host or host vs. graft disease; etc.

"Treating" such diseases includes therapy, prophylactic treatment, prevention of rejection of grafts, and induction of tolerance of grafts on a long-term basis.

The term "graft" as used herein refers to biological material derived from a donor for transplantation into a recipient. Grafts include such diverse material as, for example, isolated cells such as islet cells, tissue such as the amniotic membrane of a newborn, bone marrow, hematopoietic precursor cells, and organs such as skin, heart, liver, spleen, pancreas, thyroid lobe, lung, kidney, tubular organs (e.g., intestine, blood vessels, or esophagus), etc. The tubular organs can be used to replace damaged portions of esophagus, blood vessels, or bile duct. The skin grafts can be used not only for burns, but also as a dressing to damaged intestine or to close certain defects such as diaphragmatic hernia. The graft is derived from any mammalian source, including human, whether from cadavers or living donors. Preferably the graft is bone marrow or an organ such as heart and the donor of the graft and the host are matched for HLA class II antigens.

The term "mammal" refers to any animal classified as a mammal, including humans, domestic and farm animals, and zoo, sports, or pet animals, such as dogs, horses, cats, cows, etc. Preferably, the mammal herein is human.

The term "mammalian host" as used herein refers to any compatible transplant recipient. By "compatible" is meant a mammalian host that will accept the donated graft. Preferably, the host is human. If both the donor of the graft and the host are human, they are preferably matched for HLA class II antigens so as to improve histocompatibility.

The term "donor" as used herein refers to the mammalian species, dead or alive, from which the graft is derived. Preferably, the donor is human. Human donors are preferably volunteer blood-related donors that are normal on physical examination and of the same major ABO blood group, because crossing major blood group barriers possibly prejudices survival of the allograft. It is, however, possible to transplant, for example, a kidney of a type O donor into an A, B or AB recipient

The term "transplant" and variations thereof refers to the insertion of a graft into a host, whether the transplantation is syngeneic (where the donor and recipient are genetically identical), allogeneic (where the donor and recipient are of different genetic origins but of the same species), or xenogeneic (where the donor and recipient are from different species). Thus, in a typical scenario, the host is human and the graft is an isograft, derived from a human of the same or different genetic origins. In another scenario, the graft is derived from a species different from that into which it is transplanted, such as a baboon heart transplanted into a human recipient host, and including animals from phylogenically widely separated species, for example, a pig heart valve, or animal beta islet cells or neuronal cells transplanted into a human host.

The term "LFA-1 antagonist" as used herein generally refers to a benzoyl-amino acid (AA) derivative or a peptidomimetic thereof that acts as a competitive inhibitor of the CD11a and/or CD18 interaction with ICAM-1, soluble forms of ICAM-1 and bound or soluble forms of ICAM-2, ICAM-3 and telencephalin.

The term "immunosuppressive agent" as used herein for adjunct therapy refers to substances that act to suppress or mask the immune system of the host into which the graft is being transplanted. This would include substances that suppress cytokine production, down regulate or suppress self-antigen expression, or mask the MHC antigens. Examples of such agents include 2-amino-6-aryl-5-substituted pyrimidines (see U.S. Pat. No. 4,665,077, *supra,* the disclosure of which is incorporated herein by reference), azathioprine (or cyclophosphamide, if there is an adverse reaction to azathioprine); bromocryptine; glutaraldehyde (which masks the MHC antigens, as described in U.S. Pat. No. 4,120,649, *supra*); anti-idiotypic antibodies for MHC antigens and MHC fragments; cyclosporin A; steroids such as glucocorticosteroids, e.g., prednisone, methylprednisolone, and dexamethasone; cytokine or cytokine receptor antagonists including anti-interferon-β, or -a antibodies; anti-tumor necrosis factor-a antibodies; anti-tumor necrosis factor-β antibodies; anti-interleukin-2 antibodies and anti-IL-2 receptor antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published 7/26/90), streptokinase; TGF-β; streptodornase; RNA or DNA from the host; FK506; RS-61443; deoxyspergualin; rapamycin; T-cell receptor (Cohen et al., U.S. Pat. No. 5,114,721); T-cell receptor fragments (Offner et al., Science, 251:430-432 (1991); copending U.S. Ser. No. 07/853,362 filed March 18, 1992, the disclosure of which is incorporated herein by reference; Howell, WO 90/11294; Ianeway, Nature, 341:482 (1989); and Vandenbark, WO 91/01133); and T-cell receptor antibodies (EP 340,109) such as T10B9. These agents are administered at the same time or at separate times from the CD11a or CD18 antagonists as used in this invention, and are used at the same or lesser dosages than as set forth in the art.

The preferred adjunct immunosuppressive agent will depend on many factors, including the type of disorder being treated including the type of transplantation being performed, as well as the patient's history, but a general overall preference is that the agent be selected from cyclosporin A, a glucocorticosteroid (most preferably prednisone or methylprednisolone), OKT-3 monoclonal antibody, azathioprine, bromocryptine, heterologous anti-lymphocyte globulin, or a mixture thereof.

"Increasing tolerance of a transplanted graft" by a host refers to prolonging the survival of a graft in a host in which it is transplanted, i.e., suppressing the immune system of the host so that it will better tolerate a foreign transplant.

The term "alkyl" means a branched or unbranched, saturated aliphatic hydrocarbon radical, having the number of carbon atoms specified, or if no number is specified, having up to 12 carbon atoms. Unless otherwise specified the term also encompasses unsaturated alkyls defined as "cycloalkyl", "alkenyl" and "alkynyl" below. Examples of preferred alkyl radicals include methyl, ethyl, n-propyl, isopropyl, n-butyl, iso-butyl, sec-butyl, tert-butyl, n-pentyl, 2-methylbutyl, 2,2-dimethylpropyl, n-hexyl, 2-methylpentyl, 2,2-dimethylbutyl, n-heptyl, 2-methylhexyl, and the like. The term "C₀-C₆ alkyl" and similar terms containing "C₀" means a covalent bond when the number of carbons is zero (C₀) or C₁-C₆ alkyl. If necessary to prevent a dangling valence the term "C₀" may include a hydrogen atom. A preferred "C₁-C₆ alkyl" group is methyl.

The term "substituted Cₙ-Cₘ alkyl" where m and n are integers identifying the range of carbon atoms contained in the alkyl group, denotes the above alkyl groups that are substituted by the groups listed or if no groups are listed one, two or three halogen, hydroxy, protected hydroxy, amino, protected amino, C₁-C₇ acyloxy, nitro, carboxy, protected carboxy, carbamoyl, carbamoyloxy, cyano, methylsulfonylamino or C₁-C₄ alkoxy groups. The substituted alkyl groups may be substituted once, twice or three times with the same or with different substituents.

Examples of the above substituted alkyl groups include but are not limited to; cyanomethyl, nitromethyl, hydroxymethyl, trityloxymethyl, propionyloxymethyl, aminomethyl, carboxymethyl, alkyloxycarbonylmethyl, allyloxycarbonylaminomethyl, carbamoyloxymethyl, methoxymethyl, ethoxymethyl, t-butoxymethyl, acetoxymethyl, chloromethyl, bromomethyl, iodomethyl, trifluromethyl, 6-hydroxyhexyl, 2,4-dichloro(n-butyl), 2-amino(iso-propyl), 2-carbamoyloxyethyl and the like. A preferred group of examples within the above "C₁-C₁₂ substituted alkyl" group includes the substituted methyl group, e.g. a methyl group substituted by the same substituents as the "substituted Cₙ-Cₘ alkyl" group. Examples of the substituted methyl group include groups such as hydroxymethyl, protected hydroxymethyl (e.g. tetrahydropyranyloxymethyl), acetoxymethyl, carbamoyloxymethyl, trifluoromethyl, chloromethyl, bromomethyl and iodomethyl.

The terms "C₁-C₁₂ alkyloxy" or "C₁-C₁₂ alkoxy" are used interchangeably herein and denote groups such as methoxy, ethoxy, n-propoxy, isopropoxy, n-butoxy, t-butoxy and like groups.

The terms "C₁-C₁₂ acyloxy" or "C₁-C₁₂ alkanoyloxy" are used interchangeably and denote herein groups such as formyloxy, acetoxy, propionyloxy, butyryloxy, pentanoyloxy, hexanoyloxy, heptanoyloxy, and the like.

The terms "C₁-C₁₂ alkylcarbonyl", "C₁-C₁₂ alkanoyl" and "C₁-C₁₂ acyl" are used interchangeably herein encompass groups such as formyl, acetyl, propionyl, butyryl, pentanoyl, hexanoyl, heptanoyl, benzoyl and the like.

The term "cycloalkyl" as used herein refers to a mono-, bi-, or tricyclic saturated or unsaturated ring, each ring having from 3 to 14 carbon atoms and preferably 3 to 7 carbon atoms. Optionally any ring carbon may be oxidized to from a carbonyl.

The term "alkenyl" means a branched or unbranched hydrocarbon radical having the number of carbon atoms designated containing one or more carbon-carbon double bonds, each double bond being independently cis, trans, or a nongeometric isomer.

The term "alkynyl" means a branched or unbranched hydrocarbon radical having the number of carbon atoms designated containing one or more carbon-carbon triple bonds.

The terms "C₁-C₁₂ alkylthio" and "C₁-C₁₂ substituted alkylthio" denote C₁-C₁₂ alkyl and C₁-C₁₂ substituted alkyl groups, respectively, attached to a sulfur which is in turn the point of attachment for the alkylthio or substituted alkylthio group to the group or substituent designated.

The term "aryl" when used alone means a homocyclic aromatic radical whether or not fused having the number of carbon atoms designated. Preferred aryl groups include phenyl, napthyl, biphenyl, phenanthrenyl, naphthacenyl, and the like (see e.g. Lang's Handbook of Chemistry (Dean, J. A., ed.) 13th ed. Table 7-2 [1985]).

The term "substituted phenyl" or "substituted aryl" denotes a phenyl group or aryl group substituted with one, two or three substituents chosen from the groups listed or those selected from; halogen(F, Cl, Br, I), hydroxy, protected hydroxy, cyano, nitro, C₁-C₆alkyl, C₁-C₆alkoxy, carboxy, protected carboxy, carboxymethyl, protected carboxymethyl, hydroxymethyl, protected hydroxymethyl, aminomethyl, protected aminomethyl, trifluoromethyl N-(methylsulfonylamino) or other groups specified.

Examples of the term "substituted phenyl" includes but is not limited to a mono- or di(halo)phenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono or di(alkoxy)phenyl group, for example, 2,6-dimethoxyphenyl, 4-methoxyphenyl, 3-ethoxyphenyl, 4-(isopropoxy)phenyl, 4-(t-butoxy)phenyl, 3-ethoxy-4-methoxyphenyl and the like; 3- or 4- trifluoromethylphenyl; a mono- or dicarboxyphenyl or (protected carboxy)phenyl group such 4-carboxyphenyl; a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl and the like. Preferred substituted phenyl groups include the 2- and 3-trifluoromethylphenyl, the 4-hydroxyphenyl, the 2-aminomethylphenyl and the 3-(N-(methylsulfonylamino))phenyl groups.

The term "arylalkyl" means one, two, or three aryl groups having the number of carbon atoms designated, appended to an alkyl radical having the number of carbon atoms designated including but not limited to; benzyl, napthylmethyl, phenethyl, benzhydryl (diphenylmethyl), trityl, and the like. A preferred arylalkyl group is the benzyl group.

The term "substituted C₆-C₁₀aryl-C₁-C₈alkyl" denotes a C₁-C₈alkyl group substituted at any carbon with a C₆-C₁₀aryl group bonded to the alkyl group through any aryl ring position and substituted on the C₁-C₈alkyl portion with one, two or three groups chosen from halogen (F, Cl, Br, I), hydroxy, protected hydroxy, amino, protected amino, C₁-C₇acyloxy, nitro, carboxy, protected carboxy, carbamoyl, carbamoyloxy, cyano, C₁-C₆alkylthio, N-(methylsulfonylamino) or C₁-C₄alkoxy. Optionally the aryl group may be substituted with one, two, or three groups chosen from halogen, hydroxy, protected hydroxy, nitro, C₁-C₆alkyl, C₁-C₄alkoxy, carboxy, protected carboxy, carboxymethyl, protected carboxymethyl, hydroxymethyl, protected hydroxymethyl, aminomethyl, protected aminomethyl, or an N-(methylsulfonylamino) group. As before, when either the C₁-C₈alkyl portion or the aryl portion or both are disubstituted, the substituents can be the same or different.

Examples of the term "substituted C₆-C₁₀aryl-C₁-C₈alkyl" include groups such as 2-phenyl-1-chloroethyl, 2-(4-methoxyphenyl)ethyl, 2,6-dihydroxy-4-phenyl(n-hexyl), 5-cyano-3-methoxy-2-phenyl(n-pentyl), 3-(2,6-dimethylphenyl)n-propyl, 4-chloro-3-aminobenzyl, 6-(4-methoxyphenyl)-3-carboxy(n-hexyl), 5-(4-aminomethyl phenyl)-3-(aminomethyl)(n-pentyl), and the like.

The term "carboxy-protecting group" as used herein refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl,4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, b-(trimethylsilyl)ethyl, b-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the benzodiazepinedione molecule and can be removed at the appropriate point without disrupting the remainder of the molecule. In particular, it is important not to subject the carboxy-protected benzodiazepinedione molecule to strong nucleophilic bases or reductive conditions employing highly activated metal catalysts such as Rarey nickel. (Such harsh removal conditions are also to be avoided when removing amino-protecting groups and hydroxy-protecting groups, discussed below.) Preferred carboxylic acid protecting groups are the allyl and p-nitrobenzyl groups. Similar carboxy-protecting groups used in the cephalosporin, penicillin and peptide arts can also be used to protect a carboxy group substituents of the benzodiazepinedione. Further examples of these groups are found in E. Haslam. "Protective Groups in Organic Chemistry", J. G. W. McOmie, Ed., Plenum Press, New York, N.Y., 1973, Chapter 5, and T.W. Greene, "Protective Groups in Organic Synthesis", John Wiley and Sons, New York, NY, 1981, Chapter 5. The term "protected carboxy" refers to a carboxy group substituted with one of the above carboxy-protecting groups.

As used herein the term "amide-protecting group" refers to any group typically used in the peptide art for protecting the peptide nitrogens from undesirable side reactions. Such groups include p-methoxyphenyl, 3,4-dimethoxybenzyl, benzyl, O-nitrobenzyl, di-(p-methoxyphenyl)methyl, triphenylmethyl, (p-methoxyphenyl)diphenylmethyl, diphenyl-4-pyridylmethyl, m-2-(picolyl)-N'-oxide, 5-dibenzosuberyl, trimethylsilyl, t-butyl dimethylsilyl, and the like. Further descriptions of these protecting groups can be found in "Protective Groups in Organic Synthesis", by Theodora W. Greene, 1981, John Wiley and Sons, New York.

Unless otherwise specified, the terms "heterocyclic group" or "heterocyclic" or "HET", "het" or "heterocyclyl" are used interchangeably as used herein refer to any mono-, bi-, or tricyclic saturated, unsaturated, or aromatic ring having the number of atoms designated where at least one ring is a 5-, 6- or 7-membered ring containing from one to four heteroatoms selected from the group nitrogen, oxygen, and sulfur (*Lang's Handbook of Chemistry, supra*). Typically, the 5-membered ring has 0 to 2 double bonds and the 6- or 7-membered ring has 0 to 3 double bonds and the nitrogen, carbon or sulfur atoms in the ring may optionally be oxidized (e.g. NO₂, C=O and SO₂) and any nitrogen heteroatom may optionally be quaternized. Included in the definition are any bicyclic groups where any of the above heterocyclic rings are fused to a benzene ring. Heterocyclics in which oxygen and sulfur are the heteroatom are preferred when the heterocycly forms all or a part of "D" in Formula I.

The following ring systems are examples of the heterocyclic (whether substituted or unsubstituted) radicals denoted by the term "heterocylic" or het: thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, thiazinyl, oxazinyl, triazinyl, thiadiazinyl, oxadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl, tetrazolo[1,5-b]pyridazinyl and purinyl, as well as benzo-fused derivatives, for example benzoxazolyl, benzofuryl, benzothiazolyl, benzothiadiazolyl, benzotriazolyl, benzoimidazolyl and indolyl.

Heterocyclic 5-membered ring systems containing a sulfur or oxygen atom and one to three nitrogen atoms are also suitable for use in the instant invention. Examples of such preferred groups include thiazolyl, in particular thiazol-2-yl and thiazol-2-yi N-oxide, thiadiazolyl, in particular 1,3,4-thiadiazol-5-yl and 1,2,4-thiadiazol-5-yl, oxazolyl, preferably oxazol-2-yl, and oxadiazolyl, such as 1,3,4-oxadiazol-5-yl, and 1,2,4-oxadiazol-5-yl. A group of further preferred examples of 5-membered ring systems with 2 to 4 nitrogen atoms include imidazolyl, preferably imidazol-2-yl; triazolyl, preferably 1,3,4-triazol-5-yl; 1,2,3-triazol-5-yl, 1,2,4-triazol-5-yl, and tetrazolyl, preferably 1H-tetrazol-5-yl. A preferred group of examples of benzo-fused derivatives are benzoxazol-2-yl, benzthiazol-2-yl and benzimidazol-2-yl.

Further suitable specific examples of the above heterocylic ring systems are 6-membered ring systems containing one to three nitrogen atoms. Such examples include pyridyl, such as pyrid-2-yl, pyrid-3-yl, and pyrid-4-yl; pyrimidyl, preferably pyrimid-2-yl and pyrimid-4-yl; triazinyl, preferably 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl radicals, are a preferred group.

The substituents for the optionally substituted heterocyclic ring systems, and further examples of the 5- and 6-membered ring systems discussed above can be found in W. Druckheimer et al., U.S. Patent No. 4,278,793.

Another preferred group of "heterocyclics" or "het" include; 1,3-thiazol-2-yl, 4-(carboxymethyl)-5-methyl-1,3-thiazol-2-yl, 4-(carboxymethyl)-5-methyl-1,3-thiazol-2-yl sodium salt, 1,2,4-thiadiazol-5-yl, 3-methyl-1,2,4-thiadiazol-5-yl, 1,3,4-triazol-5-yl, 2-methyl-1,3,4-triazol-5-yl, 2-hydroxy-1,3,4-triazol-5-yl, 2-carboxy-4-methyl-1,3,4-triazol-5-yl sodium salt, 2-carboxy-4-methyl-1,3,4-triazol-5-yl, 1,3-oxazol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-thiol-1,3,4-thiadiazol-5-yl, 2-(methylthio)-1,3,4-thiadiazol-5-yl, 2-amino-1,3,4-thiadiazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 2-methyl-1H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(n-oxide)-pyridaz-3-yl, 6-hydroxypyridaz-3-yl, 1-methylpyrid-2-yl, 1-methylpyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-astriazin-3-yl, 2,5-dihydro-5-oxo-6-hydroxy-as-triazin-3-yl sodium salt, 2,5-dihydro-5-oxo-6-hydroxy-2-methyl-astriazin-3-yl sodium salt, 2,5-dihydro-5-oxo-6-hydroxy-2-methyl-as-triazin-3-yl, 2,5-dihydro-5-oxo-6-methoxy-2-methyl-as-triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5-b]pyridazin-6-yl and 8-aminotetrazolo[1,5-b]-pyridazin-6-yl.

An alternative group of "heterocyclics" includes; 4-(carboxymethyl)-5-methyl-1,3-thiazol-2-yl, 4-(carboxymethyl)-5-methyl-1,3-thiazol-2-yl sodium salt, 1,3,4-triazol-5-yl, 2-methyl-1,3,4-triazol-5-yl, 1H-tetrazol-5-yl, 1-methyl-1H-tetrazol-5-yl, 1-(1-(dimethylammo)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 1,2,3-triazol-5-yl, 1,4,5,6-tetrahydro-5,6-dioxo-4-methyl-as-triazin-3-yl, 1,4,5,6-tetrahydro-4-(2-formylmethyl)-5,6-dioxo-as-triazin-3-yl, 2,5-dihydro-5-oxo-6-hydroxy-2-methyl-as-triazin-3-yl sodium salt, 2,5-dihydro-5-oxo-6-hydroxy-2-methyl-as-triazin-3-yl, tetrazolo[1,5-blpyridazin-6-yl, and 8-aminotetrazolo[1,5-b]pyridazin-6-yl.

Bivalent radicals L, whether branched or unbranched, derived from alkanes, alkenes, alkadienes, alkynes, alkadiynes, and arenes optionally containing O, N and/or S atoms, or homo- and heterocycles either aromatic or aliphatic, are designated by adding a free valence "-" to both ends of the corresponding monovalent radical. Atoms bearing the free valences may include any C,O,N or S.

"Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic non-toxic bases are isopropylamine, diethylamine, ethanolamine, trimethamine, dicyclohexylamine, choline, and caffeine.

The term "prodrug" as used herein means a derivative or precursor of a parent drug molecule that enhances pharmaceutically desirable characteristics or properties (e.g. transport, bioavailablity, pharmacodynamics, etc.) and that requires biotransformation, either spontaneous or enzymatic, within the organism to release the active parent drug. Examples of carboxylic prodrugs include precursors such as aldehydes, alcohol's or amines or derivatives such as esters

### B. Uses

The LFA-1 and/or Mac-1 antagonists of this invention are useful for therapeutic use in those diseases and conditions for which inhibition or modulation of the LFA-1 and/or Mac-1 interaction with ICAM, especially ICAM-1, is indicated. Such diseases and conditions include: T-cell inflammatory responses such as inflammatory skin diseases including psoriasis; responses associated with inflammatory bowel disease (such as Crohn's disease and ulcerative colitis); adult respiratory distress syndrome; dermatitis; meningitis; encephalitis; uveitis; allergic conditions such as eczema and asthma, psoriasis and other conditions involving infiltration of T-cells and chronic inflammatory responses; skin hypersensitivity reactions (including poison ivy and poison oak), allergic contact dermatitis; atherosclerosis; autoimmune diseases such as rheumatoid arthritis, systemic lupus erythematosus (SLE), diabetes mellitus, multiple sclerosis, Reynaud's syndrome, autoimmune thyroiditis, experimental autoimmune encephalomyelitis, Sjorgen's syndrome, juvenile onset diabetes, and immune responses associated with delayed hypersensitivity mediated by cytokines and T-lymphocytes typically found in tuberculosis, sarcoidosis, polymyositis, granulomatosis and vasculitis; pernicious anemia; diseases involving leukocyte diapedesis; CNS inflammatory disorder, multiple organ injury syndrome secondary to septicaemia or trauma; autoimmune haemolytic anemia; myasthenia gravis; antigen-antibody complex mediated diseases; all types of transplantations, including graft vs. host or host vs. graft disease, HIV infection and the like.

Other leukocyte mediated diseases for which the instant competitive inhibitors may be used include: hemorrhagic shock, ischemia/reperfusion injury, bypass surgery, burns, stroke, post CABG surgery, vasculitis, cerebral edema (broader, restenosis, AMI and non Q wave MI.

### C. Preferred Embodiments

### 1. CD11a/CD18:ICAM-1 Competitive Inhibitors

One embodiment of the invention comprises a compound represented by Formula I capable of inhibiting binding of the leukocyte LFA-1 receptor to its native *in vivo* ligand(s), especially ICAM-1. Preferred inhibitors include compounds represented by structural Formula I:

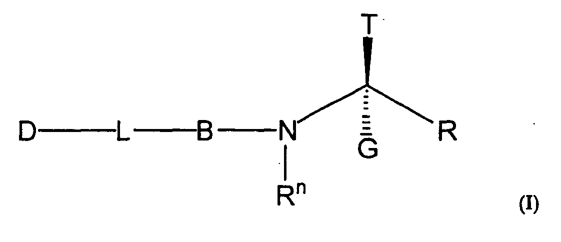

Referring to Formula I the following important structural features of the instant peptidiomimetic LFA-1 inhibitors can be identified:
a. The negatively charged acidic moiety R or prodrug form thereof;
b. The substituent T, a naturally occurring amino acid side chain and derivatives thereof;
c. The amide nitrogen(N) and substituents (Rⁿ):
d. The substituted "benzoyl" ring B;
e. substituents of the B ring, namely R^{p};
f. The spacer or linking moiety L.
g. The distal aromatic moiety D; and
h. substituents of D, namely R^{d}.

### (a) The negatively charged acidic moiety R

The preferred negatively charged acidic moiety R is the carboxyl group (-COOH) or a prodrug thereof. Generally the carboxyl group R and prodrug forms thereof is designated COR^{z}. Suitable R^{z}'s include C₁-C₈alkoxy, C₁-C₈dialkyl-aminocarbonylmethoxy and C₆-C₁₀arylC₁-C₈dialkylaminocarbonylmethoxy. Other suitable prodrugs R^{z} includes the following groups:

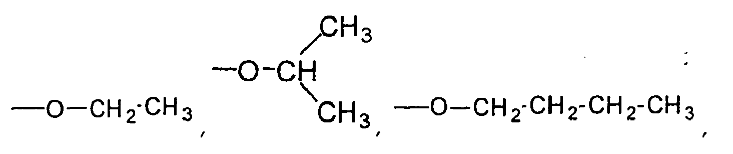

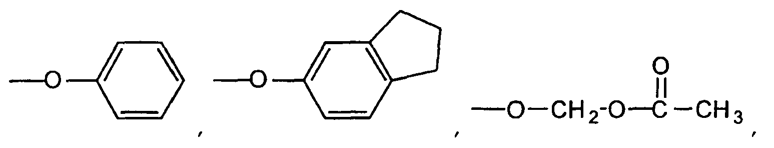

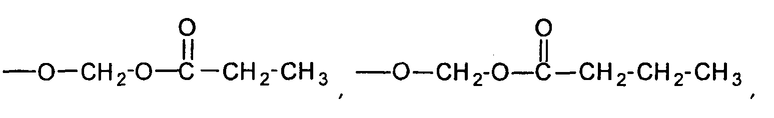

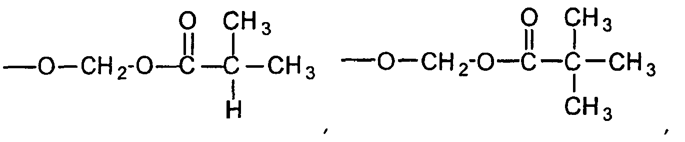

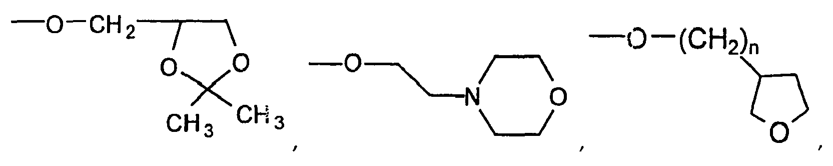

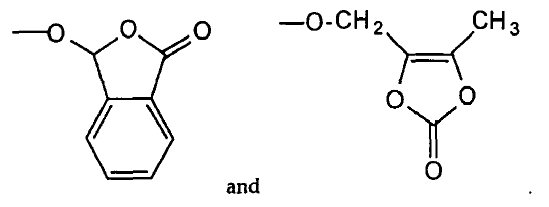

### (b) The substituent T or U-Q-V-W

T of formula I is usually the sidechain of any α-amino acid, preferably the L configuration, or a homolog or derivative thereof. Preferably T will contain a hydrogen bond donating group such as CONH₂, NHCOH, NH₂, OH or NH. T will frequently be a 1-4 carbon alkane containing an amide, carbamate, ureido, sulfonamide and an optionally substituted phenyl or heterocycle. The heterocycle will usually be a 5 or 6 member ring with 1 or 2 hetero atoms selected from N, O and S. Such heterocycles include furan, thiophene, pyrrole, pyridine and piperidine. substituents include halogens such as chloro and fluro, nitro, cyano, alkyl and halo substituted alkyl, substituted or unsubstituted amides, amines, carbamates sulfonamides, ureidos and the like.

Examples of T will also include a lower alkyl, cycloalkyl, alkenyl or alkynyl substituted with an aromatic ring, especially a heteroaryl or C₆-C₁₄aryl, substituted with 0-3 R^{d}. Suitable aromatic rings include any mono-, bi-, or tricyclic saturated, unsaturated, or aromatic ring having three to seven atoms in the ring, where at least one ring is a 5-, 6- or 7-membered ring containing from zero to four heteroatoms selected from the group nitrogen, oxygen, and sulfur, optionally substituted with R^{d}. Optionally the aromatic rings may be linked through a C₁-C₄ alkyl. Preferred ring's are substituted phenyl and het as defined above optionally substituted with R^{d}. More preferred optionally substituted aromatic ring's are selected from the group; 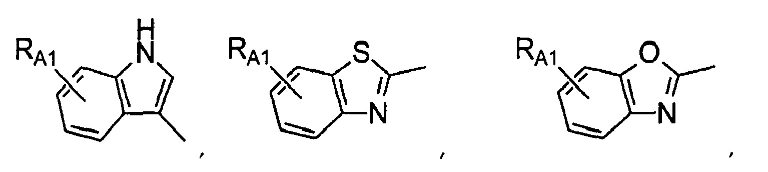 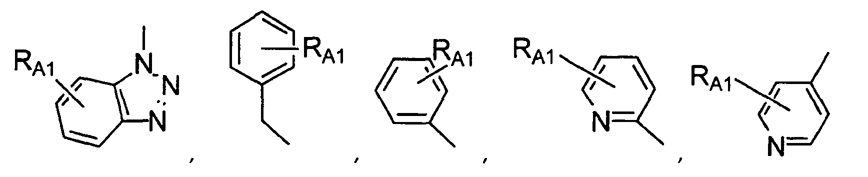 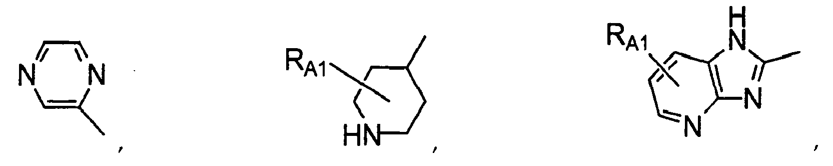 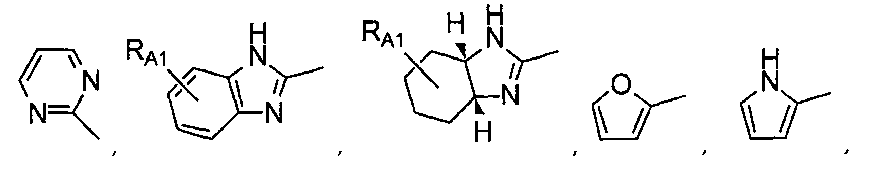 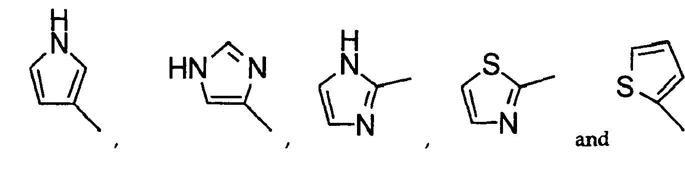 where R_{A1} is 0-3 R^{d} or U-V-W.

Other optionally prefered substituents T are U-Q-V-W defined below. Specifically, T may preferably be -C₁-C₆alkyl-Q-V-W, where Q is -N(Rⁿ)-, -C(=O)-, -N(Rⁿ)C(=O)-, -C(=O)-N(Rⁿ)-, - N(Rⁿ)C(=O)-N(Rⁿ)-, -N(Rⁿ)C(=O)-O-, -O-C(=O)-N(Rⁿ)-, -N(Rⁿ)S(=O)₂-, -S(=O)₂-N(Rⁿ)-, -C(=O)-O- or -O-; V may be het or absent and W is provided in Table 1.

Generally, each of U, Q, V and W are independently selected according to the Table 1 below. U, Q and V may also each independently be absent (i.e. one or more of U, Q, V may be a covalent bond).

**Table 1**

| U | Q | V | W |
|---|---|---|---|
| -C₁-C₆alkyl- | -O- | -C₁-C₆alkyl- | R^{a} |
| -C₂-C₆alkenyl- | -S(O)₀₋₂- | -C₃-C₈cycloalkyl- | OR° |
| -C₁-C₆alkynyl- | -SO₂N(Rⁿ)- | -C₀-C₆alkyl-het- | SR^{m} |
| -C₃-C₈cycloalkyl- | -N(Rⁿ)- | -C₀-C₆alkyl-C₆-C₁₀aryl- | NRⁿR^{n'} |
| -C₆-C₁₀aryl- | -N(Rⁿ)C(=O)- | -C₂-C₆alkenyl- | NHCOOR^{c} |
| | -N(Rⁿ)C(=O)-O- | furan | NHCONRⁿR^{n'} |
| | -N(Rⁿ)-SO₂- | thiophene | NHCOR^{c} |
| | -C(=O)- | pyrrole | NHSO₂R^{s} |
| | -C(=O)-O- | phenyl | NHSO₂NRⁿR^{n'} |
| | -het- | piperidine | NHSO₂NHCOR^{c} |
| | -C(=O)-N(Rⁿ)- | piperazine | NHCONHSO₂R^{s} |
| | -O-C(=O)-N(Rⁿ)- | morpholine | CONHCOOR^{c} |
| | -PO(OR^{c})-O- | pyridine | CONHCOR^{c} |
| | -P(O)-O- | | CONHCONRⁿRⁿ |
| | | | CONHSO₂R^{s} |
| | | | CONHSO₂NRⁿR n' |
| | | | CSNRⁿR^{n'} |
| | | | SO₂-R^{s} |
| | | | SO₃R^{s} |
| | | | SO₂NRⁿR^{n'} |
| | | | OSO₂R^{s} |
| | | | SO₂NHCOOR^{c} |

Where any alkyl, alkenyl or alkynyl is substituted with 0-3 R^{a} and any aryl or het are substituted with 0-3 R^{d}, and where R^{a}, R^{c}, R^{d}, R^{m}, Rⁿ, R^{n'}, R° and R^{s} are defined above. More specifically, each of U, Q, V and W may be independently selected according to Table 2 below.

**Table 2**

| U | Q | V | W |
|---|---|---|---|
| -CH₂- | -N(Rⁿ)C(=O)- | 2-thienyl | - |
| -CH₂- | -N(Rⁿ)C(=O)- | 2-furyl | - |
| -CH₂- | -N(Rⁿ)C(=O)-O- | -CH₂-CH=CH₂ | - |
| -CH₂- | -C(=O)-NH₂ | - | - |
| -CH₂- | -N(Rⁿ)C(=O)- | 2-thienyl | halo |
| -CH₂- | -NH-C(=O)-NH- | phenyl | -CN |
| -CH₂-CH₂-CH₂- | -N(Rⁿ)-SO₂- | 2-thienyl | - |
| -CH₂- | -O-C(=O)-NH- | phenyl | methyl |
| -CH₂-CH₂-CH₂- | -N(Rⁿ)-SO₂- | thioimidazole | -NH-C(=O)-CH₃ |
| -CH₂-CH₂-CH₂- | -NH-SO₂- | phenyl | -NH-C(=O)-CH₃ |
| -CH₂-CH₂-CH₂- | -NH-SO₂- | 2-thienyl | - |
| -CH₂- | -NH-C(=O)- | pyrrole | tri-methyl |
| -CH₂-CH₂- | -NH-C(=O)- | 3-chloro-2-thienyl | methylsulfonyl |
| -CH₂-CH₂- | -NH-C(=O)- | cyclopropyl | - |
| -CH₂-CH₂- | -NH-C(=O)- | 2-thienyl | chloro |
| -CH₂- | -NH-C(=O)- | 2-furyl | methyl |

(c) the substituents (Rⁿ) for amide nitrogen N are lower alkyl or hydrogen and preferably hydrogen.
(d) The substituted "benzoyl" ring B is preferably selected from the group: 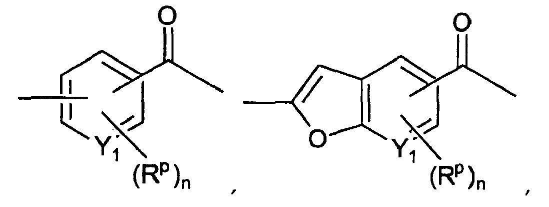 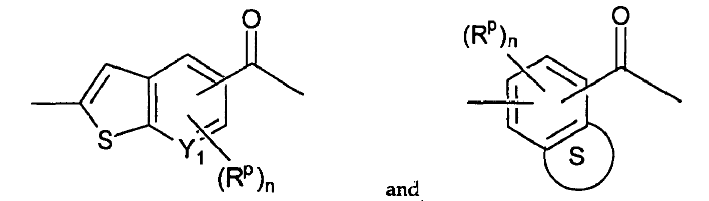 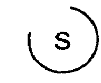 is a fused hetero- or homocyclic ring containing 5, 6 or 7 atoms, the ring being unsaturated, partially saturated or aromatic, the heteroatoms selected from 1-3 O, S or N, Y₁ is selected from CH or N, n is 0-3. Preferably B is a para-substituted benzoyl group.
(e) substituents of B (R^{p}) are defined above. Preferably when B is a para-substituted benzoyl group the remaining positions on B are substituted with one or more halo (F, Cl, Br) or lower alkyl groups.
(f) The linking group L
   The length of the bivalent radical L appears to be important to optimal biological activity. By length is meant the distance between the "B" or benzoyl moiety (eg from the para position on B), including the amide or amide isostere bonded to the benzoyl moiety, and the distal group D. Preferably L is 3, 4 or 5 methylene (-CH₂-) equivalents in length depending on the atoms in L and the nature of D. Thus L is composed of L¹-L³ and optionally L⁴ and L⁵. Each L¹⁻⁵ is independently selected from oxo (-O-), S(O)ₛ, C(=O), CR¹⁻⁵R^{1'-5'}, CR¹⁻⁵, het, NRⁿ or N, where s is 0-2. For example, functional groups in L (in addition to -CH₂- or CR¹⁻⁵R^{1'-5'}) include one or more of the following: 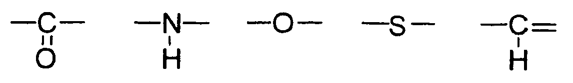 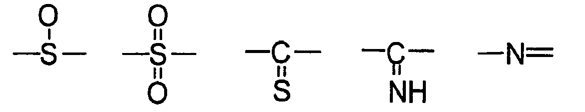 which may be located within the linker L (e.g. forming amides, imides, amidines, guanidinos, ureidos, carbamates, ethers, thioethers, ketones, sulfoxides, sulfonamides and the like) or combined in any combination, provided only that the compounds so produced are stable in aqueous solution and do not exceed the above stated length requirements. For example, preferred functional groups in L, other than a C₃-C₅ alkyl, are: ethers, diethers, ketones, alcohols, esters, amides, ureidos, carbamates, carbonates, sulfonamides, sulfoxides, sulfones, and combinations thereof. Preferred lengths for L are from 0 to 4 while most preferred lengths are 1 or 3 methylene equivalents. In counting atoms comprising L, only those atoms sequentially linking the benzoyl moiety B and the distyl group D are counted except when a homo- or heterocycle (eg het) comprises L in which case the fewest number of atoms separating these moieties are counted.
   Preferred exemplary L bivalent linking groups include: -C₃-C₅-alkyl-, -C₃-C₅-alkenyl-, - CH₂C(=O)NH-, -CH₂NH-C(=O)-, -O-CH₂-C(=O)-, -CH₂-CH₂-C(=O)-, -CH=CH-C(=O)NH-CH₂-, - CH=CH-C(=O)NH-CH-(CH₃)-, -CH(OH)-CH₂-O-, -CH(OH)-CH₂-CH₂-, -CH₂-CH₂-CH(OH)-, -O-CH₂-CH(OH)-, -O-CH₂-CH(OH)-CH₂-, -O-CH₂-CH₂-CH(OH)-, -O-CH₂-CH₂-O-, -CH₂-CH₂-CH₂-O-, -CH₂-CH(OH)-CH₂-O-, -CH₂-CH₂-O-, -CH(OH)-CH₂-O-, -CH-(CH₃)-NH-C(=O)-, -CH₂-NH-SO₂-, -NH-SO₂-CH₂-, -CH₂-SO₂NH-, -SO₂NH-CH₂-, -C(=O)-NH-C(=O)-, -NH-C(=O)-NH-,-NH-C(=O)-NH-CH₂-, -CH₂-NH-C(=O)-NH-, -C(=O)-NH-CH₂-C(=O)-NH -, -NH-C(=O)-O- and -O-C(=O)-NH-.
   Preferred exemplary L bivalent linking groups containing a heterocycle include: 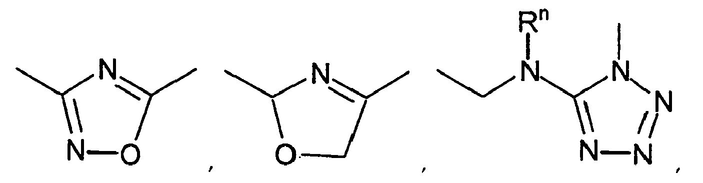 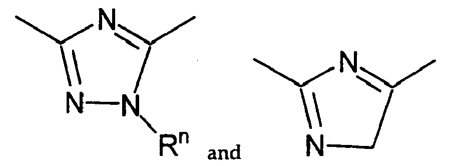 Any carbon in the bivalent linking groups may optionally be substituted with a halogen, especially fluorine.
(g) The distal moiety D may be a mono-, bi-, or tricyclic saturated, unsaturated, or aromatic ring, each ring having 5-, 6- or 7 atoms in the ring where the atoms in the ring are carbon or from 1-4 heteroatoms selected from; nitrogen, oxygen, and sulfur, each ring substituted with 0-3 R^{d}.

Optionally, D is an aromatic homocycle or aromatic heterocycle containing 1-3 heteroatoms selected from the group N, S and O, the homo- or hetero-cycles selected from: 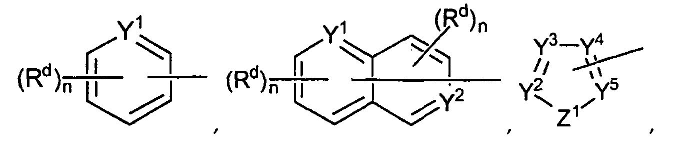 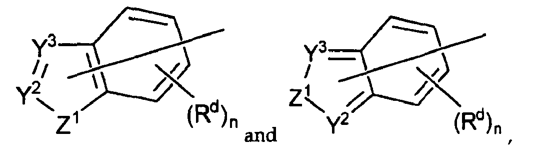 where Y¹, Y², Y³, Y⁴ and Y⁵ are CH, CR^{d} or N, Z¹ is O, S, NH or NRⁿ and n is 0-3.

More specifically, D may be:
1) a 5-member aromatic heterocycle selected from; 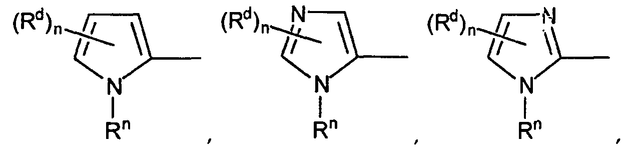 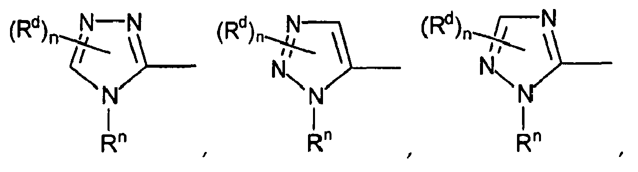 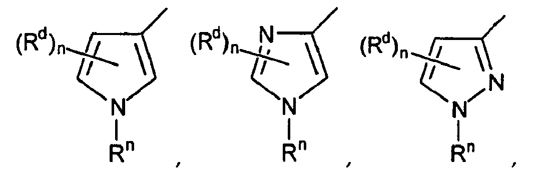 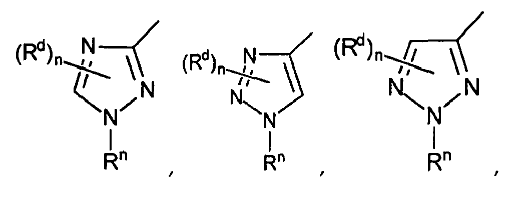 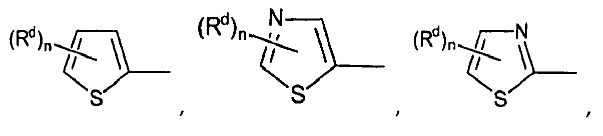 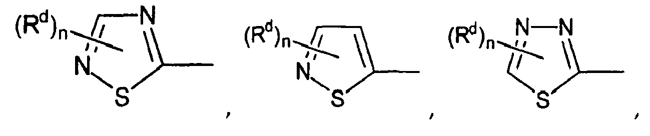 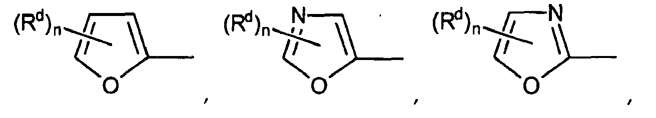 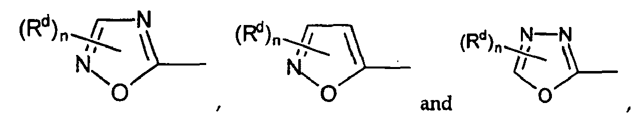
2) a 9-member aromatic heterobicycle selected from; 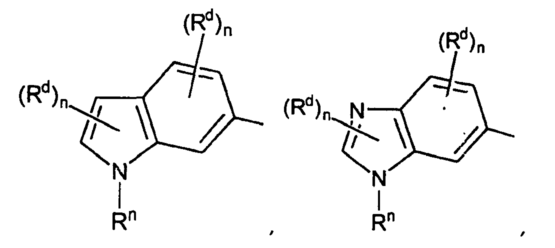 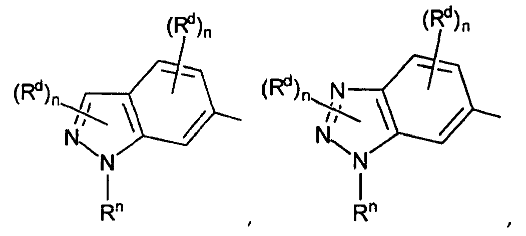 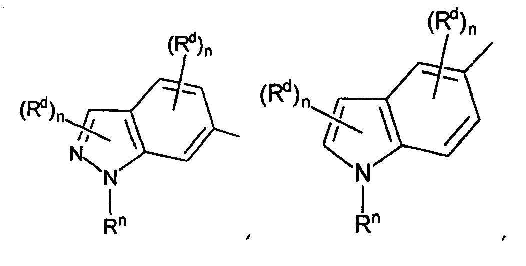 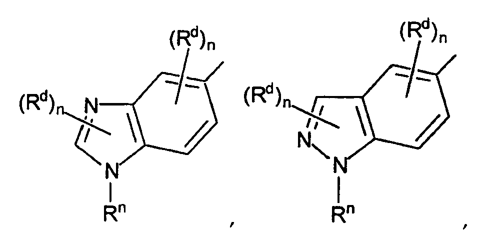 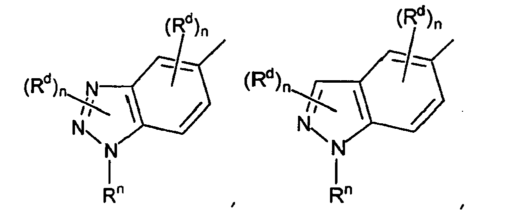 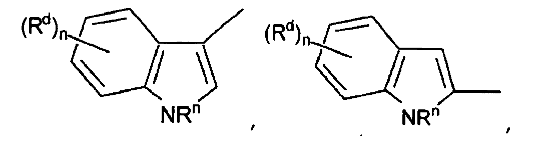 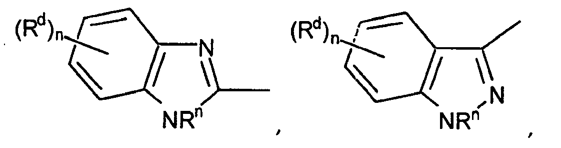 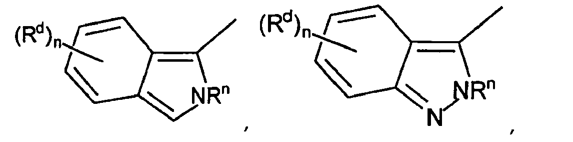 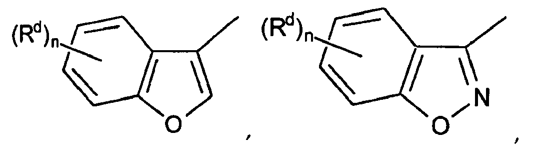 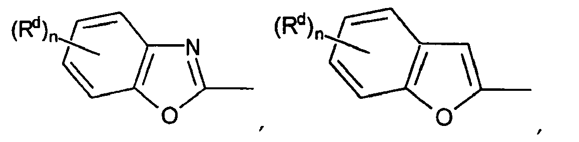 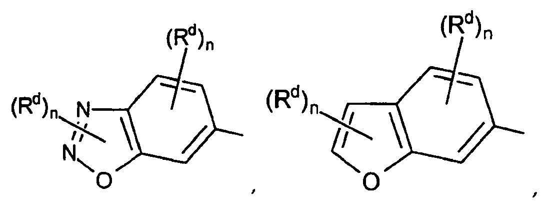 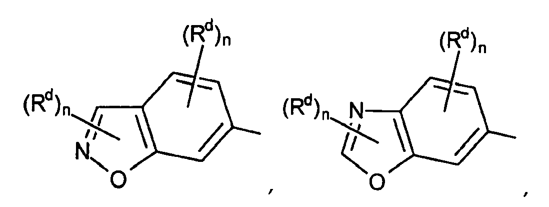 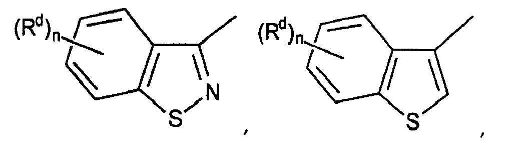 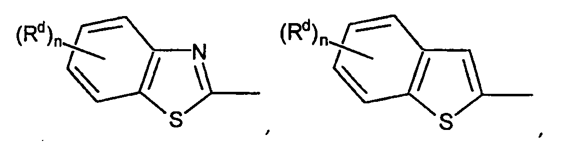 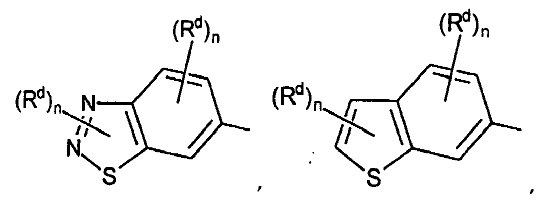 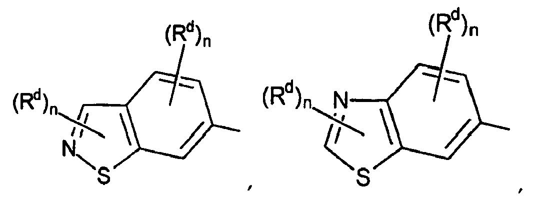 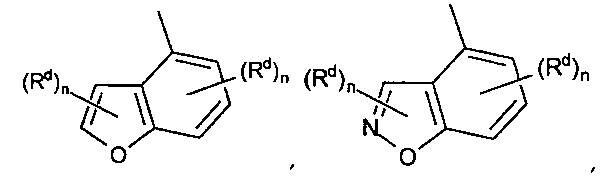 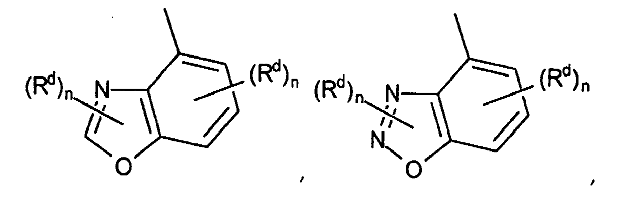 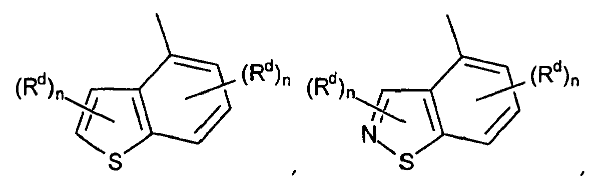 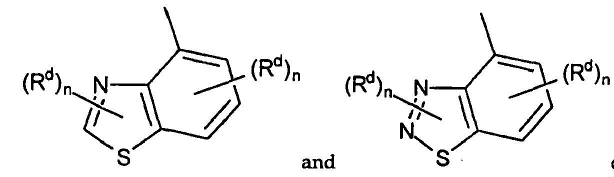 or
3) a 6-member aromatic hetero- or homocycle selected from; 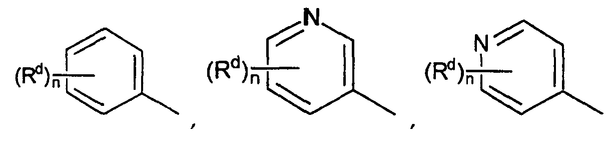 and 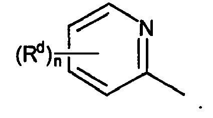 Compounds containing the foregoing preferred 5-member aromatic heterocycle and 9-member aromatic heterobicycle, 1 and 2 above, as aromatic groups D are preferred as LFA-1 specific antagonists, while the 6-member aromatic hetero- or homocycles of 3 above are preferred as D groups suitable for inhibiting both LFA-1 and Mac-1. In this latter case D is preferably substituted with a hydroxyl or precursor thereof.
(h) Preferred substituents of D are one or more groups selected from; OH, NH₂ SO₂NH₂, SO₂CH₃, CH₃, CH₂OH, CN, CH₃-C(=O)NH-, NH₂C(=O)-, NHCONH₂, CF₃, C₁-C₆ alkoxy and halo(F, Cl, Br and I).
   Exemplary preferred compounds of this invention include:

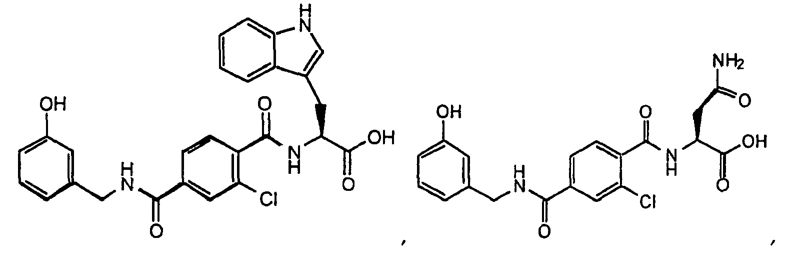

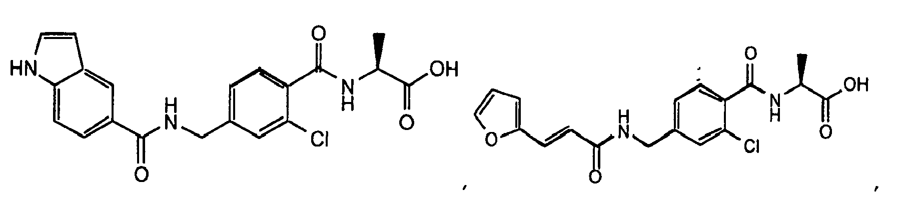

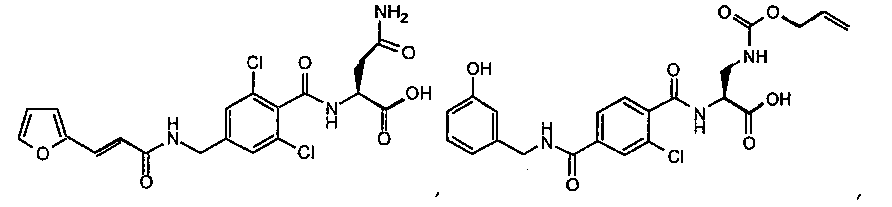

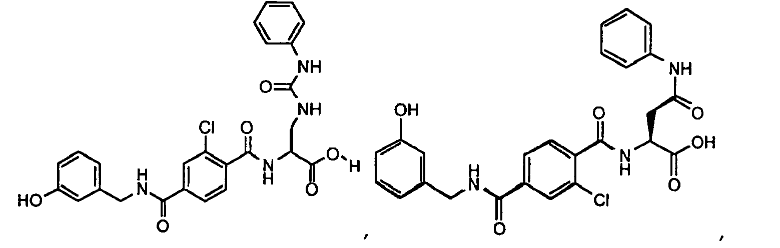

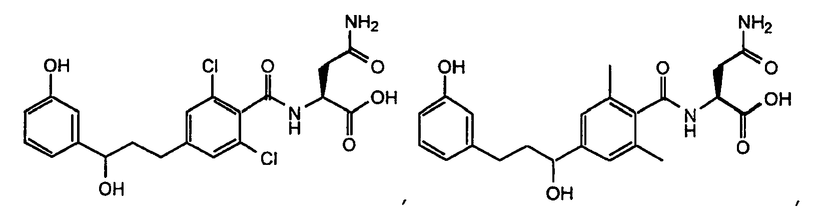

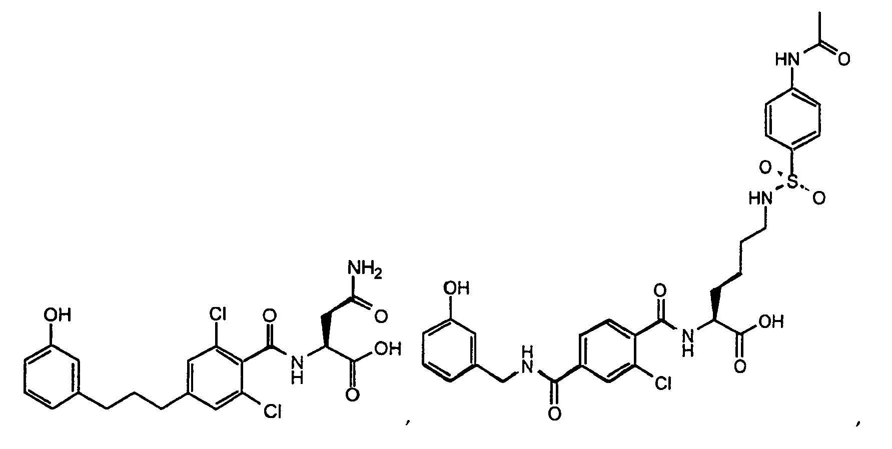

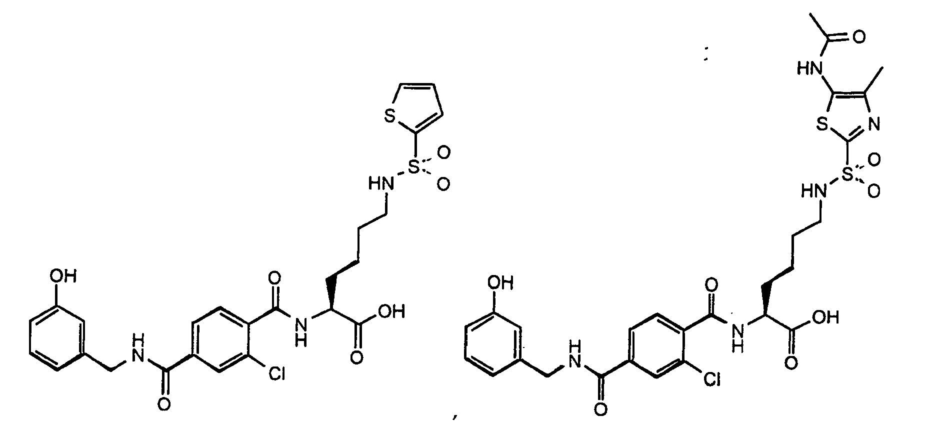

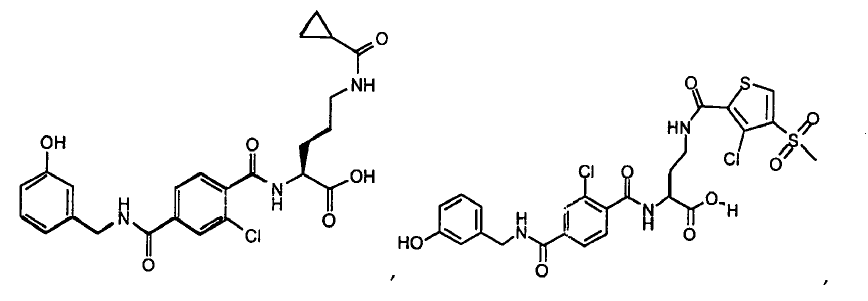

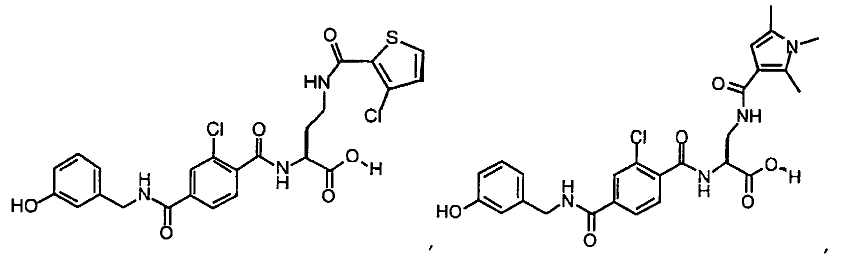

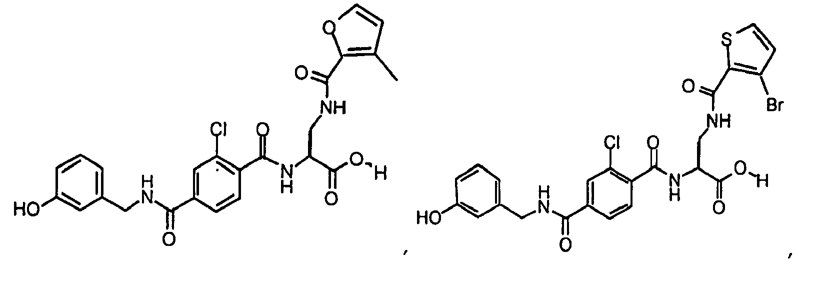

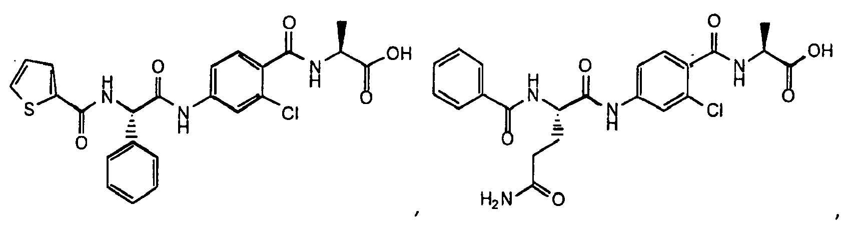

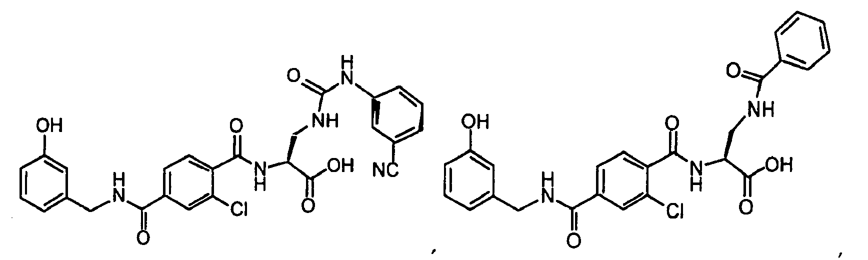

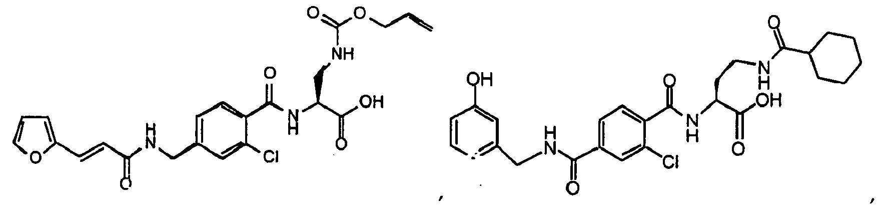

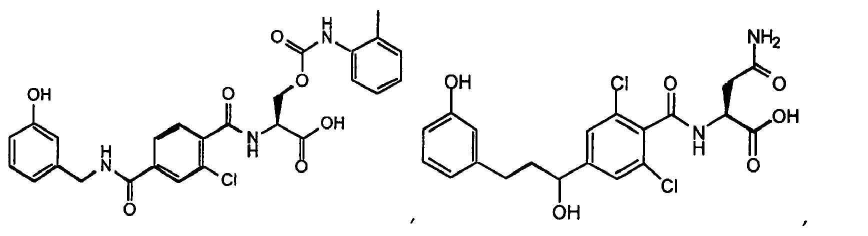

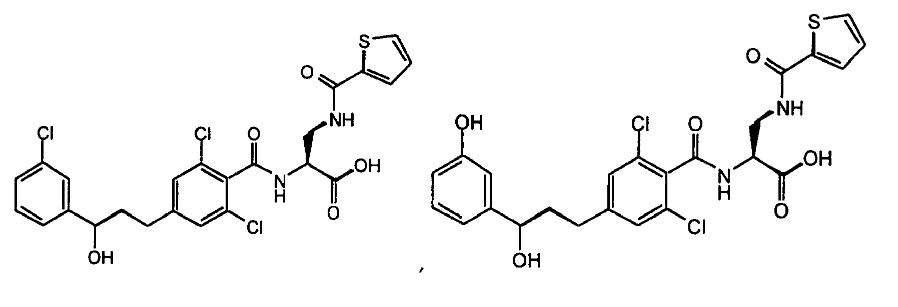

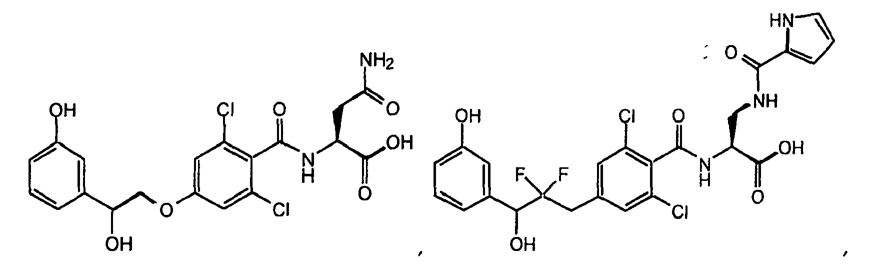

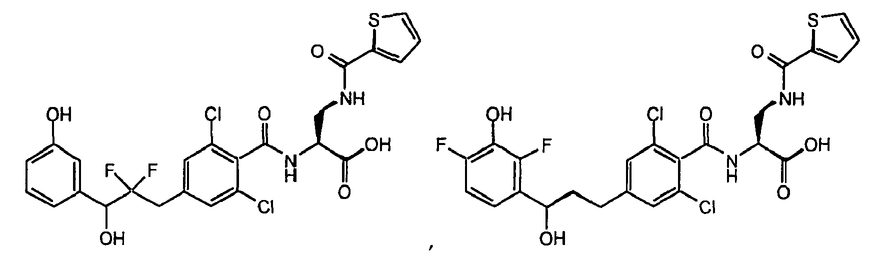

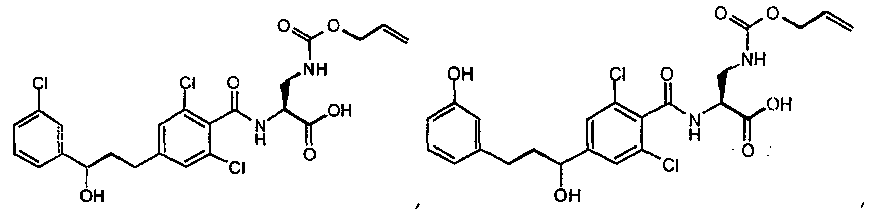

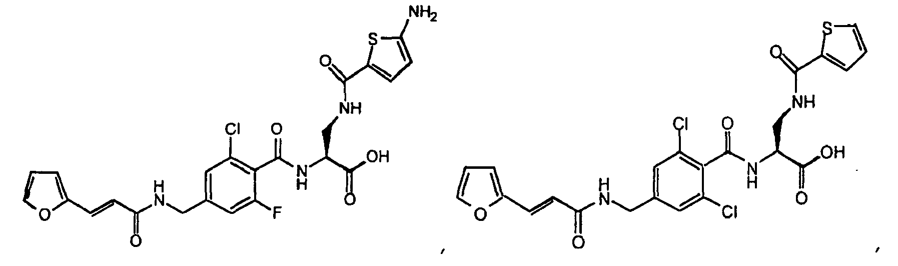

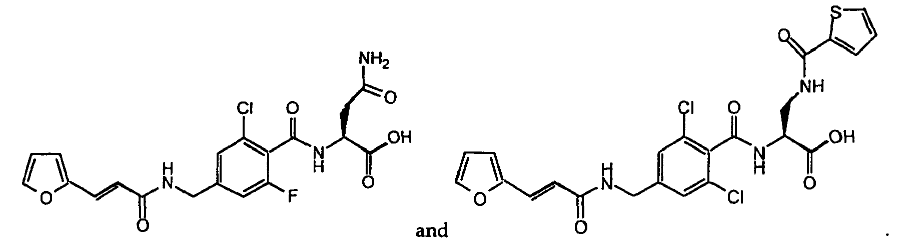

### D Methods of Making

One method of producing LFA-1 antagonists involves chemical synthesis of the "peptide" or peptidomimetic. This can be accomplished using methodologies well known to those skilled in the art (see Stewart and Young, Solid Phase Peptide Synthesis Pierce Chemical Co. Rockford, IL (1984); see also U. S. Patent No.'s 4,105,603; 3,972,859; 3,842,067; and 3,862,925)).

It will be appreciated from inspection of the compounds shown above that they all contain one or more amide or peptide bonds and thus may be considered peptidomimetics. Peptidomimetics of the invention may also be conveniently prepared using solid phase peptide synthesis (Merrifield, J. Am. Chem. Soc., 85:2149 (1964); Houghten, Proc. Natl. Acal. Sci. USA 82:5132 (1985)). Solid phase synthesis begins at the carboxy-terminus of the putative peptide by coupling a protected amino acid to a suitable resin (e.g. chloromethylated polystyrene resin) as shown in Figures 1-1 and 1-2, on pages 2 and 4 of Stewart and Young *supra.* After removal of the α-amino protecting group with, for example, trifluoroacetic acid (TFA) in methylene chloride and neutralizing in, for example TEA, the next α-amino- and sidechain protected amino acid in the synthesis is added. The remaining α-amino- and, if necessary, side-chain-protected amino acids are then coupled sequentially in the desired order by condensation to obtain an intermediate compound connected to the resin. Alternatively, some amines and acids may be coupled to one another forming a peptide prior to addition of the peptide to the growing solid phase peptide chain.

The condensation between two amino acids can be carried out according to the usual condensation methods such as the azide method, mixed acid anhydride method, DCC (N,N'-dicyclohexylcarbodiimide) or DIPC (N,N'-diisopropylcarbodiimide)methods, active ester method (p-nitrophenyl ester method, BOP [benzotriazole-1-yl-oxy-tris (dimethylamino) phosphonium hexafluorophosphate] method, N-hydroxysuccinic acid imido ester method, etc., and Woodward reagent K method.

Common to chemical syntheses of peptides is the protection of any reactive side-chain groups of the amino acids with suitable protecting groups. Ultimately these protecting groups are removed after the desired polypeptide chain has been sequentially assembled. Also common is the protection of the α-amino group on an amino acid or a fragment while that entity reacts at the carboxyl group followed by the selective removal of the α-amino-protecting group to allow subsequent reaction to take place at that location. Accordingly, it is common in peptide synthesis that an intermediate compound is produced which contains each of the amino acid residues located in the desired sequence in the peptide chain with various of these residues having side-chain protecting groups attached. These protecting groups are then commonly removed substantially at the same time so as to produce the desired resultant product following removal from the resin.

Suitable protective groups for protecting the α-and ε- amino side chain groups are exemplified by benzyloxycarbonyl (CBZ), isonicotinyloxycarbonyl (iNOC), O-chlorobenzyloxycarbonyl (2-Cl-CBZ), p-nitrobenzyloxycarbonyl [Z(NO₂], p-methoxybenzyloxycarbonyl [Z(OMe)], t-butoxycarbonyl, (BOC), t-amyloxycarbonyl (AOC), isoborrnyloxycarbonyl, adamatyloxycarbonyl, 2-(4-biphenyl)-2-propyl-oxycarbonyl (BPOC), 9-fluorenylmethoxycarbonyl (FMOC), methylsulfo-nyiethoxycarbonyl (Msc), trifluoroacetyl, phthalyl, formyl, 2-nitrophenylsulphenyl (NPS), diphenylphosphinothioyl (Ppt), dimethylophosphinothioyl (Mpt) and the like.

Protective groups for the carboxy functional group are exemplified by; benzyl ester (OBzl), cyclohexyl ester (Chx), 4-nitrobenzyl ester (ONb), t-butyl ester (OtBu), 4-pyridylmethyl ester (OPic), and the like. It is often desirable that specific amino acids such as arginine, cysteine, and serine possessing a functional group other than amino and carboxyl groups are protected by a suitable protective group. For example, the guanidino group of arginine may be protected with nitro, p-toluenesulfonyl, benzyloxycarbonyl, adamantyloxycarbonyl, p-methoxybenzenesulfonyl, 4-methoxy-2, 6-dimethylbenzenesulfonyl (Mds), 1,3,5-trimethylphenysulfonyl (Mts), and the like. The thiol group of cysteine may be protected with p-methoxybenzyl, triphenylmethyl, acetylaminomethyl ethylcarbamoyle, 4-methylbenzyl, 2, 4, 6-trimethy-benzyl (Tmb) etc., and the hydroxyl group of serine can be protected with benzyl, t-butyl, acetyl, tetrahydropyranyl and the like.

Stewart and Young *supra* provides detailed information regarding procedures for preparing peptides. Protection of α-amino groups is described on pages 14-18, and side-chain blockage is described on pages 18-28. A table of protecting groups for amine, hydroxyl and sulfhydryl functions is provided on pages 149-151.

After the desired amino acid sequence has been completed, the intermediate peptide is removed from the resin support by treatment with a reagent, such as liquid HF and one or more sulfur-containing scavengers, which not only cleaves the peptide from the resin, but also cleaves all the remaining side-chain protecting groups. Following HF cleavage, the peptide residue is washed with ether, and extracted from the resin by washing with aqueous acetonitrile and acetic acid.

Preferably in order to avoid alkylation of residues in the polypeptide, (for example, alkylation of methionine, cysteine, and tyrosine residues) a thio-cresol and cresol scavenger mixture is used.

### Other General Procedures

The peptidomimetic compounds of this invention may also be conveniently prepared by the methods for peptide synthesis described in monographs such as ("Principles of Peptide Synthesis, M. Bodanszky, Springer-Verlag, 2nd Ed., 1993; "Synthetic Peptides: A Users Guide", G. A. Grant, Ed, W. H. Freeman and Co., 1992; and references sited therein), or by other methods generally known to one skilled in the art. The synthesis of compounds of this invention that are peptidomimetic in nature (i.e. contain other than standard amide bond linkages between two or more amino acids) may be prepared by extension of the methods described in Examples 6 and by the general synthetic methods described in "Comprehensive Organic Transformations", R. C. Larock, VCH Publishers, 1989, and by methods generally known to one skilled in the art.

For compounds of Claim 1 where the amide linkages (-C(=O)-NH-) are replaced with amide isostere (Ai) linkages such as; (-C(=S)-NH-), (-S(=O)₂-NH-), -CH₂-NH-, -CH₂-S-, -CH₂-O-, - CH₂-CH₂-, -CH=CH- (cis and trans), -C(=O)-CH₂-, -CH(OH)-CH₂-, -CH(CN)-NH-, -O-C(=O)-NH-and -CH₂-SO-, amide bond replacing methods known in the art are employed. The following references describe preparation of amide isostere linkages which include these alternative-linking moieties: Spatola, A.F., Vega Data 1(3): "Peptide Backbone Modifications" (General Review) (Mar 1983), Spatola, A.F., in "Chemistry and biochemistry of Amino Acids Peptides and Proteins", B. Weinstein, ed., Marcel Dekker, New York, P. 267 (1983); Morley Trends Pharm. Sci. pp. 463-468; Hudson et al. Int. J. Pept. Prot. Res. 14:177-185 (1979) (-CH₂NH-, -CH₂CH₂-); Spatola et al., Life Sci. 38:1243-1249 (1986) (-CH₂-S-); Hann J. Chem. Soc. Perkin. Trans. 1307-314 (1982) (-CH=CH-, cis and trans); Almquist et al., J. Med. Chem. 23:1392-1398 (1980) (-C(=O)-CH₂-); Jennings-White et al., Tetrahedron Lett 23:(1982) (-C(=O)-CH₂-); Szelke et al., EP Application No. 45665 (1982) Chem Abs :9739405 (1982) (-CH(OH)-CH₂); Holladay et al., Tetrahedron Lett 24:4401-4404 (1983) (-C(OH)-CH₂-); Hruby Life Sci 31:189-199 (1982) (-CH₂S-); Cho et al., Science 261:1303-1305 (1993) (-O-C(=O)-NH-); Sherman et al., Biochem Biophys Res Comm 162(3):1126-1132 (1989) (-C(=S)-NH-); Calcagni et al., Int, J. Peptide Protein Res. 34:319-324 (1989) (-S(=O)₂-NH-); TenBrink, J. Org. Chem. 52:418-422 (1987) -CH₂-O-.

Scheme I illustrates one synthetic approach which provides access to unnatural amino acid sidechains particularly for substituent T of Formula I. The method provides for α-alkylation of the "glycine" sidechain using a solid phase approach on a commercially available machine, such as an Argonaut Nautilus 2400.

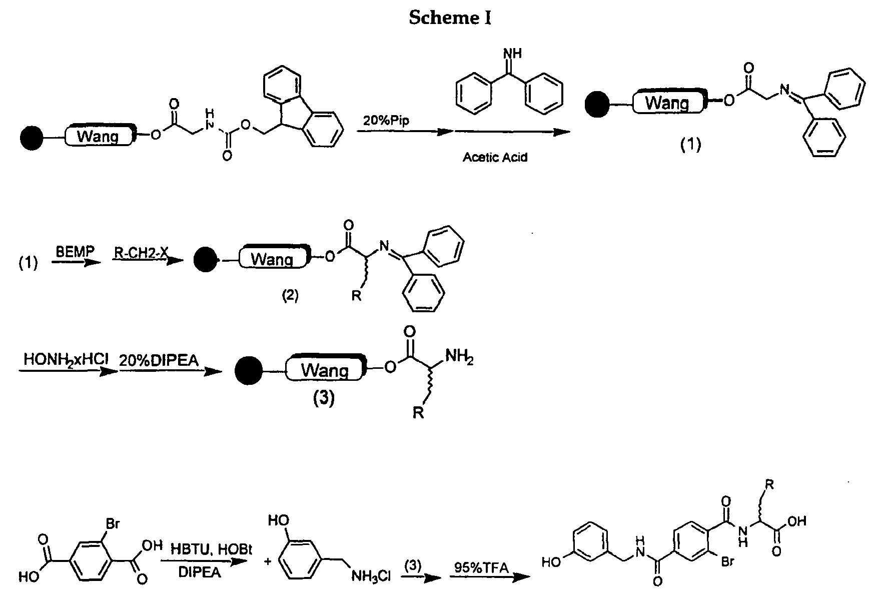

The following representative "R" groups can be introduced into the LFA-1 antagonists by the alkylation scheme above: 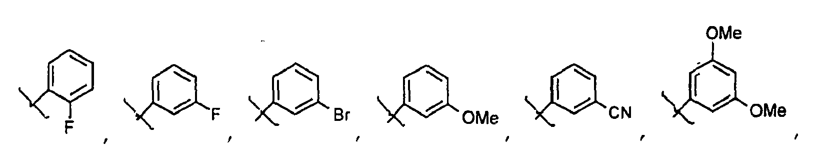 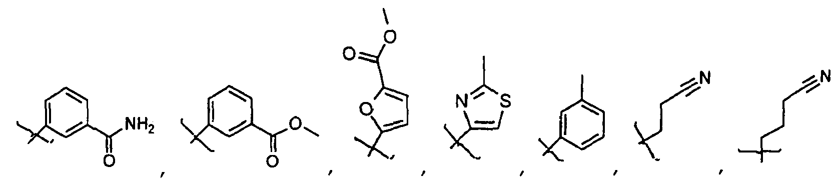 and 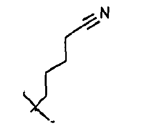 When "R" of Scheme I is an alkyl amine, prepared from the amino acids lys. orn or DAPA, reduction of the representative nitriles above or prepared from the protected (e.g. FMOC) aminoalkyl halide, synthetic routs are available to make derivatives of T including urea's, carbamates, amides and sulfonamides by known procedures.

Scheme II illustrates a solid phase approach for producing these derivatives of T.

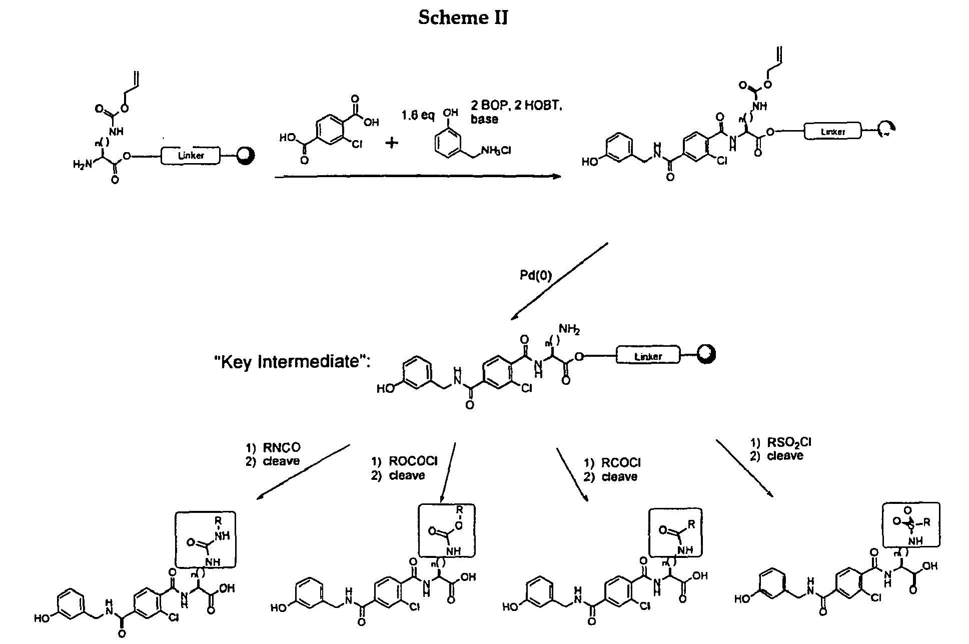

Urea's made according to Scheme II can be synthesized from representative commercially available isocyanates, RNCO's, including the following: 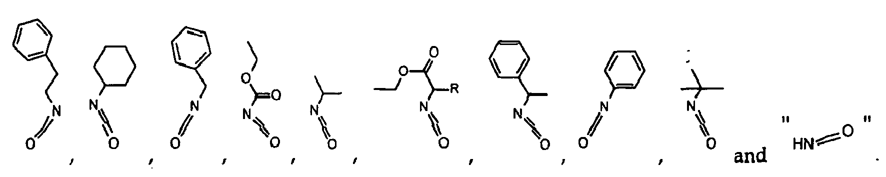 Other representative substituted aryl isocyanates suitable for use in the above scheme include:

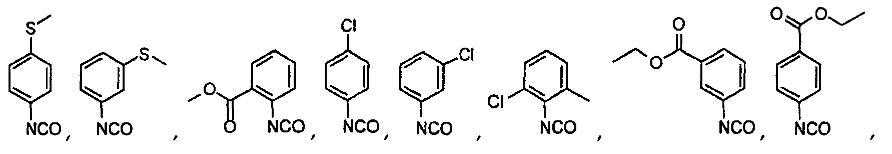

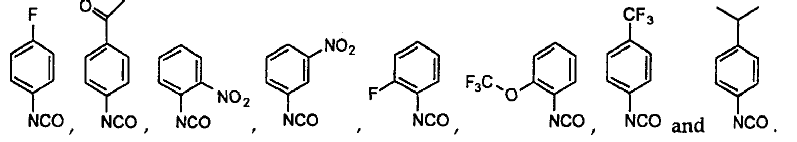

These and other isocyanates may be used to produce carbamates when the "R" in Scheme I is an alcohol (e.g. ser) according to scheme Scheme IIa below.

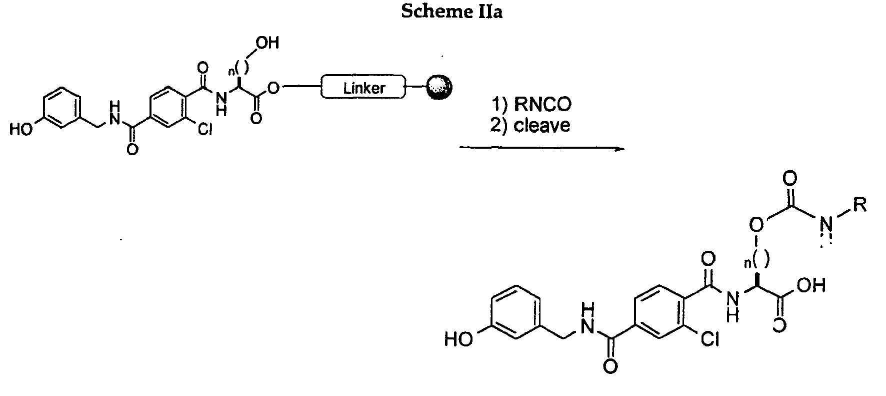

Carbamates (of the opposite orientation to Scheme IIa), amides and sulfonamides synthesized according to Scheme II can be made from representative commercially available ROCOCI's, RCOCl's and RSO₂Cl's including the following:

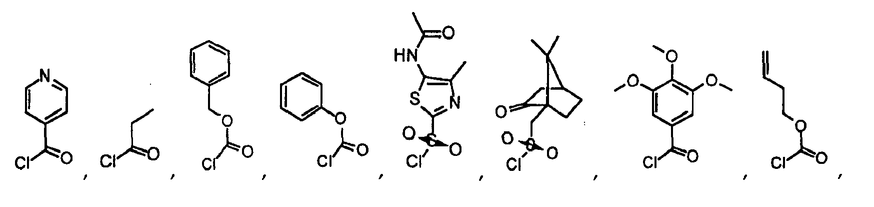

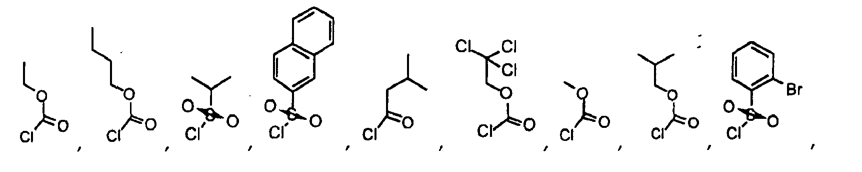

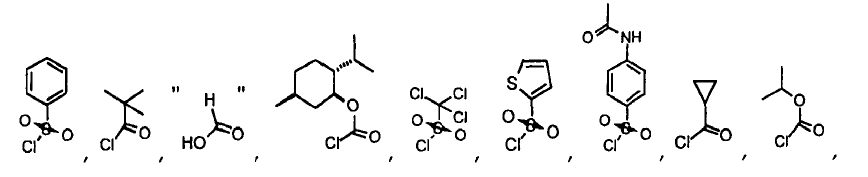

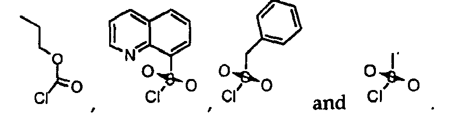

Scheme III illustrates a general synthetic route for alkyl linkers, L, for dichloro-substituted benzoyl-amino acids or derivitaves thereof. The key intermediate in this approach is the iodo, dichloro-benzoyl-AA (4).

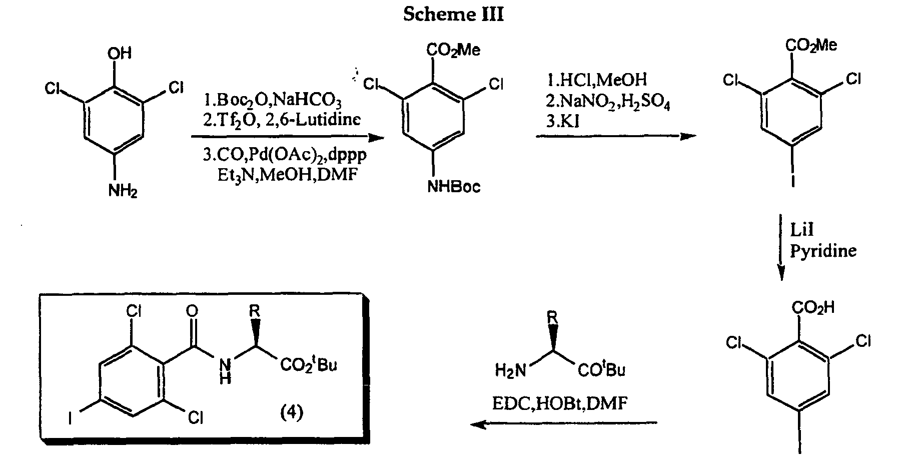

Key intermediate (4) is coupled to a variety of alkynes to produce alkyl linkers of various length. For example a 3 carbon linker can be made by coupling (4) to alkyne intermediate (5) prepared according to Scheme IIIa. 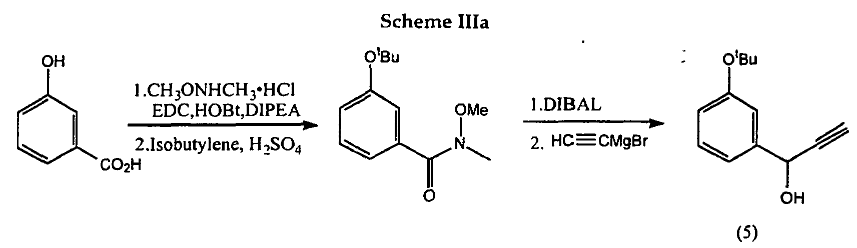 Scheme IV illustrates the synthesis of both substituted or unsubstituted alkane and substituted alkyne linkers. 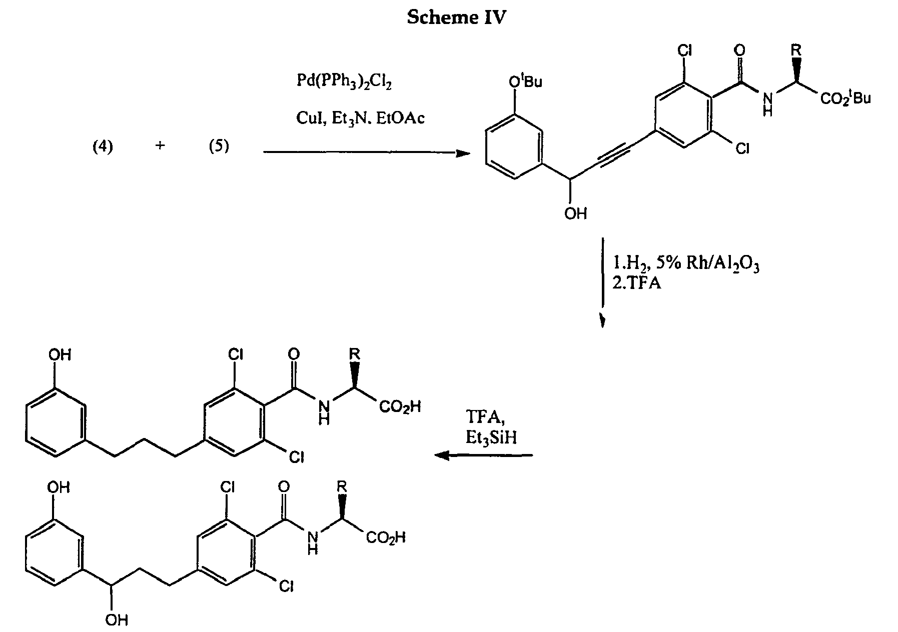 A 4 carbon linker can be made by coupling (4) to alkyne intermediate (6) prepared according to Scheme V.

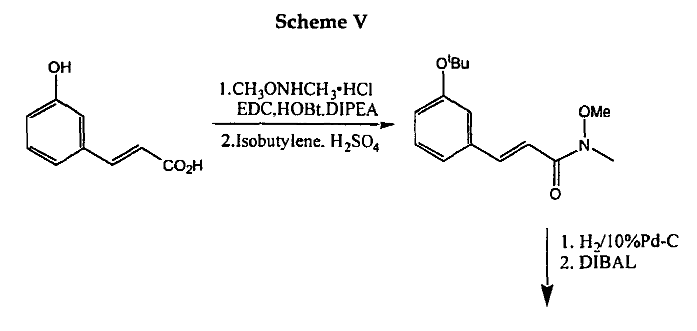

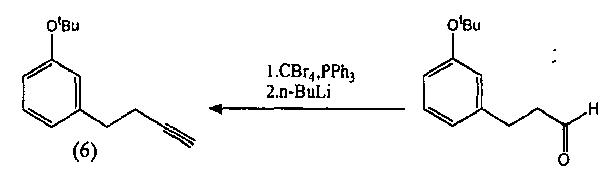

Scheme VI illustrates the synthesis of unsubstituted alkane and alkyne linkers. 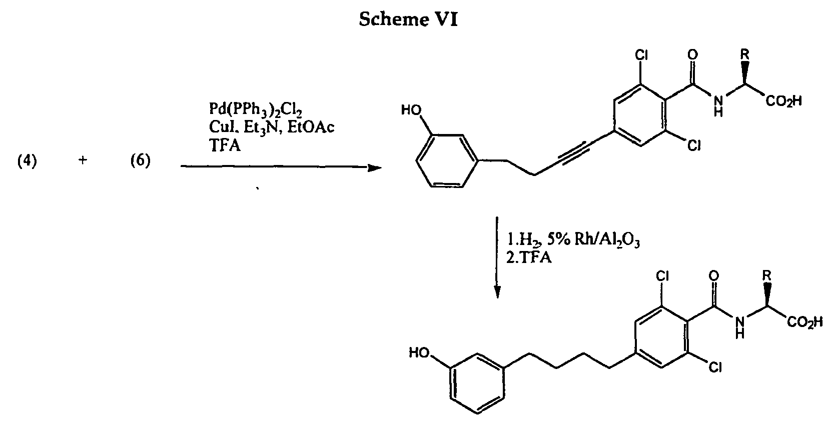 Schemes VIa and VIb illustrate the synthesis of substituted and unsubstituted alkane and alkene linkers of 3-5 carbons long. 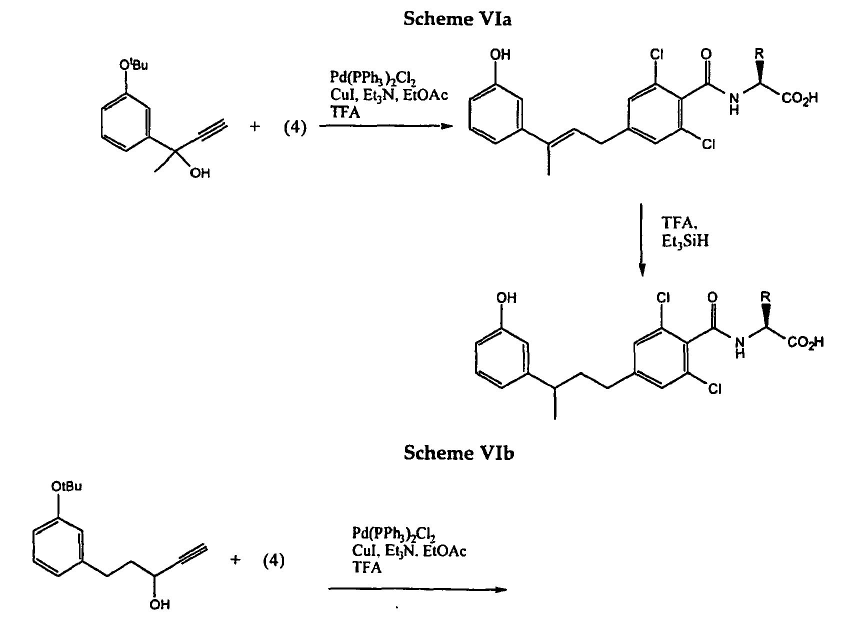 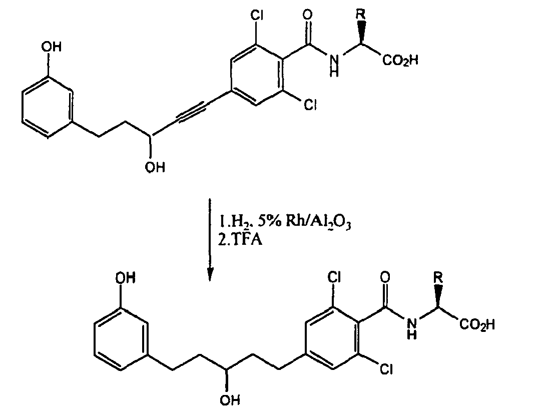 Scheme VII illustrates the synthesis of a 3-carbon alkyl linker where "B" is a dimethyl substituted benzoyl LFA-1 antagonist.

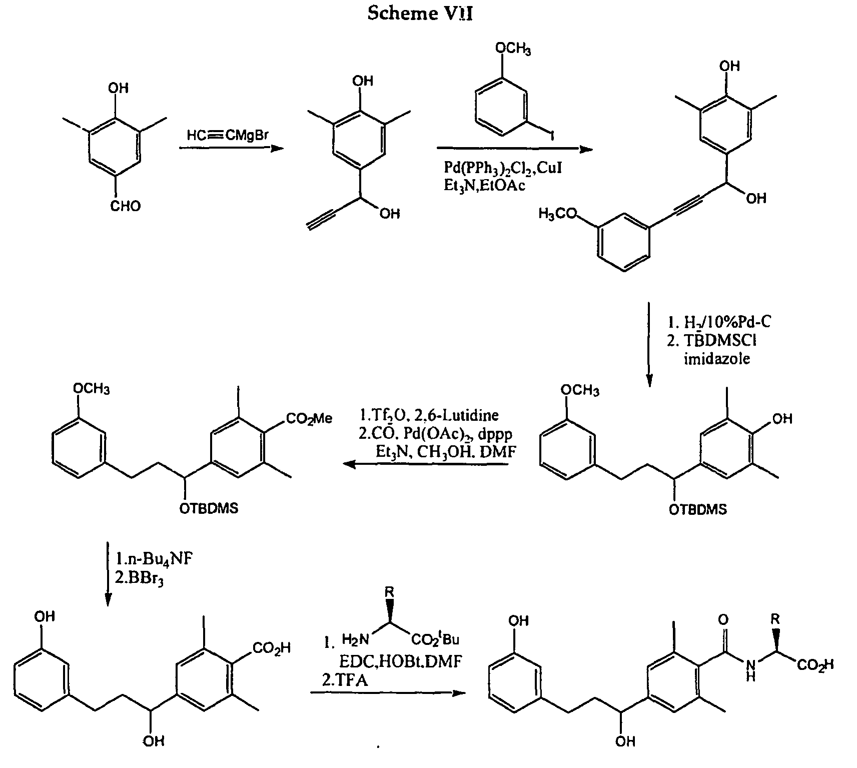

Scheme VIII illustrates the synthesis of a 3-5 atom diether linker where n is 1-3. Intermediate phenol (7) may also be used in the synthesis of monoethers described below.

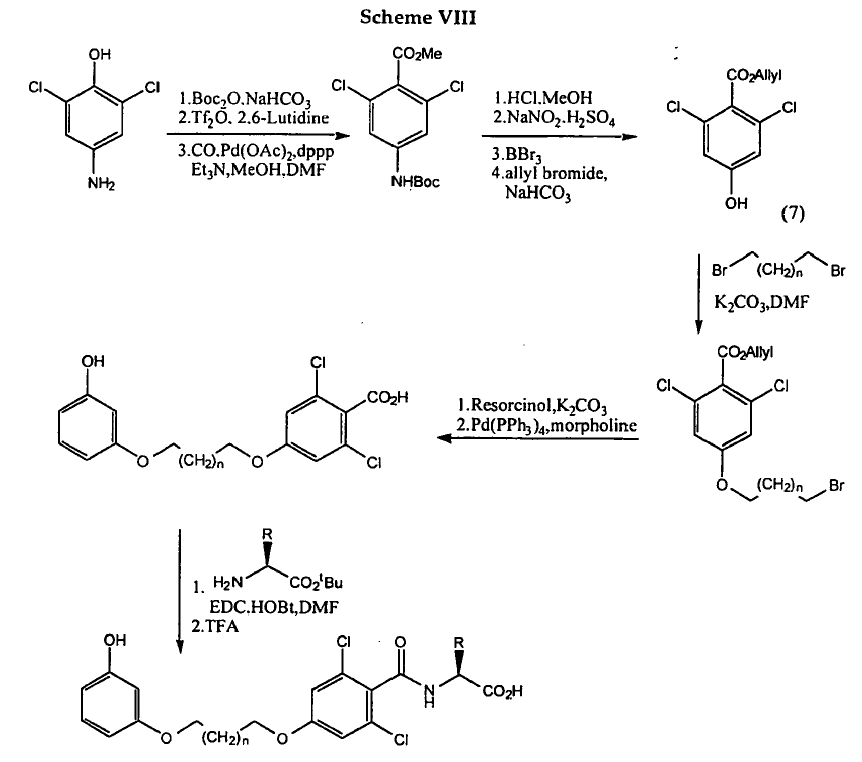

Scheme IX illustrates the synthesis of a 3-5 atom monoether linkers where n is 1-3. Intermediate phenol (7) above is employed in this method.

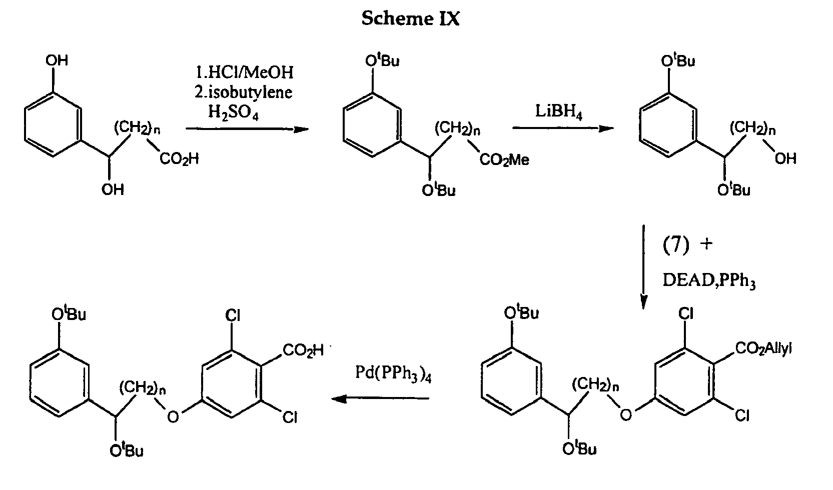

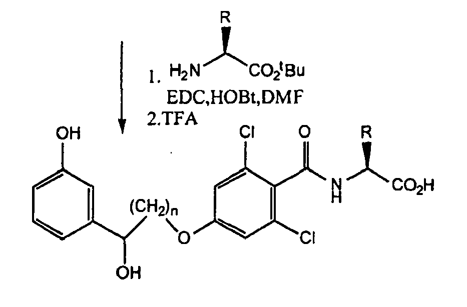

Scheme X illustrates the synthesis of a 5 atom alkyl linkers where the distyl group "D" is a 5-member aromatic ring. Preferred rings include thiophene, furan, thiazole and oxazole, where Z¹ is O or S and Y², Y³ or Y⁴ is selected from N or CH.

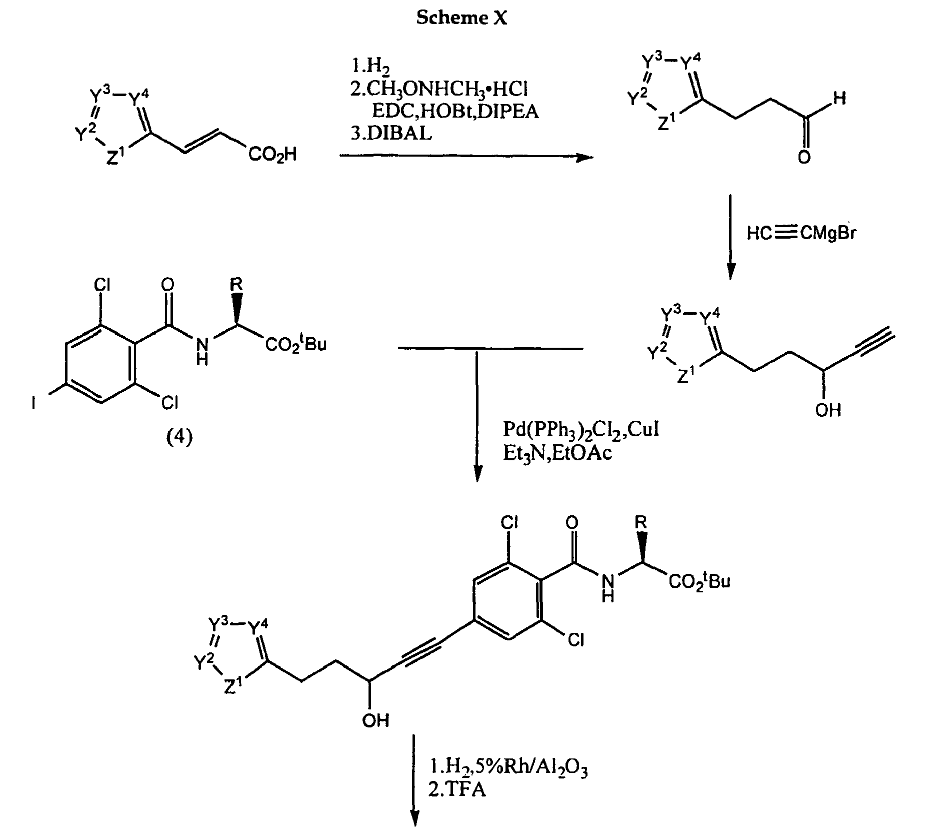

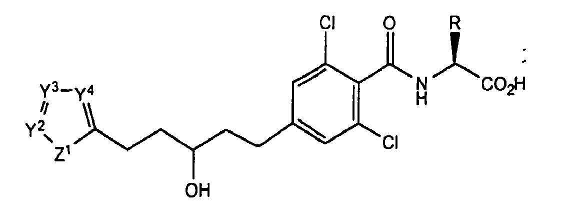

Scheme XI illustrates the synthesis of 3 atom aminoalcohol linkers where the distyl group "D" is phenyl or het.

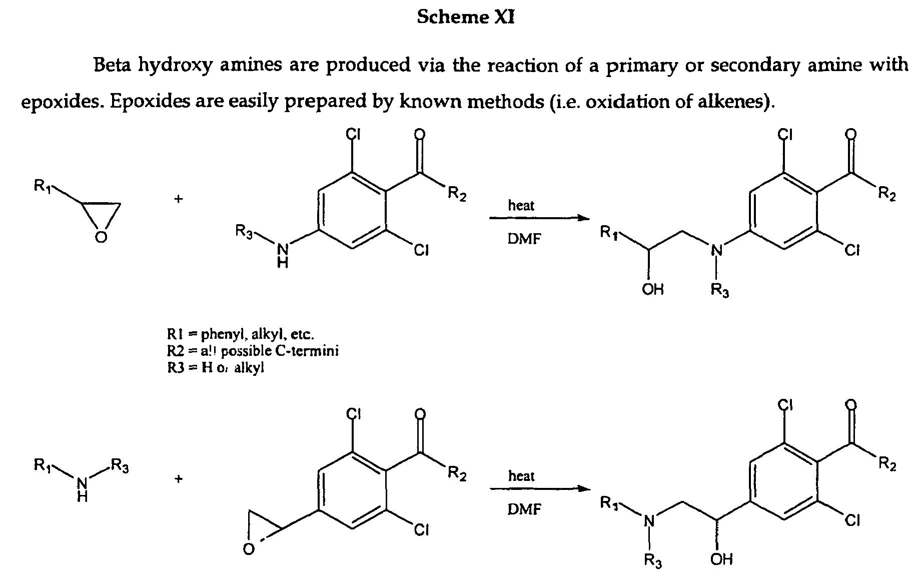

Scheme XII illustrates the synthesis of 3-5 atom oxadiazole linkers where the distyl group "D" is phenyl or het. 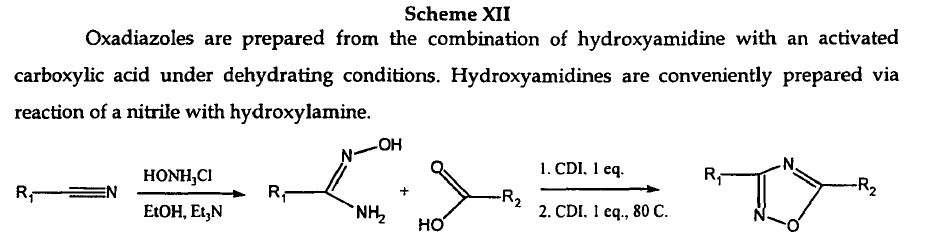 to prepare compounds such as:

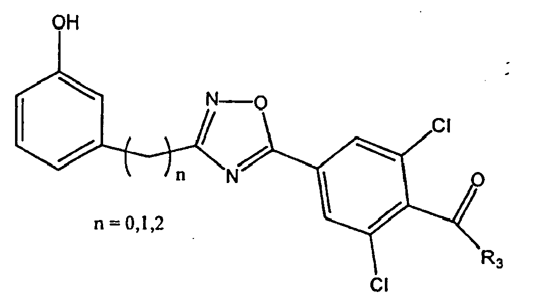

Scheme XIII illustrates the synthesis of 5 atom aminotetrazoles linkers where the distyl group "D" is phenyl or het. 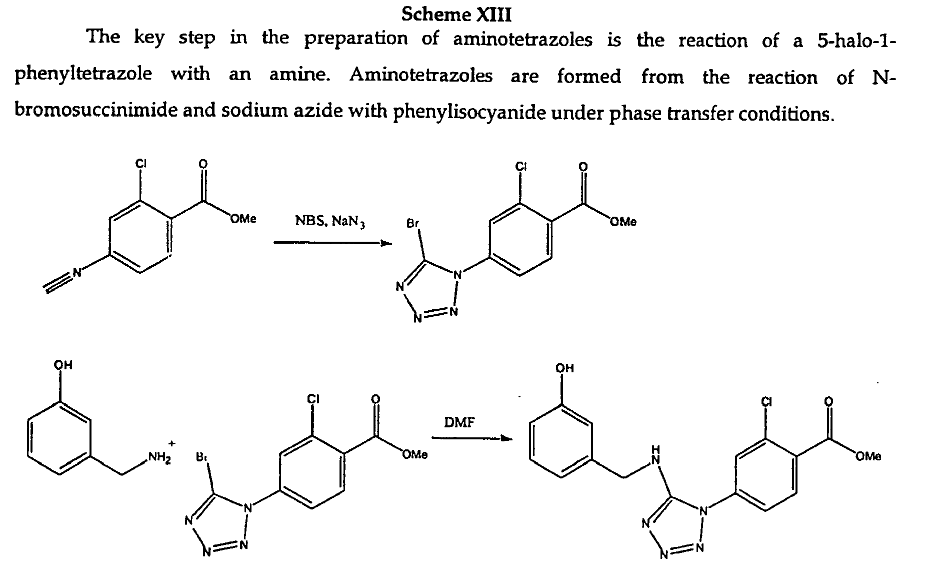 Deprotection and coupling at the carboxylate to add the left side amino acid is carried out as described previously for other compounds.

### E. Modes for Carrying Out the Invention

Superior immunosuppressive efficacy is seen with a treatment regimen that uses early induction with a high dose of LFA-1 antagonist followed by extended treatment with a lower dose of antagonist.

Typically, the LFA-1 antagonist used in the method of this invention is formulated by mixing it at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of antagonist, but preferably ranges anywhere from about 3 to about 8. Formulation in an acetate buffer at pH 5 is a suitable embodiment.

The LFA-1 antagonist for use herein is preferably sterile. LFA-1 antagonist ordinarily will be stored as a solid composition, although lyophilized formulations or aquous solutions are acceptable.

The antagonist composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of LFA-1 antagonist to be administered will be governed by such considerations, and is the minimum amount necessary to prevent, ameliorate, or treat the LFA-1-mediated disorder, including treating rheumatoid arthritis, multiple sclerosis, asthma, psoriasis (topically or systemically), reducing inflammatory responses, inducing tolerance of immunostimulants, preventing an immune response that would result in rejection of a graft by a host or vice-versa, or prolonging survival of a transplanted graft. Such amount is preferably below the amount that is toxic to the host or renders the host significantly more susceptible to infections.

As a general proposition, the initial pharmaceutically effective amount of the LFA-1 antagonist administered parenterally per dose will be in the range of about 0.1 to 20 mg/kg of patient body weight per day, with the typical initial range of LFA-1 antagonist used being 0.3 to 15 mg/kg/day.

The LFA-1 antagonist is administered by any suitable means, including oral, topical, transdermal, parenteral, subcutaneous, intraperitoneal, intrapulmonary, and intranasal, and, if desired for local immunosuppressive treatment, intralesional administration (including perfusing or otherwise contacting the graft with the antagonist before transplantation). Parenteral infusions include intramuscular, intravenous, intraarterial, intraperitoneal, or subcutaneous administration. A preferred administration method for psoriasis is topical in close proximity to the affected area.

The LFA-1 antagonist need not be, but is optionally formulated with one or more agents currently used to prevent or treat the disorder in question. For example, in rheumatoid arthritis, the LFA-1 antagonist may be given in conjunction with a glucocorticosteroid. In addition, T-cell receptor peptide therapy is suitably an adjunct therapy to prevent clinical signs of autoimmune encephalomyelitis (Offner *et al., supra.*). For transplants, the LFA-1 antagonist may be administered concurrently with or separate from an immunosuppressive agent as defined above, e.g., cyclosporin A, to modulate the immunosuppressant effect. The effective amount of such other agents depends on the amount of LFA-1 antagonist present in the formulation, the type of disorder or treatment, and other factors discussed above.

The various autoimmune disorders described above are treated with LFA-1 antagonists in such a fashion as to induce immune tolerance to the self antigen under attack as a result of the disorder. In this regard, autoimmune disorders resemble host versus graft rejection and are treated with LFA-1 antagonists in analogous fashion. However, in these disorders the patient is already mounting an immune response to the target antigen, unlike the case with transplants prior to grafting. Thus, it is desirable to first induce and maintain a transient state of immunosuppression by conventional methods in such patients, e.g. by the conventional use of cyclosporin A or other conventional immunosuppressive agents (alone or together with LFA-1 antagonist), or to monitor the patient until the occurrence of a period of remission (an absence or substantial lessening of pathological or functional indicia of the autoimmune response).

The invention will be more fully understood by reference to the following examples. They should not, however, be construed as limiting the scope of the invention. All literature citations are incorporated by reference.

### Examples

### Example 1

### Preparation and Purification of full-length LFA-1 from 293 Cells

### Construction of LFA-1 cDNA expression vector

A plasmid with both the human CD11a (α_{L}) and CD18 (β₂) sequences, each with a separate CMV promoter for expression in 293 cells, was constructed as follows. The plasmid, pRKCD18, containing the full length CD18 cDNA, was cut with restriction enzymes HpaI and Avr II. The plasmid, pRKCD11a, containing the full length CD11a cDNA, was treated with the enzyme Taq I methylase to methylate one of the two Xmn I sites, then cut with Xmn I and Spe I. The fragment from the pRKCD18 digest containing the CD18 coding sequence, the CMV promoter, the antibiotic resistance gene and other plasmid sequences was ligated to the fragment from the pRKCD11a digest containing the CD11a coding sequence and the CMV promoter. The Spe I and Avr II sticky ends are compatible and were ligated together. The Hpa I and Xmn I ends are both blunt and were ligated together to generate the pRK LFA a+b plasmid.

### Generation of LFA-1 expressing 293 cell line

A cell line expressing human LFA-1 was generated by cotransfecting 293 cells with a plasmid (pRK LFA a+b) containing the full-length cDNAs for the aL (CD11a) and b2 (CD18) subunits together with pRSVneo, which encodes the G418 resistance marker under the control of the RSV promoter, using previously described methods. (Bodary, Napier and McLean, J Biol. Chem, 264, 32, 18859-18862, 1989) Upon growth in the presence of 0.8mg/ml of G418 for 20 days a population of drug resistant cells was selected for LFA-1 expression, using two color FACS (fluoresence activated cell sorting,) with monoclonal antibodies directed against the a L subunit (Fluorescein isothiocyanate labeled monoclonal antibody clone 25.3, catalogue # 0860, AMAC, Inc.) or the b2 subunit-complex (Phycoerythrin labeled MHM23.) (MHM23 antibody reference: Hildreth JEK, and August JT, J Immunol, 134, 3272-3280, 1985) After three rounds of FACS a clonal population was isolated (clone 19) and receptor number was determined to be approximately 106 LFA-1 per cell by Scatchard analysis. This cell line was grown under serum free suspension culture conditions to generate cell pellets for the purification of LFA-1.

### Cell Extraction (All procedures are at 0-4⁻ C.)

The frozen 293 cell pellet was suspended in 5 volumes of 0.3 M sucrose/20 mM HEPES/5 mM CaCl₂/5 mM MgCl₂/2 mg/ml aprotinin pH 7.4 using a Polytron homogenizer (Brinkman) at approximately 8000 rpm. Once a uniform suspension was obtained, the cells were homogenized at approximately 20,000 rpm for 1 min. Phenylmethane sulfonyl fluoride (PMSF, 100 mM in isopropanol) was then added to the homogenate to a final concentration of 1 mM, and the homogenate was centrifuged at 21,000 x g for 40 min. The supernatant was discarded and the pellet suspended in a volume of 1% Triton X-100 (ultrapure)/0.15 M NaCl/20 mM HEPES/5 mM CaCl₂/5 mM MgCl₂/20 mg/ml aprotinin/1 mM PMSF pH 7.4 equal to the volume of sucrose buffer above. The cells were homogenized briefly at about 8000 rpm with the Polytron then placed on a rocker for 30 min. The extract was centrifuged as above and the supernatant saved.

### Lentil Lectin Column

Approximately 3 to 4 column volumes of cell extract were loaded at 15 cm/hr onto a lentil lectin Sepharose column (Pharmacia) equilibrated in 0.1% Triton X-100/0.15 M NaCl/20 mM HEPES/5 mM CaCl_{2/}5 mM MgCl₂ pH 7.4. Once the sample was loaded, the column was washed with equilibration buffer until the A₂₈₀ nm reached baseline. LFA-1 was eluted with 0.5 M a-methyl mannoside in equilibration buffer. To maximize recovery, elution was stopped when the LFA-1 started to appear, the column was left overnight in elution buffer then elution was resumed. **Q Sepharose Column**

The lentil eluate was diluted with an equal volume of 0.1 % Triton X-100/20 mM HEPES/5 mM CaCl_{2/}5 mM MgCl₂ pH 7.4 and loaded at 15 cm/hr onto a Q Sepharose High Performance column (Pharmacia) equilibrated in the same buffer. After the sample was loaded, the column was washed with equilibration buffer until the A_{280 nm} approached baseline, then with 1 % octyl glucoside/20 mM HEPES/5 mM CaCl₂/5 mM MgCl₂ pH 7.4 until the Triton X-100 was removed. The LFA-1 was eluted with a 10 column volume 0 to 0.3 M NaCl gradient in the same buffer. Fractions were analyzed by SDS PAGE and the peak fractions pooled and stored frozen at -70Z C.

### Example 2

### ICAM-1-immunoadhesin

### Plasmid for expression of a human ICAM-1-immunoadhesin

A plasmid for the expression of a human ICAM-1 immunoadhesin was constructed and named pRK.5dICAMGaIg. This plasmid contains; a CMV (cytomegalovirus) promoter and enhancer region, an SP6 promoter for making riboprobes, the five immunoglobulin-like domains of ICAM-1, a six amino acid cleavage site recognized by Genenase (a genetically engineered form of subtilisin), the Fc region from human IgG, an SV40 early polyadenylation site, an SV40 origin of replication, a bacterial origin of replication, and a bacterial gene coding for ampicillin resistance.

This plasmid was constructed using fragments from two other plasmids. The first plasmid, pRKICAMm.2, is a plasmid for the expression of full-length ICAM-1. The following two primers were used to generate a fragment containing the five immunoglobulin-like domains of ICAM-1 by PCR : 1) a 17bp forward primer which is homologous to a portion of the vector sequence 5' of the ICAM-1 coding sequence -5' TGC CTT TCT CTC CAC AG 3' and 2) a 48bp reverse primer which is homologous to 7 amino acids at the 3' end of Ig-like domain 5 and contains sequence coding for a protease cleavage site - 5' GG TGG GCA CAG AGT GTA GTG CGC AGC CTC ATA CCG GGG GGA GAG CAC A 3'. The PCR reaction used 0.2µg of pRKICAMm.2, 1 µl forward primer, at 10 OD/ml, 2µl reverse primer, at 10 OD/ml, 0.2 mM each dATP, dCTP, dGTP, and dTTP, 0.5 mM additional MgCl₂, 1x VENT polymerase buffer (New England Biolabs), and 1 µl VENT polymerase, at 2 units/µl (New England Biolabs). The reaction was denatured at 98° C for 5' then cycled 20 times through the following temperatures: 98° C 1", 98° C 10", 60° C 1", 60° C 1', 72° C 1", 72° C 1'. The reaction was extended for 20' at 72°C before being held at 4° C overnight. This reaction produces a 1579bp fragment which was purified using Qiaquick-spin PCR purification kit (Qiagen) and digested with restriction enzymes ClaI and DraIII (New England Biolabs). The resulting 1515bp fragment was gel purified on a 5% acrylamide gel in 1x TBE, electroeluted in 0.1x TBE, and purified on SpinBind columns (FMC). This insert fragment contains the first 5 immunoglobulin domains of ICAM-1 and the Genenase cleavage site.

The second plasmid, trkcfcgen, is a plasmid for the expression of the TrkC immunoadhesin containing the same portease cleavage site. This plasmid was digested with ClaI (New England Biolabs) completely. This material was then digested with DraIII (New England Biolabs) using sub optimal amounts of the enzyme such that a series of partially cut fragments was generated. The desired 5378bp fragment was isolated on a 0.6% GTG Agarose (FMC) gel run in 1x TBE (BRL) and electroeluted in 0.1X TBE. The material was extracted first with butanol, then phenol, then chloroform and precipitated with 0.1 volume 3M NaAcetate, pH 7.0 and 2.5 volumes of EtOH. This vector fragment contains all of the plasmid features listed above except the first 5 immunoglobulin domains of ICAM-1 and the protease cleavage site.

The two fragments described above were combined in an insert:vector ratio of 3:1 using approximately 50 ng of vector in 1x ligase buffer and 2 µl ligase at 400 units/µl (New England Biolabs) for 2 hrs. at room temperature. Half of the reaction was transformed into MM294 competent cells by standard methods.

### Generation of ICAM-1-immunoadhesin expressing 293 cell line

A cell line expressing the ICAM-1-immunoadhesin was generated by transfecting 293 cells with a cDNA encoding the five immunoglobulin domains of human ICAM-1 upstream from the human Fc sequence (pRK.5dICAMGaIg,) together with pRSVneo, as previously described for the LFA-1 cell line. Upon selection in 0.8mg/ml G418 individual clones of drug resistant cells were isolated. Culture supernatants from these clones were assayed for expression of the human ICAM-1-immunoadhesin by ELISA, using polyclonal antibodies directed against the human Fc (Caltag catalogue # H10507, H10700.) A clonal cell line expressing approximately 1mg/ml of ICAM-1-immunoadhesin, as measured by Fc ELISA, was found to react with a monoclonal antibody (AMAC clone 84H10, catalogue # 0544) directed against human ICAM-1. This cell line was grown under serum free culture conditions and culture supernatant was harvested for purification of the ICAM-1-immunoadhesin.

### Example 3

### ICAM-1:LFA-1 Receptor Binding Assay (protein/protein assay)

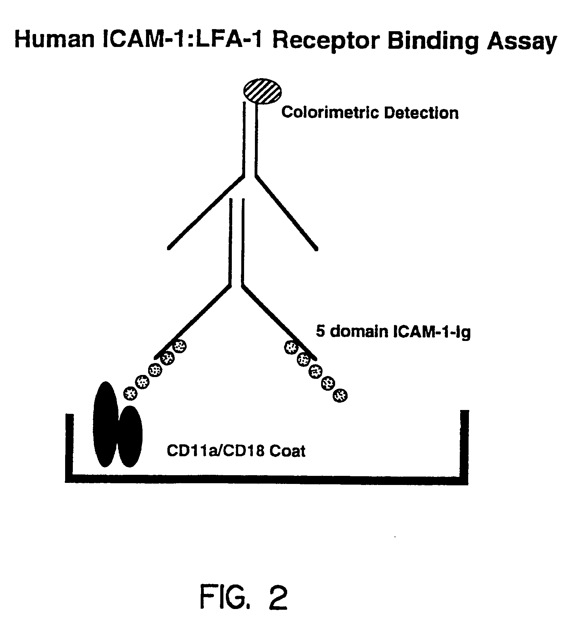

A cartoon illustrating the forward format of the human ICAM-1:LFA-1 Receptor Binding Assay (PPFF) is provided in Figure 2. Competitive inhibition of the CD11a/CD18-ICAM-1 interaction is quantitated by adding known amounts of inhibitors according to the two protein/protein assay systems described below.

### Forward Format LFA-1:ICAM-1 Assay (PPFF):

Purified full-length recombinant human LFA-1 protein is diluted to 2.5µg/ml in 0.02M Hepes, 0.15M NaCl, and 1mM MnCl₂ and 96-well plates (50µl/well) are coated overnight at 4°C. The plates are washed with wash buffer (0.05% Tween 20 in PBS) and blocked for 1h at room temperature with 1% BSA in 0.02M Hepes, 0.15M NaCl, and 1mM MnCl₂. Plates are washed. 50µl/well inhibitors, appropriately diluted in assay buffer (0.5% BSA in 0.02M Hepes, 0.15M NaCl, and 1mM MnCl2), are added to a 2X final concentration and incubated for 1h at room temperature. 50µl/well of purified recombinant human 5 domain ICAM-Ig, diluted to 50ng/ml in assay buffer, is added and incubated 2h at room temperature. Plates are washed and bound ICAM-Ig is detected with Goat anti-HuIgG(Fc)-HRP for 1h at room temperature. Plates are washed and developed with 100µl/well TMB substrate for 10-30' at room temperature. Colorimetric development is stopped with 100µl/well 1M H₃PO₄ and read at 450nM on a platereader.

An alternative protein/protein assay system described below also quantitates competitive inhibition of the CD11a/CD18-ICAM-1 interaction.

### PLM2 Antibody Capture LFA-1:ICAM-1 Assay (PLM2):

A non-function blocking monoclonal antibody against human CD18, PLM-2 (as described by Hildreth, et al., Molecular Immunology, Vol. 26, No. 9, pp. 883-895, 1989), is diluted to 5µg/ml in PBS and 96-well flat-bottomed plates are coated with 100µl/well overnight at 4°C. The plates are blocked with 0.5% BSA in assay buffer (0.02M Hepes, 0.15M NaCl, and 1mM MnCl₂) 1h at room temperature. Plates are washed with 50mM Tris pH 7.5, 0.1M NaCl, 0.05% Tween 20 and 1mM MnCl2. Purified full-length recombinant human LFA-1 protein is diluted to 2µg/ml in assay buffer and 100µl/well is added to plates and incubated 1h at 37°C. Plates are washed 3X. 50µl/well inhibitors, appropriately diluted in assay buffer, are added to a 2X final concentration and incubated for 30' at 37°C. 50µl/well of purified recombinant human 5 domain ICAM-Ig, diluted to 161ng/ml (for a final concentration of 80ng/ml) in assay buffer, is added and incubated 2h at 37°C. Plates are washed and bound ICAM-Ig is detected with Goat anti-HuIgG(Fc)-HRP for 1h at room temperature. Plates are washed and developed with 100µl/well TMB substrate for 5-10' at room temperature. Colorimetric development is stopped with 100µl/well 1M H₃PO₄ and read at 450nM on a platereader.

### Example 4

### Human T-cell Adhesion Assay

### (cell attachment assay)

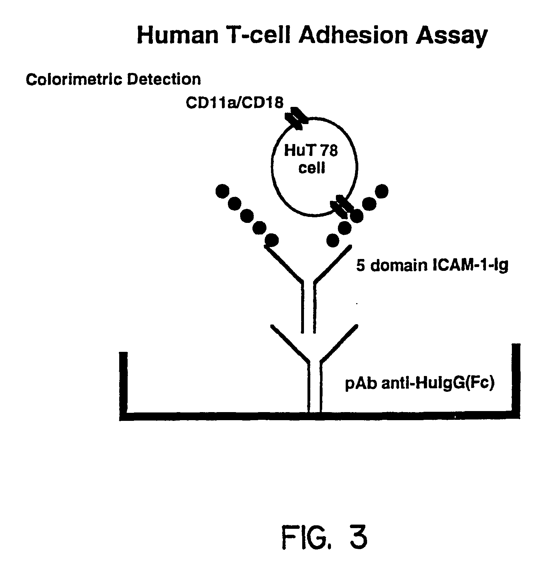

A cartoon illustrating the human T cell adhesion colorimetric assay is provided in Figure 3. The T-cell adhesion assay is performed using a human T-lymphoid cell line HuT 78. Goat anti-HuIgG(Fc) was diluted to 2µg/ml in PBS and 96-well plates were coated with 50µl/well @ 37°C for 1h. Plates were washed with PBS and blocked for 1h @ room temperature with 1% BSA in PBS. 5 domain ICAM-Ig was diluted to 100ng/ml in PBS and 50µl/well was added to the plates O/N @ 4°C. HuT 78 cells were centrifuged at 100 g and the cell pellet was treated with 5mM EDTA for -5' at 37°C in a 5% CO2 incubator. Cells were washed in 0.14M NaCl, 0.02M Hepes, 0.2% Glucose and 0.1mM MnCl2 (assay buffer) and centrifuged. The cells were resuspended in assay buffer to 3.0 x 10⁶c/ml. Inhibitors were diluted in assay buffer to a 2X final concentration and pre-incubated with HuT 78 cells for 30' at room temperature. 100µl/well of cells and inhibitors were added to the plates and incubated at room temperature for 1h. 100µl/well PBS was added and the plates were sealed and centrifuged inverted at 100 g for 5'. Unattached cells were flicked out of the plate and excess PBS was blotted on a paper towel. 60µl/well p-nitrophenyl *n*-acetyl-β-D-glucosaminide (0.257g to 100ml citrate buffer) was added to the plate and incubated for 1.5h at 37°C. The enzyme reaction was stopped with 90µl/well 50mM Glycine/5mM EDTA and read on a platereader at 405nM. HUT 78 cell adhesion to 5dICAM-Ig is measured using the *p*-nitrophenyl *n*-acetyl-β-D-glucosaminide method of Landegren, U. (1984) J. Immunol. Methods 57, 379-388

### Example 5

### T-Cell Proliferation assay (co-stimulation assay)

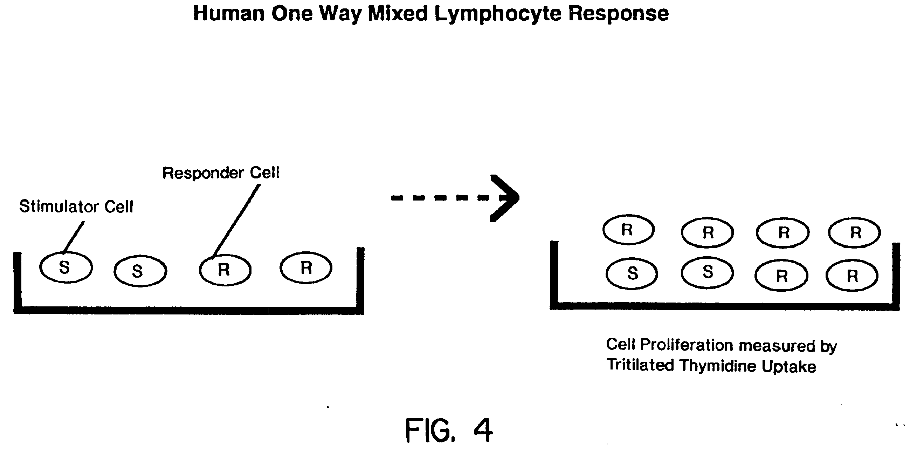

A cartoon illustrating the human T cell proliferation assay is provided in Figure 4. This assay is an in vitro model of lymphocyte proliferation resulting from activation, induced by engagement of the T-cell receptor and LFA-1, upon interaction with antigen presenting cells (Springer, Nature 346:425 (1990)).

Microtiter plates (Nunc 96 well ELISA certified) were precoated overnight at 4°C with 50µl of 2µg/ml of goat anti-human Fc (Caltag H10700) and 50µl of 0.07µg/ml monoclonal antibody to CD3 (Immunotech 0178) in sterile PBS. The next day coat solutions were aspirated. Plates were then washed twice with PBS and 100µl of 17 ng/ml 5d-ICAM-1-IgG were added for 4 hours at 37°C. Plates were washed twice with PBS prior to addition of CD4+ T cells. Lymphocytes from peripheral blood were separated from heparinized whole blood drawn from healthy donors. An alternative method was to obtain whole blood from healthy donors through leukophoresis. Blood was diluted 1:1 with saline, layered, and centrifuged at 2500 x g for 30 minutes on LSM (6.2g Ficoll and 9.4g sodium diztrizoate per 100ml) (Organon Technica, NJ). Monocytes were depleted using a myeloid cell depletion reagent method (Myeloclear, Cedarlane Labs, Hornby, Ontario, Canada). PBLs were resuspended in 90% heat-inactivated Fetal Bovine serum and 10% DMSO, aliquoted, and stored in liquid nitrogen. After thawing, cells were resuspended in RPMI 1640 medium (Gibco, Grand island, NY) supplemented with 10% heat-inactivated Fetal Bovine serum (Intergen, Purchase, NY), 1mM sodium pyruvate, 3mM L-glutamine, 1mM nonessential amino acids, 500µg/ml penicillin, 50µg/ml streptomycin, 50µg/ml gentamycin (Gibco).

Purification of CD4+ T cells were obtained by a negative selection method (Human CD4 Cell Recovery Column Kit #CL110-5 Accurate). 100,000 purified CD4+ T cells (90% purity) per microtiter plate well were cultured for 72 hours at 37°C in 5% CO₂ in 100µl of culture medium (RPMI 1640 (Gibco) supplemented with 10% heat inactivated FBS (Intergen), 0.1mM non-essential amino acids, 1nM Sodium Pyruvate, 100 units/ml Penicillin, 100 µg/ml Streptomycin, 50µg/ml Gentamicin, 10mM Hepes and 2mM Glutamine). Inhibitors were added to the plate at the initiation of culture. Proliferative responses in these cultures were measured by addition of 1µCi/well tritiated thymidine during the last 6 hours before harvesting of cells. Incorporation of radioactive label was measured by liquid scintillation counting (Packard 96 well harvester and counter). Results are expressed in counts per minute (cpm).

### Example 6

### In vitro Mixed Lymphocyte Culture Model

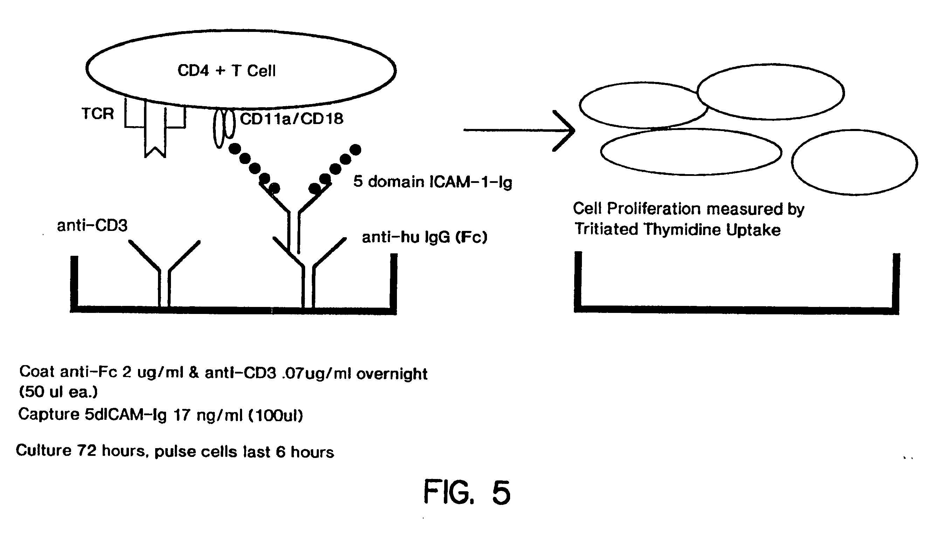

A cartoon depicting the mixed lymphocyte response assay is provided in Figure 5. This mixed lymphocyte culture model, which is an *in vitro* model of transplantation (A.J. Cunningham, "Understanding Immunology, Transplantation Immunology pages 157-159 (1978), examines the effects of various LFA-1 antagonists in both the proliferative and effector arms of the human mixed lymphocyte response.

Isolation cf Cells: Mononuclear cells from peripheral blood (PBMC) were separated from heparinized whole blood drawn from healthy donors. Blood was diluted 1:1 with saline, layered, and centrifuged at 2500 x g for 30 minutes on LSM (6.2g Ficoll and 9.4g sodium diztrizoate per 100ml) (Organon Technica, NJ). An alternative method was to obtain whole blood from healthy donors through leukophoresis. PBMCs were separated as above, resuspended in 90% heat-inactivated Fetal Bovine serum and 10% DMSO, aliquoted, and stored in liquid nitrogen. After thawing, cells were resuspended in RPMI 1640 medium (Gibco, Grand Island, NY) supplemented with 10% heat-inactivated Fetal Bovine serum (Intergen, Purchase, NY), 1mM sodium pyruvate, 3mM L-glutamine, 1mM nonessential amino acids, 500µg/ml penicillin, 50µg/ml streptomycin, 50µg/ml gentamycin (Gibco).

Mixed Lymphocyte Response (MLR): One way human mixed lymphocyte cultures were established in 96-well flat-bottomed microtiter plates. Briefly, 1.5 x 10⁵ responder PBMCs were cocultured with an equal number of allogeneic irradiated (3000 rads for 3 minutes, 52 seconds) stimulator PBMCs in 200µl of complete medium. LFA-1 antagonists were added at the initiation of cultures. Cultures were incubated at 37°C in 5% CO₂ for 6 days, then pulsed with 1µCi/well of ³H-thymidine (6.7 Ci/mmol, NEN, Boston, MA) for 6 hours. Cultures were harvested on a Packard cell harvester (Packard, Canberra, Canada). [³H] TdR incorporation was measured by liquid scintillation counting. Results are expressed as counts per minute (cpm).

### Example 7

### Compound Synthesis and Activity

Abbreviations used in the following section: Wang resin = p- alkoxybenzyl alcohol resin; Fmoc = 9- fluorenylmethyloxycarbonyl; Fmoc-OSu = 9- fluorenylmethyloxycarbonyl- N-hydroxysuccinimide; Boc = t- butyloxycarbonyl; Boc₂O = t- butyloxycarbonyl anhydride; DMA = dimethylacetimide; DMF = dimethylformamide; BOP = (benzotriazol-1-yloxy) tris (dimethyl-amino) phosphonium hexafluorophosphate; Hobt = 1-hydroxybenztriazole; NMM = 4-methylmorpholine; TFA = trifluoroacetic acid; DCM = dichloromethane; MeOH = methanol; HOAc = acetic acid; HCl = hydrochloric acid; H₂SO₄ = sulfuric acid; K₂CO₃ = potassium carbonate; Ph₃P = triphenylphosphine; THF = tetrahydrofuran; EtOAc = ethyl acetate; DIPEA = diisopropylethylamine; NaHCO₃ = sodium bicarbinate; NMP = N-methyl pyrrolidinone; DIPC = diisopropylcarbodiimide; ACN = acetonitrile; HBTU = 2-(1H-benzotriazole-1-yl)-1, 1, 3, 3-tetramethyluronium hexafluorophosphate; NCS = N-chlorosuccinimide; Na₂·EDTA = ethylenediaminetetraacetic acid sodium salt; TBAF = tetrabutyl ammonium fluoride; EDC = 1-(3-dimethylaminopropyl)-3-ethylcarbodiimide·HCl; DEAD = diethyl azocarboxylate; TEA = triethylamine; MgSO₄ = magnesium sulfate; TES = triethylsilane; Et₂O = diethyl ether; BBr₃ = boron tribromide

### General synthetic methods

### Method G1

The appropriate Boc protected molecule was dissolved in a solution of TFA in DCM (1:1). After 20 minutes, the reaction was concentrated *in vacuo.* The resulting oil was dissolved in toluene and then concentrated *in vacuo* twice.

### Method G2

The appropriate amine was dissolved in Et₂O and washed twice with a 10% solution of K₂CO₃ in H₂O and once with brine. The organic layer was then dried over MgSO₄, filtered and concentrated *in vacuo.* The product was then used with out further purification.

### Method G3

3 equivalents of the appropriate carboxylic acid was coupled to 1 equivalent of the appropriate amine using 3 equivalents EDC and 1 equivalent of Hobt in DMA. The reaction was monitored by TLC (9/1 DCM/MeOH). Upon completion, the mixture was concentrated *in vacuo.* The resulting oil was re suspended in Et₂O and washed twice with 0.1 N H₂SO₄, twice with saturated NaHCO₃, and once with brine. The organic layer was then dried over MgSO₄, filtered and concentrated *in vacuo.* The product was then used with out further purification.

### Method G4

1 equivalent of the appropriate methyl ester was dissolved in THF/H₂O (3/1) and 3 equivalents of LiOH·H₂O was added. The reaction was monitored by TLC (9/1 DCM/MeOH). Upon completion, the mixture was acidified carefully to pH 2 with concentrated HCl and then concentrated *in vacuo.* The resulting solid was re suspended in Et₂O and washed twice with 0.1 N H₂SO₄ and once with brine. The organic layer was then dried over MgSO₄, filtered and concentrated *in vacuo.*

### Method G5

1 equivalent of the appropriate amino acid and 2.5 equivalents of NaHCO₃ were dissolved in THF/H₂O (3/1). Once the solution becomes clear, 1.5 equivalents of Fmoc-OSu was added. The reaction was monitored by TLC (9/1 DCM/MeOH). Upon completion, the mixture was concentrated *in vacuo* until only the aqueous phase remained. The aqueous solution was then extracted twice with Et₂O and then acidified carefully to pH 2 with concentrated HCl to precipitate out the product. The aqueous layer and product was then extracted with EtOAc. The organic layer was then partitioned once with brine and dried over MgSO₄ filtered and concentrated *in vacuo.* The resulting product was used without further purification.

### Method G6

1 equivalent of fluorenylmethanol and 2.5 equivalents of Hobt was dissolved in NMP. The mixture was cooled to 0°C with stirring. Once cool, 1 equivalent of DIPC was added over 5 minutes with stirring followed by portion wise additions of 1 equivalent of 2- bromoterephthalic acid and then 0.01 equivalents of 4- pyrrolidinopyridine. The mixture was stirred at 0°C for 2 hours, warmed to room temperature and stirred for 4 hours, and then recooled to 0°C and quenched with the drop wise addition of H₂O. After stirring for 1 hour, the mixture was partitioned with EtOAc. The organic layer was then partitioned twice with dilute HCl, once with brine and dried over MgSO₄, filtered and concentrated *in vacuo.* The crude product (a 9:1 mixture of correct versus incorrect isomer) was purified using flash silica chromatography using 3/1 hexanes/EtOAc and 3% HOAc.

### Method G7

The appropriate methoxy containing compound was dissolved in DCM and cooled to -5°C in an ice/acetone bath under nitrogen. 2 equivalents of BBr₃ was added drop wise as a solution in DCM over 30 minutes. The reaction was warmed to room temperature and stirred until complete by TLC (DCM/2% HOAc/2% MeOH). The solution was poured onto ice, and the ice was allowed to melt. The mixture was then partitioned twice with EtOAc and the combined organic layers were dried over MgSO₄. The filtrate was then passed over a plug of silica gel and concentrated *in vacuo.*

### Method G8

1 equivalent of dimethyl 2- chloroterephthalic acid was mono hydrolyzed by Method G9 to afford the correct mono protected diacid. The mono ester was then t- butyl esterified by Method G10. The methyl ester was then removed by Method G4 to yield the carboxylic acid (Compound A).

### Method G9

The diester was dissolved in DCM and cooled to -5°C in an ice/acetone bath under nitrogen. 1 equivalent of BBr₃ was added drop wise as a solution in DCM over 30 minutes. The reaction was warmed to room temperature and stirred until complete by TLC (DCM/2% HOAc/2% MeOH). The solution was poured onto ice, and the ice was allowed to melt. The mixture was then partitioned with EtOAc and concentrated *in vacuo.* This product was dissolved in H₂O with the addition of saturated NaHCO₃ until the pH remained above 8. This solution was partitioned one time with and equal volume of DCM to remove unreacted diester. The basic solution was acidified at O°C. with concentrated HCl to pH = 1-1.5, and precipitate was extracted twice with equal volumes of EtOAc. The oraganics were partitioned once with brine and dried over MgSO₄, filtered and concentrated *in vacuo.* Product was 7:1 of the correct regioisomer by HPLC.

### Method G10

The monoester was dissolved in DCM was transferred to pre-weighed Parr flask containing a stirring bar. The flask was cooled to -5°C with a dry ice/alcohol bath under nitrogen. Once cool, ~30 equivalents of isobutylene was pumped into solution with stirring. 2.1 equivalents of concentrated sulfuric acid was added and the flask was sealed with a wired rubber stopper and allowed to warm to room temperature with stirring. The solution was stirred until clarification (1-2 days). Once the solution was clear, it was cooled to 0°C in an ice bath. The stopper was removed and the excess isobutylene was blown off with nitrogen bubbling. Saturated NaHCO₃ was added to neutralize the acid and the mixture was concentrated *in vacuo* until no DCM remained. The solution was then partitioned into EtOAc. The oraganics were partitioned twice with dilute HCl, twice with saturated NaHCO₃, once with brine, dried over MgSO₄, filtered and concentrated in *vacuo.* The resulting product was used with no further purification.

### Method G11

The t- butyl ester product was dissolved in DCM and an equal volume of TFA was added. After 30 minutes the reaction was concentrated in *vacuo* and twice redissolved and concentrated from toluene. The product was used without further purification.

### Method G12

Compound A was coupled to 3- chloro benzylamine by Method G3. The t- butyl ester was removed by Method G11 to yield the carboxylic acid (Compound B).

### Method G13

Compound A was coupled to 3- methoxy benzylamine, Method G38, by Method G3. This product was converted to the methyl ester by Method G15. The methoxy group was demethylated to the phenol by Method G7. The methyl ester was saponified to the carboxylic acid by Method G4 and the final product (Compound C) was used without further purification.

### Method G14

1 equivalent of 4- bromo 2- chloro benzoic acid was converted to the methyl ester by Method G15 and the bromine was converted to the nitrile by Method G16. After saponification by Method G4, the nitrile was reduced to the amine and Fmoc protected by Method G17. The final product (Compound D) was purified by flash silica chromatography (95/5 DCM/MeOH) and verified by electrospray mass spectrometry.

### Method G15

The appropriate carboxylic acid was dissolved in dry MeOH and 10 equivalents of HCl/dioxane was added and the mixture was stirred overnight to yield the methyl ester product. The solution was concentrated *in vacuo* and twice redissolved and concentrated from toluene. The final product was purified by flash silica chromatography (95/5 DCM/MeOH) and verified by electrospray mass spectrometry.

### Method G16

0.6 equivalents of Zinc cyanide and 0.04 equivalents of tetrakis(triphenylphosphine) palladium(0) were placed in a round bottom flask an purged for 30 minutes with circulating nitrogen. The methyl ester was dissolved in anhydrous DMF and degassed for 30 minutes with nitrogen. Upon completion of degassing, the methyl ester solution was added to the zinc cyanide and palladium via cannula and stirred over night at 80°C. Upon completion of the reaction, the solution was concentrated *in vacuo* and redissolved in EtOAc. The oraganics were partitioned twice with dilute HCl, twice with saturated NaHCO₃, once with brine, dried over MgSO₄, filtered and concentrated *in vacuo.* The product was purified by flash silica chromatography (DCM) and verified by electrospray mass spectrometry.

### Method G17

1 equivalent of the nitrile was dissolved in THF and cooled to 0°C in an ice bath. Once cool, 4 equivalents of super hydride was added quickly via cannula to the nitrile. After 5 minutes, the reaction was poured onto ice containing 5 equivalents of sulfuric acid and stirred until all of the ice melts. Two volumes of THF was added to the solution and the pH was carefully adjusted to 8 with portion wise additions of NaHCO₃. 1.5 equivalents of Fmoc-OSu was added The reaction was monitored by TLC (9/1 DCM/MeOH). Upon reaction completion, the mixture was concentrated in *vacuo* until only the aqueous phase remained. The aqueous solution was then extracted twice with Et₂O and then acidified carefully to pH 2 with concentrated HCl to precipitate out the product. The aqueous layer and product was then extracted with EtOAc. The organic layer was then partitioned once with brine and dried over MgSO₄, filtered and concentrated *in vacuo.*

### Method G18

1 equivalent of the appropriate hydroxy carboxylic acid, 2.2 equivalents oft-butyldimethyl silyl chloride and 3 equivalents of imidizole were dissolved in DMF and stirred at room temperature. The reaction was monitored by TLC (9/1 DCM/MeOH). Upon reaction completion, the mixture was concentrated *in vacuo.* The resulting oil was re suspended in Et₂O and washed twice with saturated NaHCO₃, and once with brine. The organic layer was then dried over MgSO₄, filtered and concentrated *in vacuo.* The product was then used with out further purification.

### Method G19

To resin that has been rinsed twice with DMA, a solution consisting of 20% piperidine in DMA was added. After 20 minutes, the resin was filtered and rinsed 5 times with DMA.

### Method G20

3 equivalents of the appropriate carboxylic acid was coupled with 3 equivalents of BOP, 1 equivalent of HOBt, , and 6 equivalents of NMM in DMA for 30 minutes. The coupling was monitored by the Kaiser ninhydrin test. If the Kaiser test was positive, the appropriate carboxylic acid was coupled again in the same manner.

### Method G21

The molecule was cleaved from the rinsed and dried resin in a solution consisting of 5% triisopropylsilane in TFA for 1 hour. The crude molecule was then concentrated in *vacuo,* purified by reverse phase HPLC, verified by electrospray mass spectrometry and lyophilized to a powder.

### Method G22

3 equivalents of the appropriate amine was coupled with 3 equivalents of BOP,1 1 equivalent of HOBt, , and 6 equivalents of NMM in DMA for 60 minutes.

### Method G23

The resin was washed successively with DMA, DCM, 20% HOAc in DCM, MeOH and DMF. 2 equivalents of the appropriate aldehyde was dissolved in a minimal volume of 1% HOAc in DMF and added to the freshly rinsed resin. After 5 minutes, 2 equivalents of sodium cyanoborohydride in DMF was added, and the resin was bubbled overnight. The resin was then washed with DMF, 20% DIPEA in DCM, DCM and MeOH. The coupling was monitored by the Kaiser ninhydrin test. If the Kaiser test was positive, the appropriate aldehyde was coupled again in the same manner.

### Method G24

3 equivalents of the appropriate carboxylic acid (R) was coupled with 3 equivalents of HBTU, and 3 equivalents of DIPEA, in DMA. The reaction was followed by TLC. Upon completion, the mixture was diluted with EtOAc. The organic layer was partitioned with dilute sulfuric acid, saturated NaHCO₃, dried over MgSO₄, filtered and concentrated *in vacuo.* The resulting methyl ester product was then used with out further purification.

### Method G25

The methyl ester of the appropriate carboxylic acid was made by Method G15 and the phenol was converted to the t- butyl ester by Method G10. 1 equivalent of the resulting product was dissolved in a 1:2 mixture of THF and EtOH, and 3 equivalents of lithium chloride and 3 equivalents of sodium borohydride was added and the reaction was stirred overnight. The reaction was quenched with H₂O and concentrated *in vacuo.* The residue was partitioned between EtOAc and H₂O, and the aqueous layer was extracted with EtOAc. The combined organic layers were dried over MgSO₄, filtered and concentrated *in vacuo.* The crude alcohol was purified using silica gel flash chromatography (9:1 hexane/Et₂O).

### Method G26

A solution of 1 equivalent of the alcohol and 1.1 equivalents of Ph₃P in THF was cooled to - 10°C in an ice-ethanol bath. While stirring, a solution of 1.1 equivalents of the phenol and 1.1 equivalents of DEAD in THF was added drop wise. The cold bath was removed and the reaction was stirred at room temperature overnight. The reaction was concentrated in *vacuo* and the resulting residue was taken up in a minimal amount of DCM and filtered through a plug of silica gel, using DCM as eluent. After concentrating this solution in *vacuo,* the residue was purified using silica gel flash chromatography (8/2/0.5 hexane/DCM/Et₂O) to provide the pure ether.

### Method G27

1 equivalent of the alcohol was dissolved in acetone and cooled to -10°C. 1.1 equivalents of Jones reagent was added and the reaction was stirred at room temperature for 2 hours. The reaction was filtered through a plug of silica gel and concentrated *in vacuo.* The residue was partitioned between EtOAc and H₂O. The residue was partitioned between EtOAc and H₂O, and the aqueous layer was extracted with EtOAc. The combined organic layers were dried over MgSO₄, filtered and concentrated *in vacuo.* The yellow solid was triturated with Et₂O to remove impurities, providing pure ketone.

### Method G28

1 equivalent of the appropriate dihydroxynaphthalene was dissolved in pyridine. 4 equivalents of solid sodium hydride was added followed by 2 equivalents of the bromide and 0.4 equivalents of cuprous chloride. The resulting mixture was stirred vigorously and heated in an oil bath at 100°C for two days. After concentrating *in vacuo.,* the residue was partitioned between EtOAc and 1M HCI. The aqueous layer was extracted with EtOAc. The combined organic layers were dried over MgSO₄, filtered and concentrated *in vacuo.* The residue was triturated with EtzO. After filtering the mixture and concentrating the filtrate, the resulting residue was purified using silica gel flash chromatography (5:4:1 hexane/DCM/Et₂O).

### Method G29

To a stirred -78°C solution of 1 equivalent of the appropriate methyl ester in dry toluene was added a solution of 1.5 M DIBAL in toluene (1.7 equivalents) drop wise. The reaction mixture was stirred for an additional 2 hours at -78°C or until TLC showed clean formation of product, with only a trace of starting material. The reaction was quenched by slowly adding cold (-78°C) MeOH. The resulting white emulsion was slowly poured into ice-cold 1 N HCl and EtOAc and the aqueous layer was extracted with EtOAc. The combined organic layers were dried over MgSO₄ filtered and concentrated *in vacuo.* The residue was purified using silica gel flash chromatography (9:1 hexane/Et₂O) to provide the pure aldehyde.

### Method G30

1 equivalent of the amido alcohol made by Method G28 and 1.5 equivalents of Ph₃P were dissolved in THF and cooled to -5°C. 1.5 equivalents of DEAD was added drop wise and the reaction was stirred at room temperature overnight. After concentrating the reaction in *vacuo,* the residue was taken up in a minimal amount of DCM and purified by flash chromatography (9:1 hexane/Et₂O) to provide pure oxazoline.

### Method G31

To a stirred -78°C solution of 1 equivalent of the bromide in THF was added 1.6 M n-BuLi (1.05 equivalents) drop wise. After 0.5 hour, 1.1 equivalents of the aldehyde in THF was added via cannula at -78°C and the reaction was stirred at -78°C. After 2 hours, the reaction was quenched with 2 equivalents cold (-78°C) HOAc in THF. The mixture was warmed to room temperature, concentrated *in vacuo,* and the oily residue partitioned between Et₂O and H₂O. The aqueous layer was extracted with Et₂O. The combined organic layers were dried over MgSO₄, filtered and concentrated *in vacuo.* The residue was purified using silica gel flash chromatography (7:3 hexane/Et₂O).

### Method G32

The oxazoline alcohol was dissolved in a 13:1 mixture of ethanol and sulfuric acid, then heated at reflux for 3 days. The reaction was concentrated *in vacuo,* and the residue was partitioned between Et₂O and H₂O The aqueous layer was extracted with Et₂O. The combined organic layers were dried over MgSO₄, filtered and concentrated *in vacuo.* The residue was purified using silica gel flash chromatography(1:1 hexane/Et₂O) to give the pure ethyl ester.

### Method G33

To freshly rinsed resin was added, 2.2 equivalents of DIPEA and 2.2 equivalents of the appropriate isocyanate (R) in 1, 2- dichloroethane were added and the resin agitated overnight. The resin was then washed with 10% piperidine in NMP, THF, 30% HOAc in DCM and MeOH.

### Method G34

1 equivalent of 4- benzyloxy benzyl alcohol resin (Wang resin) was washed with DMA and DCM. To the resin was added 3 equivalents of the appropriate Fmoc protected amino acid, 3 equivalents of DIPC and 0.5 equivalents of DMAP in DCM. The resin was agitated for 2 hours, rinsed with DCM and DMA. The resin was then treated with 10% acetic anhydride in DCM for 5 minutes. The resin was washed with DCM and MeOH and then dried *in vacuo.*

### Method G35

The resin was washed with DCM and chloroform. A fresh 0.14M solution of tetrakis (triphenylphosphine) palladium(0) in 2.5% NMM, 5% HOAc in chloroform was added to the resin. After agitating for 1 hour, the resin was checked by the Kaiser ninhydrin test. If the Kaiser test was negative, a new solution of Pd(0) was made and the reaction done again until a positive Kaiser test results. The resin was rinsed with DCM, MeOH and DCM.

### Method G36

The deprotected resin was treated for 1 hour with a solution of 10 equivalents of benzophenone imine and 1.3 equivalents of HOAc in DMA to form the glycine benzophenone imine. After rinsing with DMA the resin was treated with 3.5 equivalents of 2-*t*-butylimino-2-diethylamino-1, 3- dimethylperhydro- 1, 2, 3- diazaphosphorine for 1 hour. 3 equivalents of the appropriate alkylating agent was added and the reaction agitated for 2 hours. The resin was drained and washed with NMP, 20% DIPEA in DCM, DCM, 10% HOAc in DCM and DCM. The benzophenone was removed with a solution of 10 equivalents of hydroxylamine • HCl in THF/H₂O for 3 hours. The resin was the rinsed with H₂O, THF 20% DIPEA in DCM and DCM.

### Method G37

10 equivalents of 2-bromoterephthalic acid, 20 equivalents of HBTU, 20 equivalents of Hobt and 22 equivalents of DIPEA were dissolved in DMA and stirred for 15 minutes yielding the bisactivated 2-bromoterephthalic acid ester. To this solution was added 15 equivalents of 3- hydroxy benzylamine, Method G38, and 15 equivalents of DIPEA yielding the active ester of Compound E. The reaction was stirred for 30 minutes and then it was added to the resin which was then agitated over night.

### Method G38

1 equivalent of 3-cyanophenol was placed in a Parr bottle with EtOH, 0.02 equivalents of HCl and 10% (w/w) of 10% Pd on carbon. The vessel was placed in the Parr shaker, charged with 50 psi H2, and shaken for 12 hours. The reaction filtered through a pad of celite and diluted 1:10 with Et₂O. Upon standing over night, fine white needles form. The product was filtered, washed with Et₂O and dried *in vacuo.* The resulting hydrochloride salt was then used with out further purification.

### Method G39

The resin was washed with DCM and chloroform. A fresh 0.14M solution of tetrakis (triphenylphosphine) palladium(0) in 2.5% NMM, 5% HOAc in chloroform was added to the resin. After agitating for 2 hours, the resin was drained and rinsed with DCM and DMA. The resin was then treated with 10% DIPEA in DMA for 10 minutes, followed by several DMA washes and then with a 5% solution of diethyldithiocarbamic acid in DMA for 15 minutes. The resin was then rinsed with DMA, DCM, MeOH and DCM.

### Method G40

Resin was suspended in ACN and cooled to 0°C. Once cool, 3 equivalents of Ph₃P and 3 equivalents of NCS was added and the resin was agitated for 5 minutes. 6 equivalents of the appropriate aniline was added to the resin and the resin was agitated as it was warmed to room temperature. After an additional 10 minutes at room temperature, the reaction was quenched with 3 equivalents of HOAc and the resin washed with 10% HOAc in ACN, DCM and MeOH.

### Method G41

The resin was preactivated with 3 equivalents of HBTU, 3 equivalents of Hobt and 6 equivalents of DIPEA in DMA for 10 minutes. 2 equivalents of the appropriate amine was added, and the resin agitated for 30 minutes. The procedure was repeated again. The resin was rinsed with DMA and DCM.

### Method G42

The resin was rinsed with DMA, DCM and dichloroethane. 11 equivalents of the appropriate sulfonyl chloride and 3 equivalents of DIPEA were added in dichloroethane and the resin was agitated for 12 hours. The reaction can be followed by the Kiaser ninhydrin test and the procedure repeated until a negative Kiaser test results. The resin was washed with dichloroethane, and DCM.

### Method G43

The resin was rinsed with DMA, DCM and dichloroethane. 1.1 equivalents of the appropriate chloroformate and 3 equivalents of DIPEA were added in dichloroethane and the resin was agitated for 12 hours. The reaction can be followed by the Kiaser ninhydrin test and the procedure repeated until a negative Kiaser test results. The resin was washed with dichloroethane, and DCM.

### Method G44

1 equivalent of the appropriate amine was dissolved in a 3:2 THF/H₂O solution. 1.1 equivalents of solid NaHCO₃ and 1.1 equivalents of Boc₂O were added and the solution was stirred overnight. The reaction was concentrated, and the residue was partitioned between H₂O and Et₂O. The aqueous layer was extracted with Et₂O and the combined organic layers were dried over MgSO₄ and concentrated in *vacuo* to a solid. Recrystallization out of Et₂O/hexane provided pure product

### Method G45

1 equivalent of the appropriate phenol was dissolved in DCM containing 2.6 equivalents of 2, 6-lutidine and the mixture was cooled to -78°C. After adding 1.25 equivalents of triflic anhydride the stirring reaction was allowed to warm to room temperature overnight. The reaction was then concentrated, and the residue was partitioned between Et₂O and H₂O. The aqueous layer was extracted with Et₂O and the combined organic layers were dried over MgSO₄ and concentrated *in vacuo.* The residue was purified by silica gel flash chromatography (9:1 hexane/Et₂O) to provide the pure triflate.

### Method G46

To a stirring solution of 1 equivalent of the triflate in a 2/1 mixture of DMF/MeOH was added 0.15 equivalents of 1, 3-bis(diphenylphosphino)-propane and 2.5 equivalents of TEA. Carbon monoxide gas was bubbled through this solution for 15 minutes, then 0.15 equivalents of Pd(OAc)2 was added and the reaction was stirred at 70°C for 5-7 hours under an atmosphere of CO (using a balloon filled with CO). The reaction was then concentrated *in vacuo,* and the residue was partitioned between Et₂O and H₂O. The aqueous layer was extracted twice with Et₂O and the combined organic layers were dried over MgSO₄, filtered through a plug of silica gel and concentrated *in vacuo.* The residue was purified by silica gel flash chromatography (9:1:0.02 hexane/DCM/Et₂O) to provide the pure methyl ester

### Method G47

1 equivalent of the appropriate Boc-aniline was dissolved in methanol and the solution saturated with HCl. The reaction was heated at 50°C for 3h, then concentrated *in vacuo.* The pale yellow solid was heated in 35% H₂SO₄ until complete dissolution occurred. Upon cooling the mixture by the addition of ice H₂O the amine bisulfate precipitated. The reaction flask was cooled in an ice bath and the mixture stirred vigorously while 1.1 equivalent of sodium nitrite in H₂O was added drop wise. The reaction was stirred at 0°C for another 1.5 hours After diluting the reaction with H₂O, the reaction was heated at 80°C for 10 hours. The reaction was cooled to room temperature and extracted with EtOAc. The combined organic layers were dried over MgSO₄ and concentrated *in vacuo.* The residue was purified by silica gel flash chromatography (14:6:1 hexane/DCM/Et₂O) to provide the pure phenol.

### Method G48

1 equivalent of the appropriate methyl benzoate was dissolved in DCM and 1.5 equivalents of a 1.0M solution of BBr₃ was added. After stirring the reaction overnight, the reaction was quenched with ice and stirred for an additional 1.5 hours. The reaction was extracted three times with Et₂O and the combined organic layers were dried over.MgSO₄ and concentrated in *vacuo.* The residue was taken up in a minimal amount of saturated NaHCO₃. The product was precipitated from this aqueous solution by the addition of concentrated HCl and then extracted into Et₂O. The combined organic layers were dried over MgSO₄ and concentrated in *vacuo* to provide pure benzoic acid.

### Method G49

1 equivalent of the appropriate carboxylic acid was dissolved in DMF. 1.1 equivalents of solid NaHCO₃ and 5 equivalents of allyl bromide were added and the resulting mixture was stirred at 45°C overnight. The reaction was then concentrated, and the residue was partitioned between Et₂O and H₂O. The aqueous layer was extracted three times with Et₂O and the combined organic layers were dried over MgSO₄ and concentrated *in vacuo .* The residue was purified by silica gel flash chromatography (7:3 hexane/Et₂O) to provide the pure allyl ester.

### Method G50

To a solution of 1 equivalent of the appropriate allyl ester in THF was added 0.1 equivalents of tetrakis (triphenylphosphine) palladium(0) and 10 equivalents of morpholine. The reaction was stirred for 1.5 hours, then concentrated *in vacuo.* The residue was taken up in DCM, extracted three times with 1N HCl, dried over MgSO₄ and concentrated *in vacuo*. The residue was triturated with 1:1 hexane/Et₂O, filtered through a plug of glass wool and concentrated *in vacuo* to provide the pure benzoic acid.

### Method G51

1 equivalent of the phenol was dissolved in DMF and 2.05 equivalents of K₂CO₃ and 4 equivalents of 1, 3-dibromopropane were added. The reaction was stirred overnight while heating the reaction flask in an oil bath maintained at 50°C. After concentrating the mixture *in vacuo.,* the residue was partitioned between Et₂O and H₂O. The aqueous layer was extracted three times with Et₂O and the combined organic layers were dried over MgSO₄ and concentrated *in vacuo.* The residue was purified by silica gel flash chromatography (95:5 hexane/Et₂O) to provide the pure bromide.

### Method G52

1 equivalent of the appropriate hydroxy phenol and 1 equivalent of K₂CO₃ were added to a solution of 0.5 equivalents of the bromide in DMF. After stirring overnight, the reaction was concentrated *in vacuo.* The residue was partitioned between Et₂O and H₂O. The aqueous layer was extracted three times with Et₂O and the combined organic layers were dried over MgSO₄ and concentrated *in vacuo.* The residue was purified by silica gel flash chromatography (18:1 DCM/Et₂O) to provide the pure phenol.

### Method G53

To a nitrogen purged glass pressure tube was added 1 equivalent of the appropriate bromide, 5 equivalents of n-butyl vinyl ether, 15 equivalents of TEA, 0.1 equivalents of 1, 3-bis(diphenylphosphine)propane, 1 equivalent of thallium acetate, 0.09 equivalents of palladium acetate, and DMF. The tube was capped and heated to 100°C overnight. The reaction was cooled and the catalyst filtered off. The mixture was diluted with EtOAc and washed with H₂O, and dried over MgSO₄. The crude product was purified on silica (4/1 hexane/DCM). This was dissolved in THF and 4N HCl in dioxane and stirred overnight. The solvents were evaporated and the product purified on silica (4/1 hexane/EtOAc) to give pure product.

### Method G54

1 equivalent of the appropriate Boc-aniline was dissolved in methanol and the solution saturated with HCl. The reaction was heated at 50°C for 3h, then concentrated *in vacuo.* The pale yellow solid was heated in 35% H₂SO₄ until complete dissolution occurred. Upon cooling the mixture by the addition of ice H₂O the amine bisulfate precipitated. The reaction flask was cooled in an ice bath and the mixture stirred vigorously while 1.1 equivalents of sodium rutrite in H₂O was added drop wise. The reaction was stirred at 0°C for another 1.5 hours. An aqueous solution of 10 equivalents of KI was added, followed immediately with 17 equivalents CuI. The reaction was stirred at room temperature for 14 hours, then extracted 3 times with Et₂O. The combined organic layers were washed with 1M NaHCO₃, brine, and dried over MgSO₄, then concentrated *in vacuo.* The residue was purified by silica gel flash chromatography (95:5 hexane/Et₂O) to provide the pure iodide.

### Method G55

2.3 equivalents of lithium iodide was added to 1 equivalent of methyl- 2, 6-dichloro- 4-iodobenzoate in pyridine, and the mixture heated at reflux for 8 hours. The reaction was concentrated *in vacuo* and the residue was partitioned between EtOAc and 1N HCl. The aqueous layer was extracted three times with EtOAc, and the combined organic layers were washed with 1M NaHCO₃, dried over MgSO₄ and concentrated *in vacuo.* The residue was dissolved in NMM and the solution concentrated *in vacuo.* The residue was taken up in DCM and then washed three times with 1N HCl. The organic layer was dried over MgSO₄ and concentrated *in vacuo* to provide the benzoic acid in high enough purity to be used without further purification.

### Method G56

1.3 equivalents of DIPEA was added to a heterogeneous mixture of 1 equivalent of 3-hydroxybenzoic acid, 1.3 equivalents of N, O-dimethylhydroxylamine hydrochloride, 1.3 equivalents of HOBt and 1.3 equivalents of EDC stirring in DMF. All solids eventually dissolved as the mixture was stirred at room temperature for 28 hours. After concentrating the mixture, the residue was partitioned between Et₂O and H₂O. The aqueous layer was extracted three times with Et₂O and the combined organic layers were dried over MgSO₄, and concentrated *in vacuo.* The residue was purified by silica gel flash chromatography (Et₂O) to provide the pure hydroxamate. **Method G57**

To a stirred -78°C solution of 1 equivalent of the appropriate protected hydroxamate in THF was added a solution of 1.2 equivalents of 1.5 M DIBAL in toluene drop wise. The reaction mixture was stirred for an additional 3 hours at -78°C or until TLC showed clean formation of product, with only a trace of starting material. The reaction was quenched by adding to a separatory funnel containing Et₂O and 0.35M NaHSO₄. The layers were separated. The aqueous layer was extracted three times with ethyl ether. The combined organic layers were washed twice with 1N HCl, saturated aqueous NaHCO₃, and over MgSO₄, filtered through a plug of silica gel, and concentrated *in vacuo.* No further purification of the aldehyde was necessary.

### Method G58

A solution of 1 equivalent of the appropriate aldehyde in THF was cooled to -78°C and 1.1 equivalents of 0.5M ethynylmagnesium bromide/THF was added. After stirring the reaction at room temperature for 3 hours, it was diluted with Et₂O and washed twice with 10% citric acid. The combined aqueous layers were back-extracted once with Et₂O. The combined organic layers were washed twice with saturated aqueous NaHCO₃, dried over MgSO₄ and concentrated in *vacuo.* The residue was purified by silica gel flash chromatography (4:1 to 3:2 hexane/Et₂O) to provide the pure alkyne.

### Method G59

1 equivalent of the aryl iodide was dissolved in EtOAc and the solution was degassed by passing N2 through a pipette and into the solution for 10 minutes. 1.25 equivalents of the alkyne was added, followed by 0.02 equivalents of dichlorobis(triphenylphosphine)palladium(II), 0.04 equivalents of CuI and 5 equivalents TEA. The reaction was stirred for 14 hours, diluted with EtOAc, washed twice with 5% Na₂•EDTA, brine and then dried over MgSO₄ and concentrated *in vacuo.* The residue was purified by silica gel flash chromatography (gradient elution, using Et₂O to EtOAc) to provide the pure aryl alkyne.

### Method G60

1 equivalent of the aryl alkyne was dissolved in MeOH and the solution was degassed by passing N2 through a pipette and into the solution for 10 minutes. The 5% Rh/Al₂O₃ was added, one balloon-full of hydrogen was passed through the solution, and the reaction was stirred under an atmosphere of H₂ (using a balloon) for 7 hours, after which the reaction was filtered through a pad of celite and concentrated *in vacuo.* The residue was purified by silica gel flash chromatography (gradient elution, using Et₂O to EtOAc) to provide the pure product.

### Method G61

2 equivalents of the appropriate protected amino acid and 2 equivalents of Ph₃P was suspended in DCM. 2.2 equivalents of NCS was added and the mixture was stirred for 30 minutes. 1 equivalent of the aniline containing resin and 1.1 equivalents of NMM was suspended in DCM and the clear acid solution added. The resin was agitated for 2 hours, rinsed with DCM, DMA and DCM. The procedure was repeated again.

### Method G62

The appropriate benzaldehyde was converted to its corresponding hydantoin by Method G63 and then hydrolyzed to the amino acid by Method G64. The pure racemic amino acid was then protected by Method G5.

### Method G63

1 equivalent of the appropriate benzaldehyde, 2 equivalents of potassium cyanide and 4 equivalents of ammonium carbonate were refluxed in 50% EtOH for 2.5 hours. After cooling to 0°C, the solution was acidified to pH 2 will concentrated HCl. After standing in the refrigerator overnight, the crystals were filtered and washed with H₂O and recrystalized from boiling H₂O/EtOH.

### Method G64

The pure hydantoin was refluxed in 10% NaOH overnight. After cooling, activated carbon was added and the solution filtered through celite. The solution was acidified to pH 7 with concentrated HCl and allowed to stand in the refrigerator overnight. The resulting crystals were filtered, washed with H₂O and dried overnight *in vacuo* to give pure racemic amino acid.

### Method G65

4-bromo-2-chlorobenzoic acid was converted to the t-butyl ester by Method G10. *t-*Butylvinyl ether was coupled to the bromide by Method G53 to give 4-acetyl-2-chlorobenzoic acid t-butyl ester. The ketone was reduced to the alcohol by Method G66 and the racemic mixture resolved by Method G67 to give pure S isomer. Phthalamide was coupled to the alcohol by Method G68 and the product hydrolyzed by Method G69 to give the amine.

### Method G66

2 equivalents of the appropriate ketone was dissolved in MeOH and 1 equivalent of NaBH₄ was added. After stirring for 1 hour, the reaction was quenched with concentrated HCl and concentrated *in vacuo.* The residue was partitioned between Et₂O and H₂O. The organics were dried over MgSO₄ and concentrated *in vacuo.* The alcohol can be used without further purification.

### Method G67

1 equivalent of the alcohol mixture was dissolved in diisopropyl ether and 2 equivalents of vinylacetate and Amano lipase P (100mg) were added. The suspension was stirred overnight and then concentrated *in vacuo.* The residue was purified by silica get flash chromatography (5/1 EtOAc/hexane) to give pure R and S isomers.

### Method G68

A solution of 1 equivalent of the alcohol and 3 equivalents of Ph₃P in THF was cooled to - 10°C in an ice-EtOH bath. While stirring, a solution of 3 equivalents of the amine and 3 equivalents of DEAD in THF was added drop wise. The cold bath was removed and the reaction was stirred at room temperature overnight. The reaction was concentrated *in vacuo* and the resulting residue was taken up in a minimal amount of DCM and filtered through a plug of silica gel, using DCM as eluent. After concentrating this solution *in vacuo,* the residue was purified using silica gel flash chromatography (8/2/0.5 hexane/DCM/Et₂O) to provide product.

### Method G69

1 equivalent of the phthalamide was dissolved in EtOH and THF followed by addition of 8 equivalents of hydrazine hydrate. The reaction was stirred at room temperature for 1.5 hours, then at 50°C for 1 hour. The solution was cooled, filtered and the solids washed with EtOAc. The clear solution was concentrated *in vacuo* and the residue purified by silica gel flash chromatography (94/4 DCM/MeOH) to give pure amine.

### Method G70

1 equivalent of the appropriate commercially available ketone, 5 equivalents of hydroxylamine hydrochloride and 10 equivalents of sodium acetate were combined in MeOH and stirred overnight. The reaction was concentrated *in vacuo* and the residue was partitioned between EtOAc and saturated NaHCO₃. The organic layer was washed once with brine, dried over MgSO₄ and concentrated *in vacuo.* The product was purified by silica gel flash chromatography (Et₂O) to give pure oxime.

### Method G71

1 equivalent of the appropriate benzaldehyde was treated with 2.5 equivalents of the appropriate R'MgBr in THF at -20°C under an N₂ atmosphere. After warming to room temperature, the reaction was poured into a slurry of 0.1 N sulfuric acid and ice, and the product extracted with EtOAc. After partitioning and washing with brine, the organic phase was dried over MgSO₄ and concentrated *in vacuo* to give crude product. Oxidation to the ketone was carried out in dioxane with 1.1 equivalents of 2, 3-dichloro-5, 6-dicyano-1, 4-benzoquinone for 48 hours. Reaction contents were filtered, and the filtrate concentrated *in vacuo.* The residue was purified by silica gel flash chromatography (hexane/EtOAc 1:1) to yield the product as a yellow solid.

### Method G72

The resin with the S-trityl or O-trityl protecting group was washed three times with DCM. It was then washed three times for 10 minutes with a solution consisting of 1% TFA 1% TES in DCM. It was then washed 3 times with DCM. The resin was then checked by placing a small amount of resin into a test tube and treating it with concentrated TFA. If no yellow color appears the removal was complete. If a yellow color appears, the above procedure was repeated until a clear test was achieved.

### Method G73

The resin containing the appropriate free hydroxyl was washed three times with DCM. A solution of 10% DIPEA in DCM was added to the resin and a 0.3 M solution of phosgene in toluene was added to the resin. The reaction was allowed to proceed for 10 minutes at room temperature, after which it was drained and washed three times with DCM. A 0.3 M solution in DCM of the appropriate amine was added to the resin and it was allowed to react overnight. The resin was then drained and washed three times with DCM.

### Method G74

The appropriate resin was washed three times with DCM and then treated with a 0.3 M solution of the appropriate chloroformate (R) in 0.33 M DIPEA in NMP overnight. The coupling was monitored by the Kaiser ninhydrin test. If the Kaiser test was positive, the appropriate chloroformate was coupled again in the same manner. The resin was then washed three times with NMP and then three times with DCM.

### Method G75

The appropriate 2, 6- disubstituted phenol (2, 6- dichlorophenol for Compound F, 2, 6-dimethylphenol for Compound H and 2, 6- difluorophenol for Compound I) was alkylated by Method G76. The resulting phthalimide was hydrolyzed and protected by Method G77. The phenol was then converted to the triflate by Method G78 and carbonylated by Method G79 to give the desired double protected compound.

### Method G76

A round bottom flask was equipped with an efficient overhead stirrer and charged with concentrated H₂SO₄ (2.7 x volume of H₂O) and H₂O and cooled to ~-5°C with an ethanol ice bath. Once cool, 1 equivalent of the appropriate disubstituted phenol and 1 equivalent of N-(hydroxymethyl)phthalimide were added with vigorous stirring. The reaction was kept cool for 4 hours and then allowed to warm to room temperature overnight with constant stirring. The reaction generally proceeds to a point where there was just a solid in the round bottom flask. At this point EtOAc and H₂O were added and stirred into the solid. Large chunks were broken up and then the precipitate was filtered and washed with more EtOAc and H₂O. The product was then used without further purification after drying overnight in a vacuum desiccator.

### Method G77

1 equivalent of the product from Method G76 and (22.5ml x #g of starting material) of methanol was added to a round bottom flask equipped with a H₂O condenser and stirring bar. 1.2 equivalents of hydrazine mono hydrate was added and the mixture was refluxed for 4 hours. After cooling to room temperature, (4.5ml x #g of starting material) of concentrated HCl was carefully added. Upon completion of the addition, the mixture was refluxed again overnight (> 8 hours). The reaction was cooled to 0°C and the precipitated by- product filtered off. The filtrate was then concentrated *in vacuo.* The residue was then Boc protected by Method G44 with the exception that the product was recrystalized from hot methanol and H₂O.

### Method G78

1 equivalent of the appropriate phenol and 1.5 equivalents of 2, 6- lutidine was dissolved, with mild heating if necessary, in DCM in a round bottom flask. Once the starting material has completely dissolved, the mixture was cooled to -78°C under N₂ with a dry ice ethanol bath. Once cool, 2.5 equivalents of triflic anhydride was added and the reaction was allowed to slowly come to room temperature with stirring. The reaction was monitored by TLC and was generally done in 4 hours. Upon completion, the reaction was concentrated *in vacuo* and the residue partitioned between EtOAc and H₂O. The organic layer was washed twice with O.1N H₂SO₄, twice with saturated NaHCO₃, once with brine, dried over MgSO₄ and concentrated *in vacuo.* The residue was then purified on silica gel using DCM as eluent.

### Method G79

1 equivalent of triflate was dissolved in DMF and MeOH in the glass insert of a high pressure Parr bomb. The starting material was then degassed while stirring with CO for 10 minutes. 0.15 equivalents palladium(II) acetate and 0.15 equivalents of 1, 3- bis(diphenylphosphino) propane were then added and the mixture was then degassed while stirring with CO for another 10 minutes. 2.5 equivalents of diisopropyl ethyl amine was added and the Parr bomb assembled. After properly assembling the bomb, it was charged with 300 psi CO gas and heated to 70°C with stirring overnight. The bomb was then cooled and vented. The mixture was transferred to a round bottom flask and concentrated *in vacuo.* The residue was then purified on silica gel using DCM with 1% acetone and 1 % TEA as eluent.

### Method G81

1 equivalent of the appropriate alkene and 1.5 equivalents of KOH were dissolved in H₂O in an appropriately sized Parr shaker flask. A small amount (approximately 100mg per 50 mmol of alkene) of 5% Pd/C catalyst was added and the flask was charged with 50 psi H₂ and shaken overnight. The mixture was then filtered through Celite and concentrated *in vacuo.* The resulting product was used without further purification.

### Method G80

1 equivalent of the appropriate ethyl ester and 1.5 equivalents of KOH was dissolved in H₂O and refluxed for three hours. After completion, the reaction was concentrated *in vacuo* and the product used without further purification.

### Method G82

1.2 equivalents of NaH (60% mineral oil dispersion) was suspended in benzene and cooled to 0°C with and ice H₂O bath. 1.2 equivalents of triethyl phosphonoacetate was added slowly and the reaction was allowed to stir until the solution becomes clear. 1 equivalent of the appropriate ketone (R) was added slowly and the reaction was stirred for 4 hours. Upon completion, the reaction was partitioned with toluene and H₂O. The aqueous layer was back extracted. The combined organic layers were dried over MgSO₄ and concentrated *in vacuo .* The residue was purified by silica gel flash chromatography (85:15 hexane/EtOAc).

### Method G83

1.2 equivalents of NaH (60% mineral oil dispersion) was suspended in benzene and cooled to -10°C with and a dry ice H₂O bath. 1.2 equivalents of triethyl 2- phosphonopropionate was added slowly and the reaction was allowed to stir until the solution becomes clear. 1 equivalent of the appropriate aldehyde (R) was added slowly and the reaction was stirred for 4 hours. Upon completion, the reaction was partitioned with toluene and H₂O. The aqueous layer was back extracted. The combined organic layers were dried over MgSO₄ and concentrated *in vacuo .* The residue was purified by silica gel flash chromatography (85:15 hexane/EtOAc).

### Method G84

1 equivalent of the appropriately protected toluene was dissolved in acetic anhydride and HOAc, then cooled in an ice-salt bath (-5°C) before concentrated H₂SO₄ was added. A solution of CrO₃ (2.6 equivalents) in acetic anhydride and HOAc was added drop wise and the reaction was stirred for 3.5 hours at -5°C. The reaction was poured into ice H₂O and stirred for 30 min. The mixture was extracted three times with ethyl ether. The combined organic layers were washed with saturated NaHCO₃ and brine, then dried over MgSO₄ and concentrated *in vacuo* to an oil. Toluene was added to the oil and the solution concentrated *in vacuo* again. This was repeated to obtain a crystalline solid. The solid was dissolved in methanol and concentrated HCl and heated at reflux for 12 hours. The reaction was concentrated *in vacuo* and the residue was purified by silica gel flash chromatography (9:1 hexane/Et₂O) to provide the pure aldehyde.

### Method G85

1 equivalent of the appropriate alcohol was dissolved in DMF and cooled to -5°C in an ice-salt H₂O bath. 1.4 equivalents lithium bis(trimethylsilyl)amide in THF was added drop wise. The reaction was stirred for 0.5 hour, then 1 equivalent of methyl iodide was added and the reaction was stirred overnight under an atmosphere of nitrogen. The reaction was partitioned between ethyl ether and 10% citric acid. The aqueous layer was extracted with ethyl ether, the combined organic layers were washed with saturated NaHCO₃ and brine, then dried over MgSO₄ and concentrated *in vacuo* to an oil. The residue was purified by silica gel flash chromatography (9:1 hexane/Et₂O) to provide the pure methyl ether.

### Method G86

Commercially available nitroterephthalic acid was converted to its diethyl ester by Method G87. The nitro group was replaced by a benzyl mercaptan by Method G88 and deprotected by AlBr₃ using Method G89. The thiol was then alkylated with bromoacetaldehyde diethyl acetal by Method G90 and then dehydrated by Method G91. The diethyl ester was treated with LiOH, Method G4, and then coupled by Method G3 to 3-hydroxy benzyl amine, Method G38. The final ethyl ester was removed by Method G4.

### Method G87

1 equivalent of the appropriate commercially available carboxylic acid was dissolved in toluene with an excess of ethanol and 0.6 equivalents of H₂SO₄ and the mixture refluxed for 4 days. Upon completion, the reaction was concentrated *in vacuo* and partitioned between EtOAc and H₂O. The organic layer was washed with saturated NaHCO₃, brine, dried over MgSO₄ and concentrated *in vacuo.* The product was used without further purification.

### Method G88

1.25 equivalents of 95% NaH was suspended in DMF and cooled under N₂ to -5°C with an ice bath. 1.25 equivalents of benzyl mercaptan was added drop wise and the solution was allowed to stir for 40 minutes. 1 equivalent of the appropriate aryl nitro compound was added over 20 minutes and the mixture was stirred for an additional 30 minutes. After verifying that reaction was complete, the solution was poured onto ice and stirred until all the ice melts. The aqueous solution was partitioned three times with EtOAc and the combined organic layers were washed with brine, dried over MgSO₄ and concentrated *in vacuo.* The residue was purified by silica gel flash chromatography (1:4 hexane/EtOAc) to provide product.

### Method G89

1 equivalent of benzyl protected material and 2.2 equivalents of AlBr₃ were refluxed in toluene for 3 hours at which time H₂O and enough EtOAc was added to partition the mixture. The organic layer was washed three times with H₂O, brine, dried over MgSO₄ and concentrated *in vacuo.* The residue was purified by silica gel flash chromatography (4:1 hexane/EtOAc) to provide product.

### Method G90

1 equivalent of the thiol was dissolved in DMF and 2 equivalents of K₂CO₃ was added. 1.1 equivalents of bromoacetaldehyde diethyl acetal was added slowly over 20 minutes and then 0.1 equivalent of NaI was added portion wise. The reaction was stirred for 2 hours and then partitioned between EtOAc and H₂O. The organic layer was washed three times with H₂O, brine, dried over MgSO₄ and concentrated *in vacuo.* The residue was purified by silica gel flash chromatography (9:1 hexane/EtOAc) to provide product.

### Method G91

1 equivalent (by weight) of the appropriate diethyl acetal and 2 equivalents (by weight) of poly phosphoric acid were dissolved in chlorobenzene. The reaction was monitored by TLC. Upon completion of the reaction, the mixture was concentrated *in vacuo* and then partitioned between EtOAc and saturated NaHCO₃. The organic layer was washed twice more with saturated NaHCO₃, brine, dried over MgSO₄ and concentrated *in vacuo.* The residue was purified by silica gel flash chromatography (4:1 hexane/EtOAc) to provide product.

### Method G92

1 equivalent of the appropriate carboxylic acid was dissolved in DCM and cooled to 0°C with an ice H₂O bath. Once cool, 3 drops of DMF and 1.5 equivalents of oxalyl chloride were added. The reaction was stirred at 0°C for 1.5 hours and then for 0.5 hour at room temperature. At this time, the reaction was concentrated *in vacuo* and used immediately.

### Method G93

1 equivalent of bis-N-carboxybenzoyl-cystine dibenzyl ester was dissolved in HOAc/H₂O (9/1) and treated with chlorine gas for 10 minutes. The reaction was concentrated *in vacuo,* dissolved in toluene and concentrated *in vacuo* again to yield a white solid. This product was dissolved in DCM and 0.5 equivalents of the appropriate amine (R) was added. The reaction was stirred for 30 minutes and then diluted with EtOAc and partitioned with 0.1N H₂SO₄ and then brine. The organic layer was dried over MgSO₄ and concentrated *in vacuo.* The residue was purified by silica gel flash chromatography (EtOAc/hexane 1:1) to yield pure product. The protecting groups were removed by Method G38 and the product used without further purification.

### Specific Example methods

### Method S1

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-a-Fmoc-N-β-Alloc-L-diaminopropionic acid. The Fmoc group was cleaved by Method G19. Compound C, Method G13, was coupled by Method G20. The Alloc group was removed by Method G35 The appropriate isocyanate (R) was coupled by Method G33. The completed molecule was worked up by Method G21.

### Method S2

Compounds were synthesized using standard Fmoc solid phase methods on Fmoc- L-diamiobutyric acid (alloc)- p- alkoxybenzyl alcohol resin (0.5 mmol/g) (Fmoc- L- Daba(alloc)- Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35 The appropriate isocyanate (R) was coupled by Method G33. The completed molecule was worked up by Method G21.

### Method S3

Compounds were synthesized using standard Fmoc solid phase methods on Fmoc- L-Ornithine (alloc)- p- alkoxybenzyl alcohol resin (0.5 mmol/g) (Fmoc- L- Orn(alloc)- Wang resin). The resin was made by Method G34 using commercially available N-α-Fmoc-N-δ-Alloc-L-Omithine. The Fmoc group was cleaved by Method G19. Compound C, Method G13, was coupled by Method G20. The Alloc group was removed by Method G35 The appropriate isocyanate (R) was coupled by Method G33. The completed molecule was worked up by Method G21.

### Method S4

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35 The appropriate isocyanate (R) was coupled by Method G33. The completed molecule was worked up by Method G21.

### Method S5

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13 was coupled by Method G20. The Alloc group was removed by Method G35. The appropriate carboxylic acid (R) was coupled by Method G20. The completed molecule was worked up by Method G21.

### Method S6

Compounds were synthesized using standard Fmoc solid phase methods on Fmoc- L-diamiobutyric acid (alloc)- p- alkoxybenzyl alcohol resin (0.5 mmol/g) (Fmoc- L- Daba(alloc)- Wang resin). The resin was made by Method G34 using commercially available N-α-Fmoc-N-γ-Alloc-L-diaminobutyric acid The Fmoc group was cleaved by Method G19. Compound C, Method G13 was coupled by Method G20. The Alloc group was removed by Method G35. The appropriate carboxylic acid (R) was coupled by Method G20. The completed molecule was worked up by Method G21. **Method S7**

Compounds were synthesized using standard Fmoc solid phase methods on Fmoc- L-Ornithine (alloc)- p- alkoxybenzyl alcohol resin (0.5 mmol/g) (Fmoc- L- Orn(alloc)- Wang resin). The resin was made by Method G34 using commercially available N-α-Fmoc-N-δ-Alloc-L-Ornithine. The Fmoc group was cleaved by Method G19. Compound C, Method G13 was coupled by Method G20. The Alloc group was removed by Method G35. The appropriate carboxylic acid (R) was coupled by Method G20. The completed molecule was worked up by Method G21.

### Method S8

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13 was coupled by Method G20. The Alloc group was removed by Method G35. The appropriate carboxylic acid (R) was coupled by Method G20. The completed molecule was worked up by Method G21.

### Method S9

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. Commercially available Fmoc- nipecotic acid was coupled by Method G20. The Fmoc group was removed by Method G19 and the appropriate carboxylic acid (R) was coupled by Method G20. The completed molecule was worked up by Method G21.

### Method S10

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. Commercially available Fmoc- isonipecotic acid was coupled by Method G20. The Fmoc group was removed by Method G19 and the appropriate carboxylic acid (R) was coupled by Method G20. The completed molecule was worked up by Method G21.

### Method S11

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. Commercially available Fmoc- 3-aminomethyl benzoic acid was coupled by Method G20. The Fmoc group was removed by Method G19 and the appropriate carboxylic acid (R) was coupled by Method G20. The completed molecule was worked up by Method G21.

### Method S12

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. Commercially available Fmoc- 4-aminomethyl benzoic acid was coupled by Method G20. The Fmoc group was removed by Method G19 and the appropriate carboxylic acid (R) was coupled by Method G20. The completed molecule was worked up by Method G21.

### Method S13

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. Commercially available Fmoc- β alanine was coupled by Method G20. The Fmoc group was removed by Method G19 and the appropriate carboxylic acid (R) was coupled by Method G20. The completed molecule was worked up by Method G21.

### Method S14

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. Commercially available Fmoc- glycine was coupled by Method G20. The Fmoc group was removed by Method G19 and the appropriate carboxylic acid (R) was coupled by Method G20. The completed molecule was worked up by Method G21.

### Method S15

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. Commercially available Fmoc- nipecotic acid was coupled by Method G20. The Fmoc group was removed by Method G19 and the appropriate carboxylic acid (R) was coupled by Method G20. The completed molecule was worked up by Method G21.

### Method S16

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. Commercially available Fmoc- isonipecotic acid was coupled by Method G20. The Fmoc group was removed by Method G19 and the appropriate carboxylic acid (R) was coupled by Method G20. The completed molecule was worked up by Method G21.

### Method S17

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. Commercially available Fmoc- pipecolinic acid was coupled by Method G20. The Fmoc group was removed by Method G19 and the appropriate carboxylic acid (R) was coupled by Method G20. The completed molecule was worked up by Method G21.

### Method S18

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. Commercially available Fmoc- 3-aminomethyl benzoic acid was coupled by Method G20. The Fmoc group was removed by Method G19 and the appropriate carboxylic acid (R) was coupled by Method G20. The completed molecule was worked up by Method G21.

### Method S19

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. Commercially available Fmoc- 4-aminomethyl benzoic acid was coupled by Method G20. The Fmoc group was removed by Method G19 and the appropriate carboxylic acid (R) was coupled by Method G20. The completed molecule was worked up by Method G21.

### Method S20

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. Commercially available Fmoc- β alanine was coupled by Method G20. The Fmoc group was removed by Method G19 and the appropriate carboxylic acid (R) was coupled by Method G20. The completed molecule was worked up by Method G21.

### Method S21

Compounds were synthesized using standard Fmoc solid phase methods on commercially available Fmoc- L- Ornithine (alloc)- p- alkoxybenzyl alcohol resin (0.5 mmol/g) (Fmoc- L-Orn(alloc)- Wang resin). The resin was made by Method G34 using commercially available N-α-Fmoc-N-8-Alloc-L-Ornithine. The Fmoc group was cleaved by Method G19. Compound C, Method G13, was coupled by Method G20. The Alloc group was removed by Method G35. Commercially available Fmoc- glycine was coupled by Method G20. The Fmoc group was removed by Method G19 and the appropriate carboxylic acid (R) was coupled by Method G20. The completed molecule was worked up by Method G21.

### Method S22

Compounds were synthesized using standard Fmoc solid phase methods on N-α-Fmoc-N-γ-Alloc-L-diaminobutyric acid- p- alkoxybenzyl alcohol resin (0.5 mmol/g) (Fmoc- L- Daba(alloc)-Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercially available Fmoc- nipecotic acid was coupled by Method G20. The Fmoc group was removed by Method G19 and the appropriate carboxylic acid (R) was coupled by Method G20. The completed molecule was worked up by Method G21.

### Method S23

Compounds were synthesized using standard Fmoc solid phase methods on N-α-Fmoc-N-γ-Alloc-L-diaminobutyric acid- p- alkoxybenzyl alcohol resin (0.5 mmol/g) (Fmoc- L- Daba(alloc)-Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercialiy available Fmoc- isonipecotic acid was coupled by Method G20. The Fmoc group was removed by Method G19 and the appropriate carboxylic acid (R) was coupled by Method G20. The completed molecule was worked up by Method G21.

### Method S24

Compounds were synthesized using standard Fmoc solid phase methods on N-α-Fmoc-N-γ-Alloc-L-diaminobutyric acid- p- alkoxybenzyl alcohol resin (0.5 mmol/g) (Fmoc- L- Daba(alloc)- Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercially available Fmoc- pipecolinic acid was coupled by Method G20. The Fmoc group was removed by Method G19 and the appropriate carboxylic acid (R) was coupled by Method G20. The completed molecule was worked up by Method G21.

### Method S25

Compounds were synthesized using standard Fmoc solid phase methods on N-α-Fmoc-N-γ-Alloc-L-diaminobutyric acid- p- alkoxybenzyl alcohol resin (0.5 mmol/g) (Fmoc- L- Daba(alloc)-Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercially available Fmoc- 3-aminomethyl benzoic acid was coupled by Method G20. The Fmoc group was removed by Method G19 and the appropriate carboxylic acid (R) was coupled by Method G20. The completed molecule was worked up by Method G21.

### Method S26

Compounds were synthesized using standard Fmoc solid phase methods on N-α-Fmoc-N-γ-Alloc-L-diaminobutyric acid- p- alkoxybenzyl alcohol resin (0.5 mmol/g) (Fmoc- L- Daba(alloc)-Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercially available Fmoc- 4-aminomethyl benzoic acid was coupled by Method G20. The Fmoc group was removed by Method G19 and the appropriate carboxylic acid (R) was coupled by Method G20. The completed molecule was worked up by Method G21:

### Method S27

Compounds were synthesized using standard Fmoc solid phase methods on N-α-Fmoc-N-y-Alloc-L-diaminobutyric acid- p- alkoxybenzyl alcohol resin (0.5 mmol/g) (Fmoc- L- Daba(alloc)-Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercially available Fmoc- β alanine was coupled by Method G20. The Fmoc group was removed by Method G19 and the appropriate carboxylic acid (R) was coupled by Method G20. The completed molecule was worked up by Method G21.

### Method S28

Compounds were synthesized using standard Fmoc solid phase methods on N-α-Fmoc-N-γ-Alloc-L-diaminobutyric acid- p- alkoxybenzyl alcohol resin (0.5 mmol/g) (Fmoc- L- Daba(alloc)-Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. Commercially available Fmoc- glycine was coupled by Method G20. The Fmoc group was removed by Method G19 and the appropriate carboxylic acid (R) was coupled by Method G20. The completed molecule was worked up by Method G21.

### Method S29

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. Commercially available Fmoc- nipecotic acid was coupled by Method G20. The Fmoc group was removed by Method G19 and the appropriate carboxylic acid (R) was coupled by Method G20. The completed molecule was worked up by Method G21.

### Method S30

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. Commercially available Fmoc- isonipecotic acid was coupled by Method G20. The Fmoc group was removed by Method G19 and the appropriate carboxylic acid (R) was coupled by Method G20. The completed molecule was worked up by Method G21.

### Method S31

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. Commercially available Fmoc- pipecolinic acid was coupled by Method G20. The Fmoc group was removed by Method G19 and the appropriate carboxylic acid (R) was coupled by Method G20. The completed molecule was worked up by Method G21.

### Method S32

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. Commercially available Fmoc- 3-aminomethyl benzoic acid was coupled by Method G20. The Fmoc group was removed by Method G19 and the appropriate carboxylic acid (R) was coupled by Method G20. The completed molecule was worked up by Method G21.

### Method S33

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-AHoc-L-lysine. The Fmoc group was cleaved by Method G19. Compound C, Method G13, was coupled by Method G20. The Alloc group was removed by Method G35. Commercially available Fmoc- 4-aminomethyl benzoic acid was coupled by Method G20. The Fmoc group was removed by Method G19 and the appropriate carboxylic acid (R) was coupled by Method G20. The completed molecule was worked up by Method G21.

### Method S34

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-Iysine. The Fmoc group was cleaved by Method G19. Compound C, Method G13, was coupled by Method G20. The Alloc group was removed by Method G35. Commercially available Fmoc- β alanine was coupled by Method G20. The Fmoc group was removed by Method G19 and the appropriate carboxylic acid (R) was coupled by Method G20. The completed molecule was worked up by Method G21.

### Method S35

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-Iysine. The Fmoc group was cleaved by Method G19. Compound C, Method G13, was coupied by Method G20. The Alloc group was removed by Method G35. Commercially available Fmoc- glycine was coupled by Method G20. The Fmoc group was removed by Method G19 and the appropriate carboxylic acid (R) was coupled by Method G20. The completed molecule was worked up by Method G21.

### Method S36

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. The appropriate chloroformate (R) was coupled by Method G74. The completed molecule was worked up by Method G21.

### Method S37

Compounds were synthesized using standard Fmoc solid phase methods on commercially available Fmoc- L- tryptophan(Boc)- Wang resin (0.5 mmol/g). The Fmoc group was cleaved by Method G19. Compound D, Method G14, was coupled by Method G20. The Fmoc group was cleaved by Method G19. The appropriate carboxylic acid (R) was coupled by Method G20. The completed molecule was worked up by Method G21.

### Method S38

Compounds were synthesized using standard Fmoc solid phase methods on commercially available Fmoc- L- alanine- Wang resin (0.5 mmol/g). The Fmoc group was cleaved by Method G19. Compound D, Method G14, was coupled by Method G20. The Fmoc group was cleaved by Method G19. The appropriate carboxylic acid (R) was coupled by Method G20. The completed molecule was worked up by Method G21.

### Method S39

Compounds were synthesized using standard Fmoc solid phase methods on commercially available Fmoc- L- asparagine(Trt)- Wang resin (0.5 mmol/g). The Fmoc group was cleaved by Method G19. Compound D, Method G14, was coupled by Method G20. The Fmoc group was cleaved by Method G19. The appropriate carboxylic acid (R) was coupled by Method G20. The completed molecule was worked up by Method G21.

### Method S40

Compounds were synthesized using standard Fmoc solid phase methods on commercially available Fmoc- L- tryptophan(Boc)- Wang resin (0.5 mmol/g). The Fmoc group was cleaved by Method G19. 4- amino 2- methylbenzoic acid was coupled by Method G20. The appropriate carboxylic acid (R) was silyl protected, Method G18, and the acid chloride generated by Method G92 and coupled in DCM over night to the amine. After washing the resin with DCM and THF, 3 equivalents of tetrabutylammonium fluoride in THF was added. After 20 minutes, the resin was washed with THF, H₂O and dilute HOAc. The completed molecule was worked up by Method G21.

### Method S41

Compounds were synthesized using standard Fmoc solid phase methods on commercially available Fmoc- L- amino acid- Wang resins (0.5 mmol/g) (R). The Fmoc group was cleaved by Method G19. 4- amino 2- methylbenzoic acid was coupled by Method G20. The 3- hydroxy phenylacetic acid was silyl protected, Method G18, and the acid chloride generated by Method G92 and coupled in DCM over night to the amine. After washing the resin with DCM and THF, 3 equivalents of TBAF in THF was added. After 20 minutes, the resin was washed with THF, H₂O and dilute HOAc. The completed molecule was worked up by Method G21.

### Method S42

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. Fmoc- glycine was coupled to the aniline by Method G61. The Fmoc group was cleaved by Method G19. The appropriate carboxylic acid (R) was coupled by Method G20. The completed molecule was worked up by Method G21.

### Method S43

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. Fmoc- L-alanine was coupled to the aniline by Method G61. The Fmoc group was cleaved by Method G19. The appropriate carboxylic acid (R) was coupled by Method G20. The completed molecule was worked up by Method G21.

### Method S44

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. Fmoc- L-phenylglycine was coupled to the aniline by Method G61. The Fmoc group was cleaved by Method G19. The appropriate carboxylic acid (R) was coupled by Method G20. The completed molecule was worked up by Method G21.

### Method S45

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. Fmoc- L-glutamine was coupled to the aniline by Method G61. The Fmoc group was cleaved by Method G19. The appropriate carboxylic acid (R) was coupled by Method G20. The completed molecule was worked up by Method G21.

### Method S46

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. 3- chloro benzaldehyde was converted to Fmoc- 3- chloro- phenylglycine by Method G62, and coupled to the aniline by Method G61. The Fmoc group was cleaved by Method G19. The appropriate carboxylic acid (R) was coupled by Method G20. The completed molecule was worked up by Method G21.

### Method S47

Compounds were synthesized using standard Fmoc solid phase methods on commercially available Fmoc- L- diaminopropionic acid(alloc)- Wang resin (0.5 mmol/g). The Fmoc group was cleaved by Method G19. Compound D, Method G14, was coupled by Method G20. The Fmoc group was cleaved by Method G19. The appropriate carboxylic acid (R) was coupled by Method G20. The completed molecule was worked up by Method G21.

### Method S48

Compounds were synthesized using standard Fmoc solid phase methods on commercially available Fmoc- L- Lysine(Boc)- Wang resin (0.5 mmol/g). The Fmoc group was cleaved by Method G19. Compound D, Method G14, was coupled by Method G20. The Fmoc group was cleaved by Method G19. The appropriate carboxylic acid (R) was coupled by Method G20. The completed molecule was worked up by Method G21.

### Method S49

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. 3- methoxy benzaldehyde was converted to Fmoc- 3- chloro- phenylglycine by Method G62, and coupled to the aniline by Method G61. The Fmoc group was cleaved by Method G19. The appropriate carboxylic acid (R) was coupled by Method G20. The completed molecule was worked up by Method G21.

### Method S50

Compounds were synthesized using standard Fmoc solid phase methods on commercially available Fmoc- L- alanine- Wang resin (0.5 mmol/g). The Fmoc group was cleaved by Method G19. Commercially available 4- amino 2-chloro benzoic acid was coupled by Method G20. Fmoc- meta tyrosine was coupled to the aniline by Method G61. The Fmoc group was cleaved by Method G19. The appropriate carboxylic acid (R) was coupled by Method G20. The completed molecule was worked up by Method G21.

### Method S51

3-hydroxy aniline was coupled to commercially available Boc- d- serine by Method G3. The Boc group was removed by Method G1 and this amine was coupled to Compound A, Method G8. The *t*-butyl ester was removed by Method G11 and the acid coupled to the appropriate amino acid O-*t*-butyl ester (R) by Method G3. The final *t*-butyl ester was removed by Method G11, and the completed molecule was purified by reverse phase HPLC, verified by electrospray mass spectrometry and lyophilized to a powder.

### Method S52

The Boc group on Compound F, Method G75, was removed by Method G2 and furylacrylic acid was coupled to the amine after free basing, Method G2, by Method G3. The methyl ester was removed by Method G55 and the resulting acid coupled by Method G20 to the appropriate deprotected commercially available Fmoc protected amino acid Wang resin (0.5 mmol/g) (R). The completed molecule was worked up by Method G21.

### Method S53

The methyl ester of Compound F, Method G75, was removed by Method G55 and the resulting acid coupled by Method G20 to commercially available L- asparagine *t*-butyl ester. The Boc group was removed by Method G1 and the appropriate carboxylic acid (R) was coupled by Method G3. After removing the final t-butyl ester by Method G11, the molecule was purified by reverse phase HPLC, verified by electrospray mass spectrometry and lyophilized to a powder.

### Method S54

The Boc group on Compound F, Method G75, was removed by Method 2G1 and furylacrylic acid was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and the appropriate carboxylic acid (R) was coupled by Method G3. After saponification, Method G4, the molecule was purified by reverse phase HPLC, verified by electrospray mass spectrometry and lyophilized to a powder.

### Method S55

The Boc group on Compound I, Method G75, was removed by Method G1 and 3-hydroxybenzoic acid was coupled to the amine after free basing, Method G2, by Method G3. The methyl ester was removed by Method G55 and the resulting acid coupled by Method G20 to commercially available L- tryptophan methyl ester. After saponification, Method G4, the molecule was purified by reverse phase HPLC, verified by electrospray mass spectrometry and lyophilized to a powder.

### Method S56

The Boc group on Compound H, Method G75, was removed by Method G1 and 3-hydroxybenzoic acid was coupled to the amine after free basing, Method G2, by Method G3. The methyl ester was removed by Method G55 and the resulting acid coupled by Method G20 to commercially available amino acid methyl ester (R). After saponification, Method G4, the molecule was purified by reverse phase HPLC verified by electrospray mass spectrometry and lyophilized to a powder.

### Method S57

The Boc group on Compound H, Method G75, was removed by Method G1 and furylacrylic acid was coupled to the amine after free basing, Method G2, by Method G3. The methyl ester was removed by Method G55 and the resulting acid coupled by Method G20 to the appropriate commercially available amino acid methyl ester (R). The Boc group was removed by Method G1 if needed and after saponification, Method G4, the molecule was purified by reverse phase HPLC, verified by electrospray mass spectrometry and lyophilized to a powder.

### Method S58

The Boc group on Compound H, Method G75, was removed by Method G1 and 3-(2-thienyl)acrylic acid was coupled to the amine after free basing, Method G2, by Method G3. The methyl ester was removed by Method G55 and the resulting acid coupled by Method G20 to the appropriate commercially available amino acid methyl ester (R). The Boc group was removed by Method G1 if needed and after saponification, Method G4, the molecule was purified by reverse phase HPLC, verified by electrospray mass spectrometry and lyophilized to a powder.

### Method S59

The Boc group on Compound H, Method G75, was removed by Method 2G1 and furylacrylic acid was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and the appropriate carboxylic acid (R) was coupled by Method G3. After saponification, Method G4, the molecule was purified by reverse phase HPLC, verified by electrospray mass spectrometry and lyophilized to a powder.

### Method S60

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. The appropriate aldehyde (R) was coupled by Method G23. The completed molecule was worked up by Method G21.

### Method S61

Compounds were synthesized using standard Fmoc solid phase methods on Fmoc- L-diamiopropionic acid (alloc)- p- alkoxybenzyl alcohol resin (0.5 mmol/g) (Fmoc- L- Dapa(alloc)-Wang resin). The resin was made by Method G34 using commercially available N-α-Fmoc-N-β-Alloc-L-diaminopropionic acid. The Fmoc group was cleaved by Method G19. Compound C, Method G13, was coupled by Method G20. The Alloc group was removed by Method G35. The appropriate aldehyde (R) was coupled by Method G23. The completed molecule was worked up by Method G21.

### Method S62

The appropriate amine (R) was coupled to Compound A, Method G8, by Method G3. The t-butyl ester was removed by Method G11. The resulting acid was coupled by Method G3 to resin made by Method G34 using commercially available N-α-Fmoc-N-β-Alloc-L-diaminopropionic acid where the Fmoc group had been removed by Method G19. The completed molecule was worked up by Method G21.

### Method S63

The appropriate amine (R) was coupled to Compound A, Method G8, by Method G3. The *t-*butyl ester was removed by Method G11. The resulting acid was coupled by Method G3 to commercially available Fmoc- L- asparagine(Trt)- Wang resin (0.5 mmol/g) where the Fmoc group had been removed by Method G19. The completed molecule was worked up by Method G21.

### Method S64

The Boc group on Compound F, Method G75, was removed by Method G1 and reduced furylacrylic acid, Method G81, was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4, the molecule was purified by reverse phase HPLC, verified by electrospray mass spectrometry and lyophilized to a powder.

### Method S65

The Boc group on Compound F, Method G75, was removed by Method G1. 2- acetylfuran was converted to the methyl acrylic acid ethyl ester by Method G82 and after saponification by Method G80 was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4, the molecule was purified by reverse phase HPLC, verified by electrospray mass spectrometry and lyophilized to a powder.

### Method S66

The Boc group on Compound F, Method G75, was removed by Method G1. After 2-acetylfuran was converted to the methyl acrylic acid ethyl ester by Method G82, saponified by Method G80 and reduced by Method G81, it was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4, the molecule was purified by reverse phase HPLC, verified by electrospray mass spectrometry and lyophilized to a powder.

### Method S67

The Boc group on Compound F, Method G75, was removed by Method G1. Furylaldehyde was converted to the methyl acrylic acid ethyl ester by Method G83 and after saponification by Method G80 was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4 the molecule was purified by reverse phase HPLC, verified by electrospray mass spectrometry and lyophilized to a powder.

### Method S68

The Boc group on Compound F, Method G75, was removed by Method G1. After furylaldehyde was converted to the methyl acrylic acid ethyl ester by Method G83, saponified by Method G80 and reduced by Method G81, it was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4, the molecule was purified by reverse phase HPLC, verified by electrospray mass spectrometry and lyophilized to a powder.

### Method S69

Compounds were synthesized using standard Fmoc solid phase methods on the appropriate commercially available Fmoc- L- amino acid- Wang resin (0.5 mmol/g) (R). The Fmoc group was cleaved by Method G19. The commercially available 2, 6 dimethyl terephthalic acid was coupled by Method G20. 3- hydroxy benzylamine, Method G38, was coupled by Method G20. The completed molecule was worked up by Method G21 and correct stereochemistry was assigned by activity.

### Method S70

Compounds were synthesized on resin made by Method G34 using commercially available N-a-Fmoc-N-b-Alloc-L-diaminopropionic acid. The Fmoc group was cleaved by Method G19. The commercially available 2, 6 dimethyl terephthalic acid was coupled by Method G20. 3- hydroxy benzylamine, Method G38, was coupled by Method G20. The Alloc group was removed by Method G35 and the appropriate carboxylic acid (R) was coupled by Method G20. The completed molecule was worked up by Method G21 and correct stereochemistry was assigned by activity.

### Method S71

The Boc group on Compound F, Method G75, was removed by Method G1 and the appropriate carboxylic acid (R) was coupled to the amine after free basing, Method G2, by Method G3. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4, the molecule was purified by reverse phase HPLC, verified by electrospray mass spectrometry and lyophilized to a powder.

### Method S72

Compounds were synthesized using standard Fmoc solid phase methods on commercially available Fmoc- L- tryptophan(Boc)- Wang resin (0.5 mmol/g). The Fmoc group was cleaved by Method G19. Commercially available 2- bromo terephthalic acid was protected with an Fmoc group by Method G6 and the resulting product was coupled by Method G20. The appropriate amine (R) was coupled by Method G22. The completed molecule was worked up by Method G21.

### Method S73

Compounds were synthesized using standard Fmoc solid phase methods on commercially available Fmoc- L- alanine- Wang resin (0.5 mmol/g). The Fmoc group was cleaved by Method G19. Commercially available 2- bromo terephthalic acid was protected with an Fmoc group by Method G6 and the resulting product was coupled by Method G20. The appropriate amine (R) was coupled by Method G22. The completed molecule was worked up by Method G21.

### Method S74

Compounds were synthesized using standard Fmoc solid phase methods on Fmoc- L-diamiobutyric acid (alloc)- p- alkoxybenzyl alcohol resin (0.5 mmol/g) (Fmoc- L- Daba(alloc)- Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. The appropriate commercially available sulfonyl chloride (R) was coupled by Method G42. The completed molecule was worked up by Method G21.

### Method S75

Compounds were synthesized using standard Fmoc solid phase methods on N-α-Fmoc-N-δ-Alloc-L-Ornithine- p- alkoxybenzyl alcohol resin (0.5 mmol/g) (Fmoc- L- Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. The appropriate commercially available sulfonyl chloride (R) was coupled by Method G42. The completed molecule was worked up by Method G21.

### Method S76

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. The appropriate commercially available sulfonyl chloride (R) was coupled by Method G42. The completed molecule was worked up by Method G21.

### Method S77

Compounds were synthesized using standard Fmoc solid phase methods on Fmoc- L-diamiobutyric acid (alloc)- p- alkoxybenzyl alcohol resin (0.5 mmol/g) (Fmoc- L- Daba(alloc)- Wang resin). The resin was made by Method G34 using commercially available N-α-Fmoc-N-γ-Alloc-L-diaminobutyric acid. The Fmoc group was cleaved by Method G19. Compound C, Method G13, was coupled by Method G20. The Alloc group was removed by Method G35. The appropriate commercially available chloroformate (R) was coupled by Method G43. The completed molecule was worked up by Method G21.

### Method S78

Compounds were synthesized using standard Fmoc solid phase methods on N-α-Fmoc-N-δ-Alloc-L-Omithine- p- alkoxybenzyl alcohol resin (0.5 mmol/g) (Fmoc- L- Orn(alloc)- Wang resin). The resin was made by Method G34 using commercially available N-α-Fmoc-N-δ-Alloc-L-Ornithine. The Fmoc group was cleaved by Method G19. Compound C, Method G13, was coupled by Method G20. The Alloc group was removed by Method G35. The appropriate commercially available chloroformate (R) was coupled by Method G43. The completed molecule was worked up by Method G21.

### Method S79

Compounds were synthesized using standard Fmoc solid phase methods on Fmoc- L-Lysine (alloc)- p- alkoxybenzyl alcohol resin (0.5 mmol/g) (Fmoc- L- Lys(alloc)- Wang resin). The resin was made by Method G34 using commercially available N-α-Fmoc-N-ε-Alloc-L-lysine. The Fmoc group was cleaved by Method G19. Compound C, Method G13, was coupled by Method G20. The Alloc group was removed by Method G35. The appropriate commercially available chloroformate (R) was coupled by Method G43. The completed molecule was worked up by Method G21.

### Method S80

Compounds were synthesized using standard Fmoc solid phase methods on commercially available Fmoc- L- asparagine(Trt)- Wang resin (0.5 mmol/g). The Fmoc group was cleaved by Method G19. Commercially available 2- bromo terephthalic acid was protected with an Fmoc group by Method G6 and the resulting product was coupled by Method G20. The appropriate amine (R) was coupled by Method G22. The completed molecule was worked up by Method G21.

### Method S81

Compounds were synthesized on resin made by Method G34 using commercially available N-α-Fmoc-N-β-Alloc-L-diaminopropionic acid. The Fmoc group was cleaved by Method G19. Commercially available 2- bromo terephthalic acid was protected with an Fmoc group by Method G6 and the resulting product was coupled by Method G20. The appropriate amine (R) was coupled by Method G22. The completed molecule was worked up by Method G21.

### Method S82

Compounds were synthesized using standard Fmoc solid phase methods on the appropriate commercially available Fmoc- amino acid p- alkoxybenzyl alcohol resin (R) (Wang resin) (0.5 mmol/g). The Fmoc group was cleaved by Method G19. Compound C, Method G13, was coupled by Method G20. The completed molecule was worked up by Method G21.

### Method S83

Compounds were synthesized using standard Fmoc solid phase methods on the appropriate commercially available Fmoc- amino acid p- alkoxybenzyl alcohol resin (R) (Wang resin) (0.5 mmol/g). The Fmoc group was cleaved by Method G19. Compound B, Method G12, was coupled by Method G20. The completed molecule was worked up by Method G21.

### Method S84

Compounds were synthesized using standard Fmoc solid phase methods on Fmoc- L-tryptophan(Boc)- Wang resin (0.5 mmol/g). The Fmoc group was cleaved by Method G19. Commercially available 4-amino 2-chlorobenzoic acid was coupled by Method G20. The resin was treated with an excess of 0.5M 4- nitrophenyl chloroformate and 0.5M DIPEA for 45 minutes. After twice washing the resin with THF/DCM, an excess of the appropriate amine (R) in 0.5M DIPEA/ DMF was added and the resin bubbled for 20 minutes. The completed molecule was worked up by Method G21.

### Method S85

The appropriate amino acid (R) was converted to its methyl ester by Method G15. After free basing the amine by Method G2, Compound C, Method G13, was coupled to the amino acid methyl ester by Method G3. After saponification, Method G4, the molecule was purified by reverse phase HPLC, verified by electrospray mass spectrometry and lyophilized to a powder.

### Method S86

3-hydroxyacetophenone was converted to the oxime by Method G70 and then hydrogenated by Method G38 to give the amine. This amine was then coupled to Compound A, Method G8, by Method G24. After removal of the *t*-butyl ester by Method G11, the acid was coupled to commercially available L-asparagine *t*-butyl ester by Method G24. The final *t*-butyl ester was removed by Method G11 and the completed molecule was purified by reverse phase HPLC, verified by electrospray mass spectrometry and lyophilized to a powder.

### Method S87

3-hydroxyacetophenone was converted to the oxime by Method G70 and then hydrogenated by Method G38 to give the amine. This amine was then coupled to Compound A, Method G8, by Method G24. After removal of the t-butyl ester by Method G11, the acid was coupled to commercially available L-tryptophan methyl ester by Method G24. The final methyl ester was removed by Method G4 and the completed molecule was purified by reverse phase HPLC, verified by electrospray mass spectrometry and lyophilized to a powder.

### Method S88

3-hydroxybenzaldehyde and ethyl magnesium bromide were converted to the ketone by Method G71. The ketone was then converted to the oxime by Method G70 and then hydrogenated by Method G38 to give the amine. This amine was then coupled to Compound A, Method G8, by Method G24. After removal of the *t*-butyl ester by Method G11, the acid was coupled to commercially available L-asparagine t-butyl ester by Method G24. The final t-butyl ester was removed by Method G11 and the completed molecule was purified by reverse phase HPLC, verified by electrospray mass spectrometry and lyophilized to a powder.

### Method S89

3-hydroxybenzaldehyde and ethyl magnesium bromide were converted to the ketone by Method G71. The ketone was then converted to the oxime by Method G70 and then hydrogenated by Method G38 to give the amine. This amine was then coupled to Compound A, Method G8, by Method G24. After removal of the t-butyl ester by Method G11, the acid was coupled to commercially available L-tryptophan methyl ester by Method G24. The final methyl ester was removed by Method G4 and the completed molecule was purified by reverse phase HPLC, verified by electrospray mass spectrometry and lyophilized to a powder.

### Method S90

3-hydroxybenzaldehyde and N-propyl magnesium bromide were converted to the ketone by Method G71. The ketone was then converted to the oxime by Method G70 and then hydrogenated by Method G38 to give the amine. This amine was then coupled to Compound A, Method G8, by Method G24. After removal of the t-butyl ester by Method G11, the acid was coupled to commercially available L-asparagine t-butyl ester by Method G24. The final t-butyl ester was removed by Method G11 and the completed molecule was purified by reverse phase HPLC, verified by electrospray mass spectrometry and lyophilized to a powder.

### Method S91

3-hydroxybenzaldehyde and N-propyl magnesium bromide were converted to the ketone by Method G71. The ketone was then converted to the oxime by Method G70 and then hydrogenated by Method G38 to give the amine. This amine was then coupled to Compound A, Method G8, by Method G24. After removal of the *t*-butyl ester by Method G11, the acid was coupled to commercially available L-tryptophan methyl ester by Method G24. The final methyl ester was removed by Method G4 and the completed molecule was purified by reverse phase HPLC, verified by electrospray mass spectrometry and lyophilized to a powder.

### Method S92

The appropriate sulfonamide was synthesized by Method G93 using ammonia as the amine (R) and this product converted to the methyl ester by Method G15. Compound C, Method G13, was coupled to the sulfonamide methyl ester by Method G3. The finale methyl ester was removed by Method G4. The completed molecule was purified by reverse phase HPLC, verified by electrospray mass spectrometry and lyophilized to a powder.

### Method S93

Compounds were synthesized on commercially available Fmoc- L- asparagine(Trt)- Wang resin (0.5 mmol/g). The Fmoc group was removed by Method G19. The product of Method G65, with the exception that it was not resolved by Method G67, was Fmoc protected by Method G5 and the t-butyl ester removed by Method G11. This product was coupled to the resin by Method G20. The Fmoc group was removed by Method G19 and the appropriate carboxylic acid (R) was coupled by Method G20. The completed molecule was worked up by Method G21.

### Method S94

Compounds were synthesized on commercially available Fmoc- L- asparagine(Trt)- Wang resin (0.5 mmol/g). The Fmoc group was removed by Method G19. The Method S isomer of Method G65 was Fmoc protected by Method G5 and the t-butyl ester removed by Method G11. This product was coupled to the resin by Method G20. The Fmoc group was removed by Method G19 and the appropriate carboxylic acid (R) was coupled by Method G20. The completed molecule was worked up by Method G21.

### Method S95

Compounds were synthesized on commercially available Fmoc- L- alanine- Wang resin (0.5 mmol/g). The Fmoc group was removed by Method G19. The S isomer of Method G65 was Fmoc protected by Method G5 and the t-butyl ester removed by Method G11. This product was coupled to the resin by Method G20. The Fmoc group was removed by Method G19 and the appropriate carboxylic acid (R) was coupled by Method G20. The completed molecule was worked up by Method G21.

### Method S96

Compounds were synthesized using standard Fmoc solid phase methods on commercially available Fmoc- L- tryptophan(Boc)- Wang resin (0.5 mmol/g). The Fmoc group was cleaved by Method G19. The appropriate commercially available di- acid (R) coupled by Method G20. 3-hydroxy benzylamine, Method G38, was coupled by Method G20. The completed molecule was worked up by Method G21 and correct stereochemistry was assigned by activity.

### Method S97

Compounds were synthesized using standard Fmoc solid phase methods on commercially available Fmoc- L- asparagine(Trt)- Wang resin (0.5 mmol/g). The Fmoc group was cleaved by Method G19. The appropriate commercially available di- acid (R) coupled by Method G20. 3-hydroxy benzylamine, Method G38, was coupled by Method G20. The completed molecule was worked up by Method G21 and correct stereochemistry was assigned by activity.

### Method S98

The product of Method G86 was coupled by Method G20 to the appropriate commercially available Fmoc- amino acid Wang resin (R) after removing the Fmoc group by Method G19. The completed molecule was worked up by Method G21 and correct stereochemistry was assigned by activity.

### Method S99

3- hydroxymandelic acid was converted to its corresponding alcohol by Method G25 and coupled to the methyl ester of 4- hydroxy 2- chlorobenzoic acid, Method G15, by Method G26. The methyl ester removed by Method G4 and the carboxylic acid was coupled to L- asparagine t- butyl ester by Method G3. The final *t*- butyl ester was removed by Method G11 and the molecule was purified by reverse phase HPLC, verified by electrospray mass spectrometry and lyophilized to a powder.

### Method S100

3- hydroxymandelic acid was converted to its corresponding alcohol by Method G25 and coupled to the methyl ester of 4- hydroxy 2- chlorobenzoic acid, Method G15, by Method G26. The methyl ester removed by Method G4 and the carboxylic acid was coupled to L- alanine t- butyl ester by Method G3. The final t- butyl ester was removed by Method G11 and the molecule was purified by reverse phase HPLC, verified by electrospray mass spectrometry and lyophilized to a powder.

### Method S101

The methyl ester of 3-(3-hydroxyphenyl) propionic acid was made by Method G15 and converted to the aldehyde by Method G29. The oxazoline of 4- bromo 2-chloro benzoic acid was made by Method G30. The aldehyde was coupled to the bromide by Method G31 and the oxazoline converted to the ethyl ester by Method G32. After saponification by Method G4, the carboxylic acid was coupled to L- alanine methyl ester by Method G3. After saponification by Method G4, the molecule was purified by reverse phase HPLC, verified by electrospray mass spectrometry and lyophilized to a powder.

### Method S102

The methyl ester of 3-(3-hydroxyphenyl) propionic acid was made by Method G15 and converted to the aldehyde by Method G29. The oxazoline of 4- bromo 2-chloro benzoic acid was made by Method G30. The aldehyde was coupled to the bromide by Method G31 and the oxazoline converted to the ethyl ester by Method G32. The allylic alcohol was oxidized to the ketone by Method G27 and the ethyl ester was saponified by Method G4. The carboxylic acid was coupled to L- alanine methyl ester by Method G3; and after saponification by Method G4, the molecule was purified by reverse phase HPLC, verified by electrospray mass spectrometry and lyophilized to a powder.

### Method S103

The methyl ester of 4-hydroxy-2-chloro-benzoic acid was formed by Method G15. 1, 2-dibromoethane was coupled to the phenol by Method G51. The appropriate hydroxy phenol (R) was coupled by Method G52 and the methyl ester removed by Method G4. L- alanine- O-*t*-butyl ester was coupled be Method G3. The t-butyl ester was removed by Method G11 and the completed compound was purified by reverse phase HPLC, verified by electrospray mass spectrometry and lyophilized to a powder.

### Method S104

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the phenol by Method G47 and then the methyl ester removed by Method G48. The resulting carboxylic acid was then converted to its allyl ester by Method G49 (Compound G). 3- hydroxymandelic acid was converted to its corresponding alcohol by Method G25 and coupled to the phenol (Compound G) by Method G26. And the allyl ester removed by Method G50. The resulting benzoic acid was coupled to commercially available L-asparagine-O-*t*-butyl ester by Method G3. The *t*-butyl ester was removed by Method G11 without TES. The completed molecule was then concentrated *in vacuo,* purified by reverse phase HPLC, verified by electrospray mass spectrometry and lyophilized to a powder.

### Method S105

4-amino-2,6-dichlorophenol was Boc protected by Method G44 and the phenol converted to its corresponding triflate by Method G45. The triflate was converted to a carboxylic acid methyl ester by Method G46. The Boc aniline was converted to the phenol by Method G47 and then the methyl ester removed by Method G48. The resulting carboxylic acid was then converted to its allyl ester by Method G49 (Compound G). 1, 3-dibromopropane was coupled to the phenol (Compound G) by Method G51. The 3-hydroxy phenol was coupled by Method G52 and the methyl ester removed by Method G4. The allyl ester removed by Method G50. The resulting benzoic acid was coupled to commercially available L-asparagine-O-*t*-butyl ester by Method G3. The *t*-butyl ester was removed by Method G11 without TES. The completed molecule was then concentrated *in vacuo,* purified by reverse phase HPLC, verified by electrospray mass spectrometry and lyophilized to a powder.

### Method S106

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the phenol by Method G47 and then the methyl ester removed by Method G48. The resulting carboxylic acid was then converted to its allyl ester by Method G49 (Compound G). 1, 2-dibromopropane was coupled to the phenol (Compound G) by Method G51. The 3-hydroxy phenol was coupled by Method G52 and the methyl ester removed by Method G4. The allyl ester removed by Method G50. The resulting benzoic acid was coupled to commercially available L-asparagine-O-t-butyl ester by Method G3. The *t*-butyl ester was removed by Method G11 without TES. The completed molecule was then concentrated *in vacuo,* purified by reverse phase HPLC, verified by electrospray mass spectrometry and lyophilized to a powder.

### Method S107

4-amino-2, 6-dichlorophenol was Boc protected by Method G44 and the phenol converted to its corresponding triflate by Method G45. The triflate was converted to a carboxylic acid methyl ester by Method G46. The Boc aniline was converged to the phenol by Method G47 and then the methyl ester removed by Method G48. The resulting carboxylic acid was then converted to its allyl ester by Method G49 (Compound G). 1, 2-dibromopropane was coupled to the phenol (Compound G) by Method G51. The 3-hydroxy phenol was coupled by Method G52 and the methyl ester removed by Method G4. The allyl ester removed by Method G50. The resulting benzoic acid was coupled to commercially available L-alanine-O-*t*-butyl ester by Method G3. The *t*-butyl ester was removed by Method G11 without TES. The completed molecule was then concentrated *in vacuo,* purified by reverse phase HPLC, verified by electrospray mass spectrometry and lyophilized to a powder.

### Method S108

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54 and then the methyl ester removed by Method G55. This benzoic acid was then coupled to L-asparagine-O-*t-*butyl ester by Method G3. 3-hydroxybenzoic acid was converted to the hydroxamate by Method G56. The hydroxyl was protected as the t-butyl ether by Method G10 and the hydroxamate converted to the aldehyde by Method G57. The aldehyde was coupled to ethynyl magnesium bromide by Method G58 and the resulting product coupled to the above aryl iodide by Method G59. The alkyne was then reduced to the alkane by Method G60. The t-butyl ester and ether were removed by Method G11 without TES. The completed molecule was then concentrated *in vacuo,* purified by reverse phase HPLC, verified by electrospray mass spectrometry and lyophilized to a powder.

### Method S109

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54 and then the methyl ester removed by Method G55. This benzoic acid was then coupled to L-asparagine-O-*t-*butyl ester by Method G3. 3-hydroxybenzoic acid was converted to the hydroxamate by Method G56. The hydroxyl was protected as the t-butyl ether by Method G10 and the hydroxamate converted to the aldehyde by Method G57. The aldehyde was coupled to ethynyl magnesium bromide by Method G58 and the resulting product coupled to the above aryl iodide by Method G59. The alkyne was then reduced to the alkane by Method G60. The *t*-butyl ester and ether were removed by Method G11. The completed molecule was then concentrated *in vacuo,* purified by reverse phase HPLC, verified by electrospray mass spectrometry and lyophilized to a powder.

### Method S110

3, 5-dimethyl-4-hydroxybenzaldehyde was coupled to ethynyl magnesium bromide by Method G58 and this product was coupled to 3-iodoanisole by Method G59. The alkynol was hydrogenated to the alkane by Method G38 except the product was purified by silica flash chromatography (3/6/1 hexane/DCM/Et₂O) to provide pure aryl alcohol. The alcohol was silyl protected by Method G18. The phenol converted to its corresponding triflate by Method G45. The triflate was converted to a carboxylic acid methyl ester by Method G46. The methyl ether and ester were removed by Method G55. The acid was coupled to L-asparagine-O-*t*-butyl ester by Method G3. The t-butyl ester was removed by Method G11 without TES and the silyl ether was removed in the same reaction by adding 3 equivalents of TBAF. The completed molecule was then concentrated *in vacuo,* purified by reverse phase HPLC, verified by electrospray mass spectrometry and lyophilized to a powder.

### Method S111

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54 and then the methyl ester removed by Method G55. This benzoic acid was then coupled to L-asparagine-O-*t-*butyl ester by Method G3. 3'-Hydroxyacetophenone was converted to the *t*-butyl ether using Method G10. G58 resulting alkyne coupled to the aryl iodide using Method G59. The alkyne was hydrogenated to the alkane using Method G60. Reductive removal of the benzylic alcohol, as well as cleavage of the t-butyl ether and ester groups was accomplished using Method G11 (containing excess TES). The crude product was isolated by concentrating *in vacuo,* purified by reverse phase HPLC, verified by electrospray mass spectrometry and lyophilized to a powder.

### Method S112

2, 6-Dichloro-4-methyl phenol was converted to the triflate according to Method G45. This triflate was carbonylated to the methyl ester using Method G46 and then converted to the aldehyde by Method G84. The aldehyde was treated with ethynyl magnesium bromide by Method G58 and the resulting alkyne coupled to 3-iodophenol using Method G59. The alkyne was hydrogenated to the alkane using Method G60 and the methyl ester was cleaved using Method G55. The resulting carboxylic acid was coupled to L-asparagine-O-f-butyl ester using Method G3. Cleavage of the *t-*butyl ester group was accomplished using Method G11 (containing no TES). The crude product was isolated by concentrating *in vacuo,* purified by reverse phase HPLC, verified by electrospray mass spectrometry and lyophilized to a powder.

### Method S113

2, 6-Dichloro-4-methyl phenol was converted to the triflate according to Method G45. This triflate was carbonylated to the methyl ester using Method G46 and then converted to the aldehyde by Method G84. 3-Iodophenol was silylated according to Method G18 to give O-t-butyldimethylsilyl-3-iodophenol. The aldehyde was treated with ethynyl magnesium bromide by Method G58 and the resulting alkyne coupled to O-*t*-butyl-dimethylsilyl-3-iodophenol using Method G59. The alkyne was hydrogenated to the alkane using Method G60. The resulting alcohol was converted to the methyl ether by Method G85 and the methyl ester was cleaved using Method G55. The resulting carboxylic acid was coupled to L- asparagine O-*t*-butyl ester using Method G3. The *t*-butyl ester was removed by Method G11 without TES and the silyl ether was removed in the same reaction by adding 3 equivalents of TBAF. The crude product was isolated by concentrating *in vacuo,* purified by reverse phase HPLC, verified by electrospray mass spectrometry and lyophilized to a powder.

### Method S114

2, 6-Dichloro-4-methyl phenol was converted to the triflate according to Method G45. This triflate was carbonylated to the methyl ester using Method G46 and then converted to the aldehyde by Method G84. 3-Iodophenol was silylated according to Method G18 to give O-*t*-butyldimethylsilyl-3-iodophenol. The aldehyde was treated with ethynyl magnesium bromide by Method G58 and the resulting alkyne coupled to O-*t*-butyl-dimethylsilyl-3-iodophenol using Method G59. The alkyne was hydrogenated to the alkane using Method G60 and the methyl ester was cleaved using Method G55. The resulting carboxylic acid was coupled to N-β-alloc-L-α, β-diaminopropionic acid methyl ester using Method G3 (adding an equivalent of DIPEA). The silyl ether was removed by Method G11 without TES with the addition of 3 equivalents of TBAF. The methyl ester was saponified using Method G4. The crude product was isolated by concentrating *in vacuo,* purified by reverse phase HPLC, verified by electrospray mass spectrometry and lyophilized to a powder.

### Method S115

2, 6-Dichloro-4-methyl phenol was converted to the triflate according to Method G45. This triflate was carbonylated to the methyl ester using Method G46 and then converted to the aldehyde by Method G84. 3-Iodophenol was silylated according to Method G18 to give O-*t*-butyl-dimethylsflyl-3-iodophenol. The aldehyde was treated with ethynyl magnesium bromide by Method G58 and the resulting alkyne coupled to O-*t*-butyl-dimethylsilyl-3-iodophenol using Method G59. The alkyne was hydrogenated to the alkane using Method G60 and the methyl ester was cleaved using Method G55. The resulting carboxylic acid was coupled to N-ε-Boc-L-lysine methyl ester using Method G3 (adding an equivalent of DIPEA). The methyl ester was saponified using Method G4 and the Boc group was removed by Method G11 without TES and the silyl ether was removed in the same reaction by adding 3 equivalents of TBAF The crude product was isolated by concentrating *in vacuo,* purified by reverse phase HPLC, verified by electrospray mass spectrometry and lyophilized to a powder.

### Method S116

3-Hydroxybenzoic acid was converted to the N-methoxy-N-methylamide using Method G56. The hydroxyl was protected as the t-butyl ether by Method G10. The N-methoxy-N-methylamide was reduced to the aldehyde by Method G57. The aldehyde was treated with ethynyl magnesium bromide by Method G58. 4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54. The resulting aryl iodide was then coupled to the above alkyne by Method G59. The alkyne was hydrogenated to the alkane using Method G60. The methyl ester was cleaved using Method G55. The carboxylic acid was coupled to N-β-alloc-L-α, β-diaminopropionic acid methyl ester using Method G3 (adding an equivalent of DIPEA). The methyl ester was saponified using Method G4. The *t*-butyl ether was cleaved by using Method G11 (containing no TES). The crude product was isolated by concentrating *in vacuo,* purified by reverse phase HPLC, verified by electrospray mass spectrometry and lyophilized to a powder.

### Method S117

3-Hydroxybenzoic acid was converted to the N-methoxy-N-methylamide using Method G56. The hydroxyl was protected as the t-butyl ether by Method G10. The N-methoxy-N-methylamide was reduced to the aldehyde by Method G57. The aldehyde was treated with ethynyl magnesium bromide by Method G58. 4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54. The resulting aryl iodide was then coupled to the above alkyne by Method G59. The alkyne was hydrogenated to the alkane using Method G60. The resulting alcohol was converted to the methyl ether by Method G85 and the methyl ester was cleaved using Method G55. The resulting carboxylic acid was coupled to L- asparagine-O-t-butyl ester using Method G3. Cleavage of the t-butyl ester group was accomplished using Method G11 (containing no TES). The crude product was isolated by concentrating *in vacuo,* purified by reverse phase HPLC, verified by electrospray mass spectrometry and lyophilized to a powder.

### Method S118

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54. 3-Chlorobenzaldehyde was treated with ethynyl magnesium bromide by Method G58, and the resulting alkyne coupled to the above aryl iodide by Method G59. The alkyne was hydrogenated to the alkane using Method G60. The methyl ester was cleaved using Method G55. The carboxylic acid was coupled to N-β-alloc-L-α, β-diaminopropionic acid methyl ester using Method G3 (adding an equivalent of DIPEA). The methyl ester was saponified using Method G4. The crude product was isolated by concentrating *in vacuo,* purified by reverse phase HPLC, verified by electrospray mass spectrometry and lyophilized to a powder.

### Method S119

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54. 3-Chlorobenzaldehyde was treated with ethynyl magnesium bromide by Method G58, and the resulting alkyne coupled to the above aryl iodide by Method G59. The alkyne was hydrogenated to the alkane using Method G60. The methyl ester was removed by Method G55 and the resulting acid coupled by Method G20 to commercially available β- Boc- diaminopropionic acid methyl ester. The Boc group was removed by Method G1 and thiophene 2- carboxylic acid was coupled by Method G3. After saponification, Method G4, the molecule was purified by reverse phase HPLC, verified by electrospray mass spectrometry and lyophilized to a powder.

### Method S120

3-Hydroxybenzoic acid was converted to the N-methoxy-N-methylamide using Method G56. The hydroxyl was protected as the t-butyl ether by Method G10. The N-methoxy-N-methylamide was reduced to the aldehyde by Method G57. The aldehyde was treated with ethynyl magnesium bromide by Method G58. 4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54. The resulting aryl iodide was then coupled to the above alkyne by Method G59. The alkyne was hydrogenated to the alkane using Method G60. The methyl ester was removed by Method G55 and the resulting acid coupled by Method G20 to commercially available β- Boc-diaminopropionic acid methyl ester. The Boc group was removed by Method G1 and thiophene 2-carboxylic acid was coupled by Method G3. After saponification, Method G4, the molecule was purified by reverse phase HPLC, verified by electrospray mass spectrometry and lyophilized to a powder.

### Method S121

4-amino-2, 6-dichlorophenol was Boc protected by Method G44 and the phenol converted to its corresponding triflate by Method G45. The triflate was converted to a carboxylic acid methyl ester by Method G46. The Boc aniline was converted to the iodide by Method G54. 3-Chlorobenzaldehyde was treated with ethynyl magnesium bromide by Method G58, and the resulting alkyne coupled to the above aryl iodide by Method G59. The alkyne was hydrogenated to the alkane using Method G60. The methyl ester was removed by Method G55 and the resulting acid coupled by Method G20 to commercially available N-ε-Boc-L-lysine methyl ester using Method G3 (adding an equivalent of DIPEA). The methyl ester was saponified using Method G4 and the Boc group was removed by Method G11 (containing no TES). The crude product was isolated by concentrating *in vacuo,* purified by reverse phase HPLC, verified by electrospray mass spectrometry and lyophilized to a powder.

### Method S122

Compounds were synthesized using standard Fmoc solid phase methods on commercially available Fmoc- glycine- Wang resin (0.5 mmol/g). The Fmoc group was cleaved by Method G19. The α glycine α carbon was alkylated with the appropriate commercially available biomide or chloride by Method G36 resulting in the corresponding racemic amino acid. Compound E was coupled to the resin by Method G37 and the completed molecule was worked up by Method G21.

### Method S123

Compounds were synthesized using standard Fmoc solid phase methods on Fmoc- L-Aspartic acid (allyl)- p- alkoxybenzyl alcohol resin (0.5 mmol/g). The resin was made by Method G34 using commercially available N-α-Fmoc-β-Allyl-L-aspartic acid. The Fmoc group was cleaved by Method G19. Compound E was coupled to the resin by Method G37. The Allyl group was removed by Method G39. The appropriate aniline (R) was coupled by Method G40. The completed molecule was worked up by Method G21.

### Method S124

Compounds were synthesized using standard Fmoc solid phase methods on Fmoc- L-aspartic acid (allyl)- p- alkoxybenzyl alcohol resin (0.5 mmol/g) (Fmoc- L- asp(alloc)- Wang resin). The resin was made by Method G34 using commercially available N-α-Fmoc-β-Allyl-L-aspartic acid. The Fmoc group was cleaved by Method G19. Compound C, Method G13, was coupled by Method G20. The allyl group was removed by Method G39. The appropriate amine (R) was coupled by Method G41. The completed molecule was worked up by Method G21.

### Method S125

Compounds were synthesized using standard Fmoc solid phase methods on Fmoc- L-glutamic acid (allyl)- p- alkoxybenzyl alcohol resin (0.5 mmol/g) (Fmoc- L- glu(alloc)- Wang resin). The resin was made by Method G34 using commercially available N-α-Fmoc-β-Allyl-L-glutamic acid. The Fmoc group was cleaved by Method G19. Compound C, Method G13, was coupled by Method G20. The allyl group was removed by Method G39. The appropriate amine (R) was coupled by Method G41. The completed molecule was worked up by Method G21.

### Method S126

Compounds were synthesized using standard Fmoc solid phase methods on N-α-Fmoc-O-trityl-L-serine-p- alkoxybenzyl alcohol resin (0.5 mmol/g) (Fmoc- L- Ser(trityl)- Wang resin). The resin was made by Method G34 using commercially available N-α-Fmoc-O-trityl-L-serine. The Fmoc group was cleaved by Method G19. Compound C, Method G13, was coupled by Method G20. The trityl group was removed by Method G72. The appropriate amine (R) was coupled by Method G73. The completed molecule was worked up by Method G21.

### Method S127

Compounds were synthesized using standard Fmoc solid phase methods on N-α-Fmoc-O-trityl-L-threonine-p- alkoxybenzyl alcohol resin (0.5 mmol/g) (Fmoc- L- thr(trityl)- Wang resin). The resin was made by Method G34 using commercially available N-α-Fmoc-O-trityl-L-serine. The Fmoc group was cleaved by Method G19. Compound C, Method G13, was coupled by Method G20. The trityl group was removed by Method G72. The appropriate amine (R) was coupled by Method G73. The completed molecule was worked up by Method G21.

### Examples 1-39

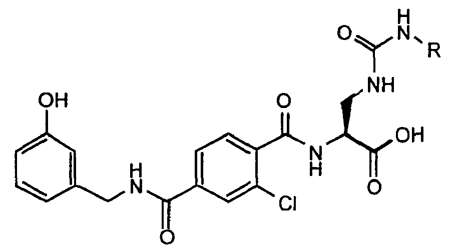

Examples 1-39 were synthesized by Method S1.

| Example # | R group |
|---|---|
| 1 | 2-isopropylphenyl isocyanate |
| 2 | phenethyl isocyanate |
| 3 | 1-napthyl isocyanate |
| 4 | (S)-(-)-a-methylbenzyl isocyanate |
| 5 | cyclohexyl isocyanate |
| 6 | ethoxycarbonyl isocyanate |
| 7 | isopropyl isocyanate |
| 8 | trans-2-phenylcyclopropyl isocyanate |
| 9 | 1-adamantyl isocyanate |
| 10 | phenyl isocyante |
| 11 | 4-(methylthio)phenyl isocyanate |
| 12 | 3-(methylthio)phenyl isocyanate |
| 13 | 3-ethoxycarbonylphenyl isocyanate |
| 14 | 4-ethoxycarbonylphenyl isocyanate |
| 15 | 4-fluorophenyl isocyanate |
| 16 | 2-fluorophenyl isocyanate |
| 17 | 2-(trifluoromethoxy)phenyl isocyanate |
| 18 | 3-fluorophenyl isocyanate |
| 19 | 3-bromophenyl isocyanate |
| 20 | 4-methoxyphenyl isocyanate |
| 21 | 4-isopropylphenyl isocyanate |
| 22 | 3-(2-hydroxy)ethyl phenyl isocyanate |
| 23 | 4-ethylphenyl isocyanate |
| 24 | 2-nitrophenyl isocyanate |
| 25 | 3-nitrophenyl isocyanate |
| 26 | 4-nitrophenyl isocyanate |
| 27 | 3-cyanophenyl isocyanate |
| 28 | 4-trifluoromethyl isocyanate |
| 29 | 3-trifluoromethyl isocyanate |
| 30 | 2-trifluoromethyl isocyanate |
| 31 | 3-methylphenyl isocyanate |
| 32 | 4-chlorophenyl isocyanate |
| 33 | 3-chlorophenyl isocyanate |
| 34 | 3-chloro-4-methylphenyl isocyanate |
| 35 | 3-ethylphenyl isocyanate |
| 36 | allyl isocyanate |
| 37 | (S)-(-)-a-methylbenzyl isocyanate |
| 38 | cyclohexyl isocyanate |
| 39 | trans-2-phenylcyclopropyl isocyanate |

### Examples 40-43

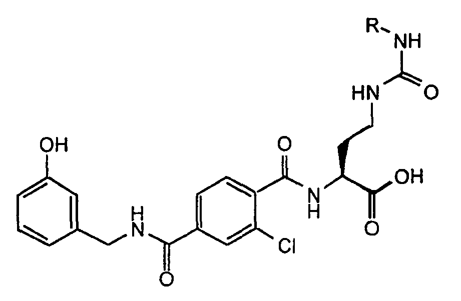

Examples 40-43 were synthesized by Method S2.

| Example # | R group |
|---|---|
| 40 | benzyl isocyanate |
| 41 | ethoxycarbonyl isocyanate |
| 42 | 2-chloro-6-methylphenyl isocyanate |
| 43 | ethoxycarbonyl isocyanate |

### Examples 44-62

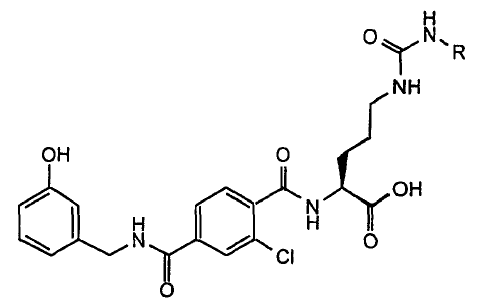

Examples 44-62 were synthesized by Method S3.

| Example # | R group |
|---|---|
| 44 | phenethyl isocyanate |
| 45 | isopropyl isocyanate |
| 46 | cyclohexyl isocyanate |
| 47 | 3-ethoxycarbonylphenyl isocyanate |
| 48 | 4-ethoxycarbonylphenyl isocyanate |
| 49 | 4-fluorophenyl isocyanate |
| 50 | 2-fluorophenyl isocyanate |
| 51 | 3-fluorophenyl isocyanate |
| 52 | 4-methoxyphenyl isocyanate |
| 53 | 4-isopropylphenyl isocyanate |
| 54 | 3-(2-hydroxyethyl)phenyl isocyanate |
| 55 | 2-nitrophenyl isocyanate |
| 56 | 4-nitrophenyl isocyanate |
| 57 | 3-cyanophenyl isocyanate |
| 58 | 3-methylphenyl isocyanate |
| 59 | 4-chlorophenyl isocyanate |
| 60 | 3-chloro-4-methylphenyl isocyanate |
| 61 | 2-chloro-6-methylphenyl isocyanate |
| 62 | 4-ethylphenyl isocyanate |

### Examples 63-71

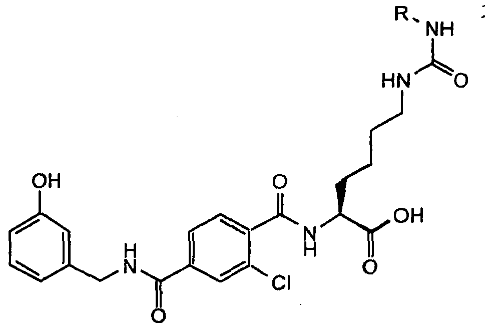

Examples 63-71 were synthesized by Method S4.

| Example # | R group |
|---|---|
| 63 | phenethyl isocyanate |
| 64 | isopropyl isocyanate |
| 65 | benzyl isocyanate |
| 66 | propyl isocyanate |
| 67 | ethoxycarbonyl isocyanate |
| 68 | ethyl 2-isocyanato-4-methylvaierate |
| 69 | (S)-(-)-a-methylbenzyl isocyanate |
| 70 | benzensulfonyl isocyanate |
| 71 | benzyl isocyanate |

### Examples 72-95

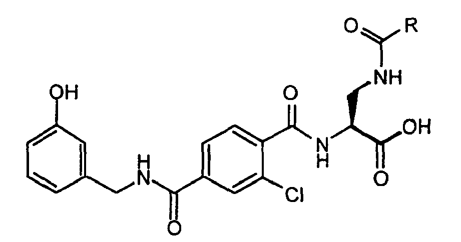

Examples 72-95 were synthesized by Method S5.

| Example # | R group |
|---|---|
| 72 | 3-methylindene-2-carboxylic acid |
| 73 | 3-methylbenzofuran-2-carboxylic acid |
| 74 | 4-Oxo-4, 5, 6, 7-tetrahydro-benzofuran-3-carboxylic acid |
| 75 | 1, 2, 5-Trimethyl-1H-pyrrole-3-carboxylic acid |
| 76 | 4-Methyl-[1, 2, 3]thiadiazole-5-carboxylic acid |
| 77 | 4-Phenyl-[1, 2, 3]thiadiazole-5-carboxylic acid |
| 78 | 3-chloro-2thiophenecarboxylic acid |
| 79 | 3, 5-Dimethyl-isoxazole-4-carboxylic acid |
| 80 | 3-methyl-2-furoic acid |
| 81 | 3-bromothiophene-2-carboxylic acid |
| 82 | 2-furoic acid |
| 83 | 3-furoic acid |
| 84 | 2-thiophene carboxylic acid |
| 85 | 3- thiophenecarboxylic acid |
| 86 | 5- chloro 2- thiophene carboxylic acid |
| 87 | 5- bromo 2- thiophene carboxylic acid |
| 88 | indole 5- carboxylic acid |
| 89 | indole 4- carboxylic acid |
| 90 | indole 6- carboxylic acid |
| 91 | benzoic acid |
| 92 | cyclohexyl carboxylic acid |
| 93 | acetic acid |
| 94 | isonipecotic acid |
| 95 | pipecolinic acid |

### Examples 96-113

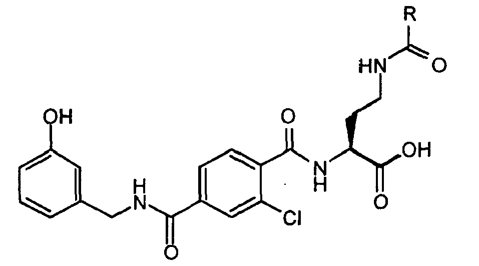

Examples 96-113 were synthesized by Method S6.

| Example # | R group |
|---|---|
| 96 | 3, 4, 5-trimethoxybenzoic acid |
| 97 | propionic acid |
| 98 | cyclopropyl carboxylic acid |
| 99 | trimethyl acetic acid |
| 100 | 1, 2, 5-Trimethyl-1H-pyrrole-3-carboxylic acid |
| 101 | 3-Chloro-4-methanesulfonyl-thiophene-2-carboxylic acid |
| 102 | 4-Methyl-[1, 2, 3]thiadiazole-5-arboxylic acid |
| 103 | 4-Phenyl-[1, 2, 3]thiadiazole-5-carboxylic acid |
| 104 | 4-Bromo-2-ethyl-5-methyl-2H-pyrazole-3-carboxylic acid |
| 105 | 3-chlorothiophene-2-carboxylic acid |
| 106 | 3, 5-Dimethyl-isoxazole-4-carboxylic acid |
| 107 | 5-Methyl-2-phenyl-2H-[1, 2,3]triazole-4-carboxylic acid |
| 108 | 3-methyl-2-furoic acid |
| 109 | 3-bromothiophene-2-carboxylic acid |
| 110 | benzoic acid |
| 111 | cyclohexyl carboxylic acid |
| 112 | acetic acid |
| 113 | none |

### Examples 114-126

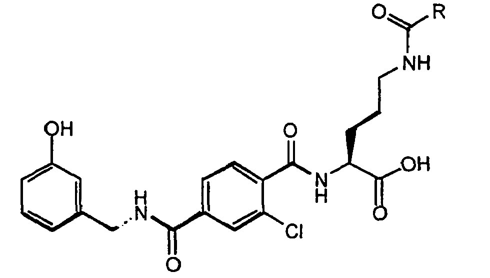

Examples 114-126 were synthesized by Method S7.

| Example # | R group |
|---|---|
| 114 | trimethyl acetic acid |
| 115 | 3-Chloro-benzo[b]thiophene-2-carboxylic acid |
| 116 | 3-chlorothiophene-2-carboxylic add |
| 117 | 3, 5-Dimethyl-isoxazole-4-carboxylic acid |
| 118 | 3-bromothiophene-2-carboxylic acid |
| 119 | 3-methylindene-2-carboxylic acid |
| 120 | 4-Oxo-4, 5, 6, 7-tetrahydro-benzofuran-3-carboxylic acid |
| 121 | 3-Chloro-4-methanesulfonyl-thiophene-2-carboxylic acid |
| 122 | 4-Methyl-[1, 2, 3]thiadiazole-5-carboxylic acid |
| 123 | 4-Bromo-2-ethyl-5-methyl-2H-pyrazole-3-carboxylic acid |
| 124 | benzoic acid |
| 125 | cyclohexane carboxylic acid |
| 126 | acetic acid |

### Examples 127-144

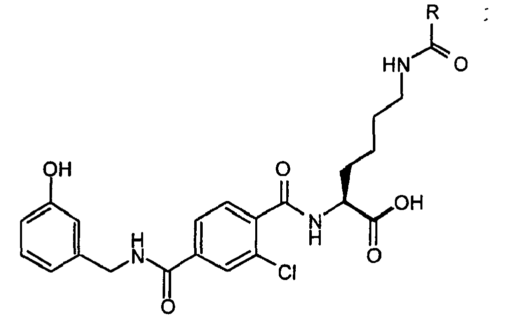

Examples 127-144 were synthesized by Method S8.

| Example # | R group |
|---|---|
| 127 | 3, 4, 5-trimethoxybenzoic acid |
| 128 | isovaleric acid |
| 129 | propionic acid |
| 130 | cyclopropyl carboxylic acid |
| 131 | 4-acetyl-3, 5-dimethyl-2-pyrrolecarboxylic acid |
| 132 | 3-methylindene-2-carboxylic acid |
| 133 | 4-Oxo-4, 5, 6, 7-tetrahydro-benzofuran-3-carboxylic acid |
| 134 | 1, 2, 5-Trimethyl-1H-pyrrole-3-carboxylic acid |
| 135 | 3-Chloro-4-methanesulfonyl-thiophene-2-carboxylic acid |
| 136 | 4-Methyl-[1, 2, 3]thiadiazole-5-carboxylic acid |
| 137 | 4-Phenyl-[1, 2, 3]thiadiazole-5-carboxylic acid |
| 138 | 4-Bromo-2-ethyl-5-methyl-2H-pyrazole-3-carboxylic acid |
| 139 | 3-chlorothiophene-2-carboxylic acid |
| 140 | 3, 5-Dimethyl-isoxazole-4-carboxylic acid |
| 141 | 5-Methyl-2-phenyl-2H-[1, 2, 3]triazole-4-carboxylic acid |
| 142 | 3-bromothiophene-2-carboxylic acid |
| 143 | benzoic acid |
| 144 | cyclohexyl carboxylic acid |

### Examples 145-147

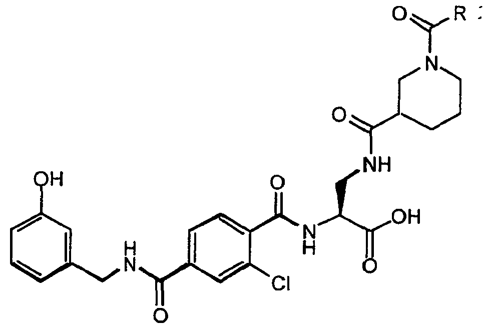

Examples 145-147 were synthesized by Method S9.

| Example # | R group |
|---|---|
| 145 | propionic acid |
| 146 | acetic acid |
| 147 | none |

### Examples 148-150

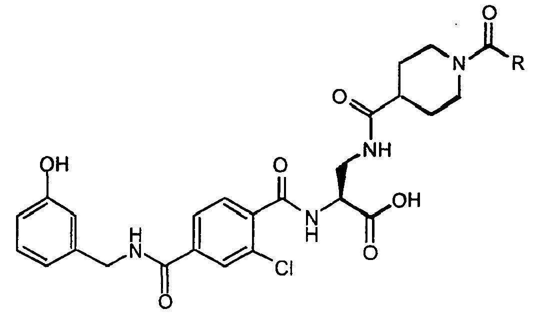

Examples 148-150 were synthesized by Method S10.

| Example # | R group |
|---|---|
| 148 | propionic acid |
| 149 | butyric acid |
| 150 | acetic acid |

### Examples 151-154

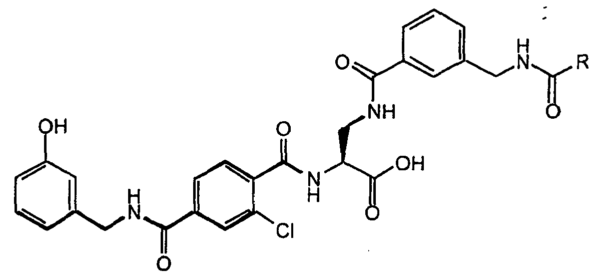

Examples 151-154 were synthesized by Method S11.

| Example # | R group |
|---|---|
| 151 | propionic acid |
| 152 | butyric acid |
| 153 | acetic acid |
| 154 | none |

### Examples 155-158

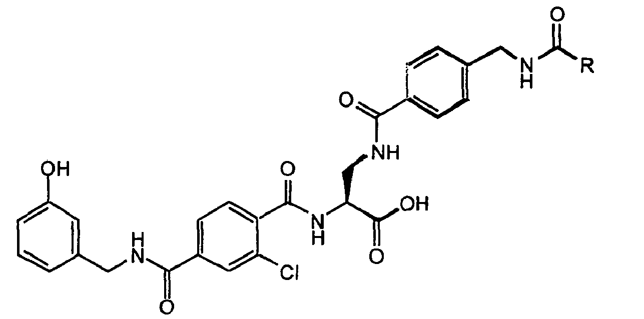

Examples 155-158 were synthesized by Method S12.

| Example # | R group |
|---|---|
| 155 | propionic acid |
| 156 | butyric acid |
| 157 | acetic acid |
| 158 | none |

### Examples 159-161

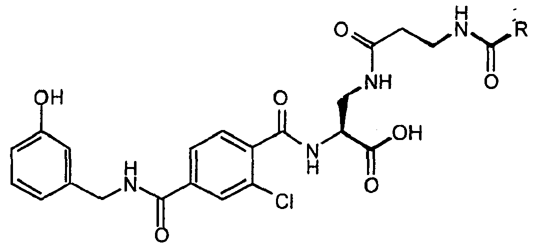

Examples 159-161 were synthesized by Method S13.

| Example # | R group |
|---|---|
| 159 | propionic acid |
| 160 | acetic acid |
| 161 | none |

### Examples 162-163

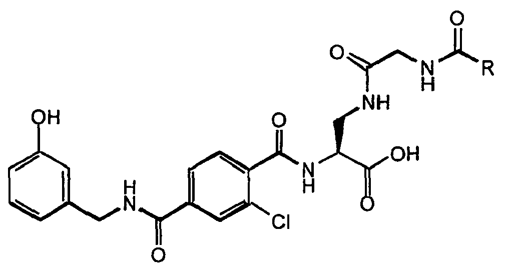

Examples 162-163 were synthesized by Method S14.

| Example # | R group |
|---|---|
| 162 | acetic acid |
| 163 | none |

### Examples 164-167

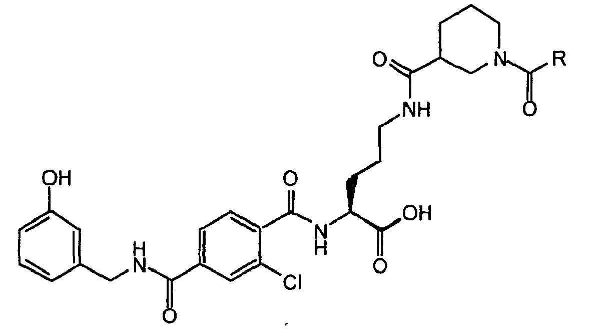

Examples 164-167 were synthesized by Method S15.

| Example # | R group |
|---|---|
| 164 | propionic acid |
| 165 | butyric acid |
| 166 | acetic acid |
| 167 | none |

### Examples 168-171

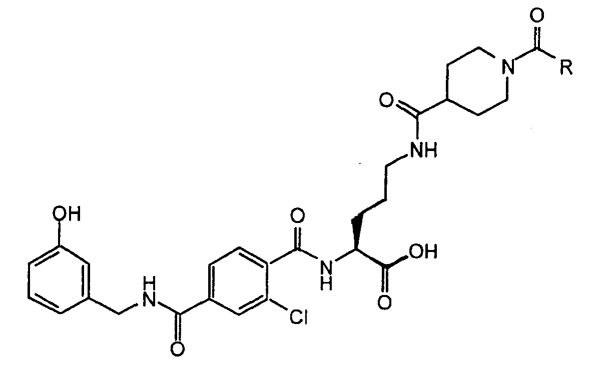

Examples 168-171 were synthesized by Method S16.

| Example # | R group |
|---|---|
| 168 | propionic acid |
| 169 | butyric acid |
| 170 | acetic acid |
| 171 | none |

### Example 172

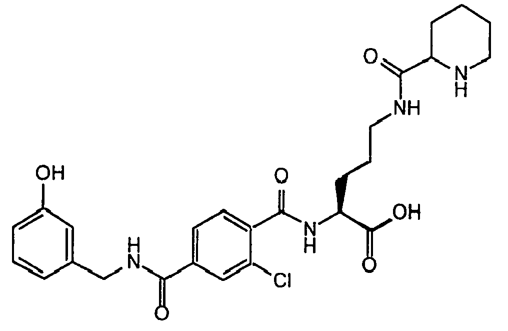

Example 172 was synthesized by Method S17.

### Examples 173-176

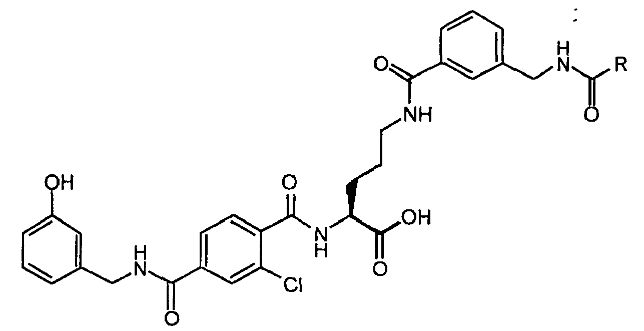

Examples 173-176 were synthesized by Method S18.

| Example # | R group |
|---|---|
| 173 | propionic acid |
| 174 | butyric acid |
| 175 | acetic acid |
| 176 | none |

### Examples 177-180

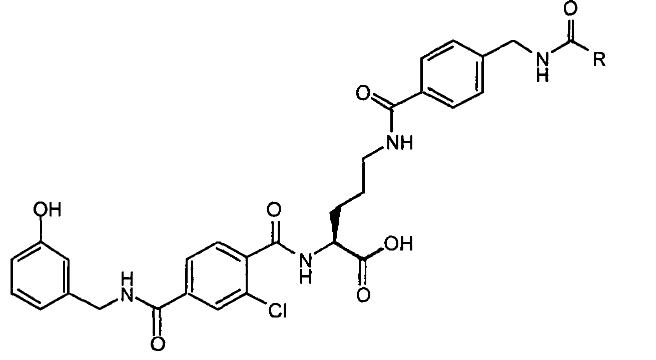

Examples 177-180 were synthesized by Method S19.

| Example # | R group |
|---|---|
| 177 | propionic acid |
| 178 | butyric acid |
| 179 | acetic acid |
| 180 | none |

### Examples 181-184

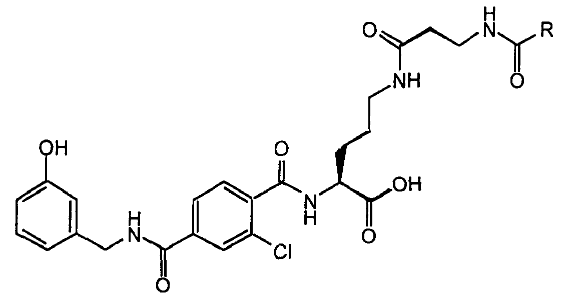

Examples 181-184 were synthesized by Method S20.

| Example # | R group |
|---|---|
| 181 | propionic acid |
| 182 | butyric acid |
| 183 | acetic acid |
| 184 | none |

### Examples 185-188

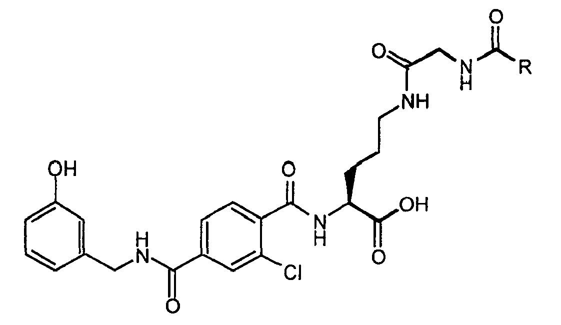

Examples 185-188 were synthesized by Method S21.

| Example # | R group |
|---|---|
| 185 | propionic acid |
| 186 | butyric acid |
| 187 | acetic acid |
| 188 | none |

### Examples 189-192

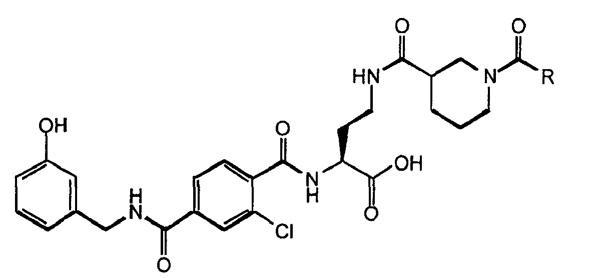

Examples 189-192 were synthesized by Method S22.

| Example # | R group |
|---|---|
| 189 | propionic acid |
| 190 | butyric acid |
| 191 | acetic acid |
| 192 | none |

### Examples 193-196

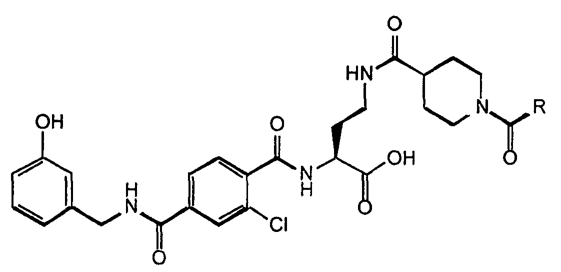

Examples 193-196 were synthesized by Method S23.

| Example # | R group |
|---|---|
| 193 | propionic acid |
| 194 | butyric acid |
| 195 | acetic acid |
| 196 | none |

### Example 197

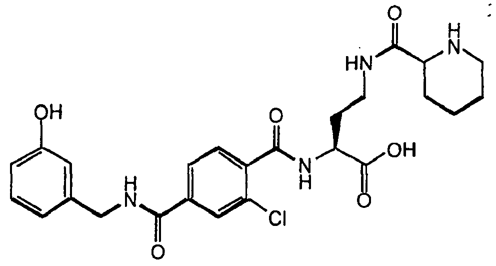

Example 197 was synthesized by Method S24.

### Examples 198-201

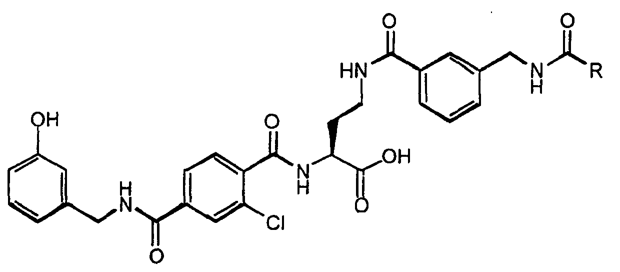

Examples 198-201 were synthesized by Method S25.

| Example # | R group |
|---|---|
| 198 | propionic acid |
| 199 | butyric acid |
| 200 | acetic acid |
| 201 | none |

### Examples 202-205

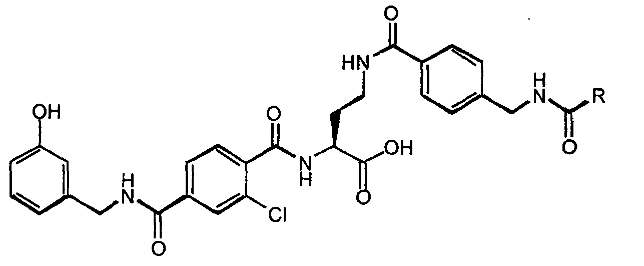

Examples 202-205 were synthesized by Method S26.

| Example # | R group |
|---|---|
| 202 | propionic acid |
| 203 | butyric acid |
| 204 | acetic acid |
| 205 | none |

### Examples 206-209

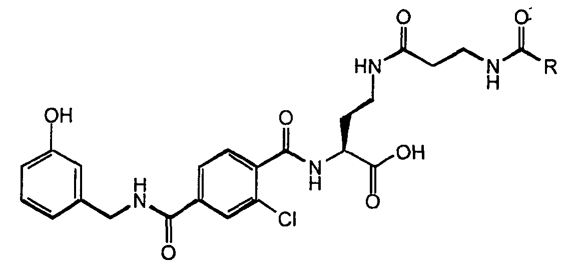

Examples 206-209 were synthesized by Method S27.

| Example # | R group |
|---|---|
| 206 | propionic acid |
| 207 | butyric acid |
| 208 | acetic acid |
| 209 | none |

### Examples 210-213

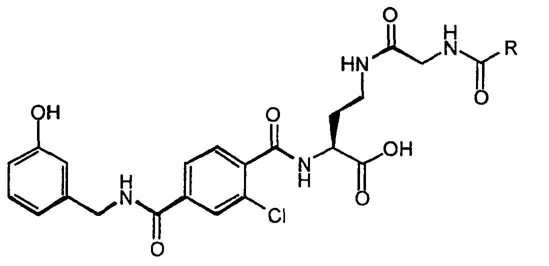

Examples 210-213 were synthesized by Method S28.

| Example # | R group |
|---|---|
| 210 | propionic acid |
| 211 | butyric acid |
| 212 | acetic acid |
| 213 | none |

### Examples 214-217

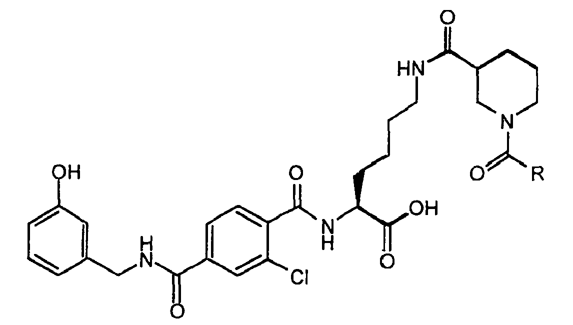

Examples 214-217 were synthesized by Method S29.

| Example # | R group |
|---|---|
| 214 | propionic acid |
| 215 | butyric acid |
| 216 | acetic acid |
| 217 | none |

### Examples 218-221

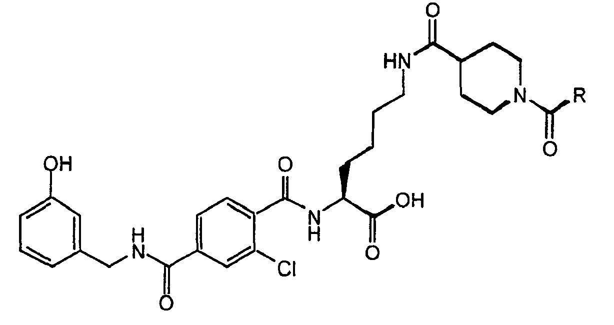

Examples 218-221 were synthesized by Method S30.

| Example # | R group |
|---|---|
| 218 | propionic acid |
| 219 | butyric acid |
| 220 | acetic acid |
| 221 | none |

### Examples 222-223

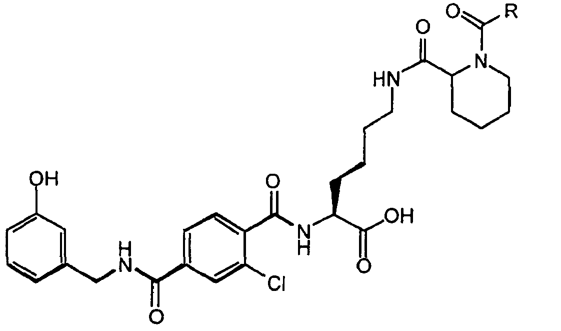

Examples 222-223 were synthesized by Method S31.

| Example # | R group |
|---|---|
| 222 | acetic acid |
| 223 | none |

### Examples 224-225

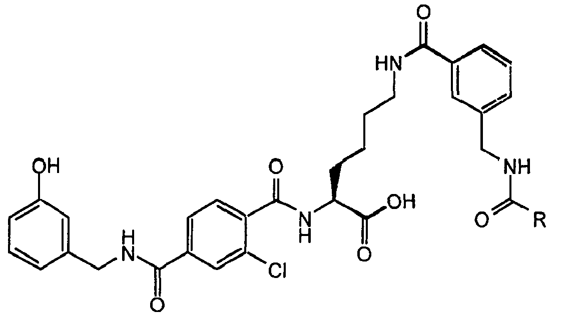

Examples 224-225 were synthesized by Method S32.

| Example # | R group |
|---|---|
| 224 | propionic acid |
| 225 | none |

### Examples 226-227

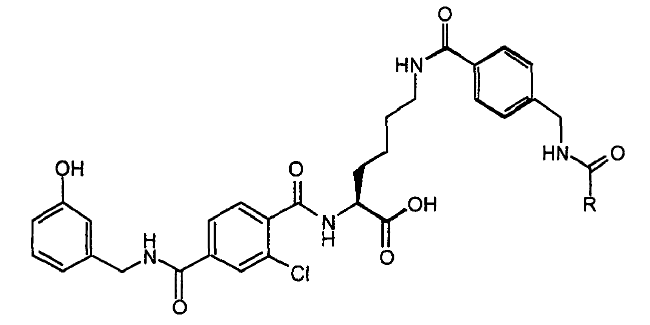

Examples 226-227 were synthesized by Method S33.

| Example # | R group |
|---|---|
| 226 | acetic acid |
| 227 | none |

### Examples 228-229

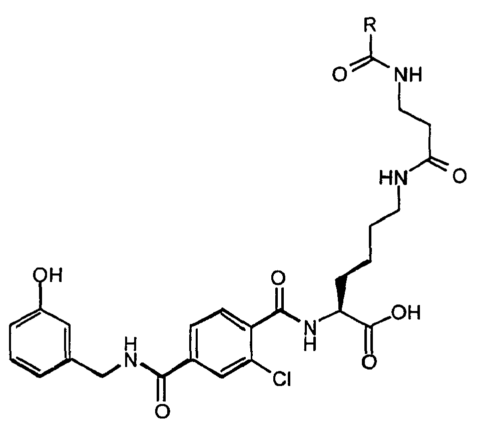

Examples 228-229 were synthesized by Method S34.

| Example # | R group |
|---|---|
| 228 | acetic acid |
| 229 | none |

### Example 230

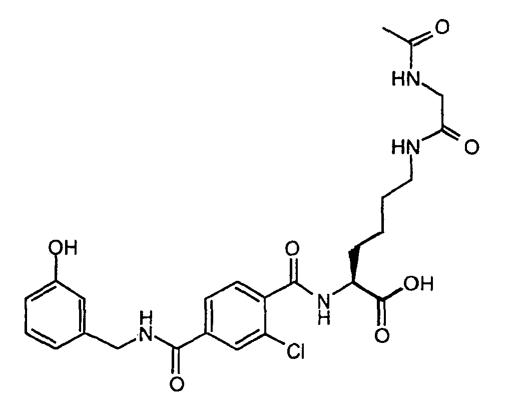

Example 230 was synthesized by Method S35.

### Examples 231-237

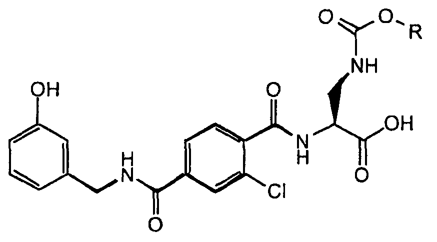

Examples 231-237 were synthesized by Method S36.

| Example # | R group |
|---|---|
| 231 | propyl chloroformate |
| 232 | benzyl chloroformate |
| 233 | isopropyl chloroformate |
| 234 | methyl chloroformate |
| 235 | ethyl chloroformate |
| 236 | butyl chloroformate |
| 237 | 3- butenyl chloroformate |

### Examples 238-240

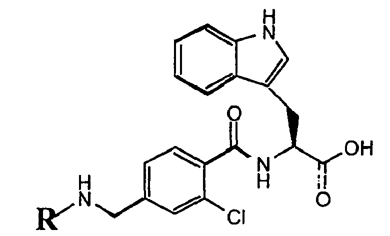

Examples 238-240 were synthesized by Method S37.

| Example # | R group |
|---|---|
| 238 | 3- hydroxy benzoic acid |
| 239 | 2- hydroxy cinnamic acid |
| 240 | 3- hydroxy benzoic acid |

### Examples 241-245

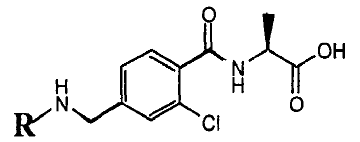

Examples 241-245 were synthesized by Method S38.

| Example # | R group |
|---|---|
| 241 | 3- hydroxy benzoic acid |
| 242 | 2- hydroxy cinnamic acid |
| 243 | 3- chloro benzoic acid |
| 244 | indole 5- carboxylic acid |
| 245 | 3- (2- thienyl)acrylic acid |

### Examples 246-253

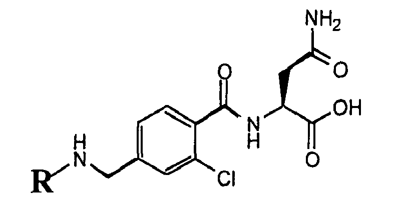

Examples 246-253 were synthesized by Method S39.

| Example # | R group |
|---|---|
| 246 | 3- chlorobenzoic acid |
| 247 | 3 - (2 - thienyl)acrylic acid |
| 248 | 2 - furanacrylic acid |
| 249 | 3- hydroxy benzoic acid |
| 250 | indole 5-carboxylic acid |
| 251 | benzofuran 5-carboxylic acid |
| 252 | benzofuran 4-carboxylic acid |
| 253 | indole 6-carboxylic acid |

### Examples 254

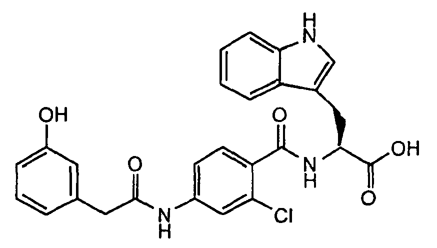

Examples 254 were synthesized by Method S40.

### Examples 255-256

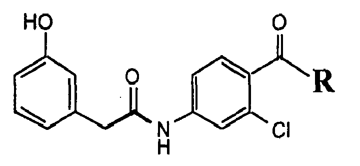

Examples 255-256 were synthesized by Method S41.

| Example # | R group |
|---|---|
| 255 | L-Ala |
| 256 | L-Thr |

### Example 257

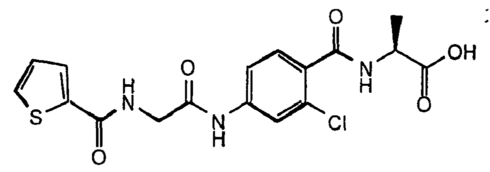

Example 257 was synthesized by Method S42.

### Examples 258-259

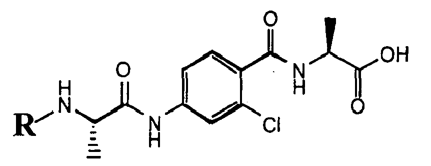

Examples 258-259 were synthesized by Method S43.

| Example # | R group |
|---|---|
| 258 | 2-thiophene carboxylic acid |
| 259 | 3-hydroxybenzoic acid |

### Examples 260-261

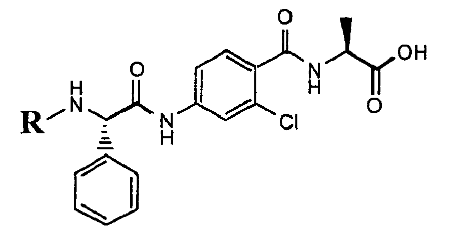

Examples 260-261 were synthesized by Method S44.

| Example # | R group |
|---|---|
| 260 | 3-hydroxybenzoic acid |
| 261 | 2-thiophene carboxylic acid |

### Examples 262-263

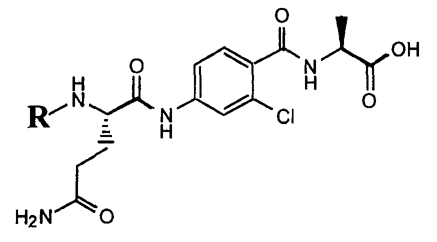

Examples 262-263 were synthesized by Method S45.

| Example # | R group |
|---|---|
| 262 | benzoic acid |
| 263 | 2-thiophene carboxylic acid |

### Examples 264-265

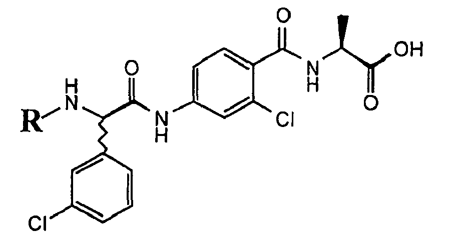

Examples 264-265 were synthesized by Method S46.

| Example # | R group |
|---|---|
| 264 | 3-hydroxybenzoic acid |
| 265 | 2-thiophene carboxylic acid |

### Examples 266-267

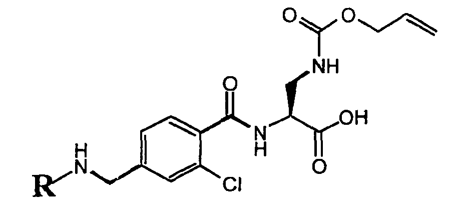

Examples 266-267 were synthesized by Method S47.

| Example # | R group |
|---|---|
| 266 | 3-(2-thienyl)-acrylic acid |
| 267 | furylacrylic acid |

### Example 268

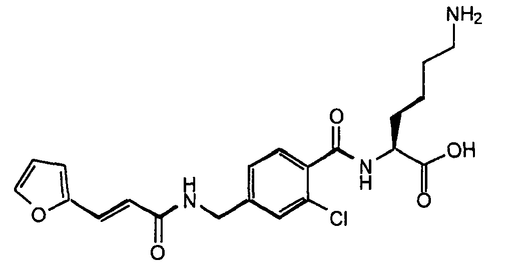

Example 268 was synthesized by Method S48.

### Example 269

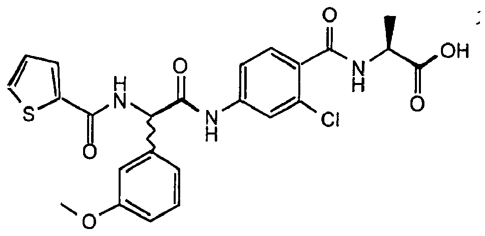

Example 269 was synthesized by Method S49.

### Examples 270-271

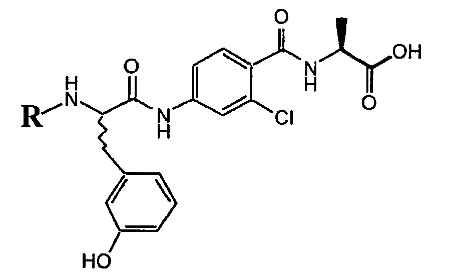

Examples 270-271 were synthesized by Method S50.

| Example # | R group |
|---|---|
| 270 | 3- hydroxybenzoic acid |
| 271 | 2- thiophene carboxylic acid |

### Example 272

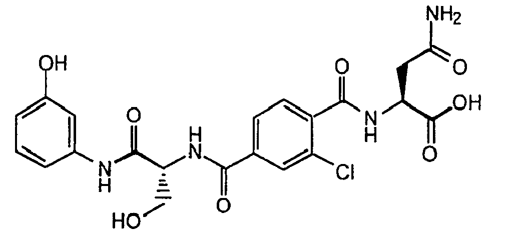

Example 272 was synthesized by Method S51.

### Examples 273-275

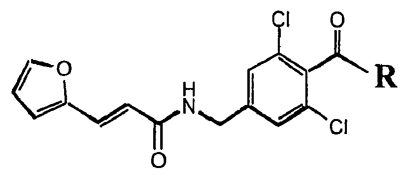

Examples 273-275 were synthesized by Method S52.

| Example # | R group |
|---|---|
| 273 | L- Ala |
| 274 | L- Asn |
| 275 | L- diaminopropionic acid (alloc) |

### Example 276

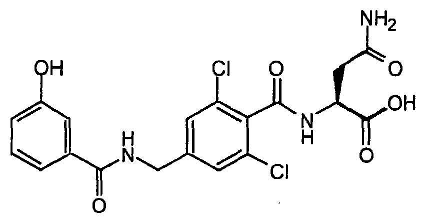

Example 276 was synthesized by Method S53.

### Examples 277-282

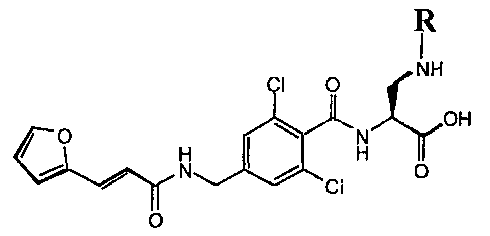

Examples 277-282 were synthesized by Method S54.

| Example # | R group |
|---|---|
| 277 | thiophene 2- carboxylic acid |
| 278 | 2- furoic acid |
| 279 | 2- pyrazinecarboxylic acid |
| 280 | 3- methyl thiophene 2- carboxylic acid |
| 281 | 3- methyl 2- furoic acid |
| 282 | 3- chloro thiophene 2- carboxylic acid |

### Example 283

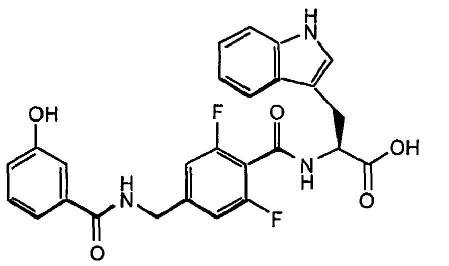

Example 283 was synthesized by Method S55.

### Examples 284-285

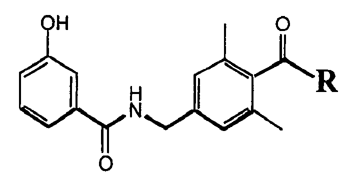

Examples 284-285 were synthesized by Method S56.

| Example # | R group |
|---|---|
| 284 | L- Ala |
| 285 | L- Asn |

### Examples 286-287

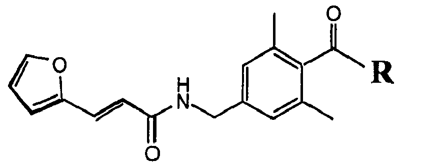

Examples 286-287 were synthesized by Method S57.

| Example # | R group |
|---|---|
| 286 | L- diaminopropionic acid (alloc) |
| 287 | L- Lys |

### Examples 288-289

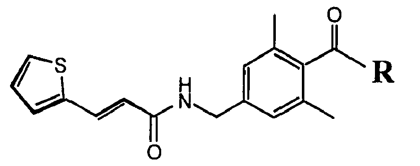

Examples 288-289 were synthesized by Method S58.

| Example # | R group |
|---|---|
| 288 | L- diaminopropionic acid (alloc) |
| 289 | L- Lys |

### Example 290

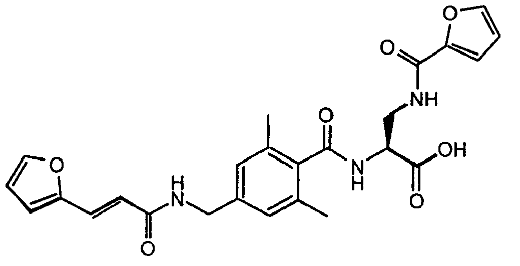

Example 290 was synthesized by Method S59.

### Examples 291-292

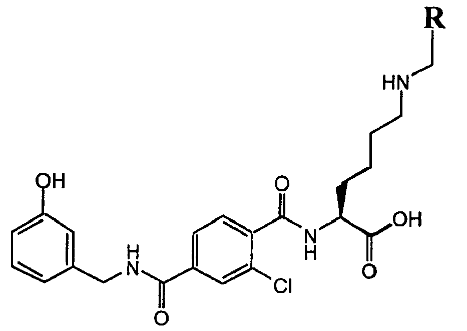

Examples 291-292 were synthesized by Method S60.

| Example # | R group |
|---|---|
| 291 | 2-furaldehyde |
| 292 | 3- methyl 2- furaldehyde |

### Examples 293-294

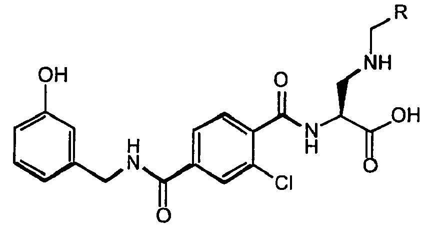

Examples 293-294 were synthesized by Method S61.

| Example # | R group |
|---|---|
| 293 | 2- furaldehyde |
| 294 | 3- methyl 2- furaldehyde |

### Examples 295-296

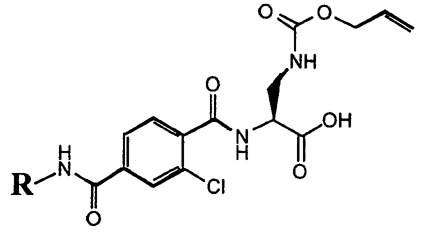

Examples 295-296 were synthesized by Method S62.

| Example # | R group |
|---|---|
| 295 | 6- aminomethyl benzofuran |
| 296 | 4- aminomethyl benzofuran |

### Example 297

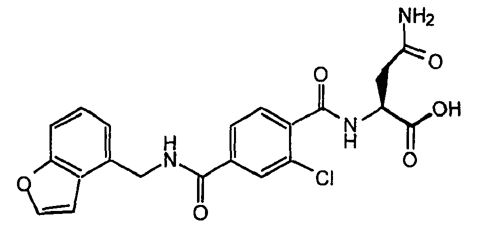

Example 297 was synthesized by Method S63.

### Example 298

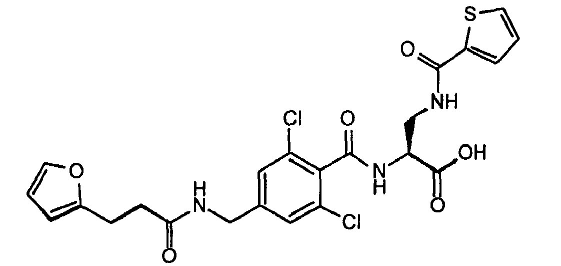

Example 298 was synthesized by Method S64.

### Example 299

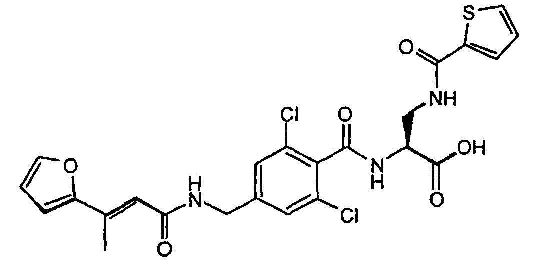

Example 299 was synthesized by Method S65.

### Example 300

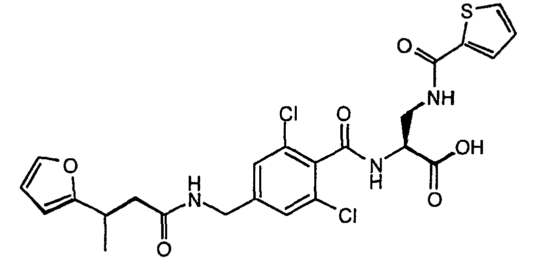

Example 300 was synthesized by Method S66.

### Example 301

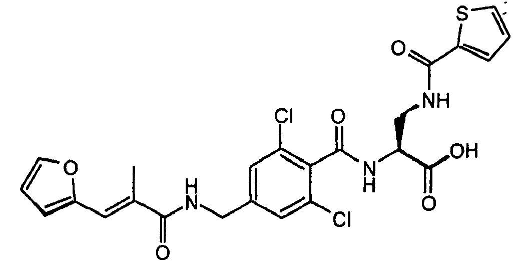

Example 301 was synthesized by Method S67.

### Example 302

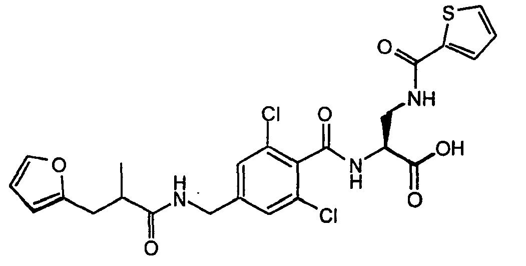

Example 302 was synthesized by Method S68.

### Examples 303-305

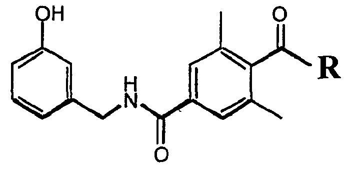

Examples 303-305 were synthesized by Method S69.

| Example # | R group |
|---|---|
| 303 | L- Asn |
| 304 | L- diaminopropionic acid (alloc) |
| 305 | L- lys |

### Example 306

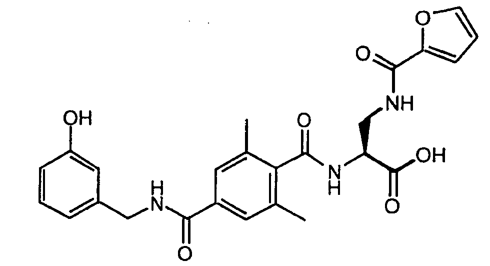

Example 306 was synthesized by Method S70.

### Example 307

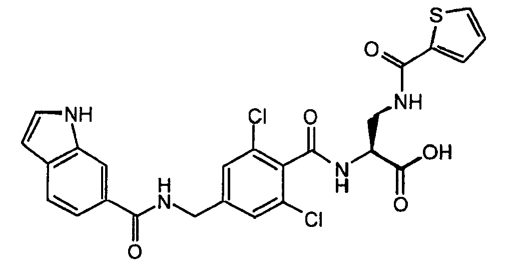

Example 307 was synthesized by Method S71.

### Examples 308-309

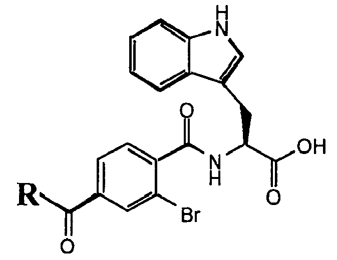

Examples 308-309 were synthesized by Method S72.

| Example # | R group |
|---|---|
| 308 | 3- hydroxy benzylamine |
| 309 | 3-(3-hydroxyphenyl)propargylamine |

### Examples 310-312

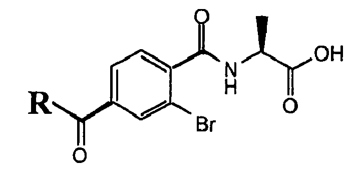

Examples 310-312 were synthesized by Method S73.

| Example # | R group |
|---|---|
| 310 | 3- flouro benzylamine |
| 311 | benzylamine |
| 312 | 3-(3-hydroxyphenyl)propargylamine |

### Examples 313-315

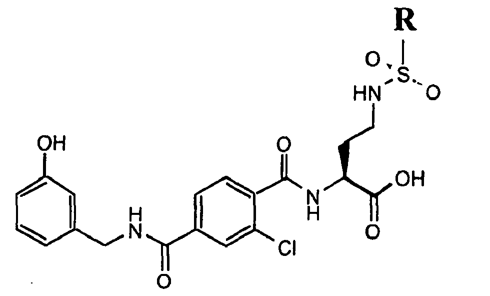

Examples 313-315 were synthesized by Method S74.

| Example # | R group |
|---|---|
| 313 | N-acetylsulfanflyl chloride |
| 314 | 2-bromobenzenesulfonyl chloride |
| 315 | 2-thiophenesulfonyl chloride |

### Examples 316-317

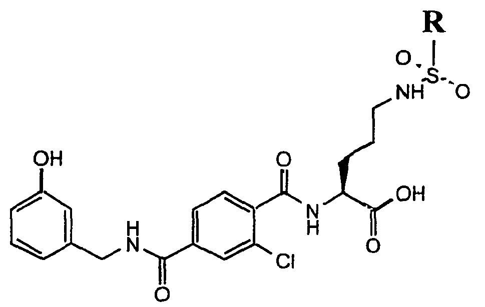

Examples 316-317 were synthesized by Method S75.

| Example # | R group |
|---|---|
| 316 | 2-thiophenesulfonyl chloride |
| 317 | 8-quinofinesulfonyl chloride |

### Examples 318-322

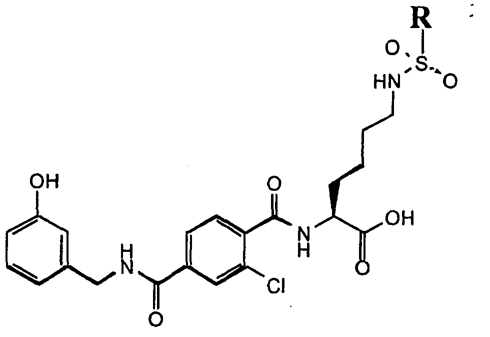

Examples 318-322 were synthesized by Method S76.

| Example # | R group |
|---|---|
| 318 | benzenesulfonyl chloride |
| 319 | N-acetylsulfanilyl chloride |
| 320 | 2-thiophenesulfonyl chloride |
| 321 | 2-bromobenzenesulfonyl chloride |
| 322 | 2-acetamido-4-methyl-5-thiazolesulfonyl chloride |

### Examples 323-328

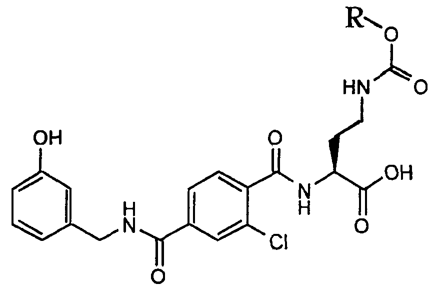

Examples 323-328 were synthesized by Method S77.

| Example # | R group |
|---|---|
| 323 | isobutyl chloroformate |
| 324 | allyl chloroformate |
| 325 | butyl chloroformate |
| 326 | ethyl chloroformate |
| 327 | isopropyl chloroformate |
| 328 | propyl chloroformate |

### Examples 329-333

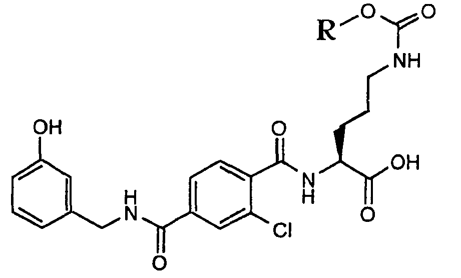

Examples 329-333 were synthesized by Method S78.

| Example # | R group |
|---|---|
| 329 | isobutyl chloroformate |
| 330 | cyclopropyl chloroformate |
| 331 | ethyl chloroformate |
| 332 | methyl chloroformate |
| 333 | 2, 2, 2-trichloroethyl chloroformate |

### Examples 334-337

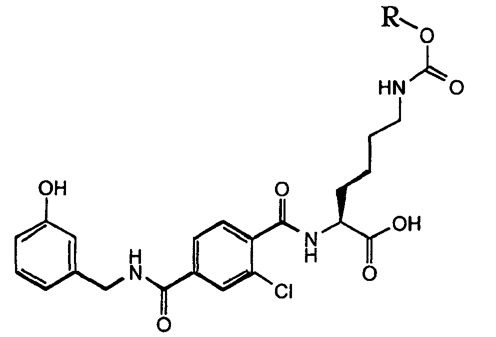

Examples 334-337 were synthesized by Method S79.

| Example # | R group |
|---|---|
| 334 | butyl chloroformate |
| 335 | propyl chloroformate |
| 336 | ethyl chloroformate |
| 337 | methyl chloroformate |

### Example 338

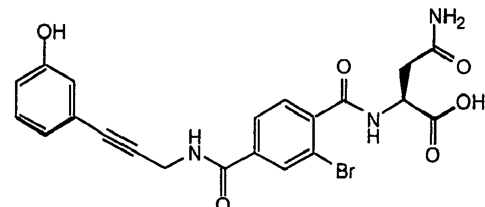

Example 338 was synthesized by Method S80.

### Example 339

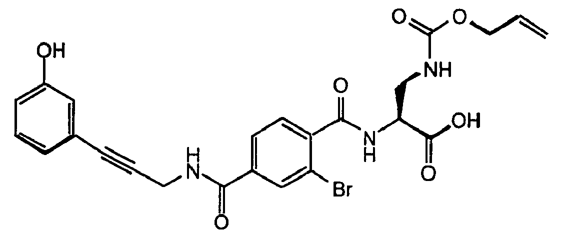

Example 339 was synthesized by Method S81.

### Examples 340-354

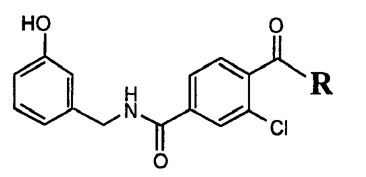

Examples 340-354 were synthesized by Method S82.

| Example # | R group |
|---|---|
| 340 | L - Ala |
| 341 | L - Thr |
| 342 | L - Trp |
| 343 | L - aza Trp |
| 344 | L - Ser(OBzl) |
| 345 | L-Asn |
| 346 | L-Lys |
| 347 | L-His |
| 348 | L-Lys(N-e-Ac) |
| 349 | L-Gln |
| 350 | L-diaminopropionic(alloc) acid |
| 351 | L-diaminobutyric(alloc) acid |
| 352 | L-lys(alloc) |
| 353 | L-orn(alloc) |
| 354 | L- Tyr |

### Examples 355-357

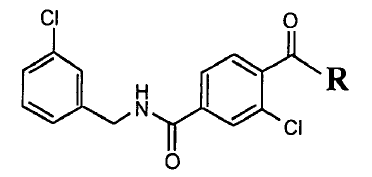

Examples 355-357 were synthesized by Method S83.

| Example # | R group |
|---|---|
| 355 | L-Ala |
| 356 | L-His |
| 357 | L-Asn |

### Example 358

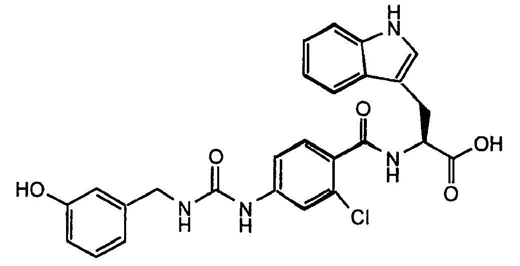

Example 358 was synthesized by Method S84.

### Examples 359-362

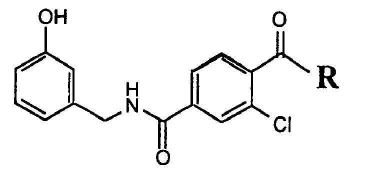

Examples 359-362 were synthesized by Method S85.

| Example # | R group |
|---|---|
| 359 | 1-amino-1-cyclopropane carboxylic acid |
| 360 | m-tyrosine |
| 361 | o-hydroxytyrosine |
| 362 | L-iodotyrosine |

### Example 363

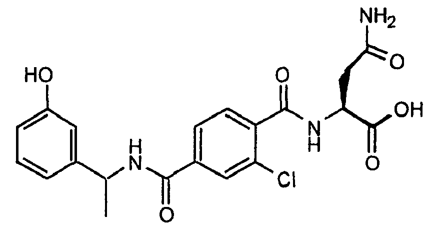

Example 363 was synthesized by Method S86.

### Example 364

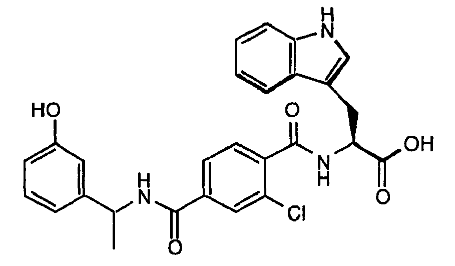

Example 364 was synthesized by Method S87.

### Example 365

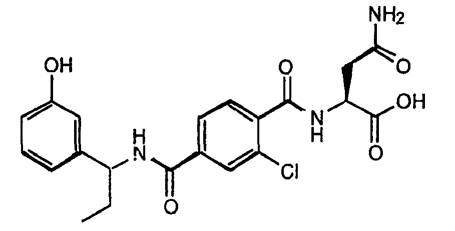

Example 365 was synthesized by Method S88.

### Example 366

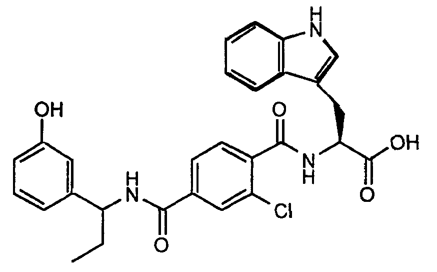

Example 366 was synthesized by Method S89.

### Example 367

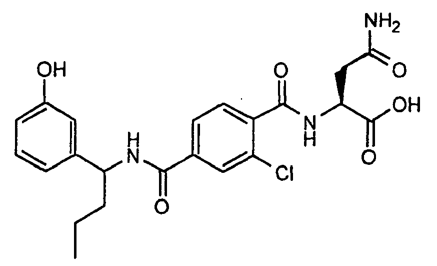

Example 367 was synthesized by Method S90.

### Example 368

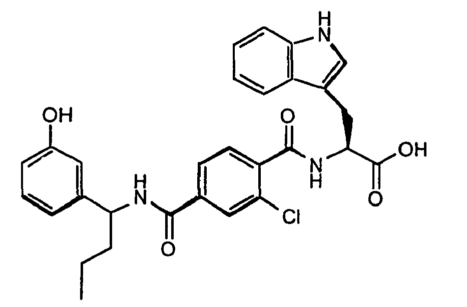

Example 368 was synthesized by Method S91.

### Example 369

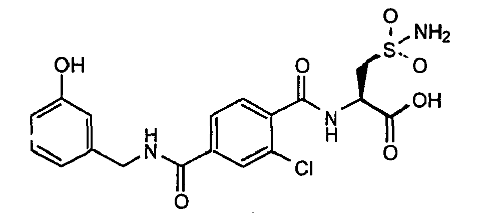

Example 369 was synthesized by Method S92.

### Examples 370-371

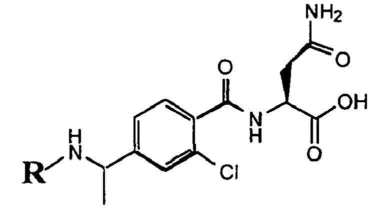

Examples 370-371 were synthesized by Method S93.

| Example # | R group |
|---|---|
| 370 | 3 - hydroxybenzoic acid |
| 371 | benzoic acid |

### Examples 372-375

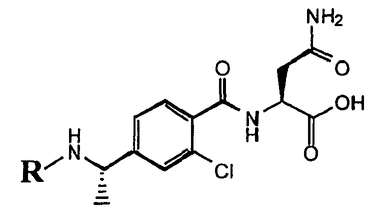

Examples 372-375 were synthesized by Method S94.

| Example # | R group |
|---|---|
| 372 | furylacrylic acid |
| 373 | 3-(2-thienyl)-acrylic acid |
| 374 | 3 - hydroxybenzoic acid |
| 375 | benzoic acid |

### Examples 376-377

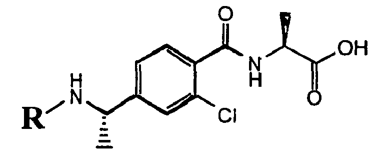

Examples 376-377 were synthesized by Method S95.

| Example # | R group |
|---|---|
| 376 | 3 - hydroxybenzoic acid |
| 377 | 3-(2-thienyl)-acrylic acid |

### Example 378

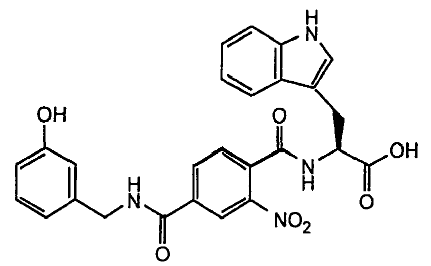

Example 378 was synthesized by Method S96.

### Example 379

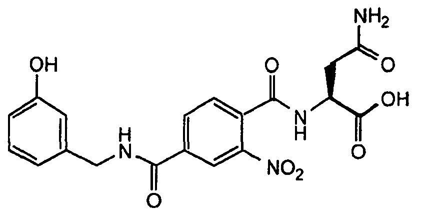

Example 379 was synthesized by Method S97.

### Examples 380-383

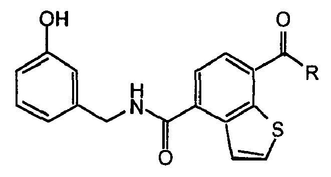

Examples 380-383 were synthesized by Method S98.

| Example # | R group |
|---|---|
| 380 | L- Trp |
| 381 | L- Asn |
| 382 | L- dapa(alloc) |
| 383 | L- Lys |

### Example 384

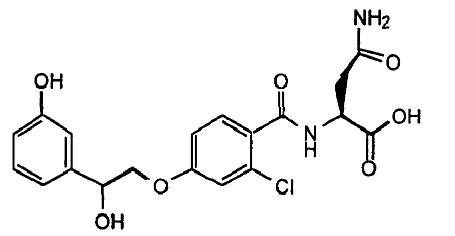

Example 383 was synthesized by Method S99.

### Example 385

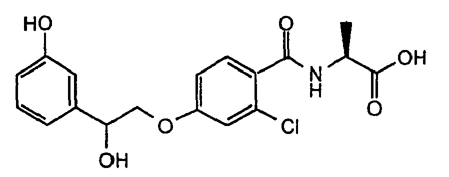

Example 385 was synthesized by Method S100.

### Example 386

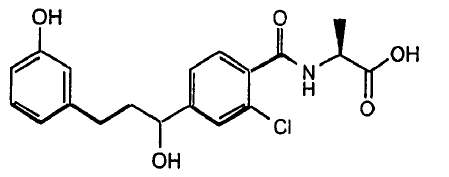

Example 386 was synthesized by Method S101.

### Example 387

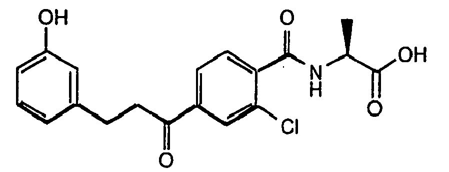

Example 387 was synthesized by Method S102.

### Example 388

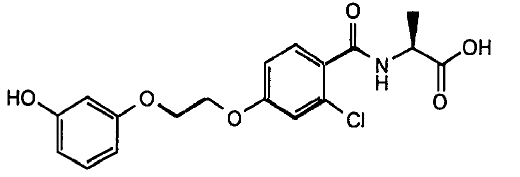

Example 388 was synthesized by Method S103.

### Example 389

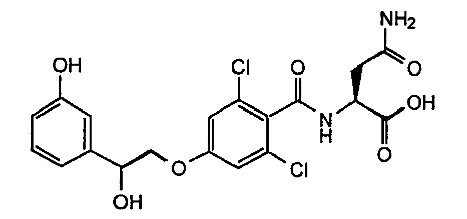

Example 389 was synthesized by Method S104.

### Example 390

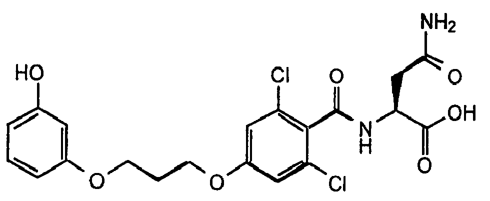

Example 390 was synthesized by Method S105.

### Example 391

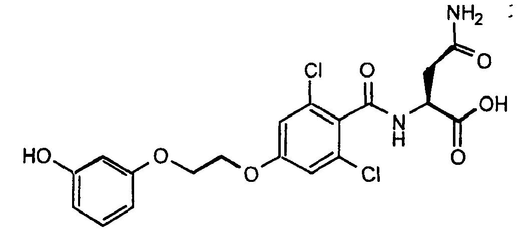

Example 391 was synthesized by Method S106.

### Example 392

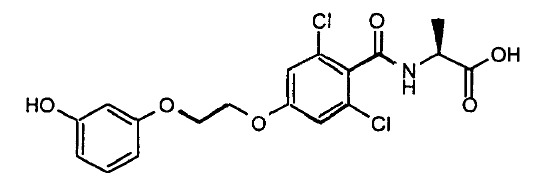

Example 392 was synthesized by Method S107.

### Example 393

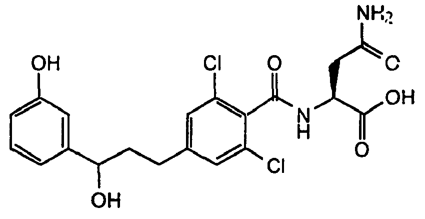

Example 393 was synthesized by Method S108.

### Example 394

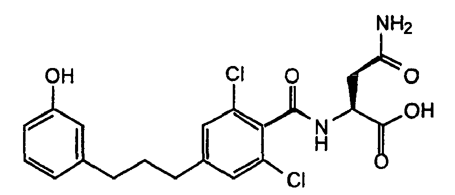

Example 394 was synthesized by Method S109.

### Example 395

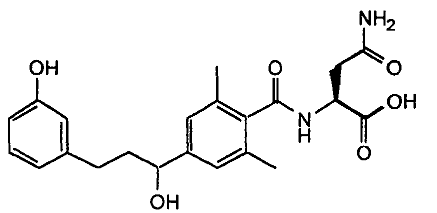

Example 395 was synthesized by Method S110.

### Example 396

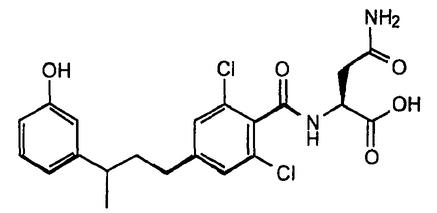

Example 396 was synthesized by Method S111.

### Example 397

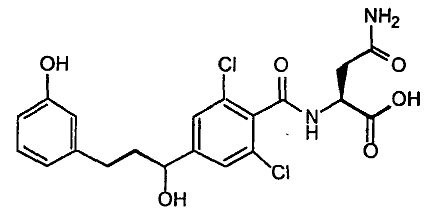

Example 397 was synthesized by Method S112.

### Example 398

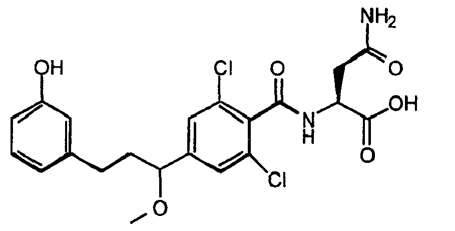

Example 398 was synthesized by Method S113.

### Example 399

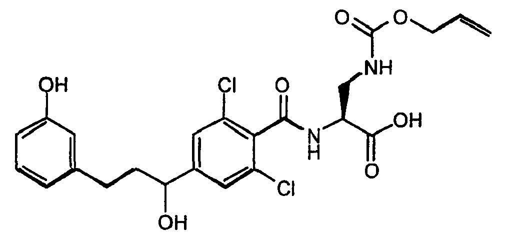

Example 399 was synthesized by Method S114.

### Example 400

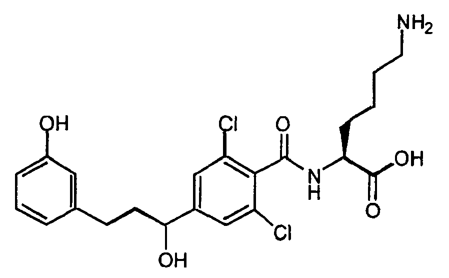

Example 400 was synthesized by Method S115.

### Example 401

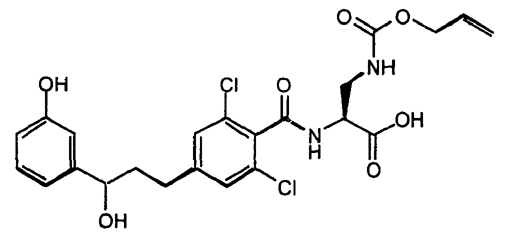

Example 401 was synthesized by Method S116.

### Example 402

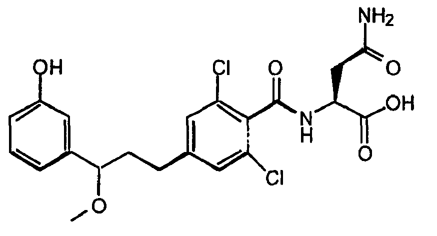

Example 402 was synthesized by Method S117.

### Example 403

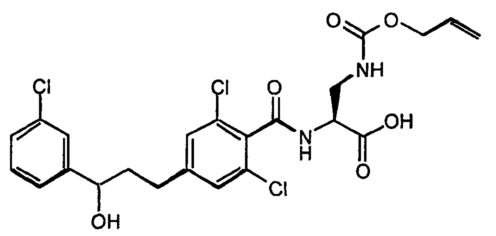

Example 403 was synthesized by Method S118.

### Example 404

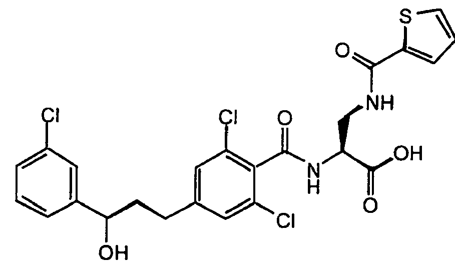

Example 404 was synthesized by Method S119.

### Example 405

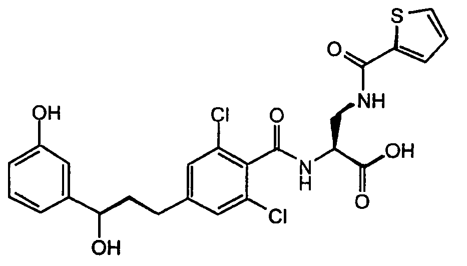

Example 405 was synthesized by Method S120.

### Example 406

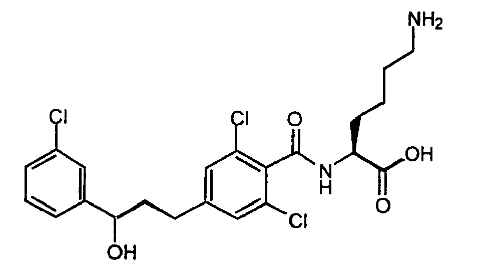

Example 406 was synthesized by Method S121.

### Examples 407-416

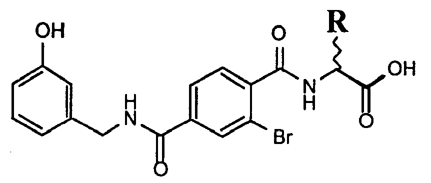

Examples 407-416 were synthesized by Method S122.

| Example # | R group |
|---|---|
| 407 | 3-methoxybenzyl bromide |
| 408 | 3-bromobenzyl bromide |
| 409 | 3, 5-dimethoxybenzyl bromide |
| 410 | 5-bromovaleronitrile |
| 411 | 6-bromochexanenitrile |
| 412 | 3-nitrobenzyl bromide |
| 413 | 3-cyanobenzyl bromide |
| 414 | 5-bromomethyl-furan-2-carboxylic acid ethyl ester |
| 415 | 5-bromomethyl-furan-2-carboxylic acid ethyl ester |
| 416 | 3-bromomethyl benzamide |

### Examples 417-423

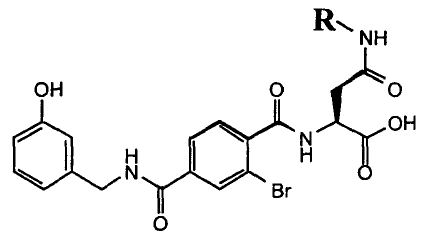

Examples 417-413 were synthesized by Method S123.

| Example # | R group |
|---|---|
| 417 | 1-aminonaphthalene |
| 418 | 2-cyanoaniline |
| 419 | 3-cyanoaniline |
| 420 | 2-fluoroaniline |
| 421 | 3-fluoroaniline |
| 422 | 4-fluoroaniline |
| 423 | 3-methoxyaniline |

### Examples 424-436

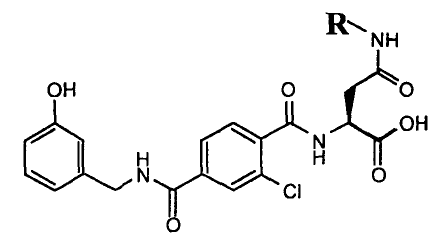

Examples 424-436 were synthesized by Method S124.

| Example # | R group |
|---|---|
| 424 | 2-(aminomethyl)pyridine |
| 425 | 3-fluorobenzylamine |
| 426 | benzylamine |
| 427 | allylamine |
| 428 | phenethyl amine |
| 429 | histamine |
| 430 | 4-fluorobenzylamine |
| 431 | 3-methoxyphenethylamine |
| 432 | 4-aminobenzylamine |
| 433 | 2-aminobenzylamine |
| 434 | 2-[1, 3]Dioxan-5-yl-ethylamine |
| 435 | piperonylamine |
| 436 | aniline |

### Examples 437-440

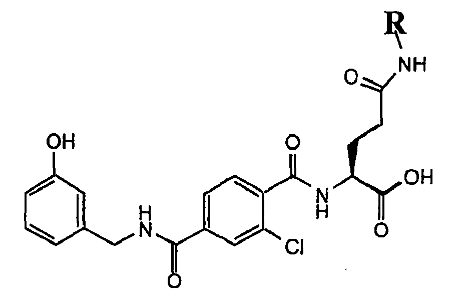

Examples 437-440 were synthesized by Method S125.

| Example # | R group |
|---|---|
| 437 | ¹soamyl amine |
| 438 | 4-(aminomethyl)pyridine |
| 439 | 2-[1,3]Dioxan-5-yl-ethylamine |
| 440 | aniline |

### Examples 441-443

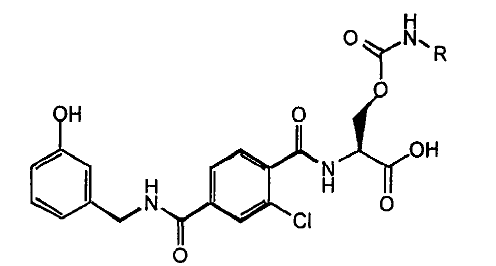

Examples 441-443 were synthesized by Method S126.

| Example # | R group |
|---|---|
| 441 | o- toluidine |
| 442 | allyl amine |
| 443 | propyl amine |

### Examples 444-459

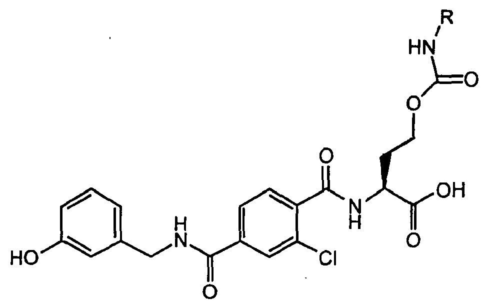

Examples 444-459 were synthesized by Method S127.

| Example # | R group |
|---|---|
| 444 | propylamine |
| 445 | 3-(aminomethyl)pyridine |
| 446 | 4-(aminomethyl)pyridine |
| 447 | 2- methylbenzylamine |
| 448 | 3- methylbenzylamine |
| 449 | 4- methylbenzylamine |
| 450 | (S)-(-)-a-methylbenzylamine |
| 451 | 2-(aminomethyl)pyridine |
| 452 | 2- fluoro benzylamine |
| 453 | 3- fluoro benzylamine |
| 454 | 4- fluoro benzylamine |
| 455 | 3- chloro benzylamine |
| 456 | 4- chloro benzylamine |
| 457 | 4- methoxy benzylamine |
| 458 | 1- naphthalenemethylamine |
| 459 | benzylamine |

Table 3 provides biological assay data for the compounds prepared by the methods described above. Data is provided for two assay formats: the forward format of LFA/ICAM assay (PPFF) and the PLM2 antibody capture format of LFA/ICAM assay (PLM2).

**Table 3**

| **PPFF and PLM2 assay data for exemplary compounds** | | |
|---|---|---|
| Example # | PPFF(µM) | PLM2(µM) |
| 1 | 0.149 | 0.028 |
| 2 | | 0.035 |
| 3 | | 0.069 |
| 4 | | 0.038 |
| 5 | | 0.013 |
| 6 | | 0.045 |
| 7 | | 0.004 |
| 8 | | 0.021 |
| 9 | | 0.033 |
| 10 | | 0.003 |
| 11 | | 0.065 |
| 12 | | 0.029 |
| 13 | | 0.064 |
| 14 | | 0.024 |
| 15 | | 0.010 |
| 16 | | 0.011 |
| 17 | | 0.036 |
| 18 | | 0.010 |
| 19 | | 0.037 |
| 20 | | 0.029 |
| 21 | | 0.023 |
| 22 | | 0.019 |
| 23 | | 0.072 |
| 24 | | 0.012 |
| 25 | | 0.019 |
| 26 | | 0.021 |
| 27 | | 0.008 |
| 28 | | 0.092 |
| 29 | | 0.055 |
| 30 | | 0.064 |
| 31 | | 0.014 |
| 32 | | 0.047 |
| 33 | | 0.023 |
| 34 | | 0.078 |
| 35 | | 0.069 |
| 36 | | 0.013 |
| 37 | | 0.038 |
| 38 | | 0.013 |
| 39 | | 0.021 |
| 40 | | 0.076 |
| 41 | | 0.098 |
| 42 | | 0.046 |
| 43 | | 0.098 |
| 44 | | 0.095 |
| 45 | | 0.059 |
| 46 | | 0.066 |
| 47 | | 0.070 |
| 48 | | 0.046 |
| 49 | | 0.038 |
| 50 | | 0.052 |
| 51 | | 0.056 |
| 52 | | 0.050 |
| 53 | | 0.094 |
| 54 | | 0.014 |
| 55 | | 0.047 |
| 56 | | 0.052 |
| 57 | | 0.036 |
| 58 | | 0.080 |
| 59 | | 0.066 |
| 60 | | 0.078 |
| 61 | | 0.052 |
| 62 | | 0.046 |
| 63 | | 0.062 |
| 64 | | 0.055 |
| 65 | | 0.044 |
| 66 | | 0.072 |
| 67 | | 0.046 |
| 68 | | 0.071 |
| 69 | | 0.084 |
| 70 | | 0.088 |
| 71 | | 0.040 |
| 72 | | 0.063 |
| 73 | | 0.063 |
| 74 | | 0.087 |
| 75 | | 0.011 |
| 76 | | 0.010 |
| 77 | | 0.017 |
| 78 | | 0.031 |
| 79 | | 0.033 |
| 80 | | 0.005 |
| 81 | | 0.008 |
| 82 | | 0.004 |
| 83 | | 0.006 |
| 84 | | 0.001 |
| 85 | | 0.003 |
| 86 | | 0.012 |
| 87 | | 0.009 |
| 88 | | 0.005 |
| 89 | | 0.004 |
| 90 | | 0.021 |
| 91 | | 0.004 |
| 92 | | 0.066 |
| 93 | | 0.024 |
| 94 | | 0.002 |
| 95 | | 0.006 |
| 96 | | 0.070 |
| 97 | | 0.042 |
| 98 | | 0.033 |
| 99 | | 0.046 |
| 100 | | 0.031 |
| 101 | | 0.022 |
| 102 | | 0.025 |
| 103 | | 0.044 |
| 104 | | 0.044 |
| 105 | | 0.004 |
| 106 | | 0.026 |
| 107 | | 0.087 |
| 108 | | 0.021 |
| 109 | | 0.026 |
| 110 | | 0.052 |
| 111 | | 0.007 |
| 112 | | 0.036 |
| 113 | | 0.086 |
| 114 | | 0.018 |
| 115 | | 0.073 |
| 116 | | 0.026 |
| 117 | | 0.045 |
| 118 | | 0.031 |
| 119 | | 0.077 |
| 120 | | 0.064 |
| 121 | | 0.055 |
| 122 | | 0.050 |
| 123 | | 0.054 |
| 124 | | 0.035 |
| 125 | | 0.058 |
| 126 | | 0.033 |
| 127 | | 0.017 |
| 128 | | 0.035 |
| 129 | | 0.029 |
| 130 | | 0.036 |
| 131 | | 0.025 |
| 132 | | 0.057 |
| 133 | | 0.020 |
| 134 | | 0.053 |
| 135 | | 0.021 |
| 136 | | 0.029 |
| 137 | | 0.039 |
| 138 | | 0.071 |
| 139 | | 0.064 |
| 140 | | 0.023 |
| 141 | | 0.068 |
| 142 | | 0.074 |
| 143 | | 0.031 |
| 144 | | 0.093 |
| 145 | | 0.004 |
| 146 | | 0.004 |
| 147 | | 0.004 |
| 148 | | 0.004 |
| 149 | | 0.004 |
| 150 | | 0.004 |
| 151 | | 0.004 |
| 152 | | 0.004 |
| 153 | | 0.003 |
| 154 | | 0.003 |
| 155 | | 0.006 |
| 156 | | 0.009 |
| 157 | | 0.007 |
| 158 | | 0.004 |
| 159 | | 0.017 |
| 160 | | 0.004 |
| 161 | | 0.004 |
| 162 | | 0.004 |
| 163 | | 0.005 |
| 164 | | 0.012 |
| 165 | | 0.015 |
| 166 | | 0.018 |
| 167 | | 0.017 |
| 168 | | 0.012 |
| 169 | | 0.006 |
| 170 | | 0.007 |
| 171 | | 0.011 |
| 172 | | 0.037 |
| 173 | | 0.010 |
| 174 | | 0.004 |
| 175 | | 0.005 |
| 176 | | 0.011 |
| 177 | | 0.006 |
| 178 | | 0.011 |
| 179 | | 0.009 |
| 180 | | 0.011 |
| 181 | | 0.016 |
| 182 | | 0.011 |
| 183 | | 0.013 |
| 184 | | 0.016 |
| 185 | | 0.016 |
| 186 | | 0.015 |
| 187 | | 0.017 |
| 188 | | 0.018 |
| 189 | | 0.018 |
| 190 | | 0.016 |
| 191 | | 0.016 |
| 192 | | 0.029 |
| 193 | | 0.014 |
| 194 | | 0.012 |
| 195 | | 0.016 |
| 196 | | 0.019 |
| 197 | | 0.017 |
| 198 | | 0.019 |
| 199 | | 0.029 |
| 200 | | 0.018 |
| 201 | | 0.013 |
| 202 | | 0.023 |
| 203 | | 0.037 |
| 204 | | 0.025 |
| 205 | | 0.082 |
| 206 | | 0.023 |
| 207 | | 0.062 |
| 208 | | 0.021 |
| 209 | | 0.053 |
| 210 | | 0.022 |
| 211 | | 0.019 |
| 212 | | 0.016 |
| 213 | | 0.035 |
| 214 | | 0.028 |
| 215 | | 0.027 |
| 216 | | 0.022 |
| 217 | | 0.031 |
| 218 | | 0.018 |
| 219 | | 0.018 |
| 220 | | 0.016 |
| 221 | | 0.042 |
| 222 | | 0.021 |
| 223 | | 0.035 |
| 224 | | 0.026 |
| 225 | | 0.029 |
| 226 | | 0.025 |
| 227 | | 0.034 |
| 228 | | 0.018 |
| 229 | | 0.026 |
| 230 | | 0.016 |
| 231 | | 0.003 |
| 232 | | 0.005 |
| 233 | | 0.001 |
| 234 | | 0.044 |
| 235 | | 0.002 |
| 236 | | 0.004 |
| 237 | | 0.003 |
| 238 | 0.099 | |
| 239 | 0.180 | 0.053 |
| 240 | 0.085 | |
| 241 | 0.053 | |
| 242 | 0.054 | |
| 243 | 0.082 | |
| 244 | 0.077 | 0.078 |
| 245 | 0.058 | 0.164 |
| 246 | 0.067 | 0.059 |
| 247 | 0.022 | 0.034 |
| 248 | 0.027 | 0.026 |
| 249 | 0.030 | |
| 250 | | 0.034 |
| 251 | | 0.038 |
| 252 | | 0.060 |
| 253 | | 0.014 |
| 254 | 0.094 | 0.036 |
| 255 | 0.042 | |
| 256 | 0.076 | |
| 257 | | 0.042 |
| 258 | | 0.038 |
| 259 | | 0.049 |
| 260 | | 0.071 |
| 261 | | 0.052 |
| 262 | | 0.075 |
| 263 | | 0.066 |
| 264 | | 0.093 |
| 265 | | 0.045 |
| 266 | | 0.046 |
| 267 | | 0.021 |
| 268 | | 0.019 |
| 269 | | 0.046 |
| 270 | | 0.055 |
| 271 | | 0.086 |
| 272 | | 0.080 |
| 273 | | 0.016 |
| 274 | | 0.006 |
| 275 | | 0.006 |
| 276 | | 0.012 |
| 277 | | 0.003 |
| 278 | | 0.002 |
| 279 | | 0.004 |
| 280 | | 0.007 |
| 281 | | 0.004 |
| 282 | | 0.024 |
| 283 | 0.092 | |
| 284 | 0.093 | 0.079 |
| 285 | 0.064 | |
| 286 | | 0.014 |
| 287 | | 0.043 |
| 288 | | 0.023 |
| 289 | | 0.074 |
| 290 | | 0.009 |
| 291 | | 0.007 |
| 292 | | 0.015 |
| 293 | | 0.083 |
| 294 | | 0.100 |
| 295 | | 0.047 |
| 296 | | 0.017 |
| 297 | | 0.028 |
| 298 | | 0.009 |
| 299 | | 0.016 |
| 300 | | 0.074 |
| 301 | | 0.025 |
| 302 | | 0.023 |
| 303 | | 0.005 |
| 304 | | 0.003 |
| 305 | | 0.015 |
| 306 | | 0.004 |
| 307 | | 0.004 |
| 308 | 0.061 | |
| 309 | | 0.057 |
| 310 | 0.082 | |
| 311 | 0.079 | |
| 312 | | 0.089 |
| 313 | | 0.069 |
| 314 | | 0.028 |
| 315 | | 0.037 |
| 316 | | 0.030 |
| 317 | | 0.055 |
| 318 | | 0.031 |
| 319 | | 0.023 |
| 320 | | 0.007 |
| 321 | | 0.020 |
| 322 | | 0.011 |
| 323 | | 0.036 |
| 324 | | 0.042 |
| 325 | | 0.056 |
| 326 | | 0.042 |
| 327 | | 0.070 |
| 328 | | 0.074 |
| 329 | | 0.033 |
| 330 | | 0.009 |
| 331 | | 0.027 |
| 332 | | 0.057 |
| 333 | | 0.090 |
| 334 | | 0.072 |
| 335 | | 0.096 |
| 336 | | 0.066 |
| 337 | | 0.079 |
| 338 | | 0.060 |
| 339 | | 0.020 |
| 340 | 0.014 | 0.006 |
| 341 | 0.031 | |
| 342 | 0.057 | 0.004 |
| 343 | 0.030 | |
| 344 | 0.183 | 0.053 |
| 345 | 0.019 | 0.004 |
| 346 | 0.071 | |
| 347 | 0.044 | 0.004 |
| 348 | 0.090 | 0.023 |
| 349 | 0.042 | |
| 350 | 0.027 | 0.005 |
| 351 | 0.067 | 0.032 |
| 352 | | 0.042 |
| 353 | | 0.074 |
| 354 | | 0.008 |
| 355 | 0.100 | 0.094 |
| 356 | 0.068 | |
| 357 | 0.057 | 0.023 |
| 358 | 0.230 | 0.032 |
| 359 | | 0.016 |
| 360 | | 0.018 |
| 361 | | 0.018 |
| 362 | | 0.005 |
| 363 | 0.014 | 0.010 |
| 364 | 0.087 | 0.035 |
| 365 | | 0.024 |
| 366 | | 0.062 |
| 367 | | 0.020 |
| 368 | | 0.043 |
| 369 | | 0.019 |
| 370 | 0.055 | 0.025 |
| 371 | 0.055 | 0.037 |
| 372 | | 0.013 |
| 373 | | 0.021 |
| 374 | | 0.021 |
| 375 | | 0.040 |
| 376 | 0.078 | 0.061 |
| 377 | 0.016 | 0.051 |
| 378 | | 0.007 |
| 379 | | 0.010 |
| 380 | | 0.096 |
| 381 | | 0.035 |
| 382 | | 0.012 |
| 383 | | 0.060 |
| 384 | 0.046 | 0.018 |
| 385 | 0.070 | 0.048 |
| 386 | 0.030 | |
| 387 | 0.098 | 0.043 |
| 388 | 0.050 | |
| 389 | 0.054 | 0.010 |
| 390 | | 0.079 |
| 391 | | 0.007 |
| 392 | | 0.025 |
| 393 | | 0.003 |
| 394 | | 0.012 |
| 395 | | 0.006 |
| 396 | | 0.062 |
| 397 | | 0.005 |
| 398 | | 0.015 |
| 399 | | 0.002 |
| 400 | | 0.007 |
| 401 | | 0.002 |
| 402 | | 0.004 |
| 403 | | 0.009 |
| 404 | | 0.002 |
| 405 | | 0.001 |
| 406 | | 0.022 |
| 407 | | 0.045 |
| 408 | | 0.071 |
| 409 | | 0.054 |
| 410 | | 0.065 |
| 411 | | 0.055 |
| 412 | | 0.074 |
| 413 | 0.051 | 0.045 |
| 414 | | 0.087 |
| 415 | | 0.059 |
| 416 | | 0.036 |
| 417 | | 0.086 |
| 418 | | 0.056 |
| 419 | | 0.079 |
| 420 | | 0.015 |
| 421 | | 0.056 |
| 422 | | 0.083 |
| 423 | | 0.032 |
| 424 | | 0.038 |
| 425 | | 0.082 |
| 426 | | 0.057 |
| 427 | | 0.044 |
| 428 | | 0.029 |
| 429 | | 0.094 |
| 430 | | 0.070 |
| 431 | | 0.070 |
| 432 | | 0.070 |
| 433 | | 0.046 |
| 434 | | 0.050 |
| 435 | | 0.074 |
| 436 | | 0.011 |
| 437 | 0.083 | 0.034 |
| 438 | | 0.082 |
| 439 | | 0.089 |
| 440 | | 0.068 |
| 441 | | 0.015 |
| 442 | | 0.006 |
| 443 | | 0.010 |
| 444 | | 0.041 |
| 445 | | 0.029 |
| 446 | | 0.020 |
| 447 | | 0.085 |
| 448 | | 0.094 |
| 449 | | 0.071 |
| 450 | | 0.061 |
| 451 | | 0.030 |
| 452 | | 0.040 |
| 453 | | 0.056 |
| 454 | | 0.046 |
| 455 | | 0.071 |
| 456 | | 0.064 |
| 457 | | 0.036 |
| 458 | | 0.083 |
| 459 | | 0.058 |

## Claims

1. A compound represented by the formula: 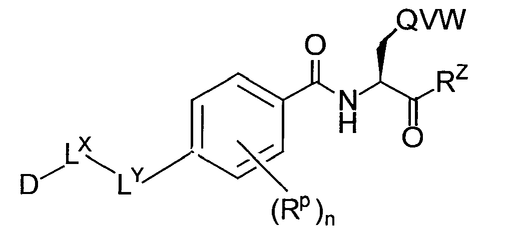 where
D is an aromatic group selected from 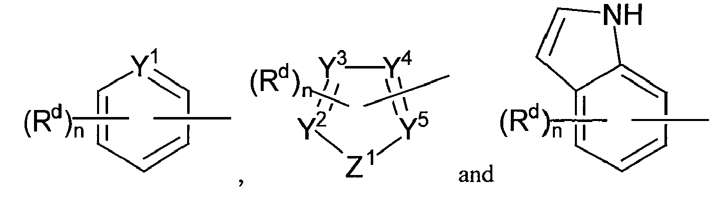 where
Y¹ is selected from NRⁿ, CH and CR^{d};
Y², Y³, Y⁴ and Y⁵ are selected from CH and CR^{d};
Z¹ is selected from NRⁿ, O and S;
n is 0-3;
L^{X} is selected from substituted or unsubstituted
C₂-C₅alkylene,
C₃-C₆cycloalkylene,
C₀-C₃alkylene-NRⁿ-(C=O)-C₀-C₃alkylene,
C ₀-C₃alkytene-(C=O)-NRⁿ-C₀-C₃alkylene,
C₀-C₃alkylene-O-C₀-C₃alkylene,
C₀-C₃alkylene-NRⁿ-C₀-C₃alkylene,
C₀-C₃alkylene-(C=O)-C₀-C₃alkylene,
C₀-C₃alkylene-S(O)₀₋₂-C₀-C₃alkylene,
C₀-C₃alkylene-NRⁿ-SO₂-C₀-C₃alkylene,
C₀-C₃alkylene-SO₂-NRⁿ-C₀-C₃alkylene,
C₀-C₃alkylene-CR¹=CR²-C₀-C₃alkylene,
C₀-C₃alkylene-C=C-C₀-C₃alkylene and
C₀-C₃alkylene-het-C₀-C₃alkylene
where the substituents are selected from one to three R¹, R² and R³;
L^{Y} is selected from substituted or unsubstituted
C₀-C₂alkylene,
C₀-C₂alkylene-NRⁿ-(C=O)-C₀-C₂alkylene,
C₀-C₂alkylene-(C=O)-NRⁿ-C₀-C₂alkylene,
C₀-C₂alkylene-O-C₀-C₂alkylene,
C₀-C₂alkylene-NRⁿ-C₀-C₂alkylene,
C₀-C₂alkylene-(C=O)-C₀-C₂alkylene,
C₀-C₃alkylene-S(O)₀₋₂-C₀-C₃alkylene,
C₀-C₃alkylene-SO₂-NRⁿ-C₀-C₃alkylene and
C₀-C₂alkylene-aryl-C₀-C₂alkylene
where the substituents are selected from one to three R¹, R² and R³;
R¹, R² and R³ are selected from
hydrogen,
C₁-C₈alkyl-hydroxy,
halo,
halo-C ₁-C₈alkyl,
cyano,
isocyanate,
carboxy,
carboxy-C₁-C₆alkyl,
amino,
amino-C ₁-C₈alkyl,
amino-di(C₁-C₈alkyl),
aminocarbonyl,
carboxamido,
carbamoyl,
carbamoyloxy,
formyl,
formyloxy,
nitro,
imidazoyl,
ureido,
thioureido,
thiocyanato,
hydroxy,
C₁-C₆alkoxy,
mercapto,
sulfonamido,
phenoxy,
phenyl, and
benzamido;
R^{a} is selected from
hydrogen,
halo,
cyano,
isocyanate,
carboxy,
carboxy-C₁-C₆alkyl,
amino,
amino-C₁-C₈alkyl,
aminocarbonyl,
carboxamido,
carbamoyl,
carbamoyloxy,
formyl,
formyloxy,
azido,
nitro,
imidazoyl,
ureido,
thioureido,
thiocyanato,
hydroxy,
C₁-C₆alkoxy,
mercapto,
sulfonamido,
C₁-C₆alkylsulfonyl,
het,
phenoxy,
phenyl,
benzamido,
tosyl,
morpholino,
morpholinyl,
piperazinyl,
piperidinyl,
pyrrolinyl.
imidazolyl and
indolyl;
R^{c} is selected from hydrogen and substituted or unsubstituted
C₁-C₁₀alkyl,
C₂-C₁₀alkenyl,
C₂-C₁₀alkynyl,
C₃-C₁₁cycloalkyl,
C₃-C₁₀cycloalkenyl,
C₁-C₆alkyl-C₆-C₁₂aryl,
C₆-C₁₀aryl-C₁-C₆alkyl,
C₁-C₆alkyl-het,
het-C₁-C₆alkyl,
C₆-C₁₂aryl,
C₁-C₁₀alkyl-O-,
C₂-C₁₀alkenyl-O-,
C₂-C₁₀alkynyl-O-,
C₃-C₁₁cycloalkyl-O-,
C₃-C₁₀cycloalkenyl-O-,
C₁-C₆alkyl-C₆-C₁₂aryl-O-,
C₆-C₁₀aryl-C₁-C₆alkyl-O-,
C₁-C₆alkyl-het-O-,
het-C₀-C₆alkyl-O-,
C₆-C₁₂aryl-O-,
C₁-C₁₀alkyl-NRⁿ-,
C₂-C₁₀alkenyl-NRⁿ-,
C₂-C₁₀alkynyl-NRⁿ-,
C₃-C₁₁cycloalkyl-NRⁿ-,
C₃-C₁₀cycloalkenyl-NRⁿ-,
C₁-C₆alkyl-C₆-C₁₂aryl-NRⁿ-,
C₆-C₁₀aryl-C₁-C₆alkyl-NRⁿ-,
C₁-C₆alkyl-het-NRⁿ-,
het-C₀-C₆alkyl-NRⁿ-,
C₆-C₁₂aryl-NRⁿ- and
het, where the substituents on any alkyl, alkenyl or alkynyl are 1-3 R^{a} and the substituents on any aryl or het are 1-3 R^{d};
het is a mono-, bi-, or tricyclic saturated, unsaturated, or aromatic ring having one to four heteroatoms selected from nitrogen, oxygen, and sulfur;
R^{p} and R^{d} are independently selected from
OH,
CN,
NO₂,
halo,
ORⁿ,
SRⁿ,
SORⁿ,
CF₃,
R^{c},
NRⁿR^{n'},
NRⁿ C(=O)-O-R^{n'},
NRⁿ C(=O)-R^{n'},
C₀-C₆alkyl-SO₂-Rⁿ,
C₀-C₆alkyl-SO₂-NRⁿR^{n'},
C(=O)-Rⁿ,
O-C(=O)-Rⁿ,
C(=O)-O-Rⁿ and
C(=O)-NRⁿR^{n'},
R^{d} is a chemical bond when het is a divalent linking group;
Rⁿ and R^{n'} are independently selected from
hydrogen,
hydroxy,
C₁-C₆alkyl,
halo-C₁-C₆alkyl,
C₁-C₆alkyl-het,
het-C ₁-C₆alkyl,
C₆-C₁₂aryl, and
het;
R^{z} is a substituted or unsubstituted group selected from
hydroxy,
C₁-C₁₁alkoxy,
C₃-C₁₂cycloalkoxy,
C₈-C₁₂aralkoxy,
C₈-C₁₂arcycloalkoxy,
C₆-C₁₀aryloxy,
C₃-C₁₀alkylcarbonyloxyalkyloxy,
C₃-C₁₀alkoxycarbonyloxyalkyloxy,
C₃-C₁₀alkoxycarbonylalkyloxy,
C₅-C₁₀cycloalkylcarbonyloxyalkyloxy,
C₅-C₁₀cycloalkoxycarbonyloxyalkyloxy,
C₅-C₁₀cycloalkoxycarbonylalkyloxy,
C₈-C₁₂aryloxycarbonylalkyloxy,
C₈-C₁₂aryloxycarbonyloxyalkyloxy,
C₈-C₁₂arylcarbonyloxyalkyloxy,
C₅-C₁₀alkoxyalkylcarbonyloxyalkyloxy,
(Rⁿ)(R^{n'})N(C₁-C₁₀alkoxy)-, 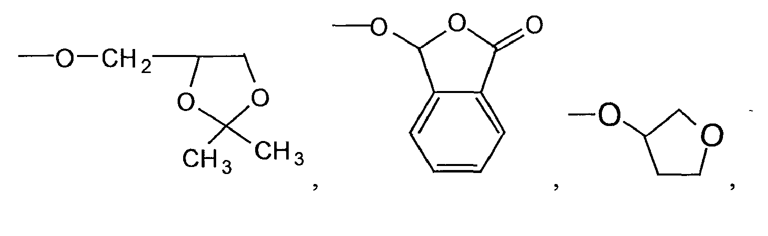 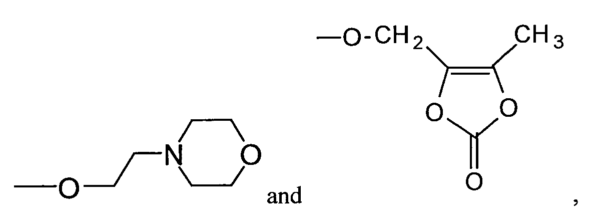 where the substituents on any alkyl, alkenyl or alkynyl are 1-3 R^{a} and the substituents on any aryl or het are 1-3 R^{d} ;
Q is absent or is C₀-C₃ alkyl substituted with a group selected from
-N(Rⁿ)-,
-N(Rⁿ)-C(=O)-,
-N(Rⁿ)-C(=O)-O-,
-N(Rⁿ)-C(=O)-N(Rⁿ)-,
-N(Rⁿ)-SO₂-,
-C(=O)-,
-O-C(=O)-N(Rⁿ)-,
-C(=O)-N(Rⁿ)-,
V is absent or is an optionally substituted bivalent group selected from
C₁-C₁₁alkylene,
C₀-C₃alkylene-O-C₀-C₃alkylene,
C₂-C₆alkenylene,
C₀-C₂alkylene-O-C₂-C₄alkenylene,
C₃-C₈cycloalkylene,
C₆-C₁₀aryl-C₀-C₆alkylene,
C₀-C₆alkyl-C₆-C₁₀arylene and
C₀-C₆alkyl-het;
where the substituents on any alkyl are 1-3 R^{a} and the substituents on any aryl or het are 1-3 R^{d};
W is a C₀-C₃ -alkyl substituted with a group selected from
R^{a},
NH-C(=O)-NRⁿR^{n'},
NH-C(=O)-R^{c},
C(=O)-R^{c},
C(=O)-NH-C(=O)-R^{c},
C(=O)-NH-C(=O)-NRⁿR^{n'},
C(=O)-NH-SO₂-R^{c},
C(=O)-NH-SO₂-NRⁿR^{n'},
C(=O)NRⁿR^{n'},
NH-C(=O)-R^{c} and
R^{c}
and pharmaceutically acceptable salts thereof.

2. A compound according to Claim 1 selected from 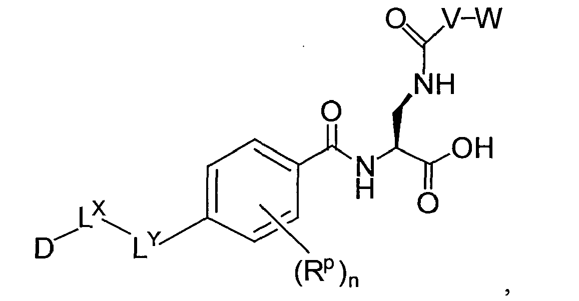 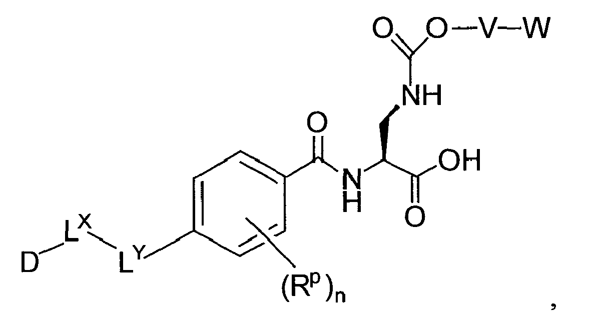 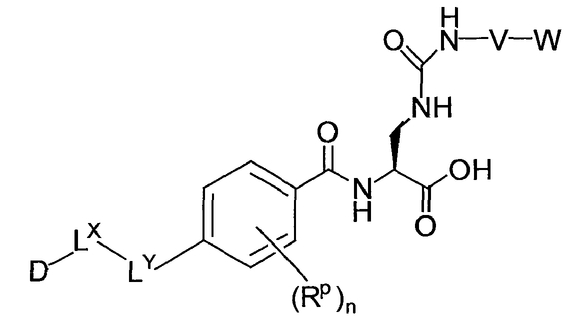 and

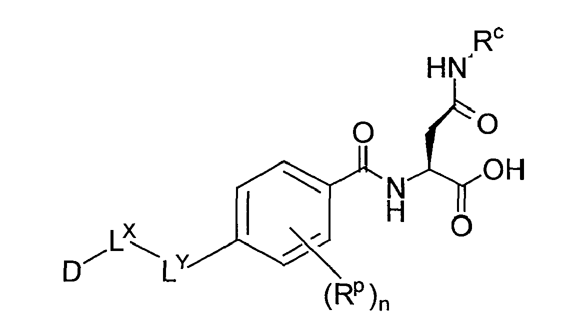

3. A compound according to Claim 1 selected from 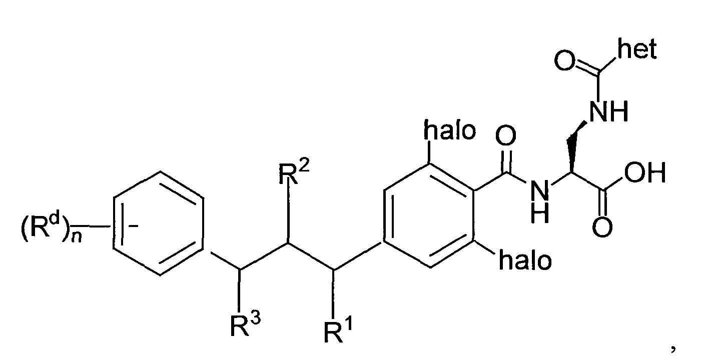 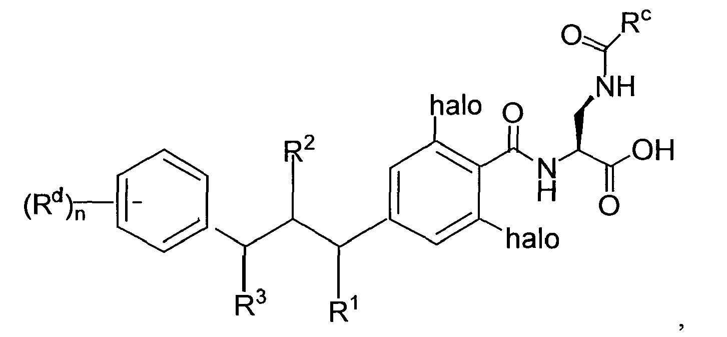 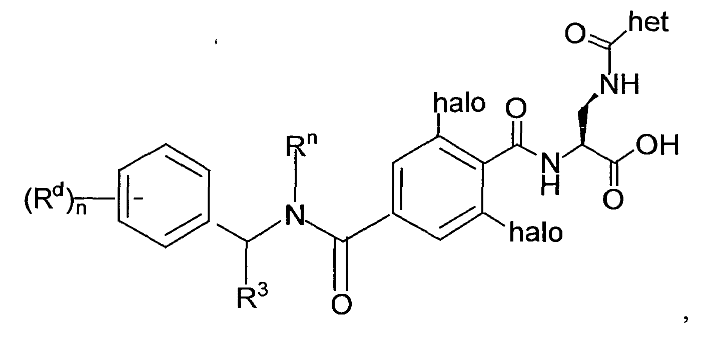 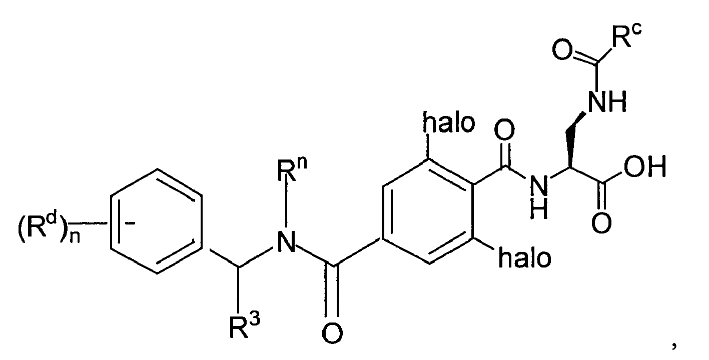 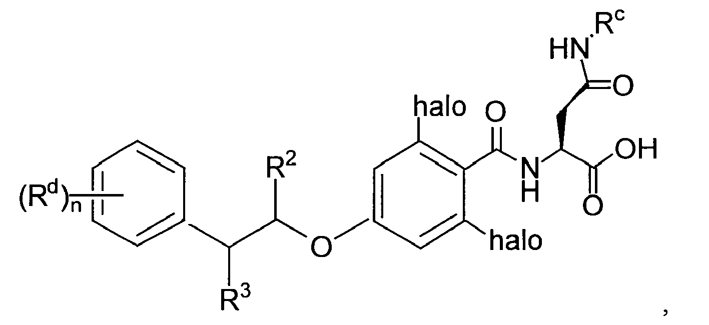 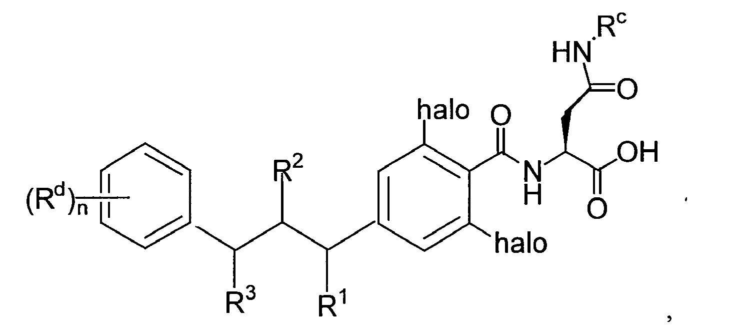 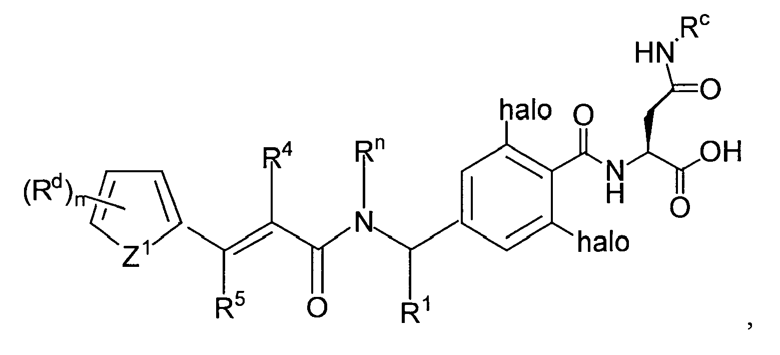 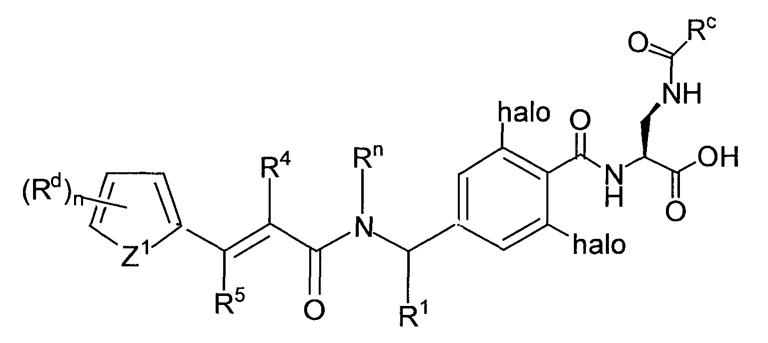 and 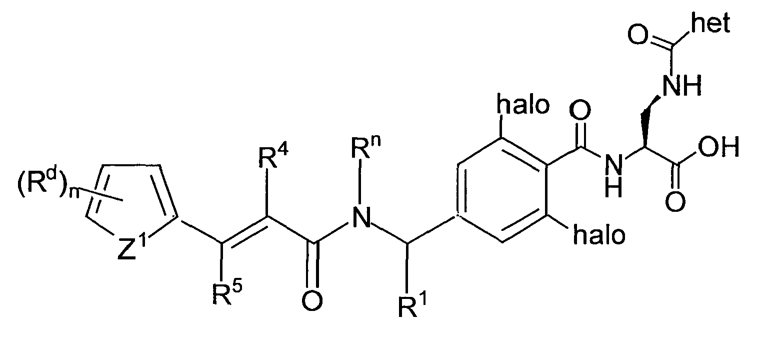 where
het is selected from

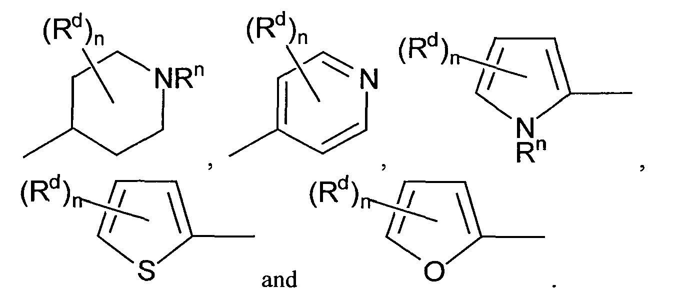

4. The compound according to claim 1 which is: 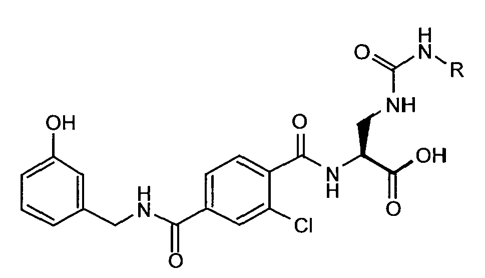 wherein R is:
2-isopropylphenyl isocyanate; phenethyl isocyanate;1-naphthyl isocyanate;
(S)-(-)-α-methylbenzyl isocyanate; cyclohexyl isocyanate; ethoxycarbonyl isocyanate;
isopropyl isocyanate; trans-2-phenylcyclopropyl isocyanate; 1-adamantyl isocyanate; phenyl isocyante; 4-(methylthio)phenyl isocyanate; 3-(methylthio)phenyl isocyanate;
3-ethoxycarbonylphenyl isocyanate; 4-ethoxycarbonylphenyl isocyanate; 4-fluorophenyl isocyanate; 2-fluorophenyl isocyanate; 2-(trifluoromethoxy)phenyl isocyanate;
3-fluorophenyl isocyanate; 3-bromophenyl isocyanate; 4-methoxyphenyl isocyanate;
4-isopropylphenyl isocyanate; 3-(2-hydroxy)ethyl phenyl isocyanate; 4-ethylphenyl isocyanate 2-nitrophenyl isocyanate; 3-nitrophenyl isocyanate; 4-nitrophenyl isocyanate;
3-cyanophenyl isocyanate; 4-trifluoromethyl isocyanate; 3-trifluoromethyl isocyanate;
2-trifluoromethyl isocyanate; 3-methylphenyl isocyanate; 4-chlorophenyl isocyanate;
3-chlorophenyl isocyanate; 3-chloro-4-methylphenyl isocyanate; 3-ethylphenyl isocyanate;
allyl isocyanate; (S)-(-)-α-methylbenzyl isocyanate; cyclohexyl isocyanate; or trans-2-phenylcyclopropyl isocyanate.

5. The compound according to claim 1 which is: 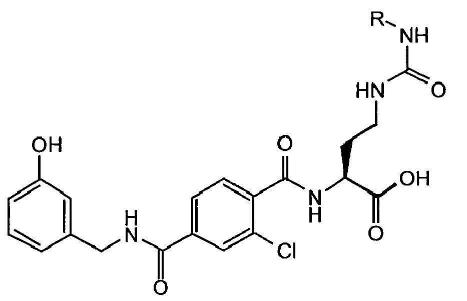 wherein R is:
benzyl isocyanate; ethoxycarbonyl isocyanate; 2-chloro-6-methylphenyl isocyanate; or ethoxycarbonyl isocyanate.

6. The compound according to claim 1 which is 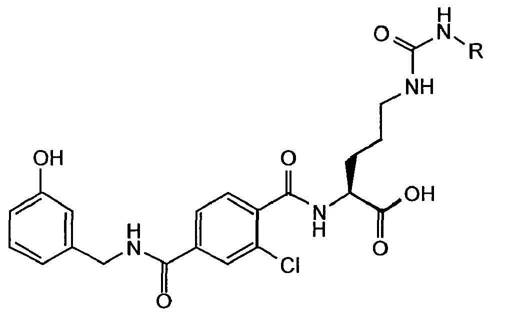 wherein R is:
phenethyl isocyanate; isopropyl isocyanate; cyclohexyl isocyanate;
3-ethoxycarbonylphenyl isocyanate; 4-ethoxycarbonylphenyl isocyanate; 4-fluorophenyl isocyanate; 2-fluorophenyl isocyanate; 3-fluorophenyl isocyanate; 4-methoxyphenyl isocyanate; 4-isopropylphenyl isocyanate; 3-(2-hydroxyethyl)phenyl isocyanate;
2-nitrophenyl isocyanate; 4-nitrophenyl isocyanate; 3-cyanophenyl isocyanate;
3-methylphenyl isocyanate; 4-chlorophenyl isocyanate; 3-chloro-4-methylphenyl isocyanate; 2-chloro-6-methylphenyl isocyanate; or 4-ethylphenyl isocyanate.

7. The compound according to claim 1 which is: 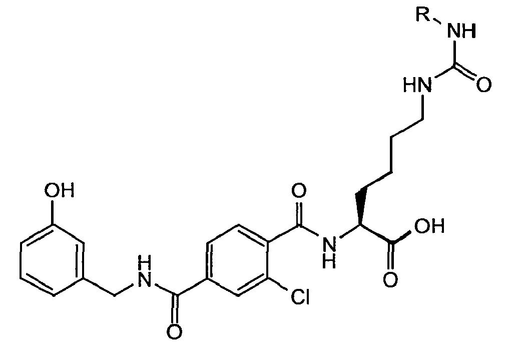 wherein R is:
phenethyl isocyanate; isopropyl isocyanate; benzyl isocyanate; propyl isocyanate;
ethoxycarbonyl isocyanate; ethyl 2-isocyanato-4-methylvalerate; (S)-(-)-α-methylbenzyl isocyanate; benzensulfonyl isocyanate; or benzyl isocyanate.

8. The compound according to claim 1 which is: 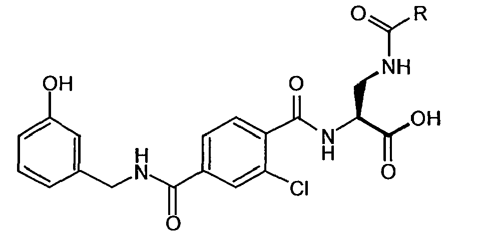
3-methylindene-2-carboxylic acid; 3-methylbenzofuran-2-carboxylic acid; 4-oxo-4, 5, 6, 7-tetrahydrobenzofuran-3-carboxylic acid; 1, 2, 5-trimethyl-1H-pyrrole-3-carboxylic acid;
4-methyl-[1, 2, 3]thiadiazole-5-carboxylic acid; 4-phenyl-[1, 2, 3]thiadiazole-5-carboxylic acid;
3-chloro-2-thiophenecarboxylic acid; 3, 5-dimethyl-isoxazole-4-carboxylic acid;
3-methyl-2-furoic acid; 3-bromothiophene-2-carboxylic acid; 2-furoic acid; 3-furoic acid;
2-thiophene carboxylic acid; 3-thiophenecarboxylic acid; 5-chloro-2-thiophene carboxylic acid;
5-bromo-2-thiophene carboxylic acid; indole-5-carboxylic acid; indole-4-carboxylic acid; indole-6-carboxylic acid; benzoic acid; cyclohexyl carboxylic acid; acetic acid; isonipecotic acid; or pipecolinic acid.

9. The compound according to claim 1 which is: 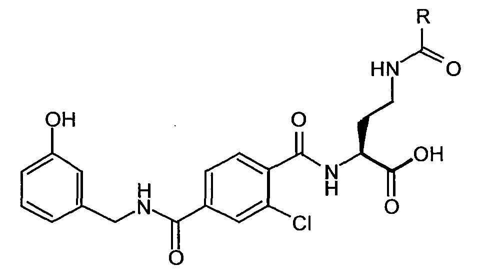 wherein R is:
3, 4, 5-trimethoxybenzoic acid; propionic acid; cyclopropyl carboxylic acid; trimethyl acetic acid;
1, 2, 5-trimethyl-1H-pyrrole-3-carboxylic acid; 3-chloro-4-methanesulfonyl-thiophene-2-carboxylic acid; 4-methyl-[1, 2, 3]thiadiazole-5-carboxylic acid; 4-phenyl-[1,2,3]thiadiazole-5-carboxylic acid; 4-bromo-2-ethyl-5-methyl-2H-pyrazole-3-carboxylic acid; 3-chlorothiophene-2-carboxylic acid; 3, 5-dimethyl-isoxazole-4-carboxylic acid;
5-methyl-2-phenyl-2H-[1, 2, 3]triazole-4-carboxylic acid; 3-methyl-2-furoic acid;
3-bromothiophene-2-carboxylic acid; benzoic acid; cyclohexyl carboxylic acid; acetic acid; or H.

10. The compound according to claim 1 which is: 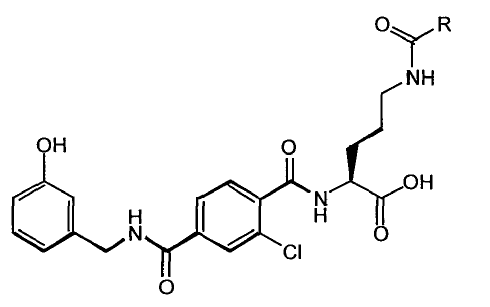 wherein R is:
trimethyl acetic acid; 3-chloro-benzo[b]thiophene-2-carboxylic acid; 3-chlorothiophene-2-carboxylic acid; 3, 5-dimethyl-isoxazole-4-carboxylic acid; 3-bromothiophene-2-carboxylic acid; 3-methylindene-2-carboxylic acid; 4-oxo-4, 5, 6, 7-tetrahydro-benzofuran-3-carboxylic acid; 3-chloro-4-methanesulfonyl-thiophene-2-carboxylic acid; 4-methyl-[1,2,3]thiadiazole-5-carboxylic acid; 4-bromo-2-ethyl-5-methyl-2H-pyrazole-3-carboxylic acid; benzoic acid; cyclohexane carboxylic acid; or acetic acid.

11. The compound according to claim 1 which is: 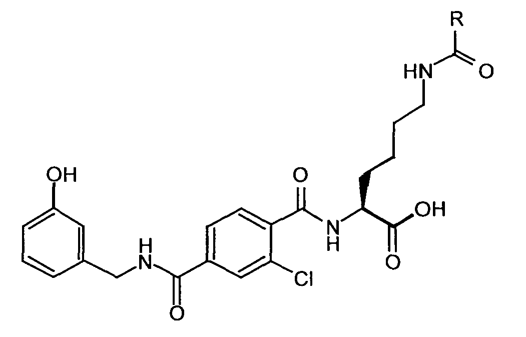 wherein R is:
3, 4, 5-trimethoxybenzoic acid; isovaleric acid; propionic acid; cyclopropyl carboxylic acid; 4-acetyl-3, 5-dimethyl-2-pyrrolecarboxylic acid; 3-methylindene-2-carboxylic acid; 4-oxo-4, 5, 6, 7-tetrahydro-benzofuran-3-carboxylic acid; 1, 2, 5-trimethyl-1H-pyrrole-3-carboxylic acid; 3-chloro-4-methanesulfonyl-thiophene-2-carboxylic acid; 4-methyl-[1, 2, 3]thiadiazole-5-carboxylic acid; 4-phenyl-[1, 2, 3]thiadiazole-5-carboxylic acid; 4-bromo-2-ethyl-5-methyl-2H-pyrazole-3-carboxylic acid; 3-chlorothiophene-2-carboxylic acid; 3, 5-dimethyl-isoxazole-4-carboxylic acid; 5-methyl-2-phenyl-2H-[1, 2, 3]triazole-4-carboxylic acid; 3-bromothiophene-2-carboxylic acid; benzoic acid; or cyclohexyl carboxylic acid.

12. The compound according to claim 1 which is: 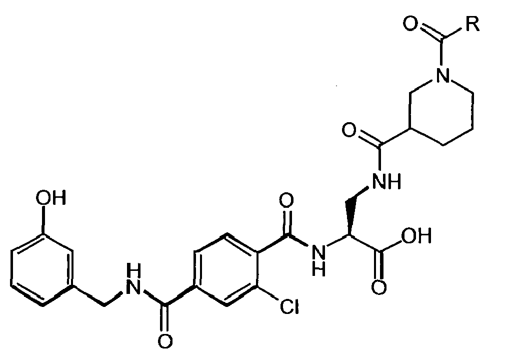 wherein R is: propionic acid; acetic acid; or H.

13. The compound according to claim 1 which is: 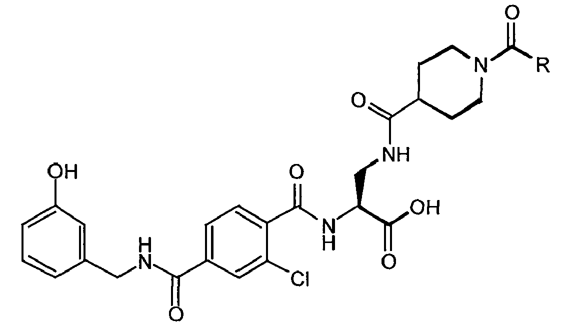 wherein R is: propionic acid; butyric acid; or acetic acid.

14. The compound according to claim 1 which is: 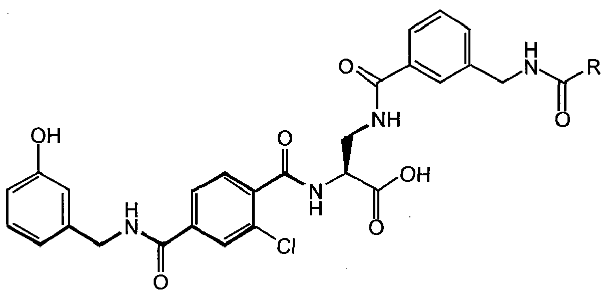 wherein R is: propionic acid; butyric acid; acetic acid; or H.

15. The compound according to claim 1 which is: 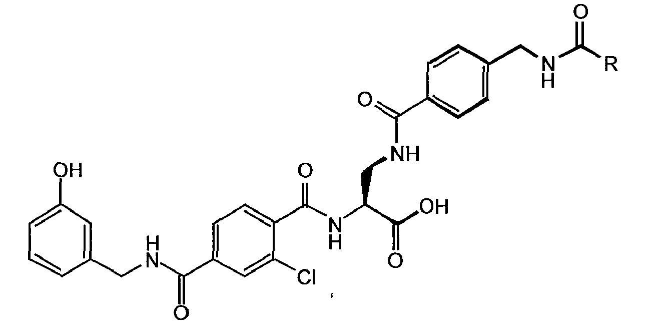 wherein R is: propionic acid; butyric acid; acetic acid; or H.

16. The compound according to claim 1 which is: 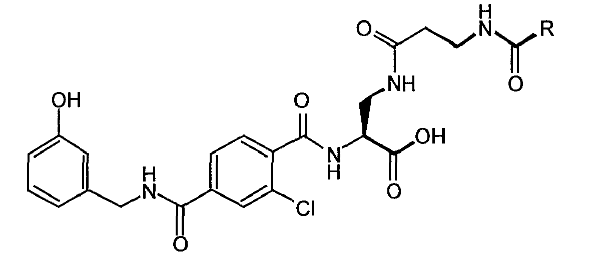 wherein R is: propionic acid; acetic acid; or H.

17. The compound according to claim 1 which is: 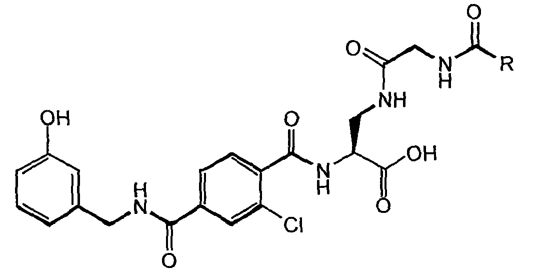 wherein R is: acetic acid; or H.

18. The compound according to claim 1 which is: 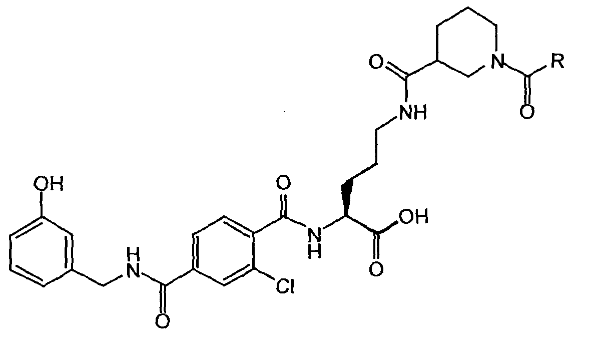 wherein R is:
propionic acid; butyric acid; acetic acid; or H.

19. The compound according to claim 1 which is: 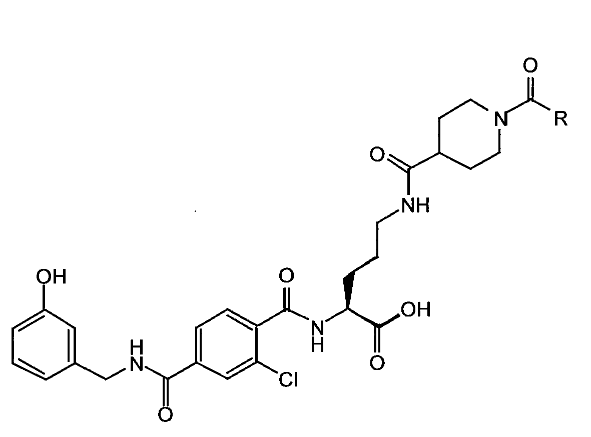 wherein R is:
propionic acid; butyric acid; acetic acid; or H.

20. The compound according to claim 1 which is: 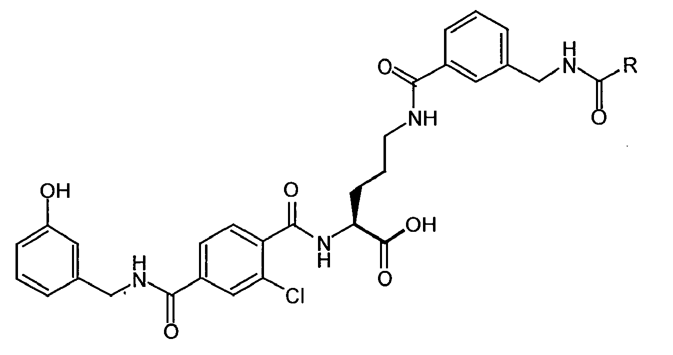 wherein R is:
propionic acid; butyric acid; acetic acid; or H.

21. The compound according to claim 1 which is: 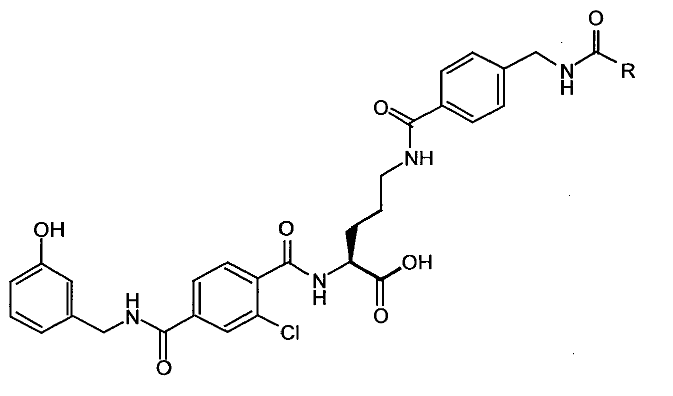 wherein R is:
propionic acid; butyric acid; acetic acid; or H.

22. The compound according to claim 1 which is: 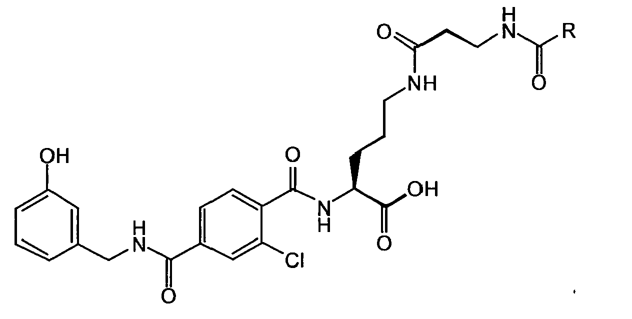 wherein R is:
propionic acid; butyric acid; acetic acid; or H.

23. The compound according to claim 1 which is: 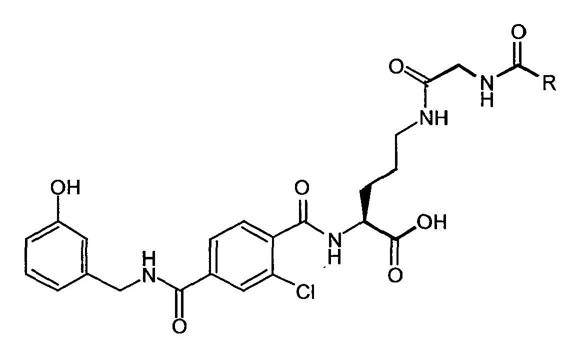 wherein R is:
propionic acid; butyric acid; acetic acid; or H.

24. The compound according to claim 1 which is: 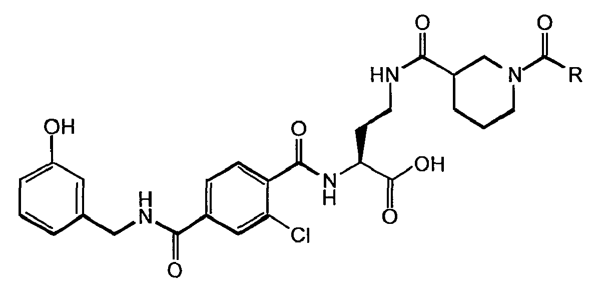 wherein R is:
propionic acid; butyric acid; acetic acid; or H.

25. The compound according to claim 1 which is: 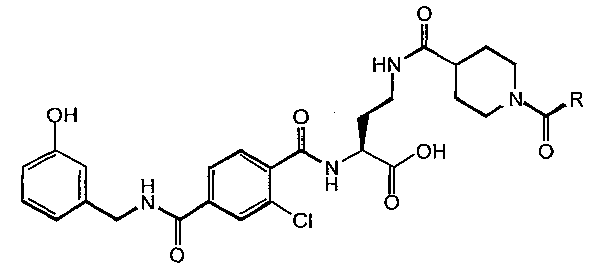 wherein R is:
propionic acid; butyric acid; acetic acid; H.

26. The compound according to claim 1 which is: 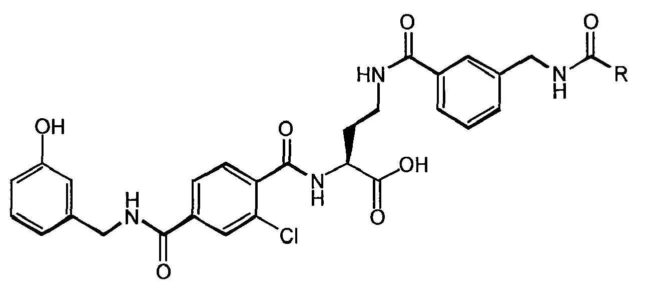 wherein R is: propionic acid; butyric acid; acetic acid; or H.

27. The compound according to claim 1 which is: 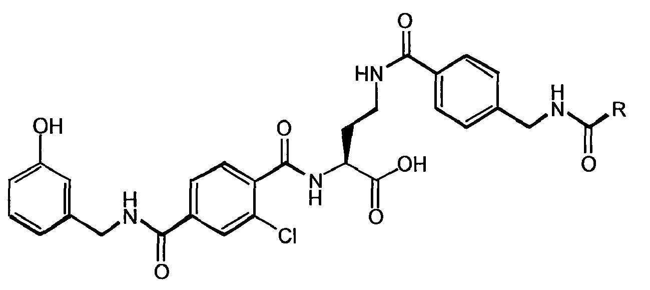 wherein R is:
propionic acid butyric acid; acetic acid; or H.

28. The compound according to claim 1 which is: 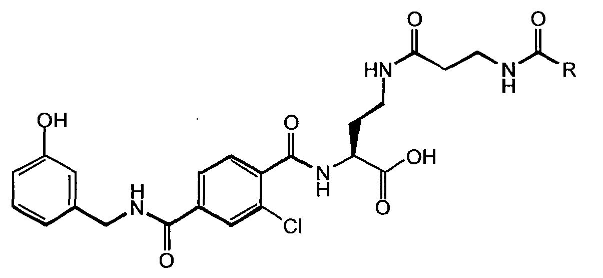 wherein R is:
propionic acid; butyric acid; acetic acid; or H.

29. The compound according to claim 1 which is: 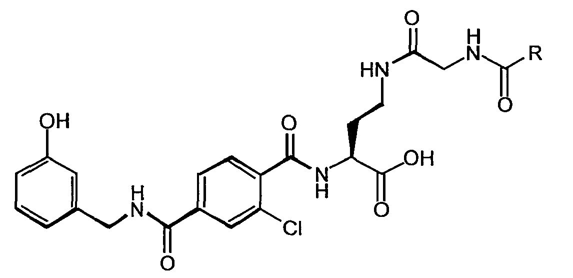 wherein R is:
propionic acid; butyric acid; acetic acid; or H.

30. The compound according to claim 1 which is: 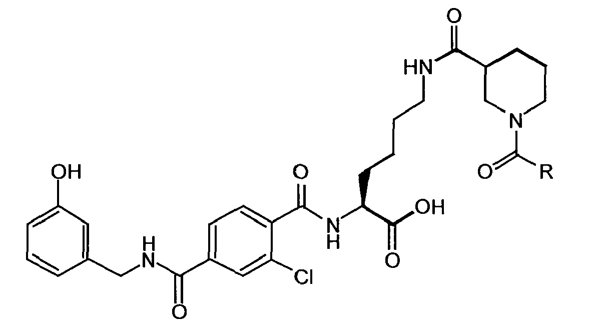 wherein R is:
propionic acid; butyric acid; acetic acid; or H.

31. The compound according to claim 1 which is: 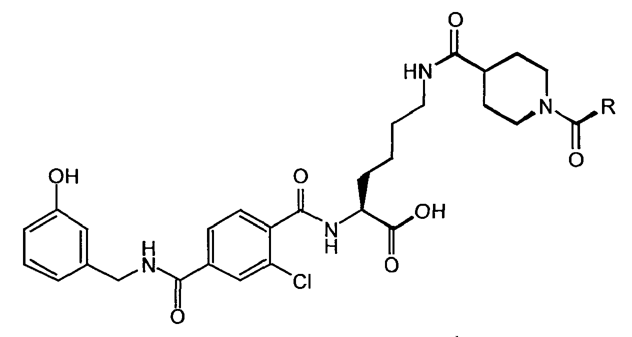 wherein R is:
propionic acid; butyric acid; acetic acid; or H.

32. The compound according to claim 1 which is: 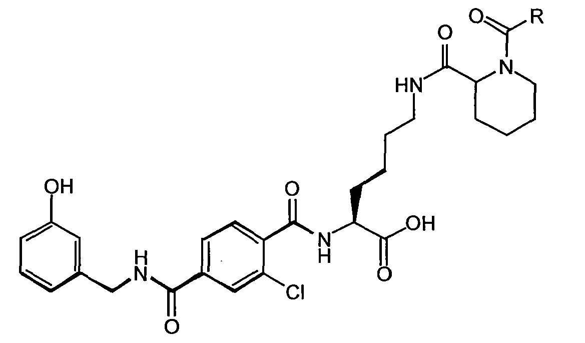 acetic acid; or H.

33. The compound according to claim 1 which is: 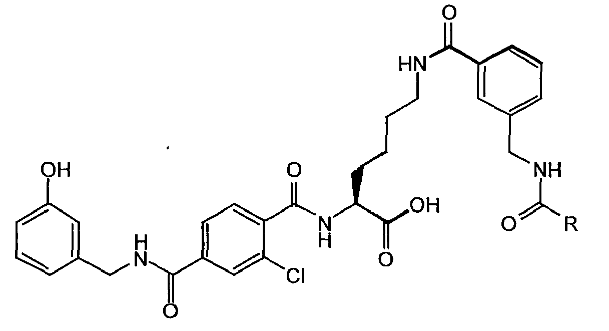 wherein R is: propionic acid; or H.

34. The compound according to claim 1 which is: 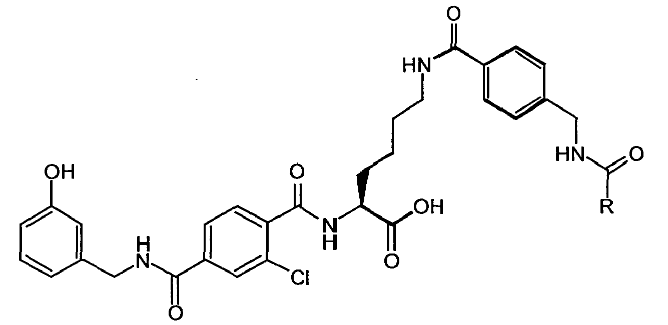 wherein R is: acetic acid; or H.

35. The compound according to claim 1 which is: 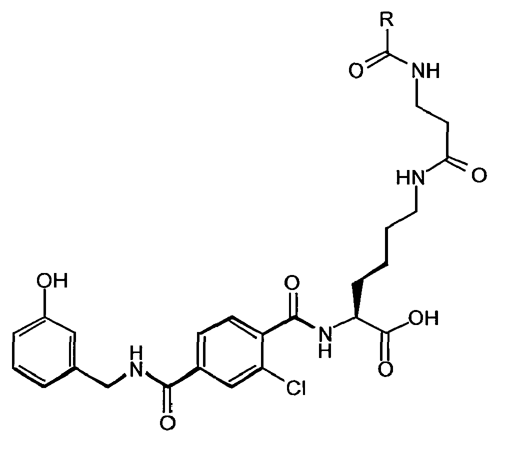 wherein R is: acetic acid; or H.

36. The compound according to claim 1 which is: 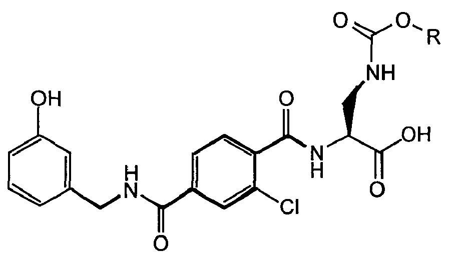 wherein R is: propyl chloroformate; benzyl chloroformate; isopropyl chloroformate; methyl chloroformate; ethyl chloroformate; butyl chloroformate; or 3-butenyl chloroformate.

37. The compound according to claim 1 which is: 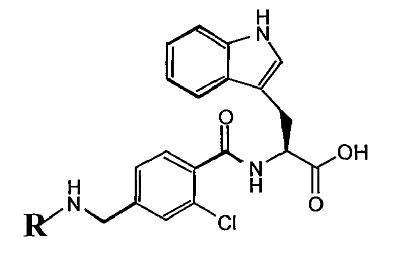 wherein R is: 3-hydroxybenzoic acid; 2-hydroxycinnamic acid; or 3-hydroxybenzoic acid.

38. The compound according to claim 1 which is: 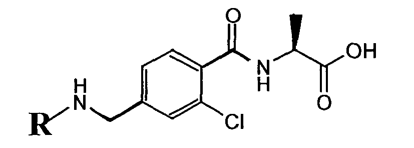 wherein R is: 3-hydroxybenzoic acid; 2-hydroxycinnamic acid; 3-chlorobenzoic acid; indole-5-carboxylic acid; or 3-(2-thienyl)acrylic acid.

39. The compound according to claim 1 which is: 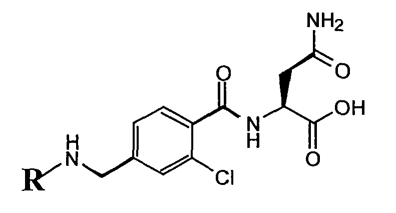 wherein R is: 3-chlorobenzoic acid; 3-(2-thienyl)acrylic acid; 2-furanacrylic acid;
3-hydroxy benzoic acid; indole-5-carboxylic acid; benzofuran-5-carboxylic acid; benzofuran-4-carboxylic acid; or indole-6-carboxylic acid.

40. The compound according to claim 1 which is: 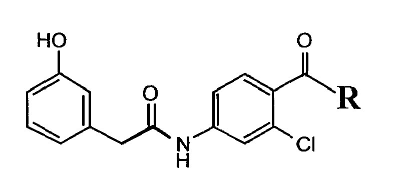 wherein R is: L -Ala; or L -Thr.

41. The compound according to claim 1 which is: 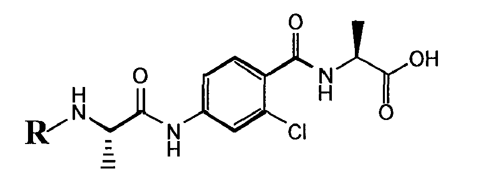 wherein R is: 2-thiophene carboxylic acid; or 3-hydroxybenzoic acid.

42. The compound according to claim 1 which is: 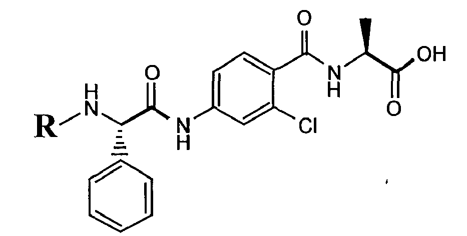 wherein R is: 3-hydroxybenzoic acid; or 2-thiophene carboxylic acid.

43. The compound according to claim 1 which is: 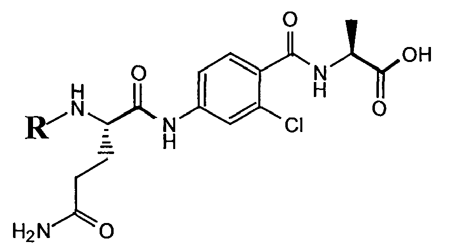 wherein R is: benzoic acid; or 2-thiophene carboxylic acid.

44. The compound according to claim 1 which is: 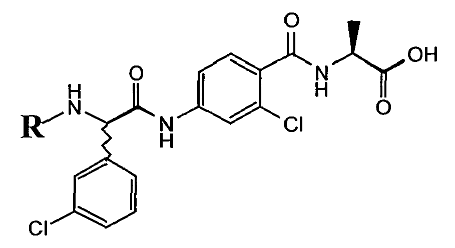 wherein R is: 3-hydroxybenzoic acid; or 2-thiophene carboxylic acid.

45. The compound according to claim 1 which is: 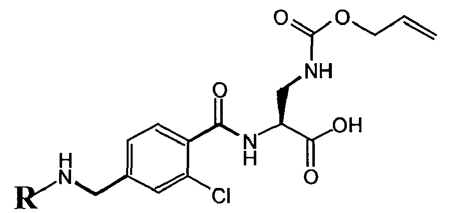 wherein R is: 3-(2-thienyl)-acrylic acid; or furylacrylic acid.

46. The compound according to claim 1 which is: 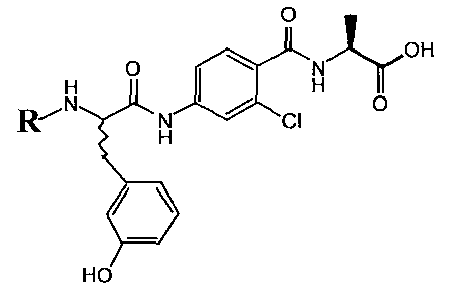 wherein R is: 3-hydroxybenzoic acid; or 2-thiophene carboxylic acid.

47. The compound according to claim 1 which is: 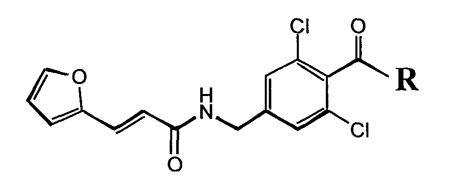 wherein R is: L-Ala; L-Asn; or L-diaminopropionic acid (alloc).

48. The compound according to claim 1 which is: 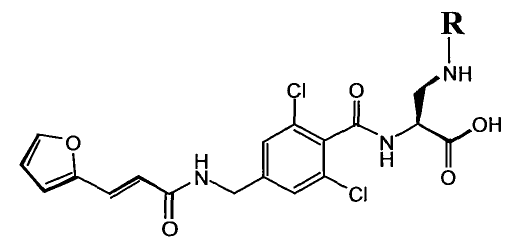 wherein R is: thiophene-2-carboxylic acid; 2-furoic acid; 2-pyrazinecarboxylic acid;
3-methylthiophene-2-carboxylic acid; 3-methyl-2-furoic acid; or 3-chlorothiophene-2-carboxylic acid.

49. The compound according to claim 1 which is: 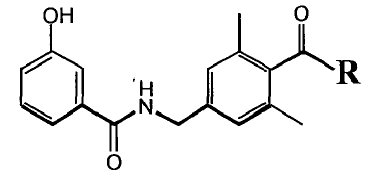 wherein R is: L-Ala; or L-Asn.

50. The compound according to claim 1 which is: 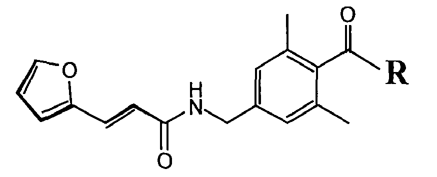 wherein R is: L-diaminopropionic acid (alloc); or L-Lys.

51. The compound according to claim 1 which is: 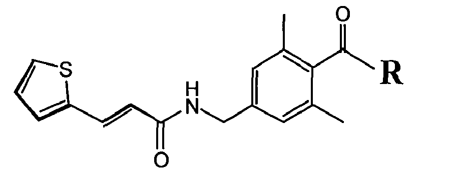 wherein R is: L-diaminopropionic acid (alloc); or L-Lys.

52. The compound according to claim 1 which is: 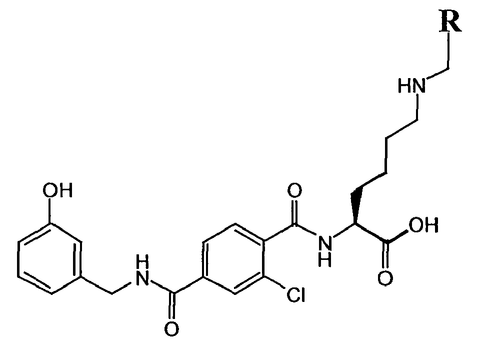 wherein R is: 2-furaldehyde; or 3-methyl 2-furaldehyde.

53. The compound according to claim 1 which is: 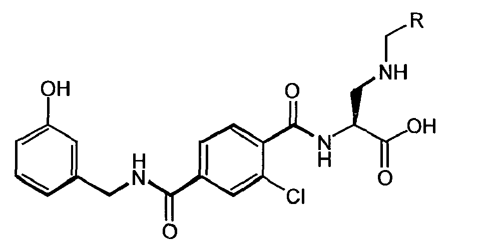 wherein R is: 2-furaldehyde; or 3-methyl-2-furaldehyde.

54. The compound according to claim 1 which is: 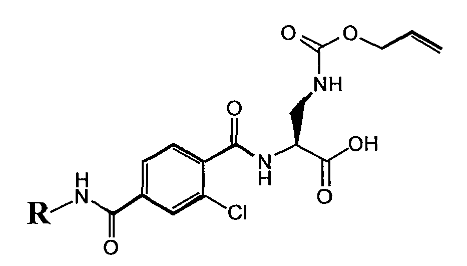 wherein R is: 6-aminomethylbenzofuran; or 4-aminomethylbenzofuran

55. The compound according to claim 1 which is: 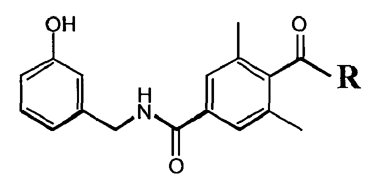 wherein R is: L-Asn; L-diaminopropionic acid (alloc); or L-lys.

56. The compound according to claim 1 which is: 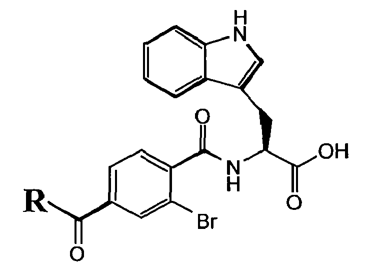 wherein R is: 3-hydroxybenzylamine; or 3-(3-hydroxyphenyl)propargylamine.

57. The compound according to claim 1 which is: 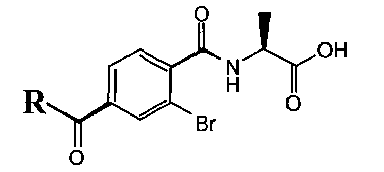 wherein R is: 3-fluorobenzylamine; benzylamine; or 3-(3-hydroxyphenyl)propargylamine.

58. The compound according to claim 1 which is: 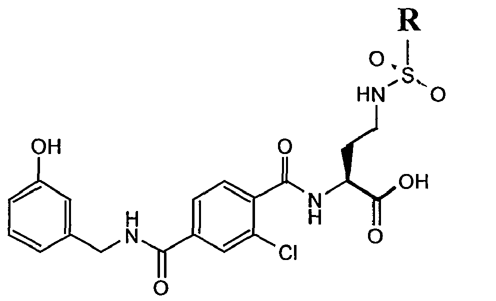 wherein R is: N-acetylsulfanilyl chloride; 2-bromobenzenesulfonyl chloride; or 2-thiophenesulfonyl chloride.

59. The compound according to claim 1 which is: 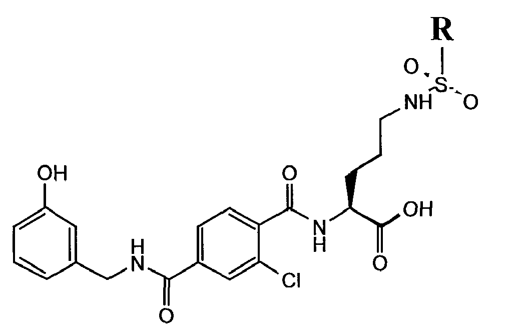 wherein R is: 2-thiophenesulfonyl chloride; or 8-quinolinesulfonyl chloride.

60. The compound according to claim 1 which is: 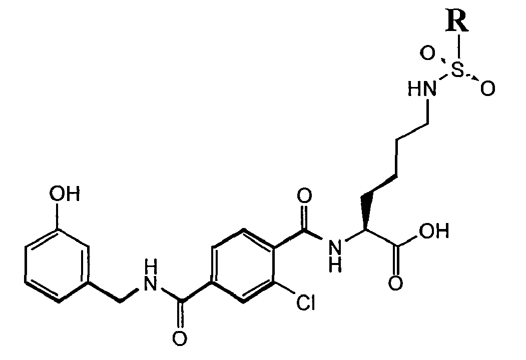 wherein R is: benzenesulfonyl chloride; N-acetylsulfanilyl chloride; 2-thiophenesulfonyl chloride; 2-bromobenzenesulfonyl chloride; or 2-acetamido-4-methyl-5-thiazolesulfonyl chloride.

61. The compound according to claim 1 which is: 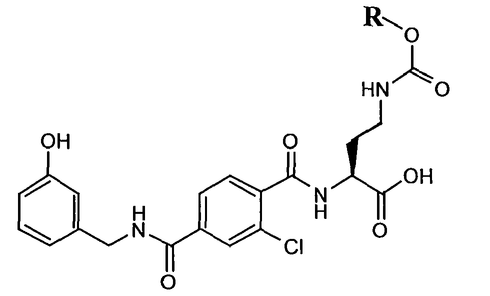 wherein R is: isobutyl chloroformate; allyl chloroformate; butyl chloroformate; ethyl chloroformate; isopropyl chloroformate; or propyl chloroformate.

62. The compound according to claim 1 which is: 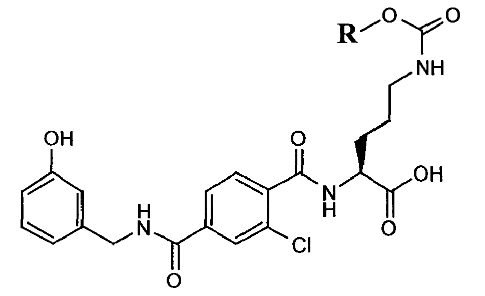 wherein R is: isobutyl chloroformate; cyclopropyl chloroformate; ethyl chloroformate; methyl chloroformate; or 2, 2, 2-trichloroethyl chloroformate.

63. The compound according to claim 1 which is: 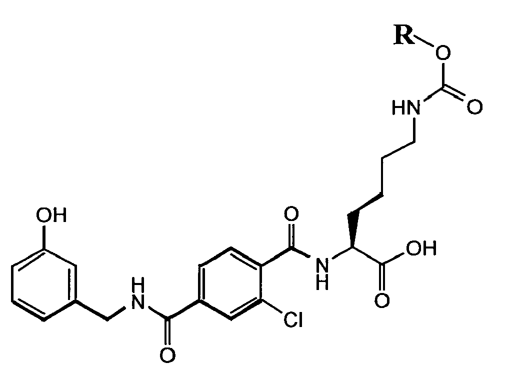 wherein R is: butyl chloroformate; propyl chloroformate; ethyl chloroformate; or methyl chloroformate.

64. The compound according to claim 1 which is: 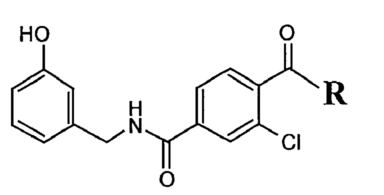 wherein R is: L -Ala; L -Thr; L -Trp; L -aza Trp; L -Ser(OBzl); L -Asn; L -Lys; L -His; L -Lys(N-e-Ac); L -Gln; L-diaminopropionic(alloc) acid; L-diaminobutyric(alloc) acid; L-lys(alloc); L-orn(alloc); or L-Tyr.

65. The compound according to claim 1 which is: 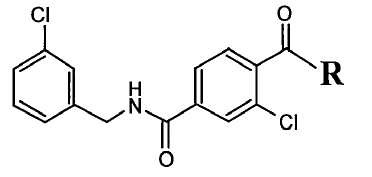 wherein R is: L -Ala; L -His; or L -Asn.

66. The compound according to claim 1 which is: 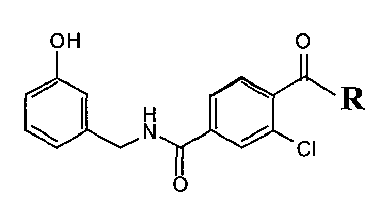 wherein R is: 1-amino-1-cyclopropane carboxylic acid; m-tyrosine; o-hydroxytyrosine; or L-iodotyrosine.

67. The compound according to claim 1 which is: 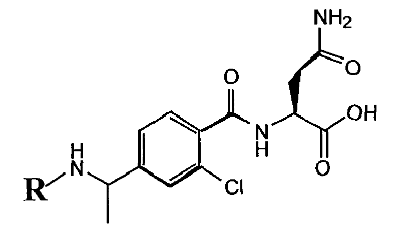 wherein R is: 3 -hydroxybenzoic acid; or benzoic acid.

68. The compound according to claim 1 which is: 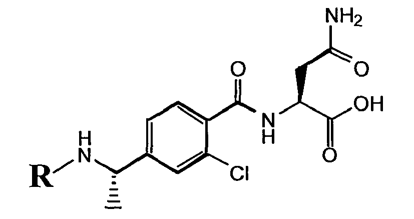 wherein R is: furylacrylic acid; 3-(2-thienyl)-acrylic acid; 3-hydroxybenzoic acid; or benzoic acid.

69. The compound according to claim 1 which is: 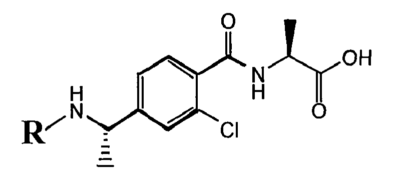 wherein R is: 3-hydroxybenzoic acid; or 3-(2-thienyl)-acrylic acid.

70. The compound according to claim 1 which is: 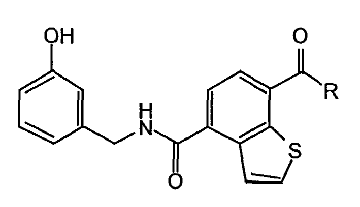 wherein R is: L-Trp; L-Asn; L-dapa(alloc); or L-Lys.

71. The compound according to claim 1 which is: 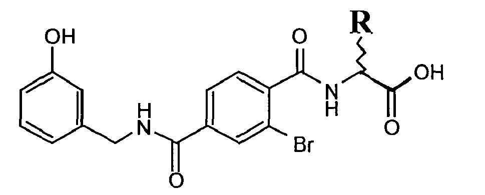 wherein R is: 3-methoxybenzyl bromide; 3-bromobenzyl bromide; 3, 5-dimethoxybenzyl bromide; 5-bromovaleronitrile; 6-bromohexanenitrile; 3-nitrobenzyl bromide; 3-cyano-benzyl bromide; 5-bromomethyl-furan-2-carboxylic acid ethyl ester; 5-bromomethyl-furan-2-carboxylic acid ethyl ester; or 3-bromomethyl benzamide.

72. The compound according to claim 1 which is: 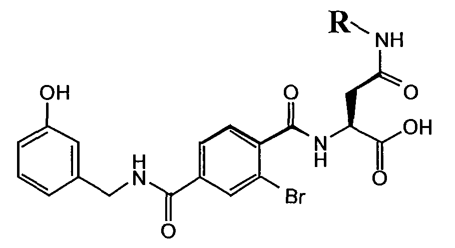 wherein R is: 1-aminonaphthalene; 2-cyanoaniline; 3-cyanoaniline; 2-fluoroaniline; 3-fluoroaniline; 4-fluoroaniline; or 3-methoxyaniline.

73. The compound according to claim 1 which is: 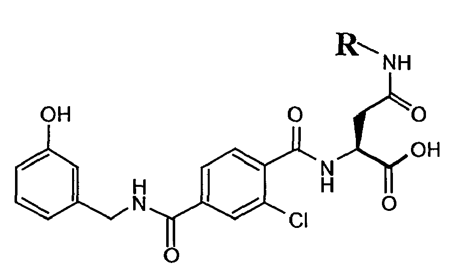 wherein R is: 2-(aminomethyl)pyridine; 3-fluorobenzylamine; benzylamine; allylamine; phenethyl amine; histamine; 4-fluorobenzylamine; 3-methoxyphenethylamine; 4-aminobenzylamine; 2-aminobenzylamine; 2-[1, 3]Dioxan-5-yl-ethylamine; piperonylamine; or aniline.

74. The compound according to claim 1 which is: 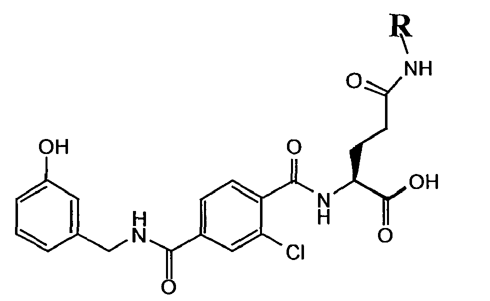 wherein R is: isoamyl amine; 4-(aminomethyl)pyridine; 2-[1, 3]dioxan-5-yl-ethylamine; or aniline.

75. The compound according to claim 1 which is: 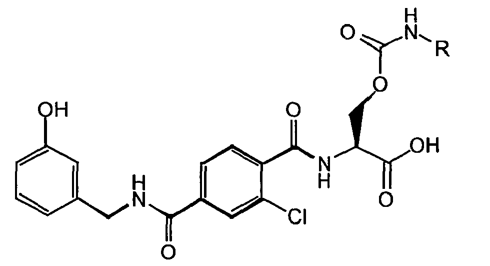 wherein R is: o-toluidine; allyl amine; or propyl amine.

76. The compound according to claim 1 which is: 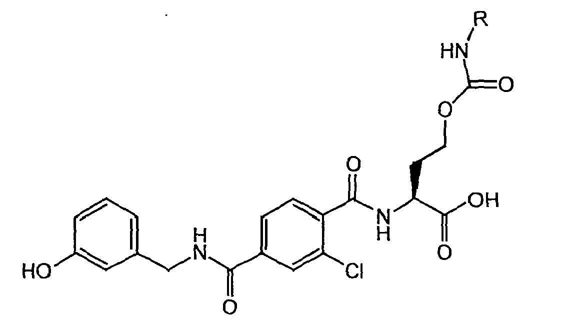 wherein R is: propylamine; 3-(aminomethyl)pyridine; 4-(aminomethyl)pyridine; 2-methylbenzylamine; 3-methylbenzylamine;4-methylbenzylamine; (S)-(-)-a-methylbenzylamine;2-(aminomethyl)pyridine; 2-fluoro benzylamine;3-fluoro benzylamine; 4-fluoro benzylamine;3-chloro benzylamine; 4-chloro benzylamine;4-methoxy benzylamine; 1-naphthalenemethylamine; or benzylamine.

77. The compound according to claim 1 which is:

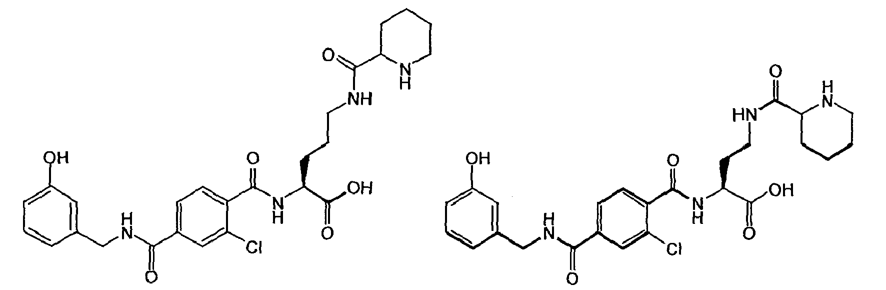

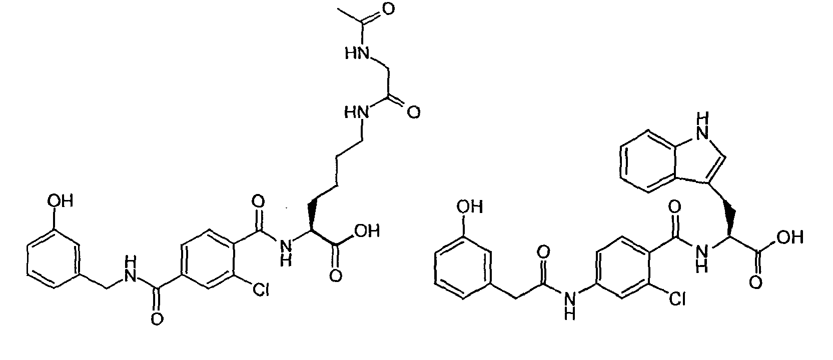

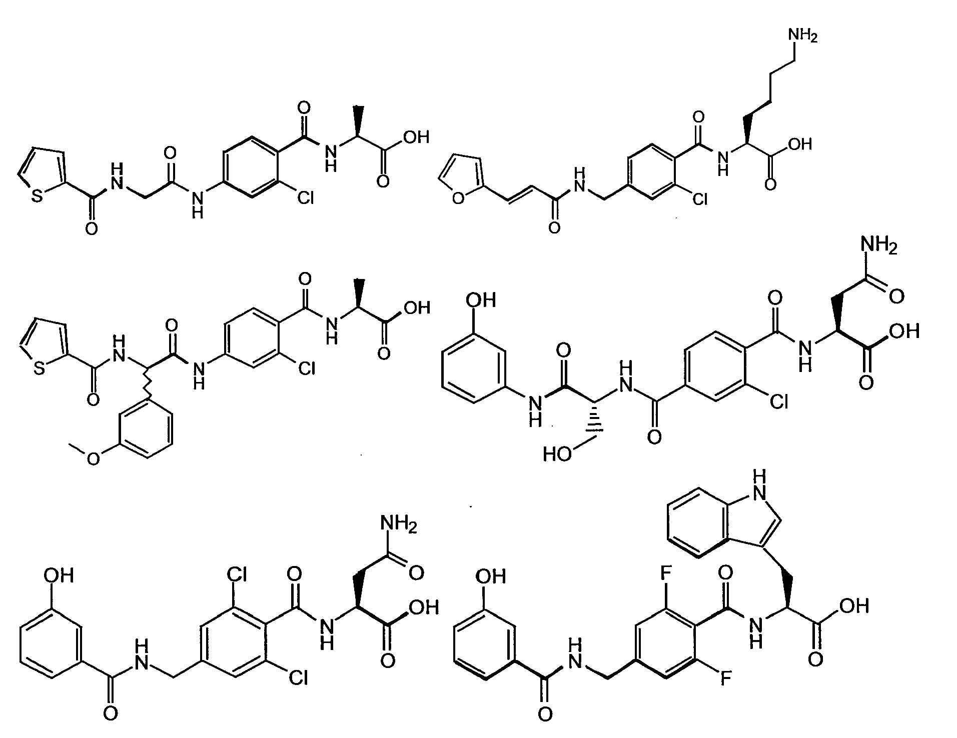

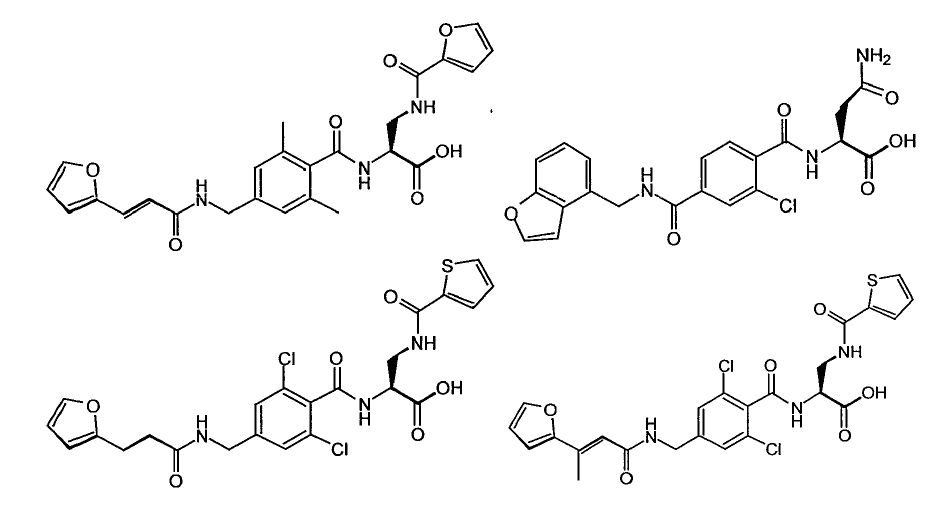

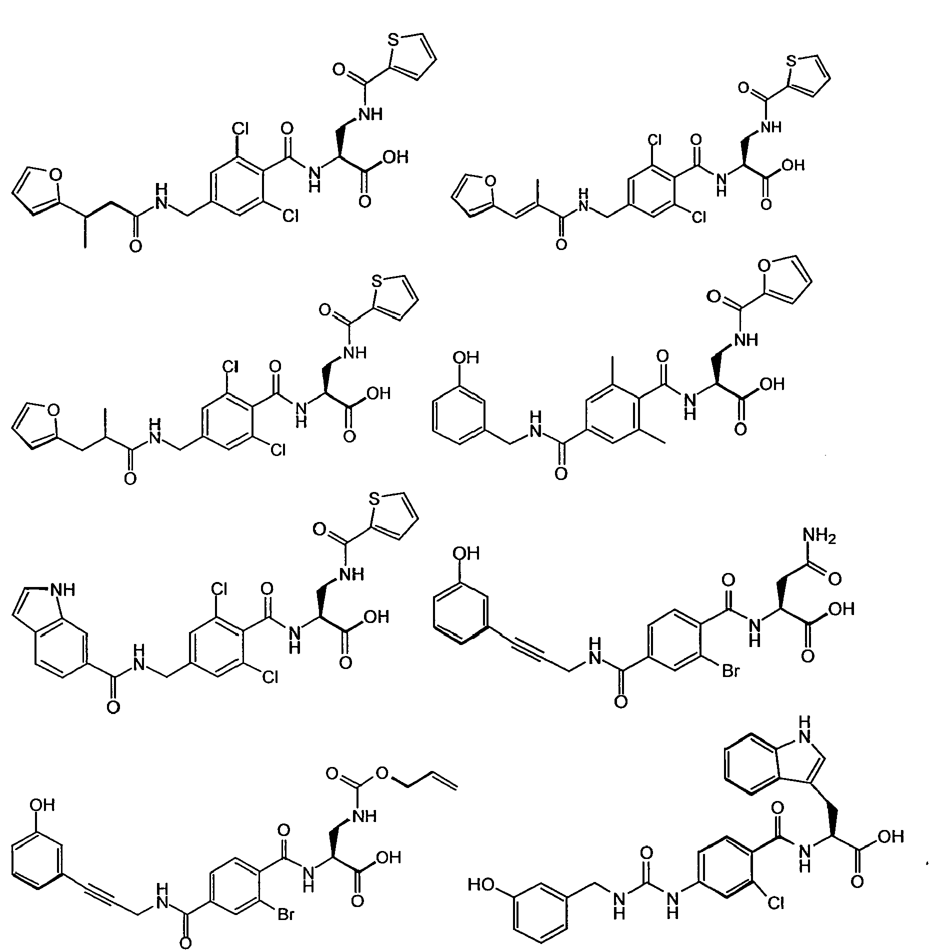

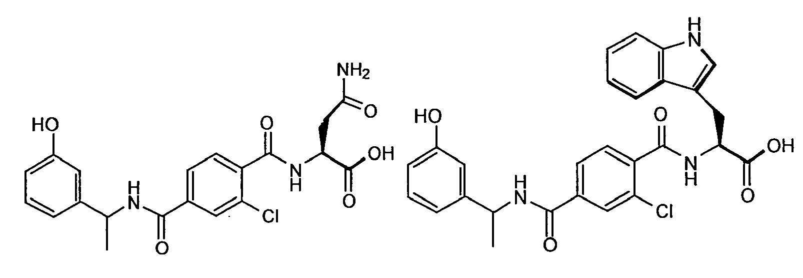

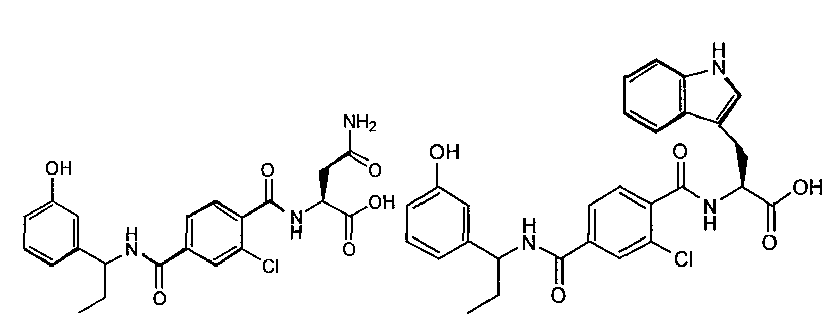

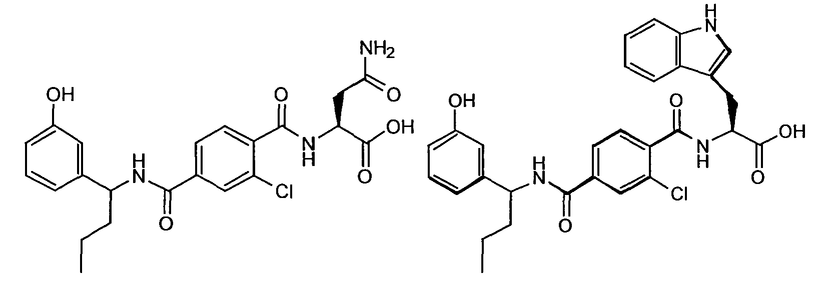

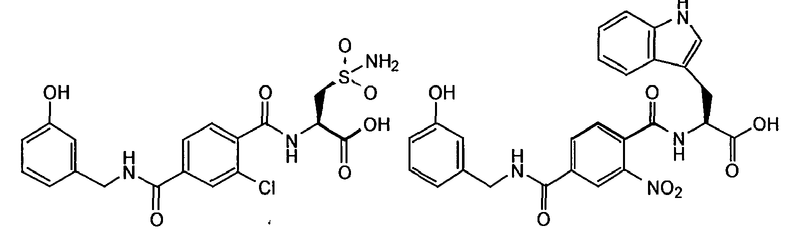

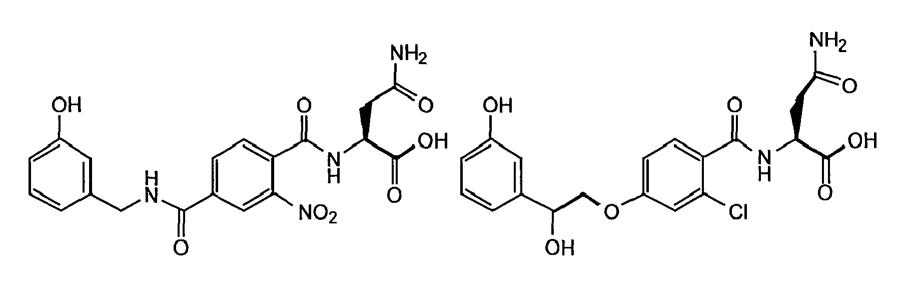

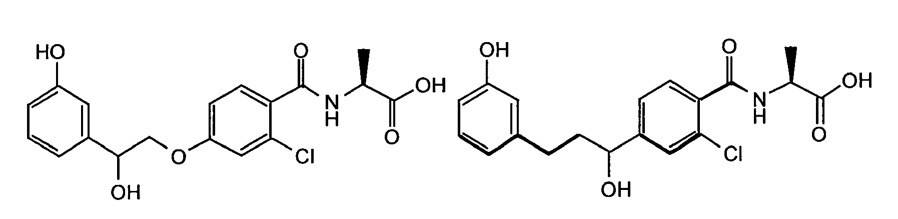

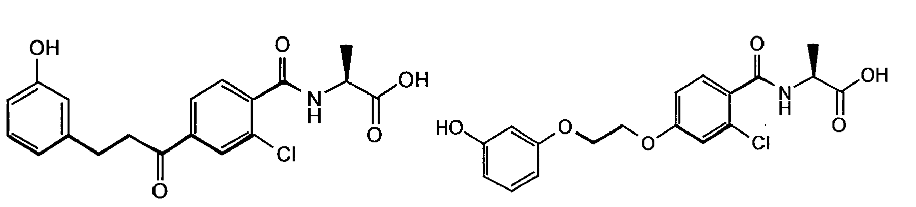

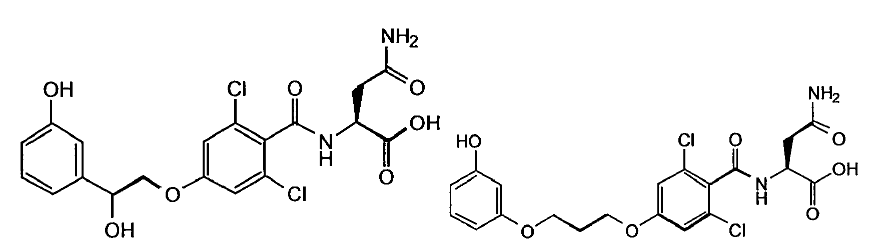

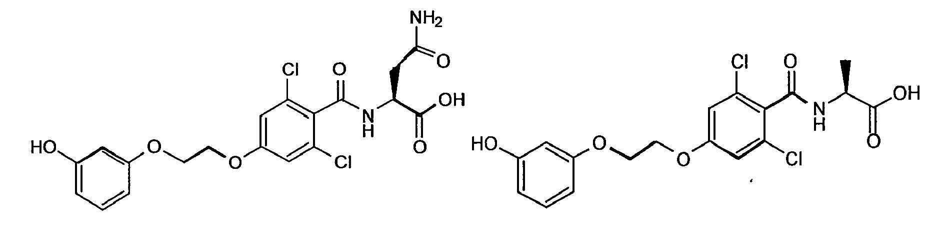

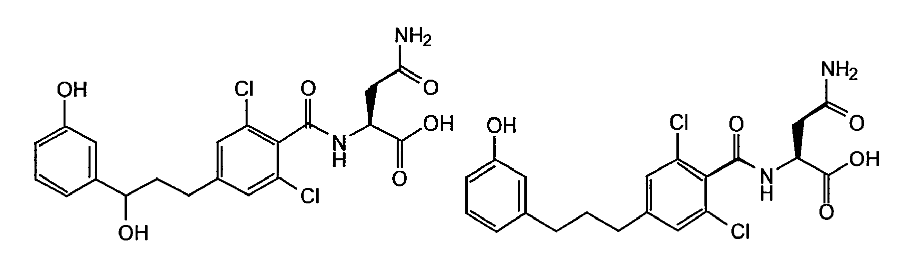

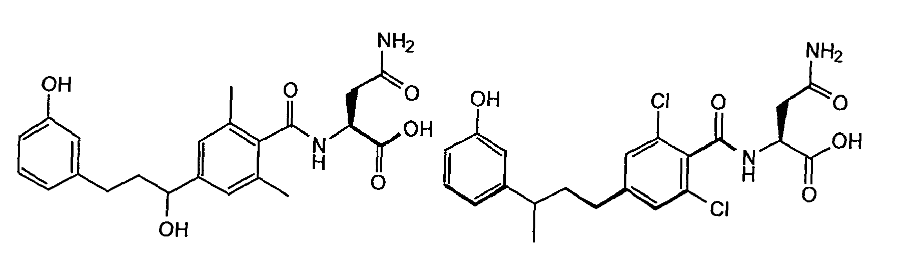

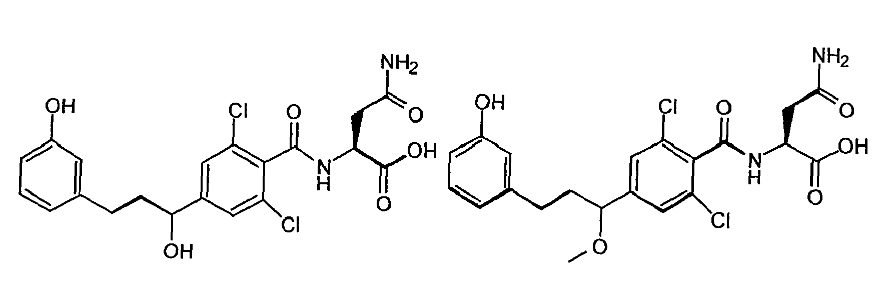

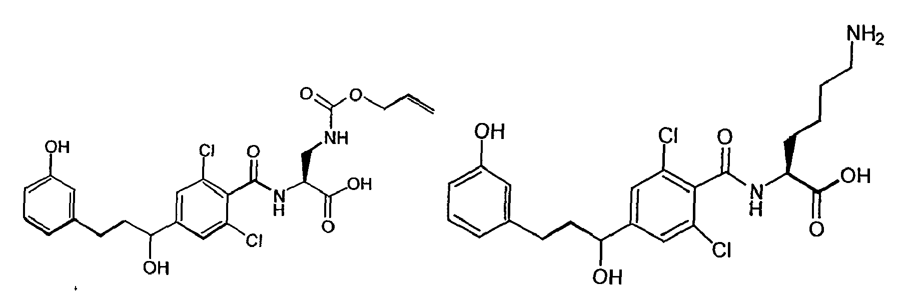

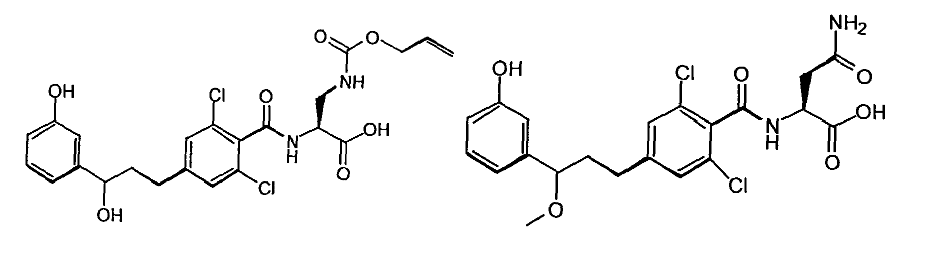

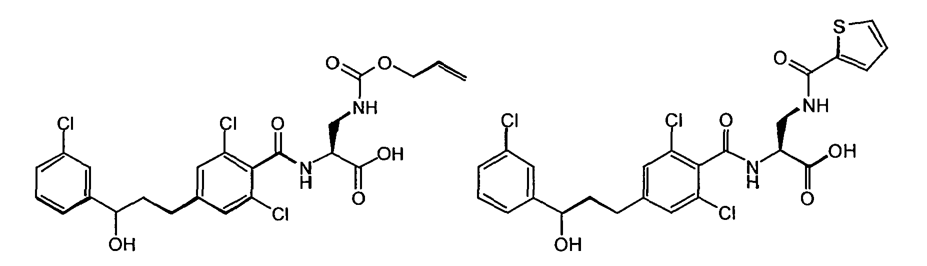

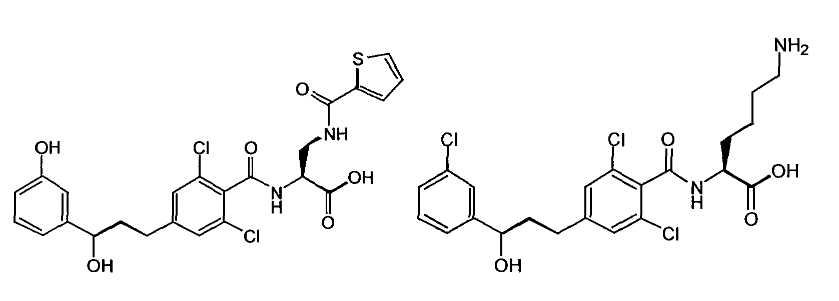

78. A pharmaceutical composition comprising a compound according to any one of Claims 1-77 and a pharmaceutically acceptable excipient.
